(19) Europäisches Patentamt
European Patent Office
Office européen des brevets

(11) EP 4 549 440 A1

(12) **EUROPEAN PATENT APPLICATION**
published in accordance with Art. 153(4) EPC

(43) Date of publication:
**07.05.2025 Bulletin 2025/19**

(21) Application number: **23830539.5**

(22) Date of filing: **03.07.2023**

(51) International Patent Classification (IPC):
***C07D 471/04*** (2006.01) ***C07D 519/00*** (2006.01)
***C07D 401/12*** (2006.01) ***C07D 401/14*** (2006.01)
***C07D 491/04*** (2006.01) ***C07D 487/04*** (2006.01)
***C07D 417/12*** (2006.01) ***A61K 31/506*** (2006.01)
***A61K 31/437*** (2006.01) ***A61P 35/00*** (2006.01)

(52) Cooperative Patent Classification (CPC):
**A61K 31/437; A61K 31/4353; A61K 31/444; A61K 31/4709; A61K 31/4985; A61K 31/506; A61K 31/519; A61K 31/5377; A61K 31/5383; A61P 35/00; C07D 401/12; C07D 401/14; C07D 417/12; C07D 471/04; C07D 487/04;** (Cont.)

(86) International application number:
**PCT/CN2023/105594**

(87) International publication number:
**WO 2024/002377 (04.01.2024 Gazette 2024/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **01.07.2022 CN 202210775564**
**23.09.2022 CN 202211168268**

(71) Applicant: **Cytosinlab Therapeutics Co., Ltd.**
**Shanghai 201203 (CN)**

(72) Inventors:
- **WANG, Long**
**Shanghai 201203 (CN)**
- **WU, Haiping**
**Shanghai 201203 (CN)**
- **MI, Yuan**
**Shanghai 201203 (CN)**
- **LIU, Yilin**
**Shanghai 201203 (CN)**
- **FU, Xingnian**
**Shanghai 201203 (CN)**
- **WANG, Meng**
**Shanghai 201203 (CN)**
- **SHI, Hui**
**Shanghai 201203 (CN)**
- **GUO, Jiannan**
**Shanghai 201203 (CN)**

(74) Representative: **Lavoix**
**Bayerstraße 83**
**80335 München (DE)**

(54) **CLASS OF PRMT5 INHIBITORS AND USE THEREOF**

(57) Provided is a class of compounds with methyltransferase inhibitory activity. Specifically, provided is a class of compounds with PRMT5 inhibitory activity. The compounds can be used for preparing a pharmaceutical composition for treating PRMT5 activity-related diseases.

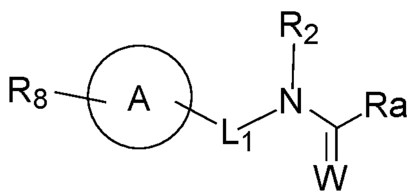

I

EP 4 549 440 A1

(52) Cooperative Patent Classification (CPC): (Cont.)
**C07D 487/14; C07D 491/04; C07D 491/048;
C07D 491/14; C07D 498/04; C07D 519/00;
C07F 5/02**

## Description

### Technical field

The present invention relates to the field of pharmaceutical compounds. Specifically, the present invention provides a class of compounds for inhibiting PRMT5 and its use in pharmaceutical compositions.

### Background

The epigenetic regulation of gene expression is an important biological determinant of protein production and cell differentiation, and plays a crucial pathological role in many human diseases.

The epigenetic regulation involves heritable modifications of genetic material without altering its nucleotide sequence. Typically, the epigenetic regulation is mediated by the selective and reversible modifications (such as methylation) of DNA and proteins (such as histones), which control conformational transitions between transcriptionally active and inactive states of chromatin. These covalent modifications can be controlled by enzymes such as methyltransferases (such as PRMT5), many of which are associated with specific genetic changes that may lead to human diseases. PRMT5 plays a role in diseases such as proliferative disorders, metabolic disorders, and hematological disorders.

PRMT5 is a known essential gene for cells. Conditional knockout of *PRMT5* and siRNA knockdown studies have shown that depletion of PRMT5 in normal tissues is associated with a range of diseases, such as pancytopenia, infertility, skeletal muscle loss, and cardiac hypertrophy. Therefore, new strategies are needed to exploit the metabolic vulnerability and priority targeting PRMT5 in MTAP-null tumors, while retaining PRMT5 activity in normal ($MTAP^{WT}$) tissues. Targeting PRMT5 with MTA in conjunction with a small molecule inhibitor can preferentially target the MTA-bound state of PRMT5, which is enriched in MTAP-null tumor cells, and provide a therapeutic index superior to that of normal cells with intact MTAP and low MTA levels.

Therefore, there is a need in the field to provide novel small molecule compounds targeting PRMT5 in MTAP-null tumors.

### SUMMARY OF THE INVENTION

The purpose of the present invention is to provide a novel class of small molecule compounds targeting PRMT5 in MTAP-null tumors.

In a first aspect of the present invention, provided is a compound of formula I shown below, or a pharmaceutically acceptable stereoisomer, a salt or a deuterated product thereof:

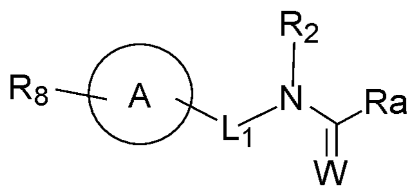

I

wherein,

Ra is selected from the group consisting of

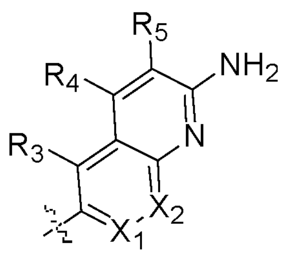

and

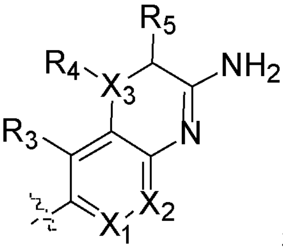

;

W is O or S;

$X_1$, $X_2$ are each independently selected from the group consisting of CR and N; $X_3$ is N;

$L_1$ is selected from the group consisting of: chemical bonds, -O-, -CHR-, and -C(R)R-;

Ring A is selected from the group consisting of: substituted or unsubstituted 8-12 membered fused bicyclic heterocyclyl (including carbocycle or heterocycle, preferably five-membered fused six-membered ring), and substituted or unsubstituted 7-10 membered fused bicyclic heteroaryl (preferably five-membered fused six-membered ring);

$R_8$ is selected from the group consisting of: H, halogen, cyano, amino, nitro, hydroxyl, thiol, aldehyde, carboxyl, $C_2$-$C_6$ alkynyl, $SF_5$, and substituted or unsubstituted or halogenated $C_1$-$C_6$ alkyl, or

$R_8$ is

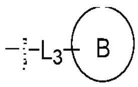

;

$L_3$ is selected from the group consisting of: chemical bonds, -O-, -CHR-, -C(R)R-, carbonyl, S, and -NH-;

Ring B is selected from the group consisting of: substituted or unsubstituted benzene ring, substituted or unsubstituted 5-6-membered heteroaromatic ring, substituted or unsubstituted $C_3$-$C_6$ carbocycle (including saturated and partially unsaturated situations), and substituted or unsubstituted 3-7-membered heterocycle (including saturated and partially unsaturated situations);

$R_2$ is selected from the group consisting of: $R_7$, and -$L_2R_7$; wherein, $L_2$ is selected from the group consisting of: -O-, -CHR-, -C(R)R-, and carbonyl; wherein, $R_7$ is selected from the group consisting of: hydrogen, none, substituted or unsubstituted $C_1$-$C_6$ alkyl, substituted or unsubstituted $C_6$-$C_{10}$ aromatic ring, substituted or unsubstituted 5-12 membered heteroaromatic ring, substituted or unsubstituted $C_3$-$C_{10}$ carbocycle (including saturated and partially unsaturated situations, including single ring, fused ring, spiro ring and bridged ring), and substituted or unsubstituted 3-10 membered heterocycle (including saturated and partially unsaturated situations, including single ring, fused ring, spirospiro ring and bridged ring);

$R_3$ is selected from the group consisting of H, halogen, cyano, and substituted or unsubstituted $C_1$-$C_6$ alkyl;

$R_4$ and $R_5$ are each independently selected from the group consisting of: H, halogen, cyano, substituted or unsubstituted $C_1$-$C_6$ alkyl, substituted or unsubstituted $C_1$-$C_6$ alkoxyl, substituted or unsubstituted $C_3$-$C_6$ carbocycle (including saturated and partially unsaturated situations), and substituted or unsubstituted 3-6 membered heterocycle; or $R_4$ and $R_5$ together with the connected ring atom form a 5-12 membered saturated or unsaturated ring, and the ring can be substituted or unsubstituted;

R is H, halogen, substituted or unsubstituted $C_1$-$C_4$ alkyl, substituted or unsubstituted $C_1$-$C_4$ alkoxyl, and substituted or unsubstituted $C_3$-$C_6$ cycloalkyl;

unless otherwise specified, in the above formulas, the "substituted" refers to hydrogen atoms on the corresponding group are substituted by one or more substituents selected from the group consisting of: deuterium, tritium, halogen, hydroxyl, carboxyl, thiol, benzyl, $C_1$-$C_{12}$ alkoxycarbonyl, $C_1$-$C_6$ aldehyde, amino, $C_1$-$C_6$ amide, nitro, cyano, unsubstituted or halogenated $C_1$-$C_6$ alkyl, unsubstituted or halogenated $C_3$-$C_8$ cycloalkyl, $C_2$-$C_{10}$ alkenyl, $C_1$-$C_6$ alkoxyl, $C_1$-$C_6$ alkyl-amino, $C_6$-$C_{10}$ aryl, five-membered or six-membered heteroaryl, five-membered or six-membered non-aromatic heterocyclyl, -O-($C_6$-$C_{10}$ aryl), -O- (five-membered or six-membered heteroaryl), $C_1$-$C_{12}$ alkylamino carbonyl, unsubstituted or halogenated $C_2$-$C_{10}$ acyl, sulfonyl (-$SO_2$-OH), phosphoryl-(-$PO_3$-OH), unsubstituted or halogenated $C_1$-$C_4$ alkyl-$S(O)_2$-, and unsubstituted or halogenated $C_1$-$C_4$ alkyl-SO-.

In another preferred embodiment, Ring A is selected from the group consisting of:

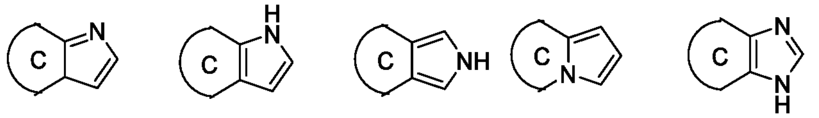

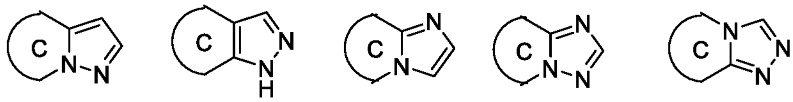

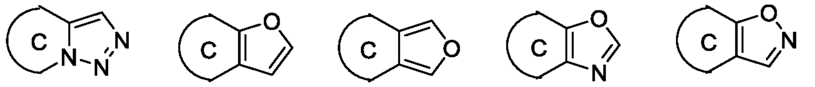

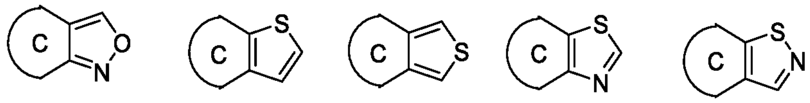

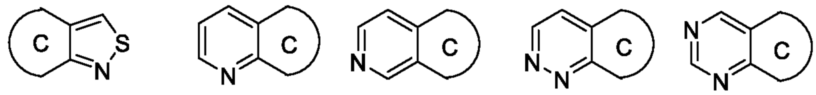

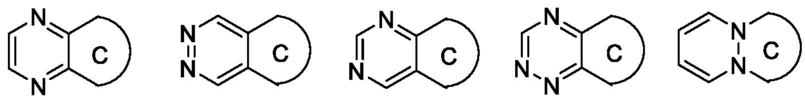

In another preferred embodiment, Ra is selected from the group consisting of:

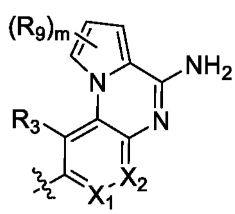
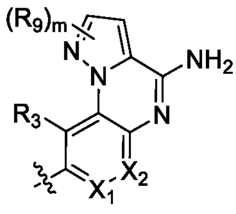
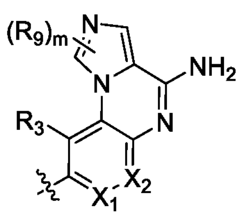
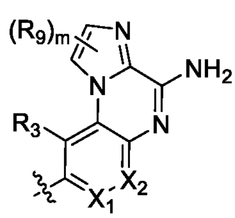

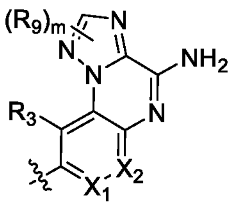
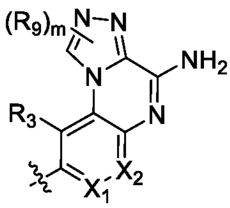
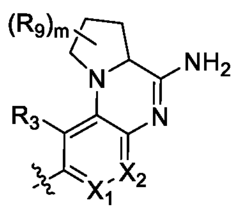
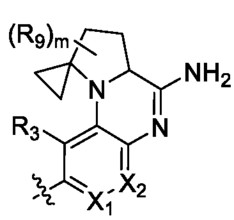

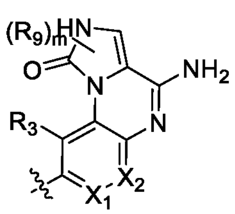
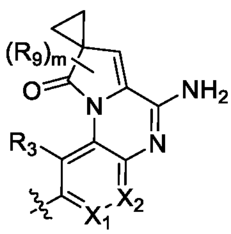
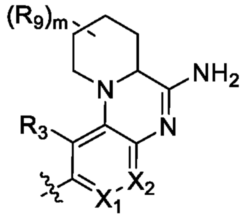
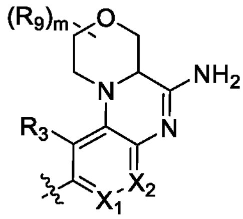

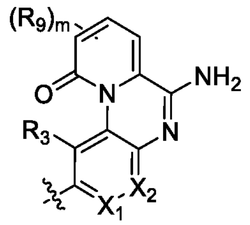
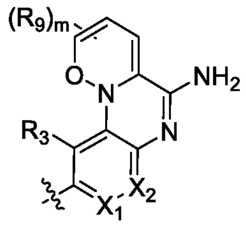
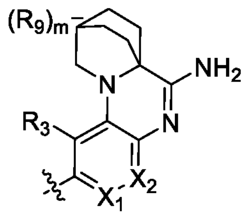
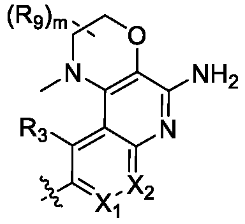
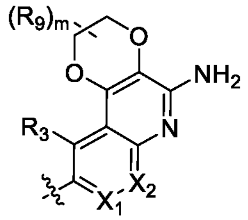
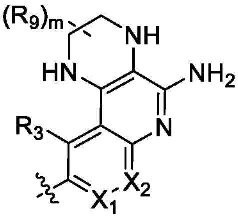
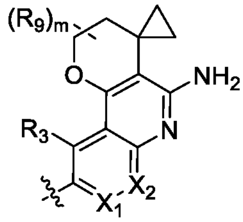
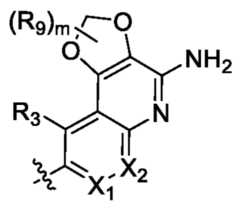
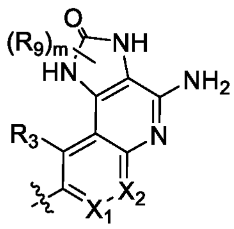
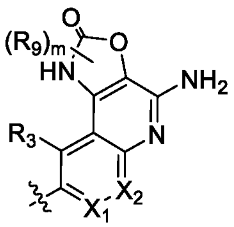
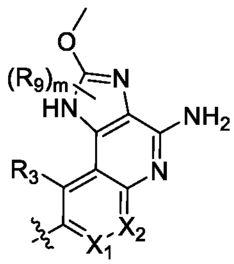
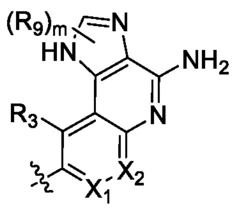
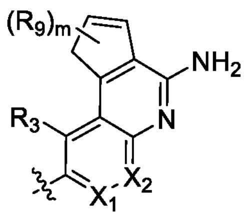
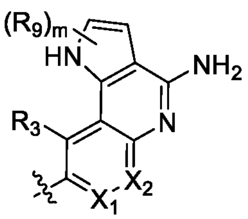
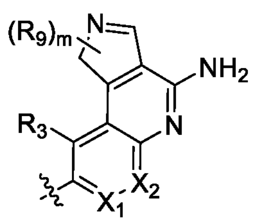
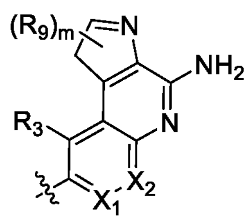
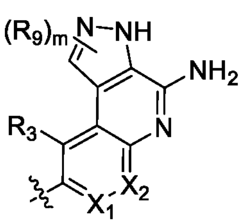
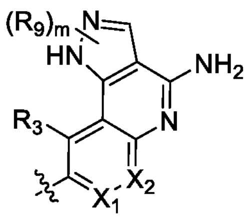
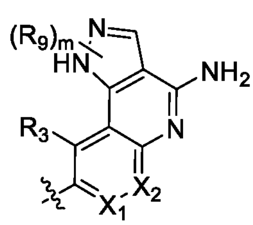
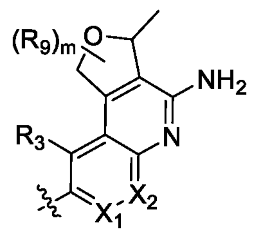
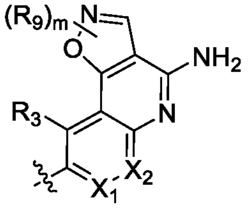
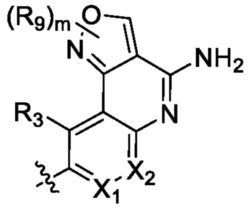
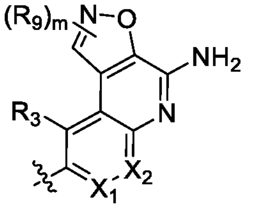
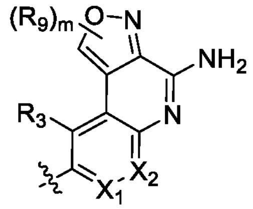

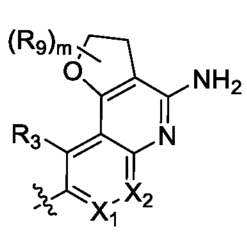 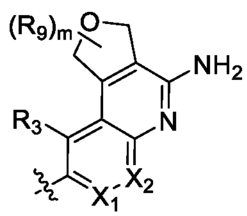 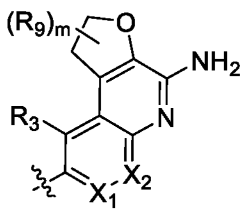 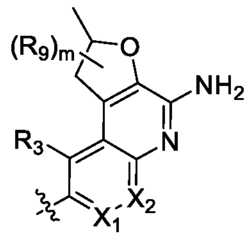

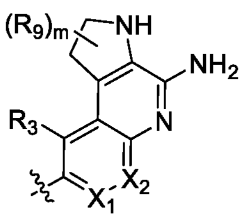 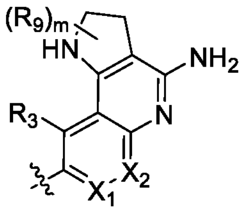 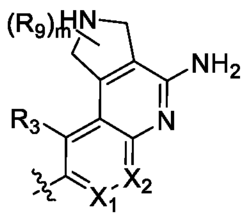 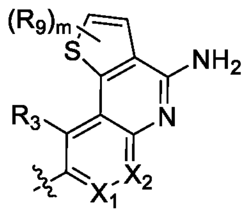

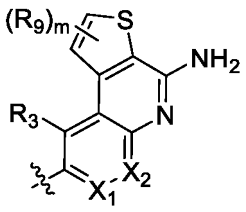 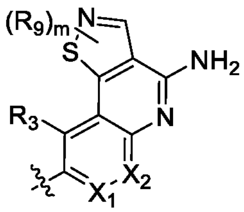 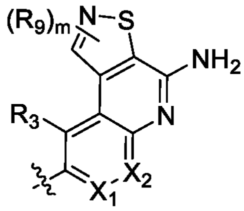 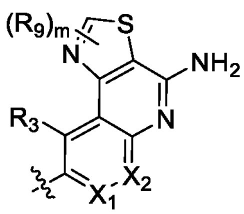

wherein, $R_9$ is selected from the group consisting of: deuterium, tritium, halogen, hydroxyl, carboxyl, unsubstituted or halogenated $C_1$-$C_6$ alkyl, unsubstituted or halogenated $C_1$-$C_6$ alkoxyl, unsubstituted or halogenated $C_1$-$C_6$ alkyl-OH, -NH (unsubstituted or halogenated $C_1$-$C_6$ alkyl), and -N (unsubstituted or halogenated $C_1$-$C_6$ alkyl)$_2$; m is selected from 0, 1, 2, and 3.

In another preferred embodiment, $L_1$ is -$CH_2$-, or $CH(CH_3)$-; ring A is selected from the group consisting of:

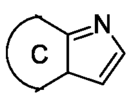 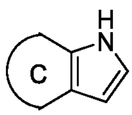 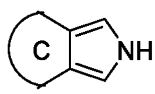 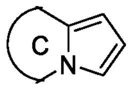 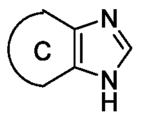

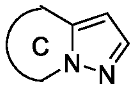 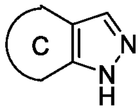 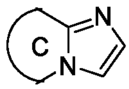 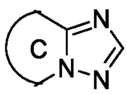 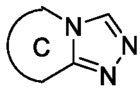

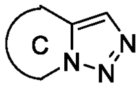 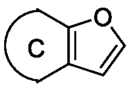 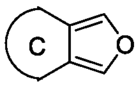 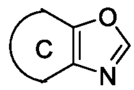 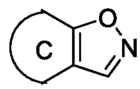

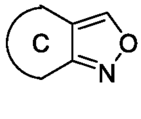 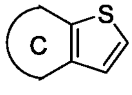 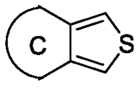 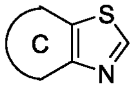 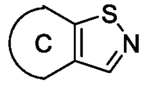

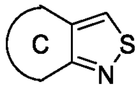 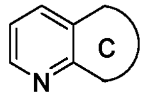 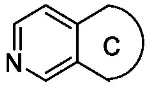 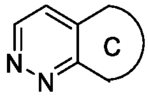 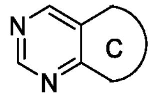

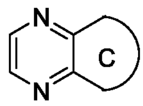 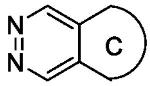 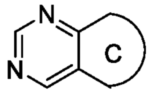 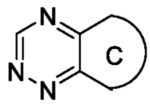 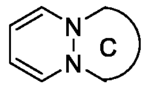

wherein ring C is selected from the group consisting of: substituted or unsubstituted benzene ring, substituted or unsubstituted 5-6-membered heteroaromatic ring, substituted or unsubstituted $C_3$-$C_6$ carbocycle (including saturated and partially unsaturated situations), and substituted or unsubstituted 3-6-membered heterocycle (including saturated and partially unsaturated situations).

In another preferred embodiment, $R_2$ is ortho-substituted 5,6-membered heteroaromatic rings, as shown below:

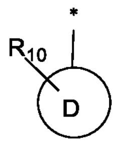

wherein, the ortho-substituent $R_{10}$ is selected from the group consisting of: hydrogen, deuterium, halogen, halogenated or unhalogenated $C_1$-$C_3$ alkyl, and halogenated or unhalogenated $C_1$-$C_3$ alkoxyl;
preferably, ring D is selected from the group consisting of substituted or unsubstituted benzene ring, and substituted or unsubstituted 5-6 membered heteroaromatic ring, more preferably, ring D is selected from the group consisting of:

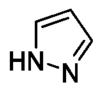 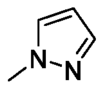 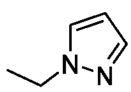 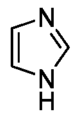 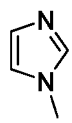

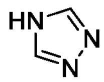 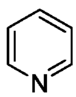 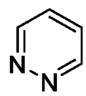 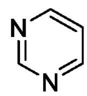

and ring A is selected from the group consisting of: substituted or unsubstituted 8-12 membered fused bicyclic heterocyclyl (including carbocycle or heterocycle, preferably five-membered fused six-membered ring), and substituted or unsubstituted 7-10 membered fused bicyclic heteroaryl (preferably five-membered fused six-membered ring). Preferably, ring A is selected from the group consisting of:

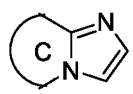 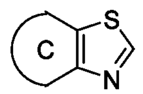 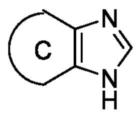

wherein ring C is selected from the group consisting of substituted or unsubstituted benzene ring, and substituted or unsubstituted 5-6 membered heteroaromatic ring; $R_8$ is $CF_3$.

In another preferred embodiment, $R_2$ is selected from the group consisting of: $R_7$, and -$L_2R_7$; wherein, $L_2$ is selected from the group consisting of: -O-, -CHR-, carbonyl, S, and -NH-; wherein, $R_7$ is selected from the group consisting of: substituted or unsubstituted $C_1$-$C_6$ alkyl, substituted or unsubstituted $C_{6-10}$ aromatic ring, and substituted or unsubstituted 5-12 membered heteroaromatic ring.

In another preferred embodiment, $R_7$ is selected from the group consisting of: substituted or unsubstituted $C_1$-$C_6$ alkyl, substituted or unsubstituted benzene ring, and substituted or unsubstituted 5-7 membered heteroaromatic ring.

In another preferred embodiment, $R_2$ is selected from the group consisting of: $R_7$, and -(CHR)$R_7$; wherein, $R_7$ is selected from the group consisting of: substituted or unsubstituted $C_{6-10}$ aromatic ring, and substituted or unsubstituted 5-12 membered heteroaromatic ring.

In another preferred embodiment, $R_2$ is substituted or unsubstituted 5-7 membered heteroaromatic ring; and ring A is selected from the group consisting of substituted or unsubstituted 7-10 membered fused bicyclic heteroaryl; $R_8$ is $CF_3$.

In another preferred embodiment, Ra has the structure shown in the following formula:

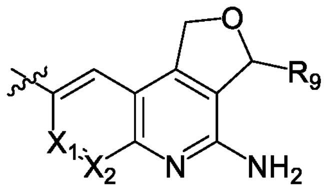 or 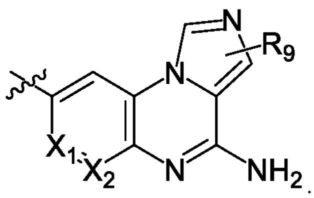

In a second aspect of the present invention, provided is a pharmaceutical composition comprising a therapeutically effective amount of one or more of the compound according to the first aspect of the present invention, or a pharmaceutically acceptable salt, a racemate, an optical isomer, a stereoisomer, or a tautomer thereof, and one or more pharmaceutically acceptable carriers, excipients, adjuvants, accessories, and/or diluents.

In a third aspect of the present invention, provided is a use of the compound according to the first aspect of the present invention, or a racemate, an optical isomer, or a pharmaceutically acceptable salt thereof in the preparation of drugs for treatment or prevention of diseases associated with abnormal gene levels or abnormal expression of PRMT5(such as corresponding nucleic acid mutations, deletions, or the methyltransferase is ectopic or fused or overexpressed).

In another preferred embodiment, the disease is selected from the group consisting of: the disease or disorder ovarian cancer, lung cancer, lymphatic cancer, glioblastoma, colon cancer, melanoma, gastric cancer, pancreatic cancer or bladder cancer.

It should be understood that, within the scope of the present invention, each of the above technical features of the present invention and each of the technical features specifically described in the following (such as the embodiments) can be combined with each other to constitute a new or preferred technical solution. Due to space limitations, It will not be repeated herein.

## EMBODIMENTS FOR CARRYING OUT THE INVENTION

After long and intensive research, the inventors unexpectedly discovered a class of compound with PRMT5 regulatory effects for the first time. The present invention is completed on this basis.

## TERMS

As used herein, halogen refers to F, Cl, Br or I.

As used herein, unless otherwise specified, the terms used have a general meaning known to those skilled in the art. As used herein, unless otherwise specified, all chemical formulas are intended to encompass any possible optical or geometric isomers (such as R-type, S-type or racemate, or cis-trans isomers of olefins, etc.).

As used herein, the term "C1-C6 alkyl" refers to a linear or branched alkyl having 1 to 6 carbon atoms, but not limited to methyl, ethyl, propyl, isopropyl, butyl, isobutyl , sec-butyl, tert-butyl, pentyl and hexyl and the like; preferably ethyl, propyl, isopropyl, butyl, isobutyl, sec-butyl and tert-butyl.

As used herein, the term "C1-C6 alkoxyl" refers to a linear or branched alkoxy having 1 to 6 carbon atoms, including but not limited to methoxy, ethoxy, propoxy, isopropoxy and butoxy and the like.

As used herein, the term "C2-C6 alkenyl" refers to a linear or branched alkenyl having 2 to 6 carbon atoms and containing a double bond, including but not limited to vinyl, propenyl, butenyl, isobutenyl, pentenyl, hexenyl, and the like.

As used herein, the term "C2-C6 alkynyl" refers to a linear or branched alkynyl group having 2 to 6 carbon atoms and containing a triple bond, including but not limited to ethynyl, propynyl, butynyl, isobutynyl, pentynyl, hexynyl, and the like.

As used herein, the term "C3-C10 cycloalkyl" refers to a cyclic alkyl having 3 to 10 carbon atoms on the ring, including but not limited to cyclopropyl, cyclobutyl, cyclopentyl, cyclohexyl, cycloheptyl, cyclooctyl, cyclodecyl and the like. The terms "C3-C8 cycloalkyl", "C3-C7 cycloalkyl", and "C3-C6 cycloalkyl" have similar meanings.

As used herein, the term "C3-C10 cycloalkenyl" refers to a cyclic alkenyl having 3 to 10 carbon atoms on the ring, including but not limited to cyclopropenyl, cyclobutenyl, cyclopentenyl, cyclohexenyl, cycloheptenyl, cyclooctenyl, cyclodecenyl and the like. The term "C3 -C7 cycloalkenyl" has a similar meaning.

As used herein, the term "C1-C12 alkoxycarbonyl" refers to an alkoxycarbonyl having 1 to 12 carbon atoms on the alkyl chain, including but not limited to methoxycarbonyl, ethoxycarbonyl, propoxycarbonyl, isopropoxycarbonyl, tert butoxycarbonyl, benzyloxycarbonyl, and the like.

As used herein, the term "C1-C12 alkylaminocarbonyl" refers to an alkylaminocarbonyl having 1 to 12 carbon atoms on the alkyl chain, including but not limited to methylamino carbonyl, ethylamino carbonyl, propylamino carbonyl, isopropylamino carbonyl, tert butylamino carbonyl, benzylamino carbonyl, dimethylamino carbonyl and the like.

As used herein, the terms "aromatic ring" or "aryl" have the same meaning, preferably "aryl" is "C6-C12 aryl" or

"C6-C10 aryl". The term "C6-C12 aryl" refers to an aromatic ring group having 6 to 12 carbon atoms without any heteroatoms on the ring, such as phenyl, naphthyl and the like. The term "C6-C10 aryl" has a similar meaning.

As used herein, the terms "heteroaromatic ring" or "heteroaryl" have the same meaning, referring to heteroaromatic groups containing one to more heteroatoms. The heteroatoms referred to herein include oxygen, sulfur, and nitrogen. For example, furyl, thienyl, pyridinyl, pyrazolyl, pyrrolyl, N-alkyl pyrrolyl, pyrimidinyl, pyrazinyl, imidazolyl, tetrazolyl, and the like. Heteroaryl may be fused onto an aromatic, heterocyclic, or cycloalkyl ring, wherein the ring connected to the parent structure is a heteroaryl ring. Heteroaryl may be optionally substituted or unsubstituted.

As used herein, the term "3-12 membered heterocyclyl" refers to a saturated or unsaturated 3-12 membered cyclic group containing 1-3 heteroatoms selected from oxygen, sulfur, and nitrogen on the ring, such as dioxocyclopentyl and the like. The term "3-7-membered heterocyclyl" has a similar definition.

As used herein, the term "substituted" indicates that one or more hydrogen atoms on a specific group are substituted by specific substituents. The specific substituents are the substituents described in the previous text, or the substituents appeared in each example. Unless otherwise specified, a substituted group may have a substituent selected from a specific group at any substitutable site of that group, and the substituents may be the same or different in each position. A cyclic substituent, such as a heterocyclicalky, can be linked to another ring, such as a cycloalkyl, thereby forming a spiro-dicyclic ring system, where the two rings share a common carbon atom. It should be understood by those skilled in the art that the combinations of substituents contemplated by the present invention are those that are stable or chemically achievable. The substituents, such as (but not limited to): C1-8 alkyl, C2-8 alkenyl, C2-8 alkynyl, C3-8 cycloalkyl, 3- to 12-membered heterocyclyl, aryl, heteroaryl, halogen, hydroxyl, carboxyl (-COOH), C1-8 aldehyde group, C2-10 acyl, C2-10 ester, C1-C12 alkoxycarbonyl, amino, alkoxyl, C1-10 sulfonyl, etc.

When using expressions such as "C1-8" and the like, it means that the functional group can have 1, 2, 3, 4, 5, 6, 7, or 8 carbon atoms.

When using expressions such as "3-12 membered" and the like, it refers to that the group has 3, 4, 5, 6, 7, 8, 9, 10, 11, or 12 carbon atoms or heteroatoms as ring atoms.

**PRMT5 regulatory compound**

The present invention provides a class of compounds with PRMT5 regulatory activity:

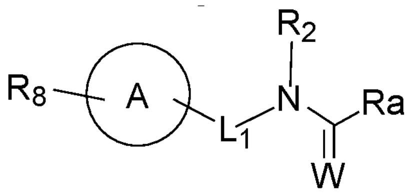

I

wherein,

Ra is selected from the group consisting of

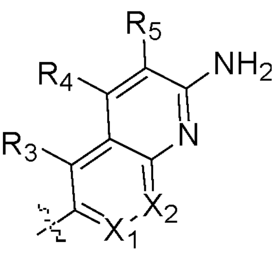

and

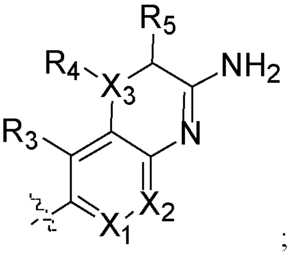

;

W is O or S;

$X_1$, $X_2$ are each independently selected from the group consisting of CR and N; $X_3$ is N;

$L_1$ is selected from the group consisting of: chemical bonds, -O-, -CHR-, and -C(R)R-;

Ring A is selected from the group consisting of: substituted or unsubstituted 8-12 membered fused bicyclic heterocyclyl (including carbocycle or heterocycle, preferably five-membered fused six-membered ring), substituted or unsubstituted 7-10 membered fused bicyclic heteroaryl (preferably five-membered fused six-membered ring);

$R_8$ is selected from the group consisting of: H, halogen, cyano, amino, nitro, hydroxyl, thiol, aldehyde, carboxyl, $C_2$-$C_6$ alkynyl, $SF_5$, and substituted or unsubstituted or halogenated $C_1$-$C_6$ alkyl, or

$R_8$ is

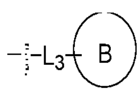

;

$L_3$ is selected from the group consisting of: chemical bonds, -O-, -CHR-, -C(R)R-, carbonyl, S, and -NH-;

Ring B is selected from the group consisting of: substituted or unsubstituted benzene ring, substituted or unsubstituted 5-6-membered heteroaromatic ring, substituted or unsubstituted $C_3$-$C_6$ carbocycle (including saturated and partially unsaturated situations), and substituted or unsubstituted 3-7-membered heterocycle (including saturated and partially unsaturated situations);

$R_2$ is selected from the group consisting of: $R_7$, and $-L_2R_7$; wherein, $L_2$ is selected from the group consisting of: -O-, -CHR-, -C(R)R-, and carbonyl; wherein, $R_7$ is selected from the group consisting of: hydrogen, none, substituted or unsubstituted $C_1$-$C_6$ alkyl, substituted or unsubstituted $C_6$-$C_{10}$ aromatic ring, substituted or unsubstituted 5-12 membered heteroaromatic ring, substituted or unsubstituted $C_3$-$C_{10}$ carbocycle (including saturated and partially unsaturated situations, including monocycle, fused ring, spiro ring and bridged ring), and substituted or unsubstituted 3-10 membered heterocycle (including saturated and partially unsaturated situations, including single ring, fused ring, spiro ring and bridged ring);

$R_3$ is selected from the group consisting of H, halogen, cyano, and substituted or unsubstituted $C_1$-$C_6$ alkyl;

$R_4$ and $R_5$ are each independently selected from the group consisting of: H, halogen, cyano, substituted or unsubstituted $C_1$-$C_6$ alkyl, substituted or unsubstituted $C_1$-$C_6$ alkoxyl, substituted or unsubstituted $C_3$-$C_6$ carbocycle (including saturated and partially unsaturated situations), and substituted or unsubstituted 3-6 membered heterocycle; or $R_4$ and $R_5$ together with the connected ring atom form a 5-12 membered saturated or unsaturated ring, and the ring can be substituted or unsubstituted;

R is H, halogen, substituted or unsubstituted $C_1$-$C_4$ alkyl, substituted or unsubstituted $C_1$-$C_4$ alkoxyl, and substituted or unsubstituted $C_3$-$C_6$ cycloalkyl;

unless otherwise specified, in the above formulas, the "substituted" refers to hydrogen atoms on the corresponding group are substituted by one or more substituents selected from the group consisting of: deuterium, tritium, halogen, hydroxyl, carboxyl, thiol, benzyl, $C_1$-$C_{12}$ alkoxycarbonyl, $C_1$-$C_6$ aldehyde, amino, $C_1$-$C_6$ amide, nitro, cyano, unsubstituted or halogenated $C_1$-$C_6$ alkyl, unsubstituted or halogenated $C_3$-$C_8$ cycloalkyl, $C_2$-$C_{10}$ alkenyl, $C_1$-$C_6$ alkoxyl $C_1$-$C_6$ alkyl-amino, $C_6$-$C_{10}$ aryl, five-membered or six-membered heteroaryl, five-membered or six-membered non-aromatic heterocyclyl, -O-($C_6$-$C_{10}$ aryl), -O- (five-membered or six-membered heteroaryl), $C_1$-$C_{12}$ alkylamino carbonyl, unsubstituted or halogenated $C_2$-$C_{10}$ acyl, sulfonyl ($-SO_2$-OH), phosphoryl-($-PO_3$-OH), unsubstituted or halogenated $C_1$-$C_4$ alkyl-$S(O)_2$-, and unsubstituted or halogenated $C_1$-$C_4$ alkyl-SO-.

**Pharmaceutical composition and mode of administration**

Due to the excellent methyltransferase inhibitory activity of the compound of the present invention, the compound of the invention and various crystal forms, pharmaceutically acceptable inorganic or organic salts, hydrates or solvates thereof, and pharmaceutical compositions containing the compound of the present invention as main active ingredients can be used in the treatment, prevention and alleviation of the related diseases induced by the abnormal expression or

activity of methyltransferase (such as PRMT5).

The pharmaceutical composition of the present invention comprises a safe and effective amount of the compound of the present invention,or a pharmaceutically acceptable salt thereof and pharmaceutically acceptable excipients or carriers. Wherein "safe and effective amount" refers to the amount of compound which is sufficient to significantly improve the condition, and not to generate severe side effects. Generally, the pharmaceutical composition contains 1-2000 mg polymorphs of the invention per dose, preferably, 5-200mg polymorphs of the invention per dose. Preferably, the "one dose" is one capsule or one pill.

"Pharmaceutically acceptable carrier" means one or more compatible solid or liquid fillers, or gelatinous materials which are suitable for human use and should be of sufficient purity and sufficiently low toxicity. "Compatible" herein refers to the ability of each component of a composition can be mixed with the compound of the present invention and can be mixed with each other without appreciably reducing the efficacy of the compound. Examples of pharmaceutically acceptable carrier include cellulose and derivatives thereof (such as sodium carboxymethylcellulose, sodium ethylcellulose, cellulose acetate, etc.), gelatin, talc, solid lubricant (such as stearic acid, magnesium stearate), calcium sulfate, vegetable oil (such as soybean oil, sesame oil, peanut oil, olive oil, etc.), polyol (such as propylene glycol, glycerol, mannitol, sorbitol, etc.), emulsifier (such as Tween®), wetting agent (such as lauryl sodium sulfate), colorant, flavoring, stabilizer, antioxidant, preservative, pyrogen-free water, etc.

There is no special limitation of administration mode for the compound or pharmaceutical compositions of the present invention, and the representative administration mode includes (but is not limited to): oral, intratumoral, rectal, parenteral (intravenous, intramuscular or subcutaneous), and topical administration.

The solid dosage forms used for oral administration include capsules, tablets, pills, powders, and granules. In these solid dosage forms, the active compounds are mixed with at least one conventional inert excipient (or carrier), such as sodium citrate or dicalcium phosphate, or mixed with any of the following components: (a) fillers or compatibilizer, for example, starch, lactose, sucrose, glucose, mannitol and silicic acid; (b) binders, for example, hydroxymethyl cellulose, alginates, gelatin, polyvinylpyrrolidone, sucrose and arabic gum; (c) humectant, such as, glycerol; (d) disintegrating agents such as agar, calcium carbonate, potato starch or tapioca starch, alginic acid, certain composite silicates, and sodium carbonate; (e) dissolution-retarding agents, such as paraffin; (f) absorption accelerators, for example, quaternary ammonium compounds; (g) wetting agents, such as cetyl alcohol and glyceryl monostearate; (h) adsorbents, for example, kaolin; and (i) lubricants such as talc, stearin calcium, magnesium stearate, solid polyethylene glycol, sodium lauryl sulfate, or the mixtures thereof. In capsules, tablets, and pills, the dosage form may also include buffers.

Solid dosage forms such as tablets, sugar pills, capsules, pills, and granules can be prepared using coating and shell materials, such as casings and other materials well-known in the art. They can contain opacifiers, and the release of active compounds or compounds in the composition can be delayed in a certain part of the digestive tract. Examples of embedding components that may be employed are polymeric substances and waxes. If necessary, the active compound may also be formed into a microcapsules with one or more of the above excipients.

Liquid dosage forms for oral administration include pharmaceutically acceptable lotion, solutions, suspensions, syrups or tinctures. In addition to the active compounds, the liquid dosage forms may contain any conventional inert diluents known in the art such as water or other solvents, solubilizers and emulsifiers, for example, ethanol, isopropanol, ethyl carbonate, ethyl acetate, propylene glycol, 1,3-butanediol, dimethyl carboxamide, as well as oil, in particular, cottonseed oil, peanut oil, corn germ oil, olive oil, castor oil and sesame oil, or the combination thereof.

In addition to these inert diluents, the composition can also include additives such as wetting agents, emulsifiers and suspensions, sweeteners, correctors, and spices.

In addition to active compounds, suspensions can include suspending agents such as ethoxylated isooctadecanol, polyoxyethylene sorbitol and dehydrated sorbitol esters, microcrystalline cellulose, methanol aluminum and agar, or mixtures of these substances.

Compositions for parenteral injection may include physiologically acceptable sterile aqueous or anhydrous solutions, dispersion liquid, suspensions or lotions, and sterile powders for re dissolution into sterile injectable solutions or dispersions. Suitable aqueous and non-aqueous carriers, diluents, solvents or excipients include water, ethanol, polyols, and their suitable mixtures.

The dosage forms of the compounds of the present invention used for local administration include ointments, powders, patches, sprays, and inhalants. The active ingredients are mixed under sterile conditions with physiologically acceptable carriers and any preservatives, buffers, and propellants if necessary.

The compounds of the present invention may be administered alone or in combination with other pharmaceutically acceptable compound. In some preferred embodiments, the compounds of the present invention can form PROTAC with other small molecule compounds, or jointly form ADC with other large molecule compounds such as monoclonal antibodies for application.

When the pharmaceutical compositions are used, a safe and effective amount of compound of the present invention is applied to a mammal (such as human) in need of, wherein the dose of administration is a pharmaceutically effective dose. For a person weighed 60 kg, the daily dose is usually 1- 2000 mg, preferably 5-500 mg. Of course, the

particular dose should also depend on various factors, such as the route of administration, patient healthy status, which are well within the skills of an experienced physician.

The present invention was further described hereafter in combination with specific embodiments. It should be understood that these examples are only used to illustrate the and not to limit the scope of the invention. The experimental methods without specific conditions in the following examples generally follow the conventional conditions or the conditions suggested by the manufacturer. Unless otherwise stated, percentages and parts are caculated by weight.

The definitions of each abbreviation are as follows:

| | |
|---|---|
| Xantphos | 4,5-Bis(diphenylphosphino)-9,9-dimethylxanthene |
| NBS | N-Bromosuccinimide |
| Pd(dppf)Cl2 | 1,1'-Bis(diphenylphosphino)ferrocene]dichloropalladium(II) |
| Pd2(dba)3 | Tris(dibenzylideneacetone)dipalladium |
| Pd(dba)2 | Bis(dibenzylideneacetone)palladium |
| BINAP | 1.1'-Binaphthyl-2,2'-diphemyl phosphine |
| X-Phos | 2-dicyclohexylphosphino-2',4',6'-triiso-propyl-1,1'-biphenyl |
| tBuXPhos | 2-Di-tert-butylphosphino-2',4',6'-triiso-propyl-1,1'-biphenyl |
| tBuXPhos Pd G3 | Methanesulfonate (2-di-tert-butylphospho-2',4',6'-triisopropyl-1,1'- biphenyl) (2'-amino-1,1'-biphenyl-2-yl) palladium (II) |
| EDCI | n-(3-dimethylaminopropyl)-n'-ethylcarbodiimide hydrochloride |
| HOBT | 1-Hydroxybenzotriazole |
| LDA | Lithium diisopropylamide |
| HATU | 2-(7-Azobenzotriazole)-N,N,N',N'-Tetramethylurea hexafluorophosphate |
| | Supercritical fluid chromatography |

The raw materials can be obtained commercially or prepared by methods already known or disclosed in the art.

Purification of intermediates and compounds were carried out by normal phase or reverse chromatography or conventional chemical laboratory operations such as recrystallisation. Normal phase chromatography was either preloaded silica gel columns or preparative thin layer chromatography. Silica gel columns are mainly glass columns or fast preparative chromatographs. The mobile phase for normal phase chromatography was selected and proportioned for elution from petroleum ether/ethyl acetate, dichloromethane/methanol or other suitable solvents. Reverse phase preparative liquid chromatography was carried out on a C18 column using a preparative liquid chromatograph or rapid preparative chromatograph. Detection was performed using 214nM and 254nM wavelength or preparative liquid chromatography-mass spectrometry instrument, using water/acetonitrile containing 0.1% hydrochloric acid, water/-acetonitrile, water/acetonitrile containing 0.1% ammonium bicarbonate, water/acetonitrile containing 0.1% formic acid, 0.1% ammonia/acetonitrile, water/acetonitrile containing 0.1% trifluoroacetic acid or other suitable solvent system as the mobile phase for gradient elution.

Structural characterisation of intermediates and compounds were carried out by nuclear magnetic resonance (NMR) and mass spectrometry (LCMS). The nuclear magnetic resonance spectrometers used for nuclear magnetic resonance are Bruker Ascend 400 or Varian 400, or ZKNJ BIXI-1 300MHz, Bruker Avance III 400 MHz, or Bruker AVANCE Neo 400 MHz. The solvents used are deuterated dimethyl sulfoxide, deuterated chloroform, deuterated methanol, or other labeled deuterated solvents. Spectral data reported in patterns: chemical shifts $\delta$ (Peak splitting number, coupling constant J (Hz), number of hydrogen). Tetramethylsilane was used as an internal standard for chemical shifts and the chemical shifts of which was set to zero ($\delta$, 0 ppm). The meanings of some of the abbreviations are: s (single peak), d (double peak), t (triple peak), q (quadruple peak), m (multiple peaks), br (broad peak).

Representative methods for liquid chromatography -mass spectrometry (LCMS) in the structural characterization of intermediates and compounds are listed below:

Method 1: Conducted on an Agilent LC1260 system coupled with a single quadrupole mass spectrometer.
column: Waters CORTECS C-18, 2.7 $\mu$m, 4.6*30 mm. Solvent A: 0.05% formic acid aqueous solution, solvent 20 B: a solution of 0.05% formic acid in acetonitrile, lasting for one minute from 5% acetonitrile to 95% acetonitrile, holding for one minute, for a total of 2.5 minutes; Flow rate: 1.8 mL/min; column temperature 40°C.
column: XSelect CSH C18, 3.5 $\mu$m, 4.6*50 mm. Solvent A: 0.05% ammonia aqueous solution, solvent B: a solution of 0.05% ammonia in acetonitrile, lasting for one minute from 5% acetonitrile to 95% acetonitrile, holding for one minute,

for a total of 2.5 minutes; Flow rate: 1.8 mL/min; column temperature 40°C.

Method 2: Conducted on an Agilent LC/MSD 1200 system coupled with a quadrupole mass spectrometer.

column: ODS2000 (50 x 4.6 mm, 5 μm) (ES (+) or (-) ionization mode), temperature 30 °C; Flow rate: 1.5 mL/min.

Equipment and methods used in SFC (Supercritical Fluid Chromatography) chiral separation and chiral compound characterisation:

Method 3:

Chromatographic column: DAICEL CHIRALPAK IC(250 mm * 30 mm, 10 um
Mobile Phase: A: $CO_2$ B: [MeOH(0.1% IPAm)]
Elution gradient: 40%

Method 4:

Chromatographic column: DAICEL CHIRALPAK AD(250 mm*30 mm, 10um);
Mobile Phase: [ACN/EtOH(0.1% NH3H2O]; B %: 69%, 20min, isocratic elution)

Method 5:

Chromatographic column: DAICEL CHIRALPAK AD(250mm*50mm, 10um);
Mobile phase: [$CO_2$-EtOH (0.1% $NH_3H_2O$)]; B%: 55%, isocratic elution mode)

Representative preparative HPLC (high-performance liquid chromatography) method:

Method 6:
Chromatographic column: Waters Xbridge BEH C18 100*30 mm*10 um; mobile phase: [$H_2O$ (10 mM $NH_4HCO_3$)-ACN]; B%: 25%-55%, 8.0min, UV 220 & 254 nm.
Method 7:
Chromatographic column: Waters Xbridge BEH C18 250 * 50 mm * 10 μm; mobile phase: [$H_2O$ ($NH_4HCO_3$ 10 mM)- ACN]; B%: 20%-45%, 10min, UV 220 & 254 nm.
Method 8:
Chromatographic column: Welch Xtimate C18 250*70 mm#10 μm; mobile phase: [$H_2O$ ($NH_4HCO_3$)-ACN]; B%: 20%-50%, 20min, UV 220 & 254 nm.

General method for Example synthesis:

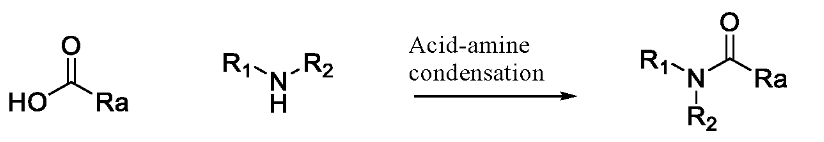


Intermediate A Intermediate B |Target compound

General method: Synthesis of intermediate A1

Synthetic route:

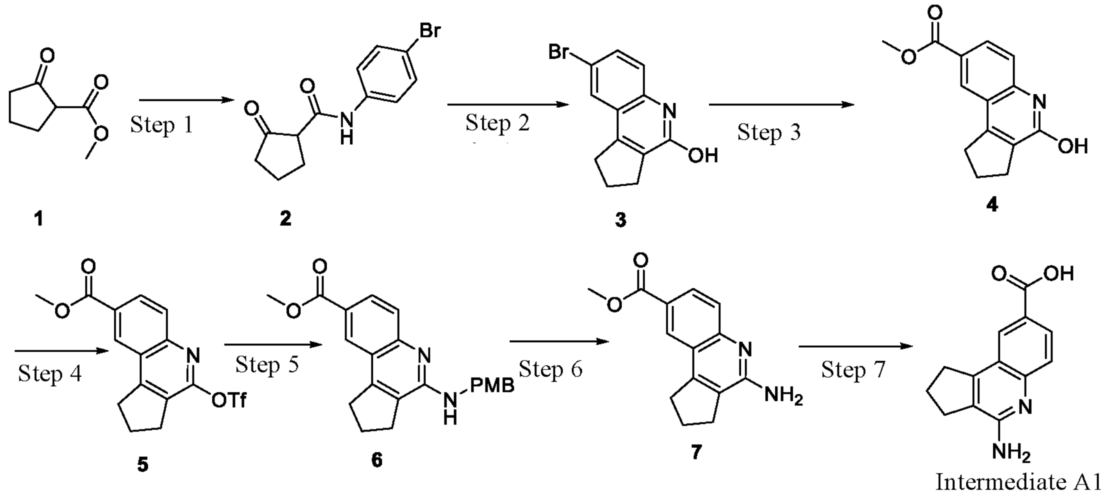


1 2 3 4 5 6 7

Intermediate A1

Step 1: N - (4-bromophenyl) -2-oxocyclopentane-1-carboxamide (2)

A mixture of methyl 2-oxocyclopentane-1-carboxylate (50g, 0.35mol) and 4-bromoaniline (121g, 0.70mol) in toluene (300mL) was stirred at 110 °C for 12 hours. Then the mixture was added to a 2M HCl aqueous solution (100 mL) and diluted with water (300 mL). The reaction was extracted with ethyl acetate (300 mL×3). The organic layer was washed with brine(200 mL×3), and dried with anhydrous sodium sulfate. After filtration, the organic layer was concentrated under reduced pressure. The residue was purified by silica gel chromatography and eluted from 0% to 30% with EA/PE solution within 20 minutes to obtain N-(4-bromophenyl) -2-oxocyclopentane-1-carboxamide (25g, yield 21%) as a light yellow hard solid. LC-MS: Rt = 1.257 min, (ESI) m/z. $[M+H]^+$ 283.0, $[M+2+H]^+$ 285.0; $C_{12}H_{12}BrNO_2$

**Step 2: 8-bromo-2,3-dihydro-1H-cyclopentadieno[c]quinoline-4-ol (3)**

A mixture of N-(4-bromophenyl) -2-oxocyclopentane-1-carboxamide(25.0g, 0.088mol) in concentrated sulfuric acid (100mL) was stirred at 100 °C for 12 hours. The mixture was poured into iced water(500mL, and $NaHCO_3$ was added until pH 7-8. The precipitate formed was filtered and washed with some cold methanol (50 mL). The filtrate was filtered. The combined solid was dried and re-crystallized in ethonal(20mL) to obtain 8-bromo-2,3-dihydro-1H-cyclopentadieno [c] quinoline-4-ol(6g, 26% yield) as a light brown solid. LC-MS: Rt = 1.299 min, (ESI) m/z. $[M+H]^+$ 264.0; $[M+2+H]^+$ 266.0, $C_{12}H_{10}BrNO$

**Step 3: methyl 4-hydroxy-2,3-dihydro-1H-cyclopentadieno [c] quinoline-8-carboxylate (4)**

A mixture of 8-bromo-2,3-dihydro-1H-cyclopentadieno [c] quinoline-4-ol (6.0 g, 23 mmol), Et3N (6.9 g, 68 mmol), and Pd(dppf)$Cl_2$ (3.3 g, 4.40 mmol in MeOH (100mL)) was stirred at 100 °C and under CO atmosphere for 12 hours. The mixture was concentrated under reduced pressure. The residue was purified by silica gel chromatography and eluted from 0% to 10% with a solution of MeOH in DCM within 20 minutes to obtain methyl 4-hydroxy-2,3-dihydro-1H-cyclopentadieno [c] quinoline-8-carboxylate (3 g, 54% yield) as a brown solid. LC-MS: Rt = 1.183 min, (ESI) m/z. $[M+H]^+$ 244.09, $C_{14}H_{13}NO_3$

**Step 4: methyl 4- (((trifluoromethyl) sulfonyl) oxy) -2,3-dihydro-1H-cyclopentadieno [c] quinoline-8-carboxylate (5)**

To a solution of methyl 4-hydroxyl-2,3-dihydro-1H-cyclopentadieno [c] quinoline-8-carboxylate (9.3 g, 0.038 mol) and pyridine (9.1 g, 0.11 mmol) in DCM (200 mL) was added trifluoromethanesulfonic anhydride (21.6 g, 0.076 mol) at 0 °C. The mixture was then stirred at 20°C for 12 hours. The reaction was quenched with $NaHCO_3$(aq.) (200 mL), and then extracted with DCM (200 mL × 3). The organic solution was washed with brine(200 mL) , dried with $Na_2SO_4$ and then filtered. The filtrate was concentrated under reduced pressure. The residue was purified by silica gel chromatography and eluted from 0% to 30% with a solution of EA in PE within 20 minutes to obtain methyl 4- (((trifluoromethyl) sulfonyl) oxy) -2,3-dihydro-1H-cyclopenta [c] quinoline-8-carboxylate (11.0 g, 77% yield) as a yellow solid. $^1H$ NMR (400 MHz, $CDCl_3$) δ: 8.57 (d, $J$ = 2.0 Hz, 1H), 8.30 (dd, $J$ = 8.8, 2.0 Hz, 1H), 8.08 (d, $J$ = 8.8 Hz, 1H), 4.01 (s, 3H), 3.43 (t, $J$ = 8.0 Hz, 2H), 3.21 (t, $J$ = 8.0 Hz, 2H), 2.55 - 2.32 (m, 2H). LC-MS: Rt =1.544 min, (ESI) m/z. 376.1 $[M+H]^+$. $C_{15}H_{12}F_3NO_5S$ 375.04.

**Step 5: methyl 4-((4-methoxybenzyl) amino) -2,3-dihydro-1H-cyclopentadieno [c] quinoline-8-carboxylate (6)**

To a stirrred solution of methyl 4- (((trifluoromethyl) sulfonyl) oxy) -2,3-dihydro-1H-cyclopenta [c] quinoline-8-carboxylate (11.0 g, 29 mmol) in dioxane (100 mL) was added $Cs_2CO_3$ (28.6 g, 88 mmol), $Pd_2(dba)_3$ (2.7 g, 2.93 mmol), Xanthhos (3.4 g, 5.86 mmol), and $PMBNH_2$ (6.0 g, 0.044 mmol) at minus 20 °C. The reaction mixture was stirred at 110°C for 12 hours under $N_2$ atmosphere. The mixture was filtered and the filtrate was concentrated under reduced pressure. The residue was purified by silica gel chromatography and eluted from 0% to 5% with a solution of MeOH in DCM within 20 minutes to obtain methyl 4-((4-methoxybenzyl) amino) -2,3-dihydro-1H-cyclopentadieno [c] quinoline-8-carboxylate (8.1 g, 76% yield) as a yellow solid. $^1H$ NMR (400 MHz, $CDCl_3$) δ: 8.33 (d, *J* = 2.0 Hz, 1H), 8.11 (dd, *J* = 8.8, 2.0 Hz, 1H), 7.79 (d, *J* = 8.8 Hz, 1H), 7.36 (d, *J* = 8.8 Hz, 2H), 6.89 (d, *J= 8.8* Hz, 2H), 4.79 (s, 2H), 3.95 (s, 3H), 3.80 (s, 3H), 3.24 (t, *J* = 8.0 Hz, 2H), 2.80 (t, *J*= 8.0 Hz, 2H), 2.36 - 2.19 (m, 2H). LC-MS: Rt =1.036 min, (ESI) m/z. 363.1 $[M+H]^+$. $C_{22}H_{22}N_2O_3$ 362.16.

**Step 6: methyl 4-Amino-2,3-dihydro-1H-cyclopentadieno [c] quinoline-8-carboxylate (7)**

A solution of methyl 4-((4-methoxybenzyl) amino) -2,3-dihydro-1H-cyclopentadieno [c] quinoline-8-carboxylate (8.1 g, 20 mmol) in $CF_3COOH$ (50 mL) was stirred at 70°C for 12 hours. Then the solvent was removed and the residue was dissolved in DCM (200mL). The organic phase was washed with $NaHCO_3$ (aq.) (300 mL) and dried with $Na_2SO_4$. Then the mixture was filtered and the filtrate was concentrated under reduced pressure. The residue was purified by silica gel chromatography and eluted from 0% to 5% with a solution of MeOH in DCM within 20 minutes to obtain methyl 4- amino -2,3-dihydro-1H-cyclopentadieno [c] quinoline-8-carboxylate (4.6 g, 85 % yield) as a yellow solid. LC-MS: Rt =0.921 min, (ESI) m/z. 243.1 $[M+H]^+$. $C_{14}H_{14}N_2O$ 242.11.

**Step 7: 4-Amino-2,3-dihydro-1H-cyclopentadieno [c] quinoline-8-carboxylate (intermediate A1)**

A solution of methyl 4- amino -2,3-dihydro-1H-cyclopentadieno [c] quinoline-8-carboxylate (0.60 g, 2.31 mmol) in HCl (4 mol/L in $H_2O$)(20 mL) was stirred at 95°C for 12 hours. Then the solvent was removed and the residue was 4-Amino-2,3-dihydro-1H-cyclopenta [c] quinoline-8-carboxylic acid (500mg, 88% yield) as a white solid, which was used in the next step without further purification. $^1H$ NMR (400 MHz, DMSO-$d_6$) δ: 8.65 (s, 2H), 8.31 (s, 1H), 8.22 (d, *J* = 8.0 Hz, 1H), 7.83 (d, *J* = 8.0 Hz, 2H), 3.32 (t, *J* = 8.0 Hz, 2H), 2.95 (t, *J* = 8.0 Hz, 2H), 2.34 - 2.20 (m, 2H). LC-MS: Rt =0.763 min, (ESI) m/z. 229.1 $[M+H]^+$. $C_{13}H_{12}N_2O_2$ 228.09.

**General method: Synthesis of intermediate A2**

**Synthetic route:**

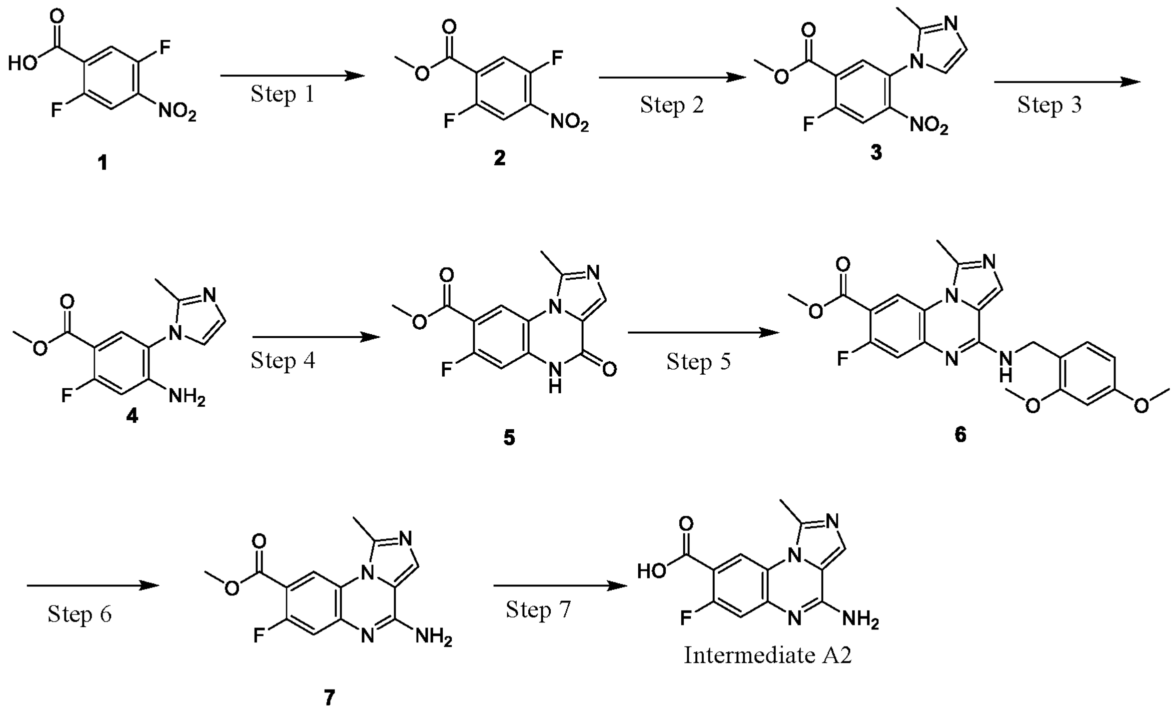


1 2 3 4 5 6 7 Intermediate A2

**Step 1: methyl 2,5-difluoro-4-nitrobenzoate (2)**

To a solution of 2,5-difluoro-4-nitrobenzoic acid (1) (50g, 246.18 mmol, 1 equiv) in methanol (500 mL) was then added thionyl chloride (43.93 g, 369.28 mmol, 26.79 mL, 1.5 equiv) at 0 °C. The reaction solution was reacted for 16 hours at 40°C. LCMS showed the reaction was completed. The reaction solution was concentrated under reduced pressure until dry, then diluted with 300 mL of water and extracted three times with 1 L of ethyl acetate. The organic phase was washed with saturated salt water (400ml), dried with anhydrous magnesium sulfate, and filtered. The filtrate was concentrated under reduced pressure until dry. The crude product was pulped with petroleum ether at 25 °C for 60 minutes to obtain white solid methyl 2,5-difluoro-4-nitrobenzoate (2) (103 g, 474.38 mmol, 96.35% yield). $^{1}$H NMR (400 MHz, CDCl3) δ ppm 7.89 (td, J=9.51, 5.63 Hz, 2 H) 4.00 (s, 3 H). $^{19}$F NMR (376 MHz, CDCl3) δ ppm -110.27 (s, 1 F) -121.56 (m, 1 F)

**Step 2: methyl 2-fluoro-5- (2-methyl-1H-imidazol-1-yl) -4-nitrobenzoate (3)**

Methyl 2,5-difluoro-4-nitrobenzoate (2) (80 g, 368.45 mmol, 1 equiv) and 2-methyl-1H-imidazole (36.30 g, 442.14 mmol, 1.2 equiv) were dissolved in dimethyl sulfoxide (1.2 L). The reaction solution was reacted at 50°C for 16 hours. LCMS showed the reaction was completed. The reaction solution was diluted with 4 L of water and extracted with 4.5 L of ethyl acetate. The organic phase was washed with saturated salt water (3 L), dried with anhydrous magnesium sulfate, and filtered. The filtrate was concentrated under reduced pressure to dryness. The crude product was pulped with methyl tert butyl ether at 25°C for 60 minutes (25.5 g). (2) The mother liquor was purified by column chromatography (silica, 50% tetrahydrofuran in petroleum ether) to obtain yellow liquid, which was then pulped in MTBE at 25 °C for 60 minutes to obtain yellow solid (8.23 g). Yellow solid methyl 2-fluoro-5-(2-methyl-1H-imidazol-1-yl) -4-nitrobenzoate (3) (25.5 g, 91.32 mmol, 24.79% yield), methyl 2,5-difluoro-4-nitrobenzoate (2) (raw material recovery) (20.34 g, 93.68 mmol, 25.43% yield). yellow solid methyl 2-fluoro-5- (2-methyl-1H-imidazol-1-yl) -4-nitrobenzoate (3) (8.2 g, 29.07 mmol, 7.89% yield, 99% purity) HNMR: ES23714-67-P1A1, 1H NMR (400 MHz, DMSO-d6) δ ppm 8.39 (d, J=9.90 Hz, 1 H) 8.08 - 8.20 (m, 1 H) 7.23 (d, J=1.32 Hz, 1 H) 6.92 (s, 1 H) 3.91 (s, 3 H) 2.12 (s, 3 H). 19F NMR (376 MHz, DMSO-d6) δ ppm -105.45 (br s, 1 F)

**Step 3: methyl 4-amino-2-fluoro-5- (2-methyl-1H-imidazol-1-yl)benzoate (4)**

Methyl 2-fluoro-5- (2-methyl-1H-imidazol-1-yl) -4-nitrobenzoate (3) (13.9 g, 49.78 mmol, 1 equiv) was dissolved in tetrahydrofuran (300 mL), and added with palladium carbon hydroxide (2.8 g, 49.78 mmol, 20% purity, 1 equiv) under hydrogen atmosphere. The reaction system was replaced three times with hydrogen gas. The reaction solution was heated to 50°Cunder hydrogen atmosphere(1 equiv) (50 psi)and reacted for 32 hours . LCMS showed the reaction was completed. The reaction solution was filtered through diatomite, and the filter cake was washed four times with 300 mL of ethyl acetate. The filtrate was concentrated to dry under reduced pressure to obtain gray solid. The crude product was

directly used in the next step. Methyl 4-amino-2-fluoro-5- (2-methyl-1H-imidazol-1-yl)benzoate (4) (12 g, 48.15 mmol, 96.72% yield) $^1$H NMR (400 MHz, DMSO-$d_6$) : 7.46 (d, J=7.63 Hz, 1H), 7.07 (d, J=1.38 Hz, 1H), 6.93 (d, J=1.25 Hz, 1H), 6.59 (d, J=13.51 Hz, 1H), 6.15 (br s, 2H), 3.68 -3.77 (m, 3H), 2.01-2.12 (m, 3H).$^{19}$F NMR (376 MHz, DMSO-$d_6$) : -109.31- -108.47 (m, 1F).

**Step 4: methyl 7-fluoro-1-methyl-4-oxo-4,5-dihydroimidazolo [1,5-a] quinoxalin-8-carboxylate (5)**

Methyl 4-amino-2-fluoro-5- (2-methyl-1H-imidazol-1-yl)benzoate (4) (12 g, 48.15 mmol, 1 equiv) was added to 1-methyl-2-pyrrolidone at 25°C, and then 1,1-carbonyldiimidazole (19.52 g, 120.37 mmol, 2.5 equiv) was added. The reaction solution was heated to 115°C and reacted for 16 hours. LCMS showed the reaction was completed. The reaction solutions of the two batches were combined for processing. The reaction solution was added with 600 mL of ethyl acetate and 600 mL of water, and pulped for 16 hours at 25°C. The slurry was filtered under reduced pressure, and the filter cake was washed with 100 mL of ethyl acetate. The solid was concentrated under reduced pressure to obtain gray solid methyl 7-fluoro-1-methyl-4-oxo-4,5-dihydroimidazolo [1,5-a] quinoxalin-8-carboxylate (5)(24.1 g, 87.56 mmol, 86.60% yield). The crude product was directly used in the next step reaction. $^1$H NMR (400 MHz, DMSO-$d_6$) : 11.71 (br s, 1H), 8.40 (d, J=6.38 Hz, 1H), 7.76 (s, 1H), 7.08 (d, J=11.38 Hz, 1H), 3.88 (s, 3H), 2.89 (s, 3H). $^{19}$F NMR (376 MHz, DMSO-$d_6$) : -111.43- -110.58 (m, 1F)

**Step 5: methyl 4- ((2,4-dimethoxybenzyl) amino) -7-fluoro-1-methylimidazolo [1,5-a] quinoxalin-8-carboxylate (6)**

Methyl 7-fluoro-1-methyl-4-oxo-4,5-dihydroimidazolo [1,5-a] quinoxalin-8-carboxylate (5) (12.0 g, 43.60 mmol, 1.0 equiv), 2,4-dimethoxybenzylamine (10.9 g, 65.19 mmol, 9.82 mL, 1.50 equiv), and 1.8-diazabicyclo [5.4.0]undec-7-ene (19.92 g, 130.80 mmol, 19.72 mL, 3.0 equiv) were added to acetonitrile (240 mL), and benzotriazol-1-oxo-tris (dimethylaminophosphate) hexafluorophosphate salt(25.07 g, 56.68 mmol, 1.3 equiv) was added in batches at 15-20 °C. The reaction solution was slightly exothermic, and became homogeneous and solids precipitated. The reaction solution was reacted for 16 hours at 15-20°C under nitrogen protection. LCMS showed the starting material was completely consumed and the target compound was detected. The reaction suspension was filtered under reduced pressure and the filter cake was washed with 100 mL of acetonitrile. The solid was collected and drained to dryness under reduced pressure to obtain offwhite solid methyl 4-((2,4-dimethoxybenzyl) amino) -7-fluoro-1-methylimidazolo [1,5-a] quinoxalin-8-carboxylate (6) (15.3 g, 36.05 mmol, 82.68% yield). LCMS: (ESI) m/z= 425.3[M+1]$^+$; RT= 1.721 min. $^1$H NMR (400 MHz, DMSO-$d_6$) Shift: 8.49 (d, J=7.00 Hz, 1H), 8.45 (t, J=5.57 Hz, 1H), 7.95 (s, 1H), 7.23 (d,J=12.51 Hz, 1H), 7.18 (d, J=8.38 Hz, 1H), 6.58 (d, J=2.38 Hz, 1H), 6.47 (dd, J=2.38, 8.38 Hz, 1H), 4.66 (d,J=5.25 Hz, 2H), 3.88 (s, 3H), 3.82 (s, 3H), 3.73 (s, 3H), 2.93 (s, 3H). $^{19}$F NMR (376.5 MHz, DMSO-$d_6$) Shift: -113.02,,,

**Step 6: methyl 4-amino-7-fluoro-1-methylimidazolo [1,5-a] quinoxalin-8-carboxylate (7)**

Methyl 4- ((2,4-dimethoxybenzyl) amino) -7-fluoro-1-methylimidazolo [1,5-a] quinoxalin-8-carboxylate (6) (16.3 g, 38.40 mmol, 1.0 equiv) was added to dichloromethane(50 mL) and trifluoroacetic acid (250 mL) was added. The reaction solution was heated to 50°C and reacted for 16 hours. LCMS showed the starting material was completely consumed and the target compound was detected. The reaction solution was concentrated to dryness under reduced pressure to obtain purple solid methyl 4-amino-7-fluoro-1-methylimidazolo [1,5-a] quinoxalin-8-carboxylate (7) (28.3 g, crude product). The crude product was directly used in the next step reaction. LCMS ES15882-1150-P1A: (ESI) m/z=275.3 [M+1]$^+$; RT= 0.607 min

**Step 7: 4-amino-7-fluoro-1-methylimidazolo [1,5-a] quinoxalin-8-carboxylic acid (intermediate A2)**

Methyl 4-amino-7-fluoro-1-methylimidazolo [1,5-a] quinoxalin-8-carboxylate (7)(crude product from the previous step) (28.3 g, 38.70 mmol, 1 equiv) was added to tetrahydrofuran (80 mL) and methanol (80 mL). Sodium hydroxide (7.74 g, 193.48 mmol, 5 equiv) was dissolved in water (80 mL) , and then added to the reaction solution. The reaction solution was heated to 50°C and reacted for 4 hours. LCMS detected the desired compound. After cooling to 20°C, the reaction solution was concentrated under reduced pressure to remove the organic solvent. The residue was diluted with water/methanol in a ratio of 10 to 1 (300 mL), and filtered through diatomite. The filter cake was washed three times with water/methanol in a ratio of 10 to 1(300 mL). All filtrates were combined and concentrated under reduced pressure to remove methanol. The pH of the residue was adjusted to 5~6 using acetic acid. The resulting slurry was stirred at 15-20°C for 12 hours and filtered under reduced pressure to obtain solid, which was washed with water. The solid was collected and lyophilized to obtain white solid 4-amino-7-fluoro-1-methylimidazolo [1,5-a] quinoxalin-8-carboxylic acid (intermediate A2) (9.9 g, 37.55 mmol, 97.05% yield, 98.71% purity). LCMS ES15882-1154-P1C: (ESI) m/z= 261.1 [M+1]$^+$; RT= 0.422

min. $^1H$ NMR (400 MHz, DMSO-$d_6$): 13.15 (br s, 1H), 8.53 (d, J=7.04 Hz, 1H), 7.85 (s, 1H), 7.68 (s, 2H), 7.16 (d, J=12.10 Hz, 1H), 2.94 (s, 3H)

**General method: Synthesis of intermediate A3**

**Synthetic route:**

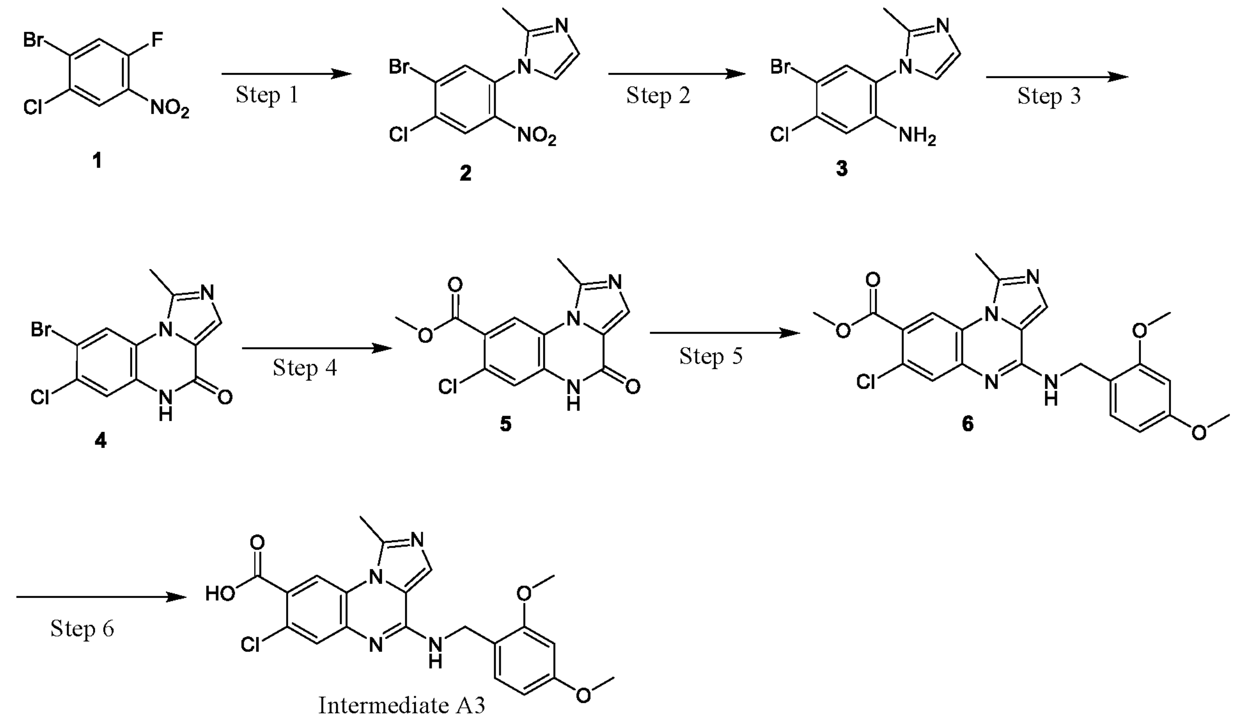


1 2 3 4 5 6 Intermediate A3

**Step 1:1- (5-bromo-4-chloro-2-nitro-phenyl) -2-methyl-imidazole (2)**

1-bromo-2-chloro-5-fluoro-4-nitrobenzene (1) (3 g, 11 mmol, 1 equiv) was addded to a solution of 2-methyl-1H-imidazole (1.2 g, 14 mmol, 1.2 equiv) in acetonitrile (50 mL), and then the reaction solution was added with potassium carbonate (4 g, 29 mmol, 2.5 equiv). The reaction solution was heated to 80°C and stirred for 16 hours. The starting material was detected to be completely consumed and the target compound was generated. The reaction solution was concentrated under reduced pressure to remove acetonitrile, added with water(40 mL) and extracted with ethyl acetate(3 x 50 mL). The organic phase was dried with magnesium sulfate anhydrous and filtered. The filtrate was concentrated to dryness under reduced pressure and purified by column chromatography(silica, 35% tetrahydrofuran in petroleum ether) to obtain white solid 1- (5-bromo-4-chloro-2-nitro-phenyl) -2-methyl-imidazole (2) (4 g). H NMR: $^1H$ NMR (400 MHz, DMSO-$d_6$) $\delta$ 8.55 (s, 1H), 8.32 (s, 1H), 7.22 (d, J=1.32 Hz, 1H), 6.91 (d, J=1.32 Hz, 1H), 2.08-2.23 (m, 3H).

**Step 2: 4-bromo-5-chloro-2- (2-methylimidazol-1-yl) aniline (3)**

1- (5-bromo-4-chloro-2-nitro-phenyl) -2-methyl-imidazole (2) (3.5 g, 11 mmol, 1 equiv) was dissolved in a mixed solution of water (8 mL), ethanol (16 mL) and tetrahydrofuran (16 mL)and ammonium chloride (8.9 g, 166 mmol, 15 equiv) was added , then iron powder (2.5 g, 44 mmol, 4 equiv) was added to the reaction solution after heating to 70°C. The reaction was stirred at 90°C for 2 hours. LC-MS detected that the starting material was completely consumed and the target compound was generated. The reaction solution was filtered through diatomite, washed with ethyl acetate (40mL x 3), and concentrated under reduced pressure to obtain black solid 4-bromo-5-chloro-2-(2-methylimidazol-1-yl) aniline (3) (3.1 g, 10.8 mmol, 98% yield). H NMR:$^1H$ NMR (400 MHz, DMSO-$d_6$) $\delta$ 7.41 (s, 1H), 7.12 (s, 1H), 7.07 (s, 1H), 6.99 (s, 1H), 5.45 (s, 2H), 2.13 (s, 3H).

**Step 3: 8-bromo-7-chloro-1-methyl-5-hydro-imidazolo [1,5-a] quinoxalin-4-one (4)**

4-bromo-5-chloro-2- (2-methylimidazol-1-yl) aniline (3) (3 g, 10.5 mmol, 1 equiv) and 1,1-carbonyl diimidazole (2.6 g, 15.7 mmol, 1.5 equiv) were dissolved successively in 1,2-dichlorobenzene solution(30 mL). The reaction solution was stirred at 130°C for 16 hours. LC-MS detected that the raw material was completely consumed and the target product was generated. The reaction solution was stirred in a solution of ethyl acetate and water (2/1 15 mL) for 30 minutes and filtered to obtain a filter cake. The filter cake was concentrated under vacuum to obtain black solid 8-bromo-7-chloro-1-methyl-5-hydro-imidazolo [1,5-a] quinoxalin-4-one (4) (2.2 g, 7 mmol, 67% yield). H NMR:$^1$H NMR (400 MHz, DMSO-$d_6$) δ 8.04 (s, 1H), 7.33 (d, J=3.30 Hz, 2H), 2.83 (s, 3H)

**Step 4: methyl 7- chloro-1-methyl-4-oxo-5H-imidazolo [1,5-a] quinoxalin-8-carboxylate (5)**

8-bromo-7-chloro-1-methyl-5-hydro-imidazolo [1,5-a] quinoxalin-4-one (4) (500 mg, 1.6 mmol, 1 equiv) was dissolved in ethanol (5 mL) solution and 1.8-diazadicyclo [5.4.0] undec-7-ene (365 mg, 2.4 mmol, 362 μL, 1.5 equiv) was added. The reaction was replaced with nitrogen gas for three times, then tributylphosphorus tetrafluoroborate (46 mg, 160 μmol, 0.1 equiv), molybdenum hexacarbonyl (232 mg, 880 μmol, 118 μL, 0.55 equiv) and palladium acetate (36 mg, 160 μmol, 0.1 equiv). The reaction was stirred at 90°C for two hours. LC-MS detected that the raw material was completely consumed and the target product was generated. The reaction solution was concentrated under reduced pressure to remove ethanol, added with water(10 mL) and extracted with 30 mL of ethyl acetate(10 mL*3). The organic phase was dried with anhydrous magnesium sulfate, and filtered. The filtrate was concentrated under reduced pressure to dryness, and purified by column chromatography(silica, 30% tetrahydrofuran in petroleum ether) to obtain yellow solid methyl 7-chloro-1-methyl-4-oxo-5H-imidazolo [1,5-a] quinoxalin-8-carboxylate (5) (500 mg).

**Step 5: methyl 7-chloro-4- ((2,4-dimethoxybenzyl) amino) -1-methylimidazolo [1,5-a]**

**quinoxalin-8-carboxylate (6)** Methyl 7- chloro-1-methyl-4-oxo-5H-imidazolo [1,5-a] quinoxalin-8-carboxylate (5) (480 mg, 1.6 mmol, 1 equiv) was dissolved in acetonitrile(5 mL), and 2,4-dimethoxybenzylamine (341 mg, 2 mmol, 306 μL,1.3 equiv), benzotriazol-1-oxo-tri (dimethylaminophosphate) hexafluorophosphate salt (1 g, 2.4 mmol, 1.2 mL, 1.5 equiv) and 1.8-diazabicyclo [5.4.0] undec-7-ene (1.2 g, 7.9 mmol, 1.2 mL, 5 equiv) were added. The reaction was stirred at room temperature for 16 hours. LC-MS detected that the raw material was completely consumed and the target product was generated. The reaction solution was concentrated under reduced pressure to remove acetonitrile, then added with water(8 mL) and extracted with 30 mL of ethyl acetate(10 mL *3). The organic phase was dried with anhydrous magnesium sulfate, and filtered. The filtrate was concentrated under reduced pressure to dryness, and purified by column chromatography(silica, 30% tetrahydrofuran in petroleum ether) to obtain yellow solid methyl 7-chloro-4- ((2,4-dimethoxybenzyl) amino) -1-methylimidazolo [1,5-a] quinoxalin-8-carboxylate (6) (500 mg, 1.1 mmol, 70% yield).

**Step 6: 7-chloro-4- ((2,4-dimethoxybenzyl)amino) -1-methylimidazolo [1,5-a] quinoxalin-8-carboxylic acid (Intermediate A3)**

Methyl 7-chloro-4- ((2,4-dimethoxybenzyl) amino) -1-methylimidazolo [1,5-a] quinoxalin-8-carboxylate (6) (500 mg, 1.1 mmol, 1 equiv) was dissolved in water(5 mL) and ethanol(5 mL), and sodium hydroxide(132 mg, 3.3 mmol, 3 equiv) was added. The reaction solution was stirred at 50°C for 5 hours. LC-MS detected that the raw material was completely consumed and the target product was generated. The reaction solution was added with 6 mol of HCl(0.5 mL) and concentrated to dryness under reduced pressure. The crude product was directly used in the next step, to obtain white solid 7-chloro-4- ((2,4-dimethoxybenzyl)amino) -1-methylimidazolo [1,5-a] quinoxalin-8-carboxylic acid (Intermediate A3) (350 mg, 820 μmol, 74.6 % yield).

**General method: Synthesis of intermediate A4**

**Synthetic route:**

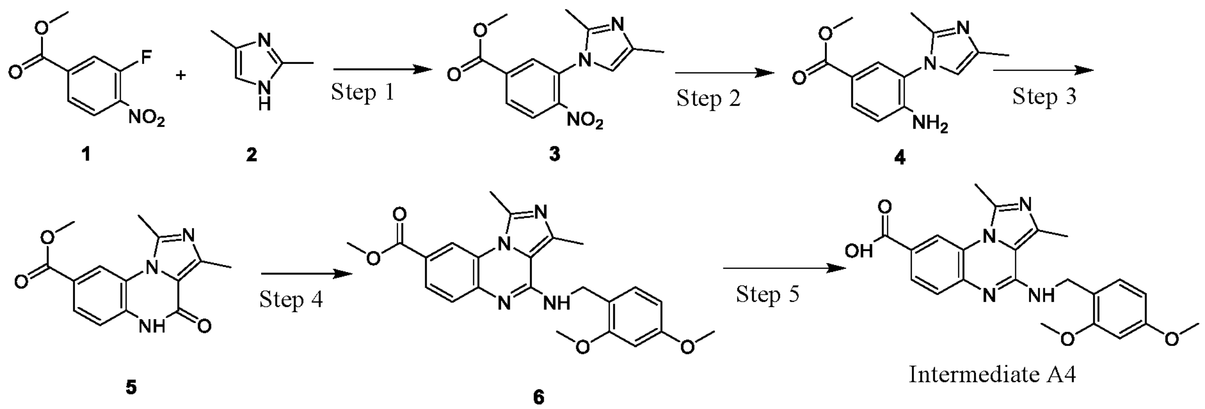


1 2 3 4 5 6 Intermediate A4

**Step 1: methyl 3- (2,4-dimethylimidazol-1-yl) -4-nitrobenzoate**

Methyl 3-fluoro-4-nitrobenzoate (1) (2.00 g, 10.04 mmol, 1 equiv) was added to acetonitrile (40 equiv), and potassium carbonate (4.16 g, 30.13 mmol, 3 equiv) and 2,4-dimethyl-1H-imidazole (2) (965 mg, 10.04 mmol, 1 equiv) were addd. The reaction solution was reacted at 85 °C for 16 hours. LC-MS showed that the raw material was completely consumed and the target product was generated. The reaction solution was concentrated under reduced pressure to dryness, and the residue was diluted with dichloromethane (80 equiv), and filtered. The filtrate was dried with magnesium sulfate, filtered and concentrated under reduced pressure to dryness to obtain yellow solid methyl 3-(2,4-dimethylimidazol-1-yl) -4-nitrobenzoate (3) (2.6 g, 9.45 mmol, 94.05% yield). The crude product was directly used in the next step without further purification.

**Step 2: methyl 4-amino-3- (2,4-dimethylimidazol-1-yl)benzoate**

Methyl 3- (2,4-dimethylimidazol-1-yl) -4-nitrobenzoate (3) (2.5 g, 9.08 mmol, 1 equiv) was dissolved in ethanol (20 equiv), tetrahydrofuran (20 equiv) and water(10 equiv). The mixture was added with iron powder(5.07 g, 90.82 mmol, 10 equiv) and ammonium chloride(2.43 g, 45.41 mmol, 5 equiv) at room temperature. The reaction solution was reacted for 16 hours at 90°C. LC-MS showed that the raw material was completely consumed and the target product was generated. The reaction solution wad filtered and concentrated under reduced pressure to dryness. The residue was diluted with water (50 equiv), and extracted with ethyl acetate (40 equiv * 3). The organic phases were combined and dried with magnesium sulfate, filtered, and concentrated under reduced pressure to dryness to obtain yellow solid methyl 4-amino-3-(2,4-dimethylimidazol-1-yl) benzoate (4) (1.9 g, 6.13 mmol, 67.49% yield, 79.13% purity). The crude product was directly used in the next step without further purification. LCMS: ES19974-375-P1C2 (ESI) m/z=246.1 $[M+1]^+$; RT=0.61 min, purity: 79.13%

**Step 3: methyl 1,3- dimethyl-4-oxo-4,5-dihydroimidazolo [1,5-a] quinoxalin-8-carboxylate**

Methyl 4-amino-3- (2,4-dimethylimidazol-1-yl) benzoate (4) (500 mg, 2.04 mmol, 1 equiv) and 1,1-carbonyl diimidazole (495 mg, 3.06 mmol, 1.5 equiv) were dissolved in 1,2-dichlorobenzene (10 equiv). The reaction solution was reacted at 120°C for 16 hours under $N_2$ protection. LC-MS showed that the raw material was completely consumed and the target product was generated. The reaction solution was sucked to filtrate and the filter cake was pulped with water and then dried under reduced pressure to obtain brown solid methyl 1,3- dimethyl-4-oxo-4,5-dihydroimidazolo [1,5-a] quinoxalin-8-carboxylate (5) (460 mg, 1.62 mmol, 79.48% yield, 95.55% Purity). The crude product was directly used in the next step without further purification.
LCMS ES19974-392-P1B1: (ESI) m/z= 272.0 $[M+1]^+$; RT=0.61 min, purity: 95.55%

**Step 4: methyl 4- ((2,4-dimethoxybenzyl) amino) -1,3- dimethylimidazolo [1,5-a] quinoxalin-8-carboxylate**

Methyl dimethyl-4-oxo-4,5-dihydroimidazolo [1,5-a] quinoxalin-8-carboxylate (5) (700 mg, 2.58 mmol, 1 equiv) was dissolved in acetonitrile (25 equiv), and benzotriazole-1-oxo-tris (dimethylaminophosphate) hexafluorophosphate salt (1.83 g, 4.13 mmol, 1.6 equiv) and 1.8 diazabicyclo [5.4.0] undec-7-ene (1.96 g, 12.90 mmol, 1.94 equiv, 5 equiv) were added. The reaction solution was reacted at room temperature for 0.5 hours and then (2,4-dimethoxyphenyl) methylamine (647.2 mg, 3.87 mmol, 583.1 μL, 1.5 equiv) was added. The reaction solution was reacted at 50°C for 15.5 hours. LC-MS showed that the raw material was completely consumed and the target product was generated. The reaction solution was filtered and the filter cake was dried under reduced pressure to obtain brown solid methyl 4- ((2,4-dimethoxybenzyl) amino)

-1,3- dimethylimidazolo [1,5-a] quinoxalin-8-carboxylate (6) (900 mg, 2.08 mmol, 80.57% yield, 97.13% purity). LCMS ES19974-397-P1B1: (ESI) m/z= 421.1 $[M+1]^+$; RT=0.78 min, purity: 97.13%

**Step 5: 4- ((2,4-dimethoxybenzyl) amino) -1,3- dimethylimidazolo [1,5-a] quinoxalin-8-carboxylic acid**

Methyl 4- ((2,4-dimethoxybenzyl) amino) -1,3- dimethylimidazolo [1,5-a] quinoxalin-8-carboxylate (6) (850 mg, 2.02 mmol, 1 equiv) was dissolved in methanol(10 equiv), tetrahydrofuran(10 equiv) and water (5 mL). Lithium hydroxide(424.2 mg, 10.11 mmol, 5 equiv) was added. The reaction solution was reacted for 16 hours at 50°C. LC-MS showed that the raw material was completely consumed and the target product was generated. The reaction solution was concentrated under reduced pressure to dryness, and the residue was adjusted to pH=6-7 with acetic acid. After filtration, the filter cake was dried under reduced pressure to obtain brown solid 4-((2,4-dimethoxybenzyl) amino) -1,3- dimethylimidazolo [1,5-a] quinoxalin-8-carboxylic acid (Intermediate A4) (800 mg, 1.97 mmol, 97.37% yield). The crude product was directly used in the next step without further purification.

**General method: Intermediate A5 and Intermediate A5b**

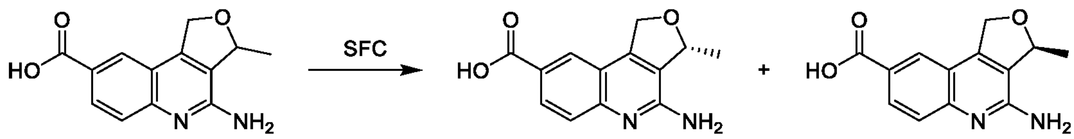


Intermediate A5 Intermediate A5a + Intermediate A5b

Intermediate A5 was a known compound. Intermediate A5 was separated by SFC (conditions: column: DAICEL CHIRALPAK IC (250 mm * 30 mm, 10 um); mobile phase: [MeOH (0.1% IPAm)]; B%: 42% - 42%, 15 min) to successively obtain intermediate A5a (600 mg, 2.46 mmol, 40.00% yield) as white solid and intermediate A5b (600 mg, 2.46 mmol, 40.00% yield) as white solid. (600 mg, 2.46 mmol, 40.00% yield). Note: Chiral centers are not absolute configurations and are specified in the order of SFC separation.

Intermediate A5a: $^1H$ NMR (400 MHz, DMSO-$d_6$) δ 8.07 (d, $J$ = 1.9 Hz, 1H), 7.98 (dd, $J$ = 1.9, 8.8 Hz, 1H), 7.54 (d, $J$ = 8.8 Hz, 1H), 6.74 (s, 2H), 5.47 - 5.35 (m, 2H), 5.34 - 5.25 (m, 1H), 1.41 (d, $J$ = 6.0 Hz, 3H). LC-MS, $[MH]^+$ 245.0
Intermediate A5b: $^1H$ NMR (400 MHz, DMSO-$d_6$) δ 8.05 (d, $J$ = 1.8 Hz, 1H), 7.98 (dd, $J$ = 1.9, 8.8 Hz, 1H), 7.52 (d, $J$ = 8.8 Hz, 1H), 6.68 (s, 2H), 5.46 - 5.35 (m, 2H), 5.33 - 5.26 (m, 1H), 1.41 (d, $J$ = 6.1 Hz, 3H) LC-MS, $[MH]^+$ 245.0

**General method: Synthesis of intermediate A6**

**Synthetic route:**

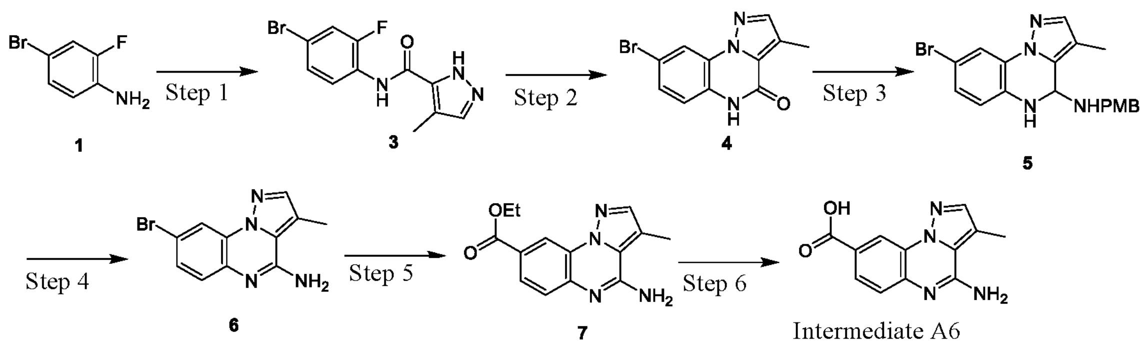


1 3 4 5 6 7 Intermediate A6

**Step 1: N - (4-bromo-2-fluorophenyl) -4-methyl-1H-pyrazol-5-carboxamide(3)**

To a solution of 4-bromo-2-fluoroaniline (5.78g, 30.4mmol, 1 equiv) and 4-methyl-1H-pyrazol-5-carboxylic acid (4.60g, 36.5mmol, 1.2 equiv) in Py (120mL) was added $POCl_3$ (4.66g, 30.4mmol, 2.82mL, 1 equiv) and stirred at 0 °C for 1

hour. LC-MS (ET63399-4-R1A1) showed that the starting material was completely consumed and a main peak with the desired m/z was detected. The reaction mixture was quenched with iced water(200mL) and extracted with ethyl acetate (60 mL×6). The combined organic layer was washed with salt water (30.0mL), dried with $Na_2SO_4$, filtered and concentrated under reduced pressure to obtain the residue. The crude product was ground with ethyl acetate (50.0mL) to obtain a white solid N - (4-bromo-2-fluorophenyl) -4-methyl-1H-pyrazol-5-carboxamide (3) (7.09g, 23.8mmol, 78.2% yield). $^1$H NMR (400 MHz, DMSO-$d_6$) δ 13.26 (br s, 1H) 9.52 (s, 1H) 7.92 (t, *J* = 8.52 Hz, 1H) 7.70 (s, 1H) 7.62 (dd, *J* = 10.31, 1.85 Hz, 1H) 7.41 (br d, *J* = 8.70 Hz, 1H) 2.25 (s, 3H). LC-MS; $[MH]^+$ 298.0

**Step 2: 8-bromo-3-methylpyrazolo [1,5-a] quinoxalin-4(5H)-one (4)**

To a solution of N - (4-bromo-2-fluorophenyl) -4-methyl-1H-pyrazol-5-carboxamide (3) (7.09g, 23.8mmol, 1 equiv) in DMA (70mL) was add NaH (1.43g, 35.7mmol, 60% purity, 1.5 equiv). The mixture was stirred at 120°C for 16 hours. LC-MS showed that the starting material was consumed completely and a main peak had the desired m/z. The reaction mixture was quenched with saturated ammonium chloride (300mL), and the precipitate waas collected and washed with water (50mL), then concentrated under reduced pressure to obtain a white solid of 8-bromo-3-methylpyrazolo [1,5-a] quinoxalin-4(5H)-one (4) (6.50g, crude product). $^1$H NMR (400 MHz, DMSO-$d_6$) δ 8.11 (d, *J* = 2.00 Hz, 1H) 7.91 (s, 1H) 7.52 (dd, *J* = 8.63, 2.00 Hz, 1H) 7.28 (d, *J* = 8.63 Hz, 1H) 2.42 (s, 3H). LC-MS; $[MH]^+$ 278.0

**Step 3: 8-bromo-N - (4-methoxybenzyl) -3-methyl-4,5-dihydropyrazolo [1,5-a] quinoxalin-4-amine (5)**

To a solution of 8-bromo-3-methylpyrazolo [1,5-a] quinoxalin-4(5H)-one (4) (1.00g, 3.60mmol, 1 equiv) in MeCN (10.0mL) was added PMBNH 2 (1.23g, 8.99mmol, 1.16mL, 2.5 equiv), BOP (3.18g, 7.19mmol, 2 equiv), and DBU (2.74g, 18.0mmol, 2.71ml, 5 equiv). The mixture was stirred at 50°C for 16 hours. LC-MS showed that the starting material was consumed completely and a main peak had the desired m/z. The reaction mixture was diluted with saturated ammonium chloride (10.0mL) and ethanol (5.00mL). The precipitate was collected and washed with water (10.0mL), then concentrated under reduced pressure to obtain a yellow solid of 8-bromo-N - (4-methoxybenzyl) -3-methyl-4,5-dihydropyrazolo [1,5-a] quinoxalin-4-amine (5) (1.09g, crude prroduct). $^1$H NMR (400 MHz, DMSO-$d_6$) δ 8.20 (d, *J*= 1.88 Hz, 1H) 7.95 (s, 1H), 7.44-7.48 (m, 2H) 7.38 (d, *J* = 8.63 Hz, 2H) 7.28 (br s, 1H) 6.87 (d, *J* = 8.75 Hz, 2H) 4.70 (d, *J* = 5.88 Hz, 2H) 3.71 (s, 3H) 2.53 (s, 3H). LC-MS; $[MH]^+$ 399.0

**Step 4: 8-bromo-3-methylpyrazolo [1,5-a] quinoxalin-4-amine (6)**

A solution of 8-bromo-N-(4-methoxybenzyl)-3-methyl-4,5-dihydropyrazolo [1,5-a] quinoxalin-4-amine (5) (500mg, 1.26mmol, 1 equiv) in TFA (5mL) was stirred at 60 °C for 12 hours. LC-MS showed that the starting material was consumed completely and a main peak had the desired m/z. The reaction mixture was concentrated under reduced pressure to obtain compound 8-bromo-3-methylpyrazolo [1,5-a] quinoxalin-4-amine (6) (400mg, crude product), as a yellow solid. LC-MS: $[MH]^+$ 277.0

**Step 5: ethyl 4-amino-3-methylpyrazolo [1,5-a] quinoxalin-8-carboxylate (7)**

To an solution of 8-bromo-3-methylpyrazolo [1,5-a] quinoxalin-4-amine (6) (400mg, 1.08mmol, 1 equiv) in EtOH (4mL) was added $Mo(CO)_6$ (143mg, 541umol, 72.9uL, 0.5 equiv), DBU (659mg, 4.33 mmol, 652 uL, 4 equiv), and (t-Bu) $3PBF_4$ (94.2 mg, 325 umol, 0.3 equiv), and $Pd(OAc)_2$ (36.5 mg, 162 umol, 0.15 equiv). The mixture was stirred at 90°C for 12 hours. LC-MS(ET63399-28-R1A1) showed that the starting material was consumed completely and a desired mass was detected. The reaction mixture was diluted with saturated ammonium chloride (10mL) and ethanol (10mL). The precipitate was collected and washed with water (10.0mL), then concentrated under reduced pressure to obtain a yellow solid of ethyl 4-amino-3-methylpyrazolo [1,5-a] quinoxalin-8-carboxylate (7) (250mg, 925umol, 85.4% yield). LC-MS; $[MH]^+$ 271.1

**Step 6: 4-amino-3-methylpyrazolo [1,5-a] quinoxalin-8-carboxylic acid (Intermediate A6)**

To a solution of ethyl 4-amino-3-methylpyrazolo [1,5-a] quinoxalin-8-carboxylate (7) (250mg, 925umol, 1 equiv) in EtOH (3mL) and $H_2O$ (1mL) was added $LiOH \cdot H_2O$ (116mg, 2.77mmol, 3 equiv). The mixture was stirred at 25°C for 12 hours. LC-MS (ET63399-32-R1A1) showed that the starting material was consumed completely and a desired mass was detected. The reaction mixture was concentrated, diluted with water (10mL), and washed with DCM (10.0mL × 3) to remove impurities. The aqueous phase was adjusted to pH=5 with 1M HCl, and the precipitate was collected and purified by preparative HPLC (column: Phenomenex Luna C18 75*30mm*3um; mobile phase: [water (FA)-ACN]; B%: 1%-35%, 8 min, UV 220 nm and 254 nm) to afford a yellow solid of 4-amino-3-methylpyrazolo [1,5-a] quinoxalin-8-carboxylic acid

(Intermediate A6) (60.0 mg, 248 umol, 26.8% yield). $^1$H NMR (400 MHz, DMSO-$d_6$) δ 12.94 (br s, 1H) 8.67 (d, *J* = 1.96 Hz, 1H) 7.96 (s, 1H) 7.89 (dd, *J* = 8.44, 1.96 Hz, 1H) 7.52 (d, *J* = 8.44 Hz, 1H) 7.19 (br s, 2H) 2.49 (br s, 3H). LC-MS, $[MH]^+$ 243.1

**General method: Synthesis of intermediate A7**

**Synthetic route:**

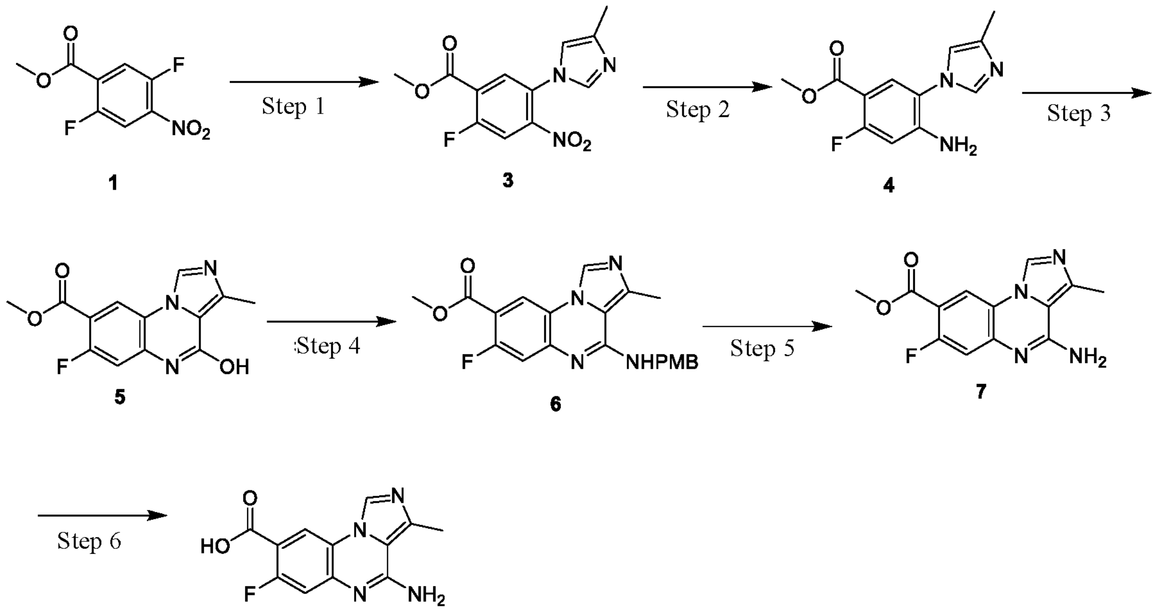


1 3 4 5 6 7

Intermediate A7

**Step 1: Methyl 2-fluoro-5-(4-methyl-1H-imidazol-1-yl)-4-nitrobenzoate (3)**

A mixture of methyl 2,5-difluoro-4-nitrobenzoate (20.0 g, 92.1 mmol, 1 eq.) and 5-Methyl-1H-imidazole (7.56 g, 92.1 mmol, 1 eq.) in DMSO (200mL) was stirred at 50°C for 12 hours. LC-MS showed the remaining starting material and detected the desired mass. The residue was diluted with $H_2O$(400mL) and extracted with EtOAc (200mL×3). The combined organic layer was washed with salt water(200mL) and dried with $Na_2SO_4$, filtered and concentrated under reduced pressure to obtain a residue. The residue was purified by column chromatography ($SiO_2$, petroleum ether/ethyl acetate=20/1 to 0/1) to obtain a yellow solid compound methyl 2-fluoro-5-(4-methyl-1H-imidazol-1-yl)-4-nitrobenzoate (9.30g, 33.3mmol, 34.2% yield). LC-MS (ESI) m/z = 280.1 $[M+H]^+$

**Step 2: methyl 4-amino-2-fluoro-5- (4-methyl-1H-imidazol-1-yl)benzoate (4)**

To a solution of methyl 2-fluoro-5-(4-methyl-1H-imidazol-1-yl)-4-nitrobenzoate (9.30g, 33.3mmol, 1 equiv) and $NH_4Cl$ (26.7g, 499mmol, 15 equiv) in EtOH (90.0mL)/THF (90.0mL)/$H_2O$ (45.0mL) was added Fe powder (7.44 g, 133 mmol, 4 equiv). The mixture was stirred at 80°C for 2 hours. LC-MS showed that the starting material was consumed and the desired mass was detected. The reaction mixture was filtered and the filtrate was diluted with water(300 mL) and extracted with EtOAc(200mL×3). The combined organic layer was washed with salt water (100mL), dried with $Na_2SO_4$, and concentrated under reduced pressure to obtain compound methyl 4-amino-2-fluoro-5- (4-methyl-1H-imidazol-1-yl) benzoate (8.00g, 32.1mmol, 95.2% yield), as a yellow solid. LC-MS (ESI) m/z = 250.0 $[M+H]^+$

**Step 3: Methyl 7-fluoro-4-hydroxy-3-methylimidazolo [1,5-a]quinoxalin-8-carboxylate (5)**

Methyl 4-amino-2-fluoro-5- (4-methyl-1H-imidazol-1-yl) benzoate (500 mg, 2.01 mmol, 1 equiv) and CDI (487 mg, 2.41 mmol, 1.2 equiv) was added to 1,2-dichlorobenzene (20.0 mL) in a microwave tube. The sealed tube was heated under microwave at 150 °C for 3 hours. Eleven parallel reactions were performed. LCMS showed that the starting material was consumed and the desired mass was detected. The reaction mixture was filtered, and the filter cake was dried under reduced pressure to obtain the residue. The crude product was ground with MTBE(20 mL) at 25°C for 30min to obtain a

yellow solid of methyl 7-fluoro-4-hydroxy-3-methylimidazolo [1,5-a]quinoxalin-8-carboxylate (6.00 g, 21.80 mmol, 85.0% yield). LC-MS (ESI) m/z = 276.0 $[M+H]^+$

**Step 4: methyl 7-fluoro-4-((4-methoxybenzyl)amino)-3-methylimidazo[1,5-a]quinoxalin-8-carboxylate (6)**

To a solution of methyl 7-fluoro-4-hydroxy-3-methylimidazolo [1,5-a]quinoxalin-8-carboxylate 5 (6.00g, 21.8mmol, 1 equiv) in MeCN (120mL) was added BOP (19.2g, 43.6mmol, 2 equiv), DBU (16.5g, 109mmol, 16.4mL, 5 equiv), and $PMBNH_2$ (7.48g, 54.5mmol, 7.05ml, 2.5 equiv) . The mixture was stirred at 70°C for 16 hours. LC-MS showed that the starting material was consumed completely and the desired mass was detected. The reaction mixture was diluted with saturated ammonium chloride (20.0mL). The filter cake was washed with water(10.0mL) and concentrated under reduced pressure to obtain yellow solid methyl 7-fluoro-4-((4-methoxybenzyl)amino)-3-methylimidazo[1,5-a]quinoxalin-8-carboxylate 6 (5.00 g, 12.6 mmol, 50.0% yield). LC-MS (ESI) m/z = 395.1 $[M+H]^+$

**Step 5: methyl 4-amino-7-fluoro-3-methylimidazolo [1,5-a] quinoxalin-8-carboxylate (6)**

A solution of methyl 7-fluoro-4-((4-methoxybenzyl)amino)-3-methylimidazo[1,5-a]quinoxalin-8-carboxylate 6 in TFA(20mL) was stirred at 75°C for 16 hours. LC-MS showed that the starting material was consumed and the desired mass was detected. The reaction mixture was concentrated under reduced pressure to obtain a yellow solid of methyl to methyl 4-amino-7-fluoro-3-methylimidazolo [1,5-a] quinoxalin-8-carboxylate 7 (1.39 g, crude product). LC-MS (ESI) m/z = 275.0 $[M+H]^+$

**Step 6: 4-amino-7-fluoro-3-methylimidazolo [1,5-a] quinoxalin-8-carboxylic acid (Intermediate A7)**

To a solution of methyl 4-amino-7-fluoro-3-methylimidazolo [1,5-a] quinoxalin-8-carboxylate 7 (1.39g, 5.07mmol, 1 equiv) in EtOH (15mL)/$H_2O$ (5.00mL) was added LiOH (638mg, 15.2mmol, 3 equiv). The mixture was stirred at 25°C for 12 hours. LC-MS showed that the starting material was consumed and a main peak with the desired mass was detected. The reaction mixture was diluted with $H_2O$ (20.0mL) and extracted with (DCM 10.0mL $\times$ 2) to remove impurities. The aqueous layer was adjusted to pH=6 with 2M HCl and the precipitate was collected and dried under reduced pressure to obtain 4-amino-7-fluoro-3-methylimidazolo [1,5-a] quinoxalin-8-carboxylic acid (Intermediate A7) (1.30 g, 5.00 mmol, 98.5% yield) as a white solid. $^1H$ NMR (400MHz, DMSO-$d_6$) $\delta$ 13.15 (br s, 1H), 9.12 (s, 1H), 8.56 (br d, $J$ = 6.4 Hz, 1H), 7.35 (br s, 2H), 7.13 (br d, $J$ = 12.1 Hz, 1H), 2.62 (s, 3H). LC-MS (ESI) m/z = 261.0 $[M+H]^+$

**General method: Synthesis of intermediate B1**

**Synthetic route:**

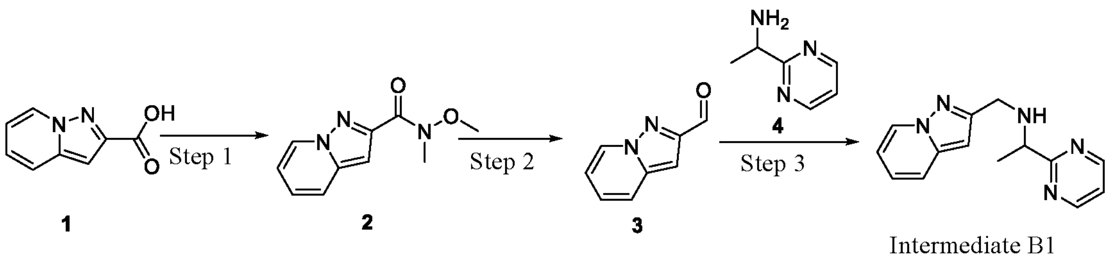


Intermediate B1

**Step 1: N-methoxy-N-methylpyrazolo[1,5-a]pyridine-2-carboxamide (1)**

To a solution of pyrazolo [1,5-a] pyridin-2-carboxylic acid (2.0 g, 12.3 mmol) and HATU (7.0 g, 18.5 mmol) in DMF (100 mL) was added Et3N (6.2 g, 61.7 mmol) and methoxy (methyl) amine (1.9 g, 30.83 mmol) at 25 °C. The mixture was then stirred at 25°C for 12 hours. The reaction was quenched with water(100 mL) and extracted with EA(100mL$\times$3). The organic solution was washed with salt water(100 mL). The organic phase was dried with $Na_2SO_4$ and then filtered. The filtrate was concentrated under reduced pressure. The residue was purified by silica gel chromatography and eluted from 0% to 10% with a solution of MeOH in DCM within 20 minutes to obtain N-methoxy-N-methylpyrazolo[1,5-a]pyridin-2-carboxamide (2.01 g, 79% yield) as a yellow solid. LC-MS: Rt = 1.049 min, (ESI) m/z. $[M+H]^+$ 206.1; $C_{10}H_{11}N_3O_2$.

**Step 2: Pyrazolo[1,5-a]pyridin-2-carbaldehyde (3)**

To a solution of N-methoxy-N-methylpyrazolo[1,5-a]pyridin-2-carboxamide (1.5 g, 7.31 mmol) in THF (20 mL) was added $LiAlH_4$ (439 mg, 10.96 mmol)at -60 °C. The mixture was then stirred at -60°C for 2 hours. The reaction was quenched with water(40mL) and then filtered. The filtrate was concentrated under reduced pressure. The residue was purified by silica gel chromatography and eluted from 0% to 10% with a solution of MeOH in DCM within 20 minutes to obtain yellow oily pyrazolo[1,5-a]pyridin-2-carbaldehyde (450 mg, 42% yield). LC-MS: Rt = 1.071 min, (ESI) m/z. $[M+H]^+$ 147.1; $C_8H_6N_2O$

**Step 3: N-(pyrazolo[1,5-a]pyridin-2-ylmethyl) -1- (pyrimidin-2-yl) ethan-1-amine(Intermediate B1)**

To a solution of pyrazolo [1,5-a] pyridin-2-carbaldehyde (100mg, 0.68mmol) in MeOH (5mL) was added 1-(pyrimidin-2-yl) ethylamine (126mg, 1.03mmol) and NaBH3CN (86mg, 1.37mmol) and reacted at 25 °C. The mixture was then stirred at 25°C for 2 hours. The reaction was quenched with water(1mL) and concentrated under reduced pressure. The residue was purified by silica gel chromatography and eluted from 0% to 5% with a solution of MeOH in DCM within 20 minutes to obtain N-(pyrazolo[1,5-a]pyridin-2-ylmethyl) -1- (pyrimidin-2-yl) ethylamine (450 mg, 23% yield) as yellow oil. LC-MS: Rt = 0.521 min, (ESI) m/z. $[M+H]^+$ 254.2; $C_{14}H_{15}N_5$

**General method: Synthesis of intermediate B2**

**Synthetic route:**

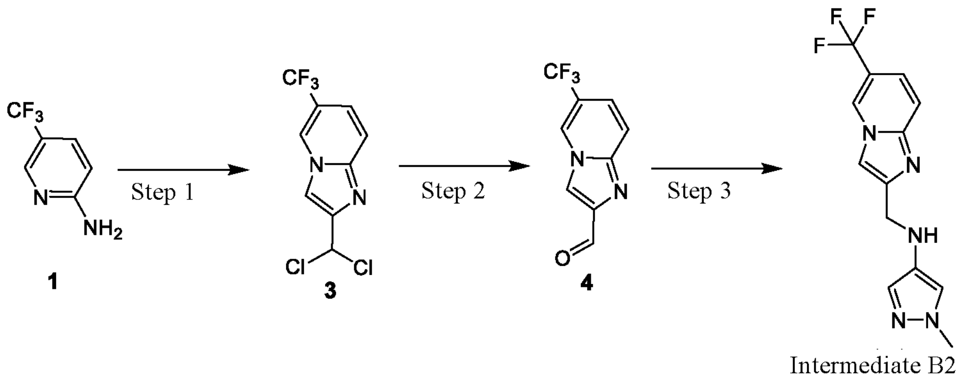


**Step 1: 2-Dichloromethyl-6-trifluoromethylimidazolo[1,2-a]pyridine(3)**

A mixture of 5- (trifluoromethyl) pyridin-2-amine (1) (30 g, 185 mmol, 1 equiv), chlorobenzene (450 mL), and 1,1,3-trichloro-2-acetone (45 g, 277 mmol, 1.5 equiv) was reacted at 135 °C for 4 hours. LC-MS detected that the target product was generated. The reaction solution was adjusted to pH about 8 with sodium carbonate, and extracted with ethyl acetate (500 mL * 3). The combined organic phase was dried with magnesium sulfate and filtered. The filtrate was concentrated under reduced pressure to dryness, and purified by column chromatography (silica, 15% ethyl acetate in petroleum ether) to obtain a yellow solid of 2-dichloromethyl-6-trifluoromethylimidazolo [1,2-a] pyridine (3) (30 g, 111 mmol, 60% yield). H NMR: ES19506-784-P1A,$^1$H NMR (400 MHz, DMSO-$d_6$) δ 9.24 (s, 1H), 8.27 (s, 1H), 7.79 (d, J=9.68 Hz, 1H), 7.65 (s, 1H), 7.56 (dd, J=1.65, 9.57 Hz, 1H).

**Step 2: 6-(trifluoromethyl)imidazolo[1,2-a]pyridin-2-carbaldehyde (4)**

2-dichloromethyl-6-trifluoromethylimidazolo [1,2-a] pyridine (3) (30 g, 111 mmol, 1 equiv), water (600 mL), and calcium carbonate (33 g, 334 mmol, 3 equiv) was heated to 100 °C to react for 2 hours. LC-MS detected that the target product was generated. The reaction solution was added with diatomite and ethyl acetate (600ml) and stirred at room temperature for 30 minutes, filtered, and extracted with ethyl acetate (600ml * 2). The combined organic phase was dried with magnesium sulfate, and filtered. The filtrate was concentrated under reduced pressure to dryness to obtain a brown solid of 6-(trifluoromethyl)imidazolo[1,2-a]pyridin-2-carbaldehyde (4) (35 g). The crude product was directly used in the next step without further purification. H NMR: ES19506-789-P1A1, $^1$H NMR (400 MHz, CHLOROFORM-d) δ: 10.09-10.29 (m, 1H), 8.59 (s, 1H), 8.27 (s, 1H), 7.82 (br d, J=9.46 Hz, 1H), 7.44 (br d, J=9.02 Hz, 1H).

**Step 3: 1-methyl-N-((6-(trifluoromethyl)imidazolo[1,2-a]pyridin-2-yl)methyl)-1H-pyrazol-4-amine (Intermediate B2)**

To a solution of 6- (trifluoromethyl) imidazolo [1, 2-a] pyridin-2-carbaldehyde (4) (100mg, 467umol, 1 equiv) in DCM (2.00mL) was added KOAc (91.7mg, 934umol, 2 equiv) and 1-methyl-1H-pyrazol-4-amine (45.4mg, 467umol, 1 equiv) at -5 °C, and the reaction mixture was stirred at -5 °C for 1 hour, then added with $NaBH(OAc)_3$ (198mg, 934umol, 2 equiv) and stirred at -5 °C for another 3 hours. LCMS (ET63219-45-P1A1) showed that Cpd.4 was consumed completely and several new peaks were displayed. The reaction mixture was diluted with saturated aqueous $Na_2CO_3$ solution (3.00 mL) and extracted with dichloromethane (2.00mL×4). The combined organic layer was dried with $Na_2SO_4$ and concentrated under reduced pressure to obtain a residue. The residue was purified by preparative TLC (ethyl acetate/methanol=8/1) to obtain a yellow solid of 1-methyl-N-((6-(trifluoromethyl)imidazolo[1,2-a]pyridin-2-yl)methyl)-1H-pyrazol-4-amine

(Intermediate B2) (80.0mg, 271umol, 58.0% yield). $^1H$ NMR (400 MHz, $CDCl_3$) δ 8.47 (s, 1H), 7.69 (d, *J* = 9.5 Hz, 1H), 7.64 (s, 1H), 7.35 (d, *J* = 1.5, 9.5 Hz, 1H), 7.30 (s, 2H), 6.97 (s, 1H), 4.39 (s, 2H), 3.81 (s, 3H); LC-MS, $[MH]^+$ 217.0.

**General method: Synthesis of intermediate B3**

**Synthetic route:**

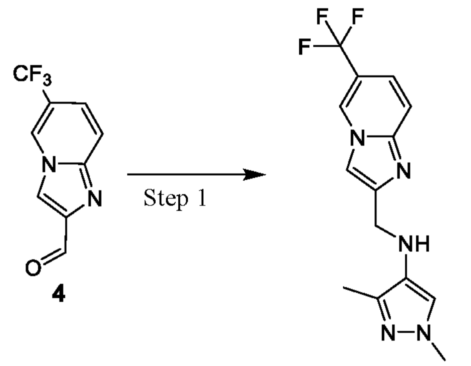


4

Intermediate B3

**Step 1: 1,3-Dimethyl-N-((6-(trifluoromethyl)imidazolo[1,2-a]pyridin-2-yl)methyl)-1H-pyrazol-4-amine (Intermediate B3)**

6- (trifluoromethyl) imidazolo [1,2-a] pyridin-2-carbaldehyde (4) (10 g, 46 mmol, 1 equiv) and 1,3-dimethylpyrazol-4-amine (6 g, 56 mmol, 1.2 equiv) were dissolved in dichloromethane (150 mL), and then added with acetic acid (3 g, 56 mmol, 3 mL, 1.2 equiv). The reaction solution was reacted at 25 °C for 1 hour, then added with sodium triacetoxyborohydride (25 g, 117 mmol, 2.5 equiv), and reacted at 25 °C for 3 hours. LCMS detection showed that the target product was generated. The reaction solution was quenched with 200 mL of sodium bicarbonate and extracted with ethyl acetate (150 mL * 2). The combined organic phase was washed with saturated salt water (400ml), dried with anhydrous magnesium sulfate, and filtered. The filtrate was concentrated under reduced pressure to dryness, and purified by column chromatography(silica, 35% ethyl acetate: ethanol (3:1) in petroleum ether) to obtain a brown solid of 1,3-Dimethyl-N-((6-(trifluoromethyl)imidazolo[1,2-a]pyridin-2-yl)methyl)-1H-pyrazol-4-amine (Intermediate B3) (14 g, 45 mmol, 97% yield). H NMR: ES19506-795-P1A, $^1H$ NMR (400 MHz, DMSO-$d_6$) δ 9.19 (s, 1H), 7.92 (s, 1H), 7.67 (d, J=9.46 Hz, 1H), 7.41 (dd, J=1.76, 9.46 Hz, 1H), 6.93 (s, 1H), 4.56 (br s, 1H), 4.17 (br d, J=3.96 Hz, 2H), 3.52-3.61 (m, 3H), 2.04 (s, 3H).LCMS: ES19506-795-P1B, (ESI) m/z=310.3 , RT= 0.64 min.

**General method: Synthesis of intermediate B4**

**Synthetic route:**

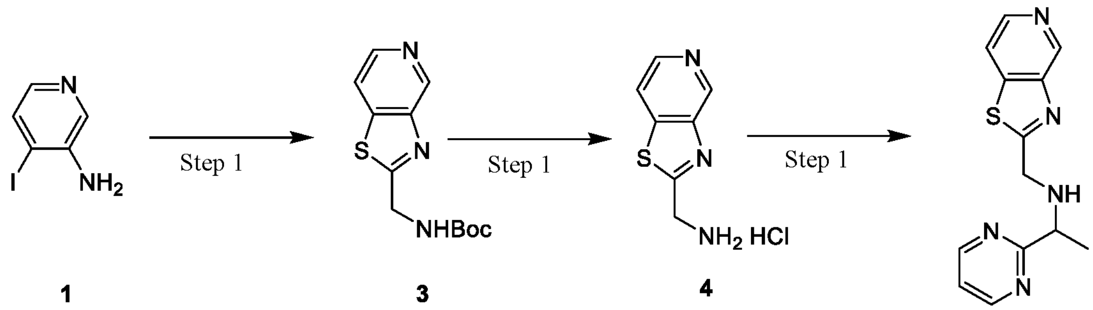


1 3 4 Intermediate B4

**Step 1: tert-butyl (thiazolo[4,5-c]pyridin-2-ylmethyl)carbamate (3)**

To a solution of 4-iodopyridin-3-amine (20.0g, 90.9mmol, 1 equiv) and tert-Butyl (2-amino-2-thioethyl)carbamate(20.7g, 109mmol, 1.2 equiv) in MeCN (200mL) was added $Pd_2(dba)_3$ (4.16g, 4.55mmol, 0.05 equiv), DPPF (10.1g, 18.2mmol, 0.2 equiv), and CaO (10.2g, 182mmol, 3.09mL, 2 equiv). The mxiture was stirred at 80 °C for 16 hours under $N_2$. LC-MS showed that the starting material was consumed completely and the desired mass was detected. The reaction mixture was filtered and the filtrate was concentrated under reduced pressure to obtain a residue. The residue was purified by column chromatography (petroleum ether/ethyl acetate=30/1 to 1/1) to obtain a brown solid of tert-butyl (thiazolo[4,5-c] pyridin-2-ylmethyl)carbamate (3) (17g, 64.0mmol, 70.5% yield). $^1H$ NMR (400 MHz, CHLOROFORM-d) δ 9.28 (s, 1H), 8.53 (d, *J* = 5.4 Hz, 1H), 7.84 (d, *J* = 5.4 Hz, 1H), 5.41 (br s, 1H), 4.77 (br d, *J* = 5.8 Hz, 2H), 1.50 (s, 9H); LC-MS, $[MH]^+$ 266.1

**Step 2: Thiazolo[4,5-c]pyridin-2-ylmethylamine hydrochloride(4)**

To a solution of tert-butyl (thiazolo[4,5-c]pyridin-2-ylmethyl)carbamate (3) (3g, 11.3mmol, 1 equiv) in DCM (15mL) was added HCl/EtOAc (4M, 15mL). The mixture was stirred at 25°C for 16 hours. LC-MS (ET63218-11-P1A2) showed that the starting material was consumed and the desired mass was detected. The reaction mixture was concentrated under reduced pressure to obtain thiazolo[4,5-c]pyridin-2-ylmethylamine hydrochloride(4) (2g, crude product, HCl), as a light yellow solid. LC-MS, $[MH]^+$ 166.1

**Step 3: 1- (pyrimidin-2-yl) -N-(thiazolo [4,5-c] pyridin-2-ylmethyl) ethan-1-amine (Intermediate B4)**

To a solution of thiazolo[4,5-c]pyridin-2-ylmethylamine hydrochloride(4) (2g, 9.92mmol, 1 equiv) and 1- (pyrimidin-2-yl) ethan-1-one (1.21g, 9.92mmol, 1 equiv) in DCM (20mL) was added KOAc (1.17g, 11.9mmol, 1.2 equiv). The mixture was stirred at 25 °C for 0.5 hours, and $NaBH(OAc)_3$ (2.73g, 12.9mmol, 1.3 equiv) was added to the above-mentioned mixture at 25 °C, and the mixture was stirred at 25 °C for 2 hours. LC-MS showed that the starting material was consumed and the desired mass was detected. The mixture was adjusted to pH=8-9 using saturated $NaHCO_3$, and then extracted with DCM (20mL × 3). The organic phase was concentrated under reduced pressure to obtain a residue. The residue was purified by column chromatography ($SiO_2$, dichloromethane/methanol=50/1 to 10/1) to obtain a brown oil of 1-(pyrimidin-2-yl) -N-(thiazolo [4,5-c] pyridin-2-ylmethyl) ethan-1-amine (Intermediate B4) (320mg, 1.18mmol, 11.9% yield). $^1H$ NMR (400 MHz, CHLOROFORM-d) δ = 9.22 (s, 1H), 8.74 (d, *J* = 4.9 Hz, 2H), 8.50 (d, *J* = 5.4 Hz, 1H), 7.85 (d, *J* = 5.4 Hz, 1H), 7.22 (t, *J* = 4.9 Hz, 1H), 4.27 (d, *J* = 16.4 Hz, 1H), 4.16 - 4.04 (m, 2H), 1.54 (d, *J* = 6.9 Hz, 3H); LC-MS, $[MH]^+$ 272.0

**General method: Synthesis of intermediate B5**

**Synthetic route:**

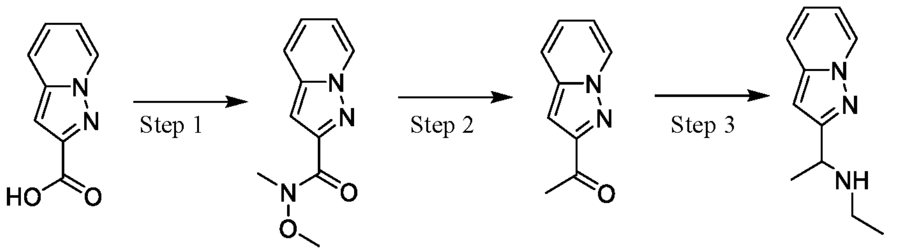


1 2 3 Intermediate B5

**Step 1: Pyrazolo [1,5-a] pyridin-2-carboxylic acid (2)**

To a solution of pyrazolo [1,5-a] pyridin-2-carboxylic acid (10.0g, 61.67 mmol, 1 equiv), HATU (28.14 g, 74.01 mmol, 1.2 equiv), and DIEA (31.88 g, 246.69 mmol, 4 equiv) in DCM (500mL) was added N, O-dimethylhydroxylamine hydrochloride (12.03 g, 123.35 mmol, 2 equiv) . The mixture was stirred at 25°C for 16 hours. LC-MS (ET63565-18-P1A) showed that the starting material was consumed completely and the product was detected. The reaction mixture was concentrated under reduced pressure to obtain a residue. The residue was purified by column chromatography (petroleum ether/ethyl acetate=1/1 to 0/1) to obtain a white solid of compound pyrazolo [1,5-a] pyridin-2-carboxylic acid (2) (12.1g, 58.9mmol, yield 95.6%). $^1$H NMR (400 MHz, CHLOROFORM-d) δ 8.49 *(d, J*= 7.1 Hz, 1H), 7.57 *(d, J*= 8.9 Hz, 1H), 7.15 (dd, *J* = 7.3, 8.4 Hz, 1H), 7.00 (s, 1H), 6.85 (t, *J* = 6.9 Hz, 1H), 3.79 (s, 3H), 3.49 (s, 3H); LC-MS, $[MH]^+$ 206.22.

**Step 2: 1- (pyrazolo [1,5-a] pyridin-2-yl) ethan-1-one (3)**

To a solution of pyrazolo [1,5-a] pyridin-2-carboxylic acid (2) (2g, 9.75mmol, 1 equiv) in THF (20.0mL) was added MeLi (11.70mL, 1.2 equiv) at -60 °C, and then stirred at 25 °C for 16 hoursunder $N_2$ hr. LC-MS (ET63565-13-P1A1) showed that the starting material was consumed completely and the product was detected. The residue was purified by column chromatography (petroleum ether/ethyl acetate=50/1 to 3/1) to obtain a white solid of 1- (pyrazolo [1,5-a] pyridin-2-yl) ethan-1-one (3) (277mg, 1.73mmol, 17.7% yield). LC-MS, $[MH]^+$ 161.1.

**Step 3: N-ethyl-1- (pyrazolo [1,5-a] pyridin-2-yl)ethan-1-amine (Intermediate B5)**

A mixture of 1- (pyrazolo [1,5-a] pyridin-2-yl) ethan-1-one (3) (0.1 g, 624.33 umol, 1 equiv) and ethylamine (112.58 mg, 2.50 mmol, 163.39 uL, 4 equiv) in DCM (2 mL) was stirred at -60 ° C for 0.5 hours, then added with $NaBH_4$ (264.64 mg, 1.25 mmol, 2 equiv) at 0 °C and stirred at 25 °C for 16 hours under $N_2$ atmosphere. LC-MS (ET63565-9-P1A2) showed that the starting material was consumed completely and the product was detected. The reaction mixture was quenched with $H_2O$ (5mL) and diluted with DCM (5mL × 3). The combined organic layer was concentrated under reduced pressure to obtain the residue. The residue was purified by preparative TLC (DCM: MeOH=10:1) to obtain a white solid of N-ethyl-1- (pyrazolo [1,5-a] pyridin-2-yl) ethan-1-amine (Intermediate B5) (50mg, 264umol, 42.3% yield). $^1$H NMR (400 MHz, CHLOROFORM-d) δ 8.39 (br d, *J* = 6.9 Hz, 1H), 7.46 (br d, *J* = 8.5 Hz, 1H), 7.12-7.03 (m, 1H), 6.70 (br t, *J* = 6.8 Hz, 1H), 6.44 (s, 1H), 4.13 (br d, *J* = 6.8 Hz, 1H), 3.32-3.21 (m, 1H), 2.77-2.56 (m, 2H), 1.53 (d, *J* = 6.6 Hz, 4H), 1.17-1.14 (m, 3H): LC-MS, $[MH]^+$ 190.1.

**General method: Synthesis of intermediate B6**

**Synthetic route:**

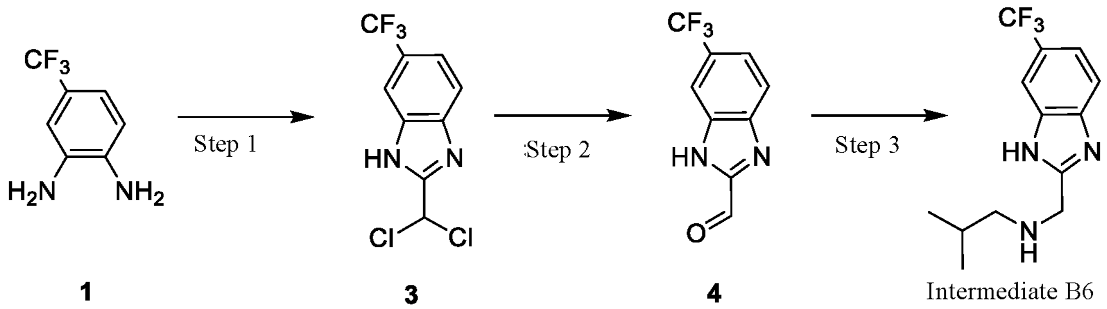


**Step 1: 2-(Dichloromethyl)-6-(trifluoromethyl)-1H-benzo[d]imidazole(3)**

A mixture of 4- (trifluoromethyl) benzene-1,2-diamine (5 g, 28.3 mmol, 1 equiv) and 2,2-dichloroacetic acid (7.32 g, 56.7 mmol, 4.66 mL, 2 equiv) in HCl (125 mL) (4 M) was stirred at 100 °C for 10 minutes. LC-MS (ET60224-68-P1A) showed that Cpd.1was consumed and the desired mass was detected. The reaction mixture was filtered and the filter cake was washed with water. The combined filtrate was extracted with DCM (20ml * 3). The combined organic layer was washed with salt water(100mL) and dried with $MgSO_4$, filtered and concentrated under reduced pressure to obtain a residue. The residue was purified by column chromatography (petroleum ether/ethyl acetate=20/1 to 5/1) to obtain a yellow oil of compound 2-(dichloromethyl) -6- (trifluoromethyl) -1H-benzo [d] imidazole (3) (4.4g, 16.3mmol, yield 57.6%). $^1H$ NMR (400 MHz, CHLOROFORM-d) δ 8.01 (s, 1H), 7.79 (d, *J* = 8.6 Hz, 1H), 7.66 (d, *J* = 8.2 Hz, 1H), 7.26 (s, 1H); LCMS: $[M+H]^+$ 268.9

**Step 2: 6- (trifluoromethyl) -1H-benzo [d] imidazol-2-carbaldehyde (4)**

To a suspension of 2- (dichloromethyl) -6- (trifluoromethyl) -1H-benzo [d] imidazole (3) (1g, 3.72mmol, 1 equiv) in $H_2O$ (20mL) was added $CaCO_3$ (1.12g, 11.1mmol, 3 equiv). The mixture was stirred at 100°C for 8 hours. LC-MS (ET60224-74-P1B) showed that Cpd.3 was consumed and the desired mass was detected. The reaction mixture was diluted with water(50 mL) and extracted with ethyl acetate(30mL×3). The combined organic layer was concentrated under reduced pressure to obtain a white solid of 6-(trifluoromethyl) -1H-benzo [d] imidazol-2-carbaldehyde (4) (310mg, 1.45mmol, 38.9% yield). $^1H$ NMR (400 MHz, DMSO-d6) δ 14.28-13.59 (m, 1H), 10.02 (s, 1H), 8.40-8.30 (m, 2H), 8.23-8.05 (m, 3H), 8.00 (dd, *J*= 5.4, 8.5 Hz, 1H), 7.89 (br s, 1H), 7.83-7.61 (m, 3H), 7.34 (br d, *J* = 7.9 Hz, 1H), 7.27 *(d, J*= 7.6 Hz, 1H), LCMS: $[M+H]^+$ 215.2

**Step 3: 2-methyl-N- ((6- (trifluoromethyl) -1H-Benzo [d] imidazol-2-yl) methyl) propan-1-amine (Intermediate B6)**

To a solution of 6- (trifluoromethyl) -1H-benzo [d] imidazol-2-carbaldehyde (4) (0.31g, 1.45mmol, 1 equiv) and 2-methylpropan-1-amine (105mg, 1.45mmol, 143uL, 1 equiv) in DCM (6.2mL) was added KOAc (170mg, 1.74mmol, 1.2 equiv) at 25°C. The mixture was stirred at 25 °C for 0.5 hours, and then $NaBH(OAc)_3$ (398mg, 1.88mmol, 1.3 equiv) was added to the above-mentioned mixture at 25, and the mixture was stirred at 25 °C for 15.5 hours. LC-MS (ET60224-77-P1A) showed that Cpd.4 was consumed and the desired mass was detected. The reaction mixture was diluted with water(10 mL) and extracted with DCM (2mL×3). The combined organic layers were washed with salt water(10mL) and dried with $MgSO_4$, filtered and concentrated under reduced pressure to obtain a residue. The residue was purified by preparative TLC (petroleum ether/ethyl acetate=0/1) to obtain a colorless oil of 2-methyl-N- ((6- (trifluoromethyl) -1H-Benzo [d] imidazol-2-yl) methyl) propan-1-amine (Intermediate B6) (100mg, 368umol, 25.4% yield, 100% purity). $^1H$ NMR (400 MHz, CHLOROFORM-d) 7.80 (s, 1H), 7.56 (d, *J*= 8.4 Hz, 1H), 7.43 (d, *J* = 8.5 Hz, 1H), 4.07 (s, 2H), 2.45 *(d, J*= 6.8 Hz, 2H), 1.82-1.68 (m, 1H), 0.89 (d, *J* = 6.6 Hz, 6H); LCMS: $[M+H]^+$ 272.0

**General method: Synthesis of intermediate B7**

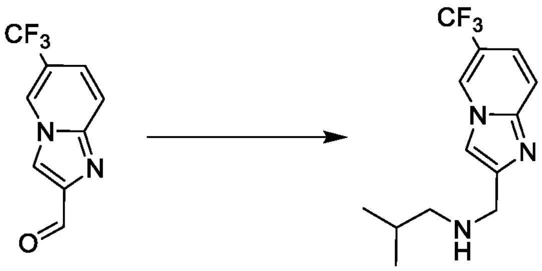

**4**

Intermediate B7

**Step: 2-Methyl-N-((6-(trifluoromethyl)imidazolo [1,2-a]pyridin-2-yl)methyl)propan-1-amine (Intermediate B7)**

To a solution of 2-methylpropan-1-amine (68.3mg, 933umol, 92.8uL, 1 equiv) and 6- (trifluoromethyl) imidazolo [1,2-a] pyridin-2-carbaldehyde (0.2g, 933umol, 1 equiv) in DCM (4mL) was added KOAc (109mg, 1.12mmol, 1.2 equiv) at 25 °C. The mixture was stirred at 25 °C for 0.5 hours, and then NaBH $(OAc)_3$ (257mg, 1.21mmol, 1.3 equiv) was added to the above-mentioned mixture at 25 °C, and the mixture was stirred at 25 ° C for 15.5 hours. LC-MS (ET60224-75-P1A) showed that Cpd.4 was consumed and the desired mass was detected. The reaction mixture was diluted with water(10 mL) and extracted with DCM (2mL×3) to remove impurities. The aqueous layer was alkalized with saturated $Na_2CO_3$ to pH=8, and then extracted with DCM (10mL * 3). The combined organic layer was dried with $MgSO_4$, filtered and concentrated under reduced pressure to obtain a colorless oil of Cpd. A6 (108mg, 398umol, 42.6% yield, 100% purity). $^1$H NMR (400 MHz, CHLOROFORM-d) δ 8.49 (s, 1H), 7.70-7.62 (m, 2H), 7.33 (br d, *J* = 9.2 Hz, 1H), 4.01 (s, 2H), 2.54 (d, *J* = 6.7 Hz, 2H), 1.84 (quind, *J* = 6.6, 13.3 Hz, 1H), 0.97 (d, *J* = 6.6 Hz, 6H); LC-MS, $[MH]^+$ 272.0.

**Synthesis of Example 1**

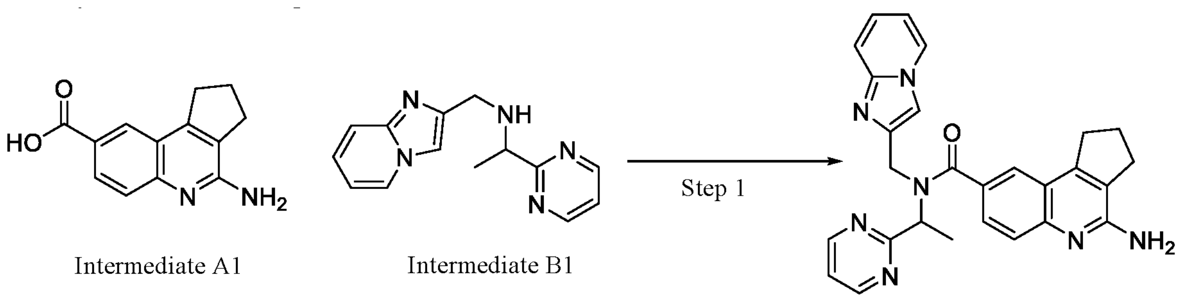


Intermediate A1

Intermediate B1

Example 1

**Step 1: 4-Amino-N-(pyrazolo[1,5-a]pyridin-2-ylmethyl)-N-(1-(pyrimidin-2-yl)ethyl)-2,3-dihydro-1H-cycl opentadieno[c]quinolin-8-carboxamide (Example 1)**

A solution of 4-amino-2,3-dihydro-1H-cyclopentadieno[c]quinolin-8-carboxylic acid (80 mg, 0.35 mmol) in $SOCl_2$ (2 mL) was stirred at 70 °C for 12 hours. The solvent was concentrated to dryness and used directly in the next step. In a solution of N - (pyrazolo [1,5-a] pyridin-2-ylmethyl) -1-(pyrimidin-2-yl) ethan-1-amine (35 mg, 0.14 mmol) and Et3N (140 mg, 1.38), 4-amino-2,3-dihydro-1H-cyclopentadieno[c]quinolin-8-carbonyl chloride (80 mg, 0.32 mmol) was added to THF (15 mL) at 0 C. The mixture was then stirred at 25°C for 12 hours. The reaction was quenched with $NaHCO_3$(aq.) (20 mL), and then extracted with EA (20 mL × 3). The organic solution was washed with salt water(20 mL) and dried with $Na_2SO_4$ then filtered. The filtrate was concentrated under reduced pressure. The residue was purified by silica gel chromatography and eluted from 0% to 5% with a solution of MeOH in DCM within 20 minutes to obtain 4-amino-N-(pyrazolo [1,5-a] pyridin-2-ylmethyl)-N-(1-(pyrimidin-2-yl) ethyl) -2,3-dihydro-1H-cyclopentadieno [c] quinolin-8-carboxamide (2.3 mg, 4% yield) as a yellow solid (Example 1). $^1$H NMR (400 MHz, MeOD) δ: 8.75 (s, 2H), 8.40 (s, 2H), 7.87 - 7.61 (m, 2H), 7.55 (d, J = 8.8 Hz, 1H), 7.32 (s, 1H), 7.18 (s, 1H), 6.83 (s, 1H), 6.51 (s, 1H), 5.68 (s, 1H), 5.40 (s, 1H), 4.73 - 4.53 (m, 2H), 3.24 (s, 2H), 2.94 (s, 2H), 2.41 - 2.13 (m, 2H), 1.84 - 1.65 (m, 3H). LC-MS: Rt =0.981 min, (ESI) m/z. 464.2 $[M+H]^+$. $C_{27}H_{25}N_7O$

**Synthesis of Example 3**

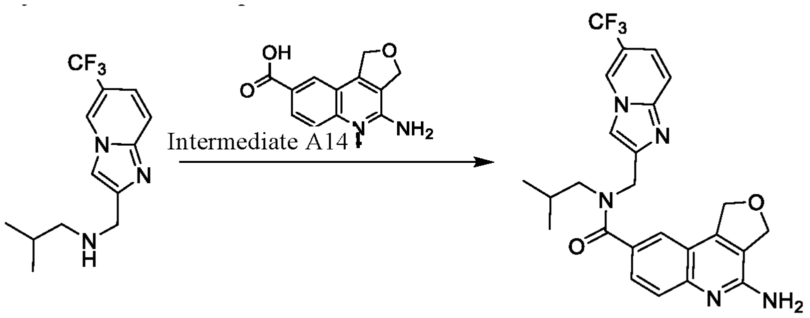


Intermediate B7

Example 3

**Step: 4-Amino-N-isobutyl-N- ((6- (trifluoromethyl) imidazolo [1,2-a] pyridin-2-yl) methyl) -1,3-dihydrofurano [3,4-c] quinolin-8-carboxamide (Example 3)**

To a mixture of 4-amino-1,3-dihydrofurano [3,4-c] quinolin-8-carboxylic acid (intermediate A14) (30 mg, 130 umol, 1 equiv) and 2-methyl-N-((6-(trifluoromethyl) imidazolo [1,2-a] pyridin-2-yl) methyl) propan-1-amine (intermediate B7) (35.3 mg, 130 umol, 1 equiv) in DMF (2 mL) was added TCFH (43.8 mg, 156 umol, 1.2 equiv) and NMI (32.1mg, 390 umol, 31.1 uL, 3 equiv). The mixture was stirred at 20°C for 16 hours under $N_2$ atmosphere. LC-MS showed that most of the starting material was consumed and the desired m/z was detected. The reaction mixture was concentrated under reduced pressure, and the residue was purified by preparative HPLC (column: Waters Xbridge BEH C18 100 * 30mm * 10um; mobile phase: [Water (NH4HCO3) - ACN]; B%: 30% -60%, 8 minutes, UV 220&254 nm) to obtain a white solid of 4-amino-N-isobutyl-N-((6- (trifluoromethyl) imidazolo [1,2-a] pyridin-2-yl) methyl)-1,3-dihydrofurano [3,4-c] quinolin-8-carboxamide (Example 3) (37 mg, 76.5 umol, 58.7% yield, 100% purity). $^1$H NMR (400 MHz, DMSO-$d_6$) δ = 9.20 (br s, 1H), 7.96 (br s, 1H), 7.91 - 7.63 (m, 2H), 7.48 (br d, $J$ = 9.4 Hz, 2H), 7.56 (br d, $J$ = 6.4 Hz, 1H), 6.66 (br s, 2H), 5.27 (br d, $J$ = 0.9 Hz, 2H), 5.00 (br s, 2H), 4.86 - 4.51 (m, 2H), 3.30 - 3.19 (m, 2H), 2.16 - 1.90 (m, 1H), 1.00 - 0.61 (m, 6H); LC-MS, $[MH]^+$.484.2

**Synthesis of Example 13**

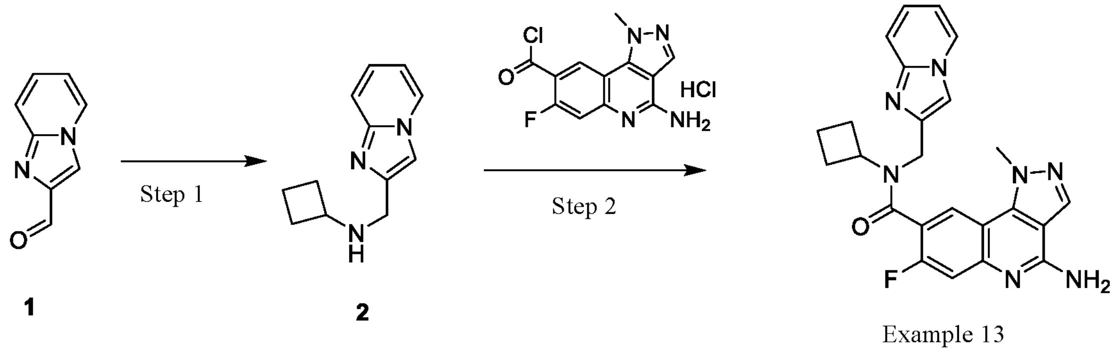


1

2

Example 13

**Step 1: (N-imidazolo[1,2-a]pyridin-2-ylmethyl)cyclobutanamine (2)**

Imidazolo [1,2-a] pyridin-2-carbaldehyde (1) (300 mg, 2.05 mmol, 1 equiv) and cyclobutylamine (300 mg, 4.22 mmol, 361.45 µL, 2.05 equiv) were dissolved in methanol (5 mL). The mixture was stirred at room temperature for 12 hours, and added with sodium borohydride (118 mg, 3.12 mmol, 1.52 equiv), and then stirred at room temperature for 4 hours. LC-MS detected that the raw material was completely consumed and the target product was generated. The reaction solution was quenched with acetic acid (60 µL), concentrated under reduced pressure to dryness, and added with 10% sodium carbonate aqueous solution (25 mL) and dichloromethane/ethanol (10:1, 25 mL). After partition, the aqueous phase was extracted with dichloromethane/ethanol (10:1, 25 mL * 3). The combined organic phase was dried with sodium sulfate, filtered, and concentrated under reduced pressure to dryness to obtain a brown slurry of N-imidazolo [1,2-a]

pyridin-2-ylmethyl) cyclobutanamine (2) (401 mg, crude product) LCMS ES15882-916-P1A: (ESI) m/z= 202.2 $[M+1]^+$; RT= 1.249 min

$^1$H NMR (400 MHz, C) δ: 8.02-8.09 (m, 1H), 7.46-7.57 (m, 2H), 7.13 (ddd, J=1.22, 6.79, 8.99 Hz, 1H), 6.73 (dt, J=0.98, 6.79 Hz, 1H), 3.88 (s, 2H), 3.29-3.47 (m, 1H), 2.15-2.27 (m, 2H), 1.57-1.84 (m, 4H)

**Step 2: 4-Amino-N-cyclobutyl-7-fluoro-N-imidazolo[1,2-a]pyridin-2-ylmethyl)-1-methylpyrazololo[4,3-c] quinolin-8-carboxamide (Example 13)**

To a solution of N-imidazolo[1,2-a]pyridin-2-ylmethyl)cyclobutanamine (2) (60 mg, 298.11 μL, 1 equiv) and N-ethyl-N-isopropyl-2-propylamine (193 mg, 1.50 μmol, 261.29 μL, 5.03 equiv) in tetrahydrofuran (5 mL) was added 4-amino-7-fluoro-1-methylpyrazolo [4,3-c] quinolin-8 formyl chloride (104 mg, 295.80 μL, 9.92e-1 equiv, 2 hydrochloride). The reaction solution was stirred at room temperature for 16 hours. LCMS detected that the raw material was completely consumed and the target product was generated. The reaction solution was concentrated under reduced pressure to dryness, diluted with dimethyl sulfoxide and purified by reverse phase preparative liquid chromatography (alkaline conditions; Boston Prime C18 column 150 * 30 mm* 5 μm; mobile phase: [water (ammonia v/v) - acetonitrile]; B% gradient: 28% -48%, 9 minutes). A white solid of 4-amino-N-cyclobutyl-7-fluoro-N-imidazolo [1,2-a] pyridin-2-ylmethyl) -1-methylpyrazolo [4,3-c] quinolin-8-carboxamide (Example 13) (72 mg, 161.69 μmol, 54.24% yield, 99.59% purity) was obtained. $^1$H NMR (400 MHz, DMSO-$d_6$): 8.40-8.61 (m, 1H), 8.11-8.38 (m, 2H), 7.73-7.90 (m, 1H), 7.44-7.58 (m, 1H), 7.13-7.42 (m, 4H), 6.87 (t, J=6.65 Hz, 1H), 3.86-5.01 (m, 6H), 2.04-2.31 (m, 3H), 1.78-1.96 (m, 1H), 1.26-1.68 (m, 2H). $^{19}$F NMR (376.5 MHz, DMSO-$d_6$): -116.26, -116.59

**Synthesis of Example 49**

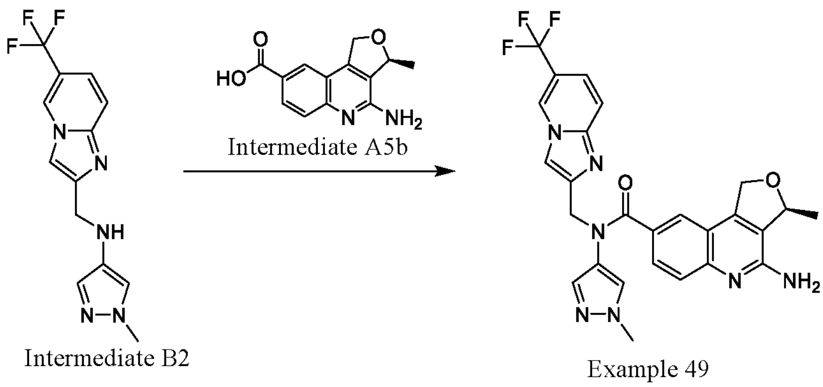


Intermediate B2

Example 49

**Step: (S)-4-Amino-3-methyl-N-(1-methyl-1H-pyrazol-4-yl)-N-((6-(trifluoromethyl)imidazolo[1,2-a]pyri din-2-yl)) methyl)-1,3-dihydrofurano[3,4-c]quinolin-8-carboxamide (Example 49)**

To a solution of 1-methyl-N-((6-(trifluoromethyl)imidazolo[1,2-a]pyridin-2-yl)methyl)-1H-pyrazol-4-amine (Intermediate B2) (500 mg, 1.69 mmol, 1.00 equiv) and (S) -4-amino-3-methyl-1,3-dihydrofurano [3,4-c] quinolin-8-carboxylic acid (intermediate A5b) (434 mg, 1.78 mmol, 1.05 equiv) in DMF (5.00 mL) was added TCFH (713 mg, 2.54 mmol, 1.50 equiv) and NMI (695 mg, 8.47 mmol, 675 μL, 5.00 equiv). The mixture was stirred at 25°C for 16 hours. LC-MS (ET68149-2-P1A1) showed that A29 was retained and the desired mass was detected. The solution was purified by preparative HPLC (chromatography column: Waters Xbridge BEH C18 250 * 50 mm * 10 μ M; Mobile phase: [water ($NH_4HCO_3$ 10 mM) - ACN]; B%: 20% -45%, 10 minutes, UV 220&254 nm) to afford a white solid of (S)-4-amino-3-methyl-N-(1-methyl-1H-pyrazol-4-yl)-N-((6- (trifluoromethyl) imidazolo [1,2-a] pyridin-2-yl))methyl)-1,3-dihydrofurano [3,4-c] quinolin-8-carboxamide (Example 49) (285 mg, 535 μ Mol, 31.6% yield, 97.9% purity). T$^1$H NMR (400 MHz, DMSO-$d_6$) δ: 9.18 (s, 1H), 8.03 (s, 1H), 7.73 (d, *J* = 9.5 Hz, 1H), 7.69-7.49 (m, 2H), 7.46 (dd, *J* = 1.3, 9.4 Hz, 2H), 7.42-6.94 (m, 2H), 6.61 (s, 2H), 5.43-5.35 (m, 1H), 5.27-5.19 (m, 1H), 5.18-5.10 (m, 1H), 5.05 (s, 2H), 3.66 (br s, 3H), 1.38 (d, *J* = 6.3 Hz, 3H); LC-MS, $[MH]^+$ 522.2.

**Synthesis of Example 90**

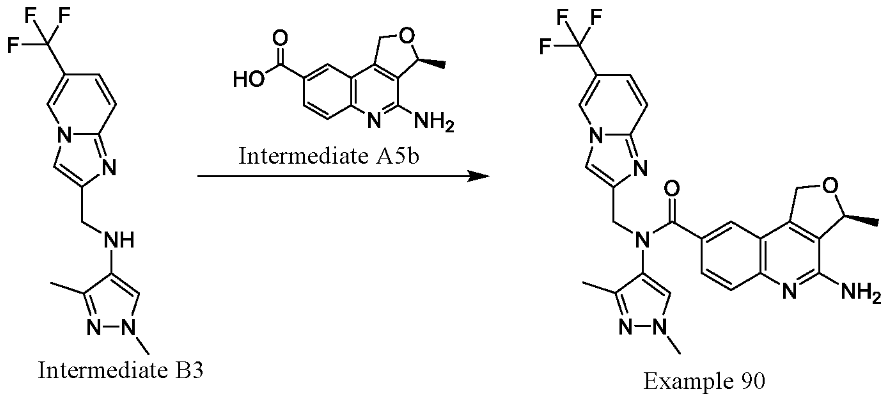


Intermediate B3

Example 90

**Step: (S)-4-amino-N-(1,3-dimethylpyrazol-4-yl)-3-methyl-N-(6-trifluoromethyl)imidazolo[1,2-a]pyridi n-2-yl) methyl)-1,3-dihydrofurano[3,4-c]quinolin-8-carboxamide (Example 90)**

To a mixture of (S)-4-Amino-3-methyl-1,3-dihydrofurano[3,4-c]quinolin-8-carboxylic acid (Intermediate A5b) (3.12 g, 12.76 mmol, 1 equiv), 1,3-dimethyl-N-(6-(trifluoromethyl)imidazolo[1,2-a]pyridin-2-yl)methyl)-1H-pyrazol-4-amine (Intermediate B1) (3.95 g, 12.77 mmol, 1.0 equiv) and 1-methylpyrrolidin-2-one (30 ml) was added N,N,N,N-tetramethylchloroformamidinium hexafluorophosphate (4.30 g, 15.32 mmol, 1.2 equiv) and N-methylimidazole (3.14 g, 38.29 mmol, 3.05 ml, 3.0 equiv); the reaction solution was stirred at room temperature for 16 hours. LC-MS detected that the raw material was completely consumed and the target product was generated. The reaction solution was diluted with acetonitrile (10 ml) and water (10 ml), and filtered. The filtrate was purified by reversed-phase preparative liquid chromatography (column: C18 150×40 mm; mobile phase: [water (ammonia + ammonium bicarbonate)-acetonitrile]; gradient: 17%-57%). Fractions were lyophilized to afford a white solid of (S)-4-amino-N-(1,3-dimethylpyrazol-4-yl)-3-methyl-N-(6-trifluoromethyl)imidazolo[1,2-a]pyridin-2-yl)methyl)-1,3-dihydrofurano[3,4-c]quinolin-8-carboxamide (Example 90) (100% purity). H NMR: ES13683-1863-P1A, $^{1}$HNMR (400 MHz, DMSO-$d_6$) Shift: 9.21 (s, 1H), 8.05 (br s, 1H), 7.71 (br d, J=9.24 Hz, 1H), 7.59 (s, 1H), 7.28-7.57 (m, 4H), 6.63 (s, 2H), 5.33-5.49 (m, 1H), 5.07-5.30 (m, 2H), 4.99 (br s, 2H), 3.59 (s, 3H), 1.66 (br s, 3H), 1.38 (d, J=6.16 Hz, 3H). FNMR: $^{19}$F NMR (376 MHz, DMSO-$d_6$) Shift: -60.41 (br s, 1F). LCMS: ES13683-1863-P1C, (ESI) m/z= 536.3 $[M+1]^+$, RT=0.802 min.

Method 2:

**Synthesis of Example 119**

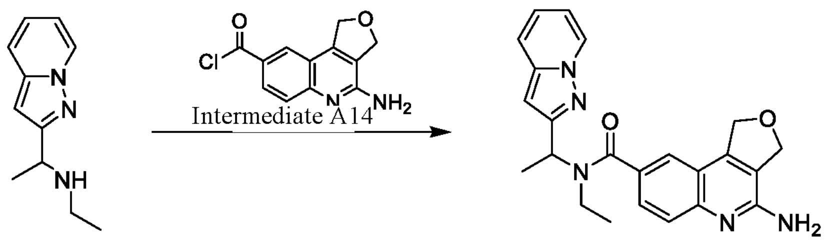


Intermediate B5

Example 119

**Step: 4-Amino-N-ethyl-N-(1-(pyrazolo[1,5-a]pyridin-2-yl)ethyl)-1,3-dihydrofurano[3,4-c]quinolin-8-ca rboxamide (Example 119)**

To a solution of N-ethyl-1- (pyrazolo [1,5-a] pyridin-2-yl) ethan-1-amine (intermediate B5) (50mg, 264.19umol, 1equiv) in THF (1mL) was added DIEA (136.58mg, 1.06mmol, 184.06uL, 4equiv) and 4-amino-1,3-dihydrofurano [3,4-c] quinolin-8-carbonyl chloride (65.69mg, 264.19umol, 1equiv) at 0 °C. The mixture was then stirred at 0°C for 1 hour under N2 atmospheres. LC-MS showed that the starting material was consumed completely and the product was detected. The reaction mixture was concentrated under reduced pressure to obtain a residue. The residue was purified by preparative HPLC (column: Waters Xbridge BEH C18 100 * 30 mm * 10 um; mobile phase: [Water (NH4HCO3) - ACN]; B%: 10% -50%,

8 minutes, UV 220 nm&254 nm) to afford a white solid of 4-Amino-N-ethyl-N-(1-(pyrazolo[1,5-a]pyridin-2-yl)ethyl)-1,3-dihydrofurano[3,4-c]quinolin-8-carbox amide (Example 119) (41.9mg, 104 umol, 39.5% yield). T$^1$H NMR (400 MHz, DMSO-$d_6$) δ 8.69 (d, $J$ = 7.0 Hz, 1H), 7.82-7.50 (m, 4H), 7.36-7.14 (m, 1H), 6.88 (s, 1H), 6.69 (s, 3H), 5.42-5.18 (m, 3H), 5.02 (br s, 2H), 1.82-1.54 (m, 3H), 1.18 - 0.83 (m, 3H); LC-MS, $[MH]^+$ 402.1.

**Synthesis of Example 210**

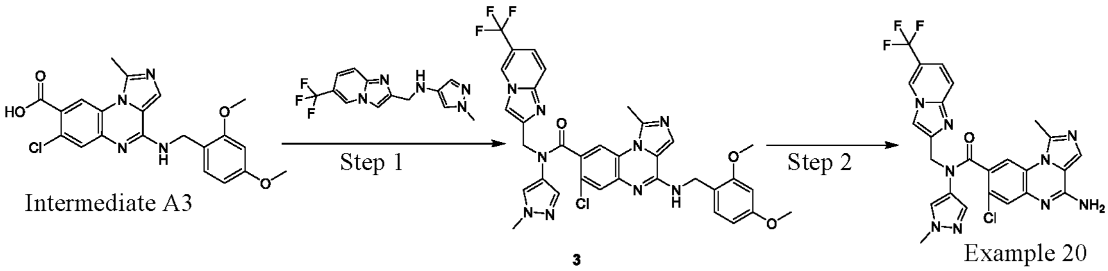


Intermediate A3

3

Example 20

Step 1: **7-chloro-4-[(2,4-dimethoxyphenyl)methylamino]-1-methyl-N-(1-methylpyrazol-4-yl)-N-[[6-(triflu oromethyl)imidazolo[1,2-a]pyridin-2-yl] methyl]imidazolo[1,5-a]quinoxalin-8-carboxamide (3)**

7-chloro-4- ((2,4-dimethoxybenzyl)amino)-1-methylimidazolo [1,5-a] quinoxalin-8-carboxylic acid (Intermediate A3) (145 mg, 339 μmol, 1 equiv) was added to a solution of 1-methyl-N - [[6-(trifluoromethyl) imidazolo [1,2-a] pyridin-2-yl] methyl] pyrazol-4-amine (intermediate B2) (100 mg, 339μmol, 1 equiv) in acetonitrile (2 mL). After dissolution, N,N,N,N-tetramethylchloroformamidinium hexafluorophosphate (285 mg, 1 mmol, 3 eq.) and N-methylimidazole (139 mg, 1.7 mmol, 135 μL, 5 eq.) were added sequentially. The reaction was stirred at 50°C for 16 hours. LC-MS detected that the raw material was completely consumed and the target product was generated. The reaction solution was filtered, and concentrated under reduced pressure to obtain crude product. The crude product was purified by column chromatographic separation (silica, 10% methanol in dichloromethane) to afford a yellow oil of 7-chloro-4-[(2,4-dimethoxyphenyl)methylamino]-1-methyl-N-(1-methylpyrazol-4-yl)-N-[[6-(trifluoro methyl)imidazolo[1,2-a]pyridin-2-yl]methyl]imidazolo[1,5-a] quinoxalin-8- carboxamide (3) (130 mg, 185 μmol, 54.5% yield).

**Step 2: 4-Amino-7-chloro-1-methyl-N-(1-methyl-1hydro-pyrazol-4-yl)-N-((6-(trifluoromethyl)imidazolo[ 1,2-a] pyridin-2-yl] )methyl)imidazolo[1,5-a]quinoxalin-8-carboxamide (Example 210)**

7-Chloro-4-[(2,4-dimethoxyphenyl)methylamino]-1-methyl-N-(1-methylpyrazol-4-yl)-N-[[6-(trifl uoromethyl)imidazolo[1,2-a]pyridin-2-yl]methyl]imidazolo[1,5-a]quinoxalin-8-carboxamide (3) (120 mg, 170 μmol, 1 eq.) was added to a mixed solution of trifluoroacetic acid (0.4 ml. ) and dichloromethane (1 mL) and dissolved. The reaction was stirred at 50°C for 16 hours. LC-MS showed that the raw material was completely consumed and the target product was generated. The reaction was concentrated under reduced pressure to dryness, then purified by reverse phase liquid chromatography purification (Boston Prime C18 column, 5 μ m silica, a diameter of 30 mm and a length of 150 mm, using a mixture of water and acetonitrile with decreasing polarity as eluent) to give white 4-Amino-7-chloro-1-methyl-N-(1-methyl-1hydro-pyrazol-4-yl)-N-((6-(trifluoromethyl) imidazolo[1,2-a]pyridin-2-yl] )methyl)imidazolo[1,5-a]quinoxalin-8-carboxamide (Example 210) (30 mg, 53 mmol, 31% yield, 98.% purity). H NMR 1H NMR (400 MHz, DMSO-d6) δ: 9.15-9.31 (m, 1H), 8.33 (s, 1H), 8.07-8.16 (m, 1H), 7.92-8.03 (m, 1H), 7.76-7.86 (m, 1H), 7.58-7.75 (m, 2H), 7.41-7.55 (m, 3H), 7.18-7.33 (m, 1H), 4.71-5.17 (m, 2H), 3.53-3.83 (m, 3H), 2.80-2.97 (m, 3H).LCMS: (ESI) m/z=554.2, RT= 1.544 min, purity of 98.7 %.

**Synthesis of Example 241**

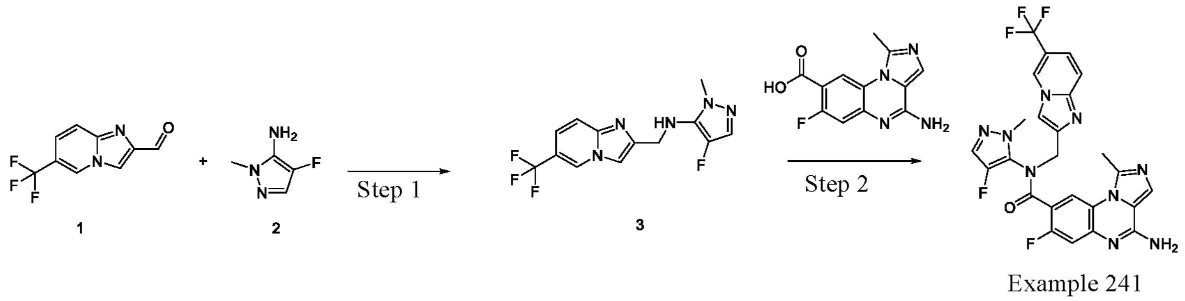


**Step 1: 4-Fluoro-2-methyl-N-[[6-(trifluoromethyl)imidazolo[1,2-a]pyridin-2-yl]methyl]pyrazol-3-amine (3)**

6- (trifluoromethyl) imidazolo [1,2-a] pyridin-2-carbaldehyde (1) (700 mg, 3.27 mmol, 1 equiv) was dissolved in methanol (14 mL), and 4-fluoro-1-methyl-1H-pyrazol-5-amine (2) (402.61 mg, 3.50 mmol, 1.07 equiv) and acetic acid (255.18 mg, 4.25 mmol, 243.26μL, 1.3 equiv) were added. The reaction was stirred at 25°C for 1 hour. Sodium cyanoborohydride (616.24 mg, 9.81 mmol, 3 equiv) was added and the reaction was stirred at 25°C for 15 hours. LC-MS detected that the raw material was completely consumed and the target product was generated. The reaction solution was concentrated and spun-dried, and added with 14 mL of 2 mol/L sodium carbonate aqueous solution and 14 mL of ethyl acetate for partition, then extracted with ethyl acetate (14 mL * 3). The organic phase was dried with anhydrous magnesium sulfate, and filtered. The filtrate was concentrated under reduced pressure to dryness and purified by column chromatography (12 g+4 g silica gel column, eluent 0-30% ethyl acetate: ethanol 3:1/petroleum ether, flow rate 30 mL/min), and then concentrated under reduced pressure to afford a light yellow solid of 4-fluoro-2-methyl-N - [[6- (trifluoromethyl) imidazolo [1,2-a] pyridin-2-yl] methyl] pyrazol-3-amine (3) (970 mg, 3.10 mmol, 94.73% yield). LCMS: ES13685-1213-P1A, (ESI) m/z = 314.0$[M+1]^+$; RT= 1.537 min. NMR: ES13685-1213-R2A,$^1$H NMR (400 MHz, CHLOROFORM-d):7.11 (d, J=4.38 Hz, 1H), 3.57 (s, 3H), 3.24 (br s, 2H). HNMR: ES13685-1213-R2A, $^{19}$F NMR (376 MHz, CHLOROFORM-d):-185.48 (s, 1F)

**Step 2: 4-Fluoro-2-methyl-N-[[6-(trifluoromethyl)imidazolo[1,2-a]pyridin-2-yl]methyl]pyrazol-3-amine (Example 241)**

To a reaction solution of 4-amino-7-fluoro-methyl-imidazolo[1,5-a]quinoxalin-8-carboxylic acid (500 mg, 1.92 mmol, 1 eq.), 4-fluoro-2-methyl-N-[[6-(trifluoromethyl)imidazolo[1,2-a]pyridin-2-yl]methyl]pyrazol-3-amine (3) (601.89 mg, 1.92 mol, 1 eq.) and N,N- diisopropylethylamine (993.30 mg, 7.69 mol, 1.34 mL, 4 eq.) in 1-methyl-2-pyrrolidinone (10 mL) was added 2-chloro-1,3-dimethyl-4,5-dihydroimidazol-1-chloride (487.23 mg, 2.88 mmol, 1.5 eq.). The reaction was stirred at 50°C for 16 hours. LC-MS showed that 47% raw material remained and 36% target product was generated. Then 2-chloro-1,3-dimethyl-4,5-dihydroimidazol-1-chloride (74.71 mg, 441.93 mmol, 0.23 equiv) was added. The reaction was stirred at 50°C for 16 hours. LC-MS showed that 49% raw material remained and 38% target product was generated. Then purified by reversed-phase preparative liquid chromatography (Boston Prime C18 column, 5 μm silica, a diameter of 30 mm, and a length of 150 mm, using a mixture of water (containing 0.05% formic acid) and acetonitrile (20%-40% ) with decreasing polarity as eluent) to afford a light yellow solid. Partial formate formation was detected by nuclear magnetic resonance, and the crude product was purified by reversed-phase preparative liquid chromatography (Boston Prime C18 column, 5μm silica, a diameter of 30 mm, and a length of 150 millimeters, using a mixture of water (containing 0.05% ammonia) and acetonitrile (32% -52%) with decreasing polarity as eluent) to obtain a white solid of 4-amino-7-fluoro-N - (4-fluoro-2-methyl-pyrazol-3-yl) -1-methyl-N - [[6- (trifluoromethyl) imidazolo [1,2-a] pyridin-2-yl] methyl] imidazolo [1,5-a] quinoxalin-8-carboxamide (Example 241) (155 mg, 276.26 μmol, 14.38% yield). LCMS: ES13685-1217-P1D1, (ESI) m/z= 556.1 $[M+1]^+$; RT= 1.929min NMR: ES13685-1217-P1B $^1$H NMR (400 MHz, DMSO-$d_6$): 9.28 (br s, 1H), 8.10 (br s, 1H), 7.92 (br s, 1H), 7.82 (s, 1H), 7.73 (d, J=9.76 Hz, 1H), 7.55 (s, 2H), 7.47 (br d, J=8.63 Hz, 1H), 7.29 (br s, 1H), 7.11 (br d, J=10.38 Hz, 1H), 5.19 (br s, 1H), 5.04-5.14 (m, 1H), 3.59 (s, 3H), 2.86 (br s, 3H). NMR: ES13685-1217-P1B, $^{19}$F NMR (376 MHz, DMSO-$d_6$): -60.47 (s, 3F), -118.15 (s, 1F), -173.54 (s, 1F)

**Synthesis of Example 285**

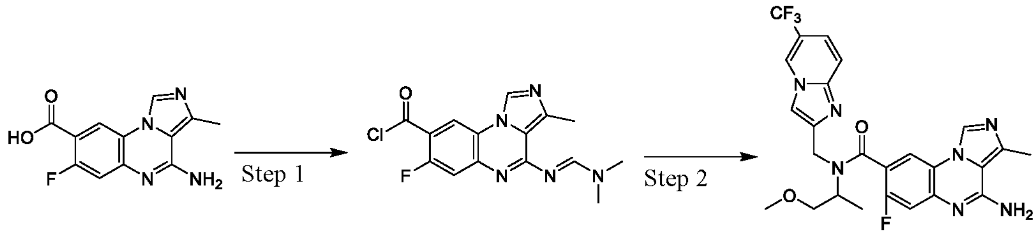


Intermediate A7 Intermediate A7-1 Example 285

**Step 1: (E)-4-(((dimethylamino)methylene)amino)-7-fluoro-3-methylimidazolo[1,5-a]quinoxalin-8-carbo nyl chloride (Intermediate A7-1)**

To a stirred solution of 4-Amino-7-fluoro-3-methylimidazolo[1,5-a]quinoxalin-8-carboxylic acid (Intermediate A7) (270mg, 1.04mmol, 1 equiv) in DCM was added HCl/dioxane(4M, 778uL, 3 equiv). The mixture was stirred at 25°C for 0.5 hours. Then the reaction mixture was concentrated, steamed with toluene (10mL x 3) and dried. The crude product was dissolved in DCM (3.00mL) and then cooled to 0 °C and added with oxalyl dichloride (790mg, 6.23mmol, 544uL, 6 equiv dropped at 0 ° C) and DMF (75.8 mg, 1.04 mmol, 79.8uL, 1 equiv). The mixture was stirred at 25°C for 12 hours. LC-MS showed that the starting material was consumed and a main peak with the desired mass was detected. The reaction mixture was concentrated, steamed with hexane(10mL×3) and dried under reduced pressure to obtain a yellow solid of (E)-4-(((dimethylamino)methylene)amino)-7-fluoro-3-methylimidazolo[1,5-a]quinoxalin-8-carbonyl chloride (Intermediate A7-1) (340mg, crude product). LC-MS (ESI) m/z = 330.0 $[M+H]^+$

**Step 2: 4-Amino-7-fluoro-N-(1-methoxypropan-2-yl)-3-methyl-N-((6-(trifluoromethyl)imidazolo[1,2-a]py ridin-2-yl)methyl)imidazo[1,5-a]quinoxalin-8-carboxamide (Example 285)**

To a solution of 1-methoxy-N-(6- (trifluoromethyl) imidazolo [1,2-a] pyridin-2-yl) methyl) propan-2-amine (50.0 mg, 174 umol, 1 equiv) and DIEA (89.9 mg, 696 umol, 121 uL, 4 equiv) in THF (1.00 mL) was added Intermediate A7-1 (63.9 mg, 191 umol, 1.1 equiv) at 0 °C. The mixture was stirred at 25°C for 3 hours. The reaction mixture was quenched with MeOH(1.00mL) at 25°C and concentrated under reduced pressure to obtain a residue. The residue was dissolved in MeOH (1mL) and NH3/MeOH (7M, 1mL). The mixture was stirred at 70°C for 2 hours. LC-MS showed that the starting material was consumed and a main peak with the desired mass was detected. The reaction mixture was concentrated under reduced pressure and the residue was purified by preparative HPLC (column: Waters Xbridge BEH C18 100*30 mm*10 um; mobile phase: [water (NH4HCO3)-ACN]; B%: 30%-60%, 8 min, UV 220 nm & 254 nm) to afford a yellow solid of 4-amino-7-fluoro-N -(1-methoxypropan-2-yl)-3-methyl-N-((6-(trifluoromethyl)imidazolo[1,2-a]pyridin-2-yl)methyl)imida zo [1,5-a]quinoxalin-8-carboxamide (Example 285) (58.1 mg, 109 umol, 64.2% yield). $^1H$ NMR (400MHz, DMSO-$d_6$) δ 9.29-8.54 (m, 2H), 8.18-7.78 (m, 2H), 7.66 (br s, 1H), 7.38 (br d, $J$ = 7.9 Hz, 1H), 7.13 (br s, 1H), 6.72 (br s, 2H), 4.95-4.39 (m, 2H), 4.22-3.82 (m, 1H), 3.52 (br s, 2H), 3.31-3.10 (m, 3H), 2.63 (s, 3H), 1.21 (br s, 3H) LC-MS (ESI) m/z = 530.2 $[M+H]^+$

**Synthesis of Example 304**

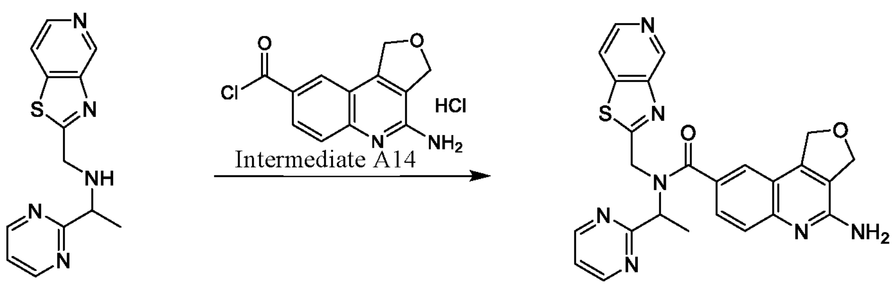


Intermediate B4 Example 304

**Step: 4-Amino-N-(1-(pyrimidin-2-yl)ethyl)-N-(thiazolo[4,5-c]pyridin-2-ylmethyl)-1,3-dihydrofurano[3,4-c]quinolin-8-carboxamide**

**(Example 304)**

To a solution of 1- (pyrimidin-2-yl)-N-(thiazolo [4,5-c] pyridin-2-ylmethyl) ethan-1-amine (Intermediate B4) in THF(1 mL) was added DIEA (51.5 mg, 398 umol, 69.4 uL, 4 equiv) and 4-amino-1,3-dihydrofurano [3,4-c] quinolin-8-formyl chloride hydrochloride (28.4 mg, 99.6 umol, 1 eq, HCl) at 0 °C. The mixture was stirred at 25°C for 2 hours. LC-MS showed that the starting material was consumed completely and the desired mass was detected. The mixture was quenched with MeOH(2mL) and concentrated under reduced pressure to obtain a residue. The residue was purified by preparative HPLC (column: Waters Xbridge Prep OBD C18 150 * 40 mm * 10 um; mobile phase: [Water (NH4HCO3) - ACN]; B%: 15% -55%, 8 minutes, UV220 nm&254 nm) to afford a white solid of 4-amino-N-(1- (pyrimidin-2-yl) ethyl)-N-(thiazolo[4,5-c]pyridin-2-ylmethyl)-1,3-dihydrofurano [3,4-c]quinolin-8-carboxamide (Example 304) (19mg, 38.9 umol, 39.0% yield, 98.9% purity). $^1$H NMR (400 MHz, DMSO-$d_6$) δ: 9.26 - 9.11 (m, 1H), 8.81 (d, *J* = 4.9 Hz, 2H), 8.49 (br d, *J* = 1.1 Hz, 1H), 8.13 (br d, *J= 5.1* Hz, 1H), 7.74 - 7.57 (m, 3H), 7.42 (t, *J* = 4.9 Hz, 1H), 6.73 (br s, 2H), 5.50 - 5.09 (m, 4H), 5.00 (br s, 2H), 4.96 - 4.87 (m, 1H), 1.68 (br d, *J* = 7.0 Hz, 3H); LC-MS, $[MH]^+$ 484.2

The following compounds were synthesized according to general methods

The corresponding structure of the product was shown in Table 1, and the characterization was shown in Table 2:

Table 2

| | HNMR/LCMS | | HNMR/LCMS |
|---|---|---|---|
| 1 | $^1$H NMR (400 MHz, DMSO-$d_6$) δ 8.82 - 8.78 (m, 2H), 8.50 (d, *J* = 6.4 Hz, 1H), 8.02 - 7.67 (m, 2H), 7.64 - 7.37 (m, 4H), 7.29 - 7.10 (m, 1H), 6.84 (s, 1H), 6.43 (s, 2H), 5.39 (s, 1H), 4.91-4.32 (m, 2H), 3.29 - 3.02 (m, 1H), 2.95 - 2.76 (m, 3H), 2.45 - 1.89 (m, 2H), 1.64 (d, *J* = 6.4 Hz, 3H).$^{19}$F NMR (377 MHz, DMSO) δ -60.66 (s, 1H). LC-MS: Rt =0.858 min, (ESI) *m/z.* $[M+H]^+$ 465.3; $C_{27}H_{25}N_7O$ | 2 | $^1$H NMR (400 MHz, dmso-$d_6$) δ 8.90 - 8.71 (m, 1H), 8.53 (s, 3H), 8.00 - 7.76 (m, 1H), 7.59 - 7.43 (m, 3H), 7.23 (s, 2H), 5.64 - 5.45 (m, 1H), 5.30 - 5.09 (m, 1H), 5.06 - 4.99 (m, 1H), 4.95 - 4.89 (m, 1H), 3.29 - 3.18 (m, 2H), 3.00 - 2.77 (m, 2H), 2.31 (s, 1H), 2.03 (s, 1H), 1.79 - 1.20 (m, 3H). LC-MS: Rt = 0.918 min, (ESI) m/z. $[M+H]^+$, 464.2. $C_{27}H_{25}N_7O$. |
| 3 | 1H NMR (400 MHz, DMSO-d6) δ = 9.20 (br s, 1H), 7.96 (br s, 1H), 7.91 - 7.63 (m, 2H), 7.48 (br d, J = 9.4 Hz, 2H), 7.56 (br d, J = 6.4 Hz, 1H), 6.66 (br s, 2H), 5.27 (br d, J = 0.9 Hz, 2H), 5.00 (br s, 2H), 4.86 - 4.51 (m, 2H), 3.30 - 3.19 (m, 2H), 2.16 - 1.90 (m, 1H), 1.00 - 0.61 (m, 6H)<br>LC-MS: Rt = 2.688 min, (ESI) *m/z.* $[M+H]^+$ 484.2; $C_{25}H_{24}F_3N_5O_2$ | 4 | 1H NMR (400 MHz, DMSO-d6) δ = 9.35 - 9.08 (m, 1H), 8.74 - 8.35 (m, 1H), 8.29 - 8.17 (m, 1H), 8.12 - 7.96 (m, 1H), 7.83 - 7.69 (m, 1H), 7.66 - 7.54 (m, 2H), 7.53 - 7.45 (m, 1H), 7.18 - 7.01 (m, 2H), 4.94 - 4.48 (m, 2H), 4.43 - 4.10 (m, 3H), 3.30 - 3.21 (m, 2H), 2.23 - 1.92 (m, 1H), 1.06 - 0.58 (m, 6H)<br>LC-MS: Rt = 2.684 min, (ESI) *m/z.* $[M+H]^+$ 496.3; $C_{25}H_{24}F_3N_7O$ |
| 5 | 1H NMR (400 MHz, DMSO-d6) δ 8.81 (br s, 2H), 8.49 (br d, J = 6.1 Hz, 1H), 7.91 (br s, 1H), 7.65 (br d, J = 8.6 Hz, 2H), 7.58 - 7.48 (m, 1H), 7.59 - 7.35 (m, 1H), 7.29 - 7.10 (m, 1H), 6.92 - 6.78 (m, 1H), 6.59 (br s, 2H), 5.47 - 5.33 (m, 2H), 5.32 - 5.17 (m, 1H), 5.15 - 4.95 (m, 1H), 4.94 - 4.22 (m, 2H), 1.62 (d, J = 7.1 Hz, 3H), 1.38 (br d, J = 2.2 Hz, 3H) LC-MS: Rt = 2.172 min, (ESI) *m/z.* $[M+H]^+$ 480.3; $C_{27}H_{25}N_7O_2$ | 6 | 1H NMR (400 MHz, DMSO-d6) δ = 8.52 (d, J = 6.8 Hz, 1H), 8.15 - 7.83 (m, 2H), 7.77 - 7.60 (m, 1H), 7.55 (br d, J = 5.6 Hz, 2H), 7.25 (br s, 1H), 6.89 (br t, J = 6.6 Hz, 1H), 6.65 (s, 2H), 5.36 - 5.20 (m, 2H), 4.99 (br s, 2H), 4.77 - 4.53 (m, 2H), 3.47 - 3.40 (m, 2H), 1.12 (br t, J = 6.9 Hz, 3H) LC-MS: Rt = 2.080 min, (ESI) *m/z.* $[M+H]^+$ 388.1; $C_{22}H_{21}N_5O_2$ |
| 7 | 1H NMR (400 MHz, DMSO-d6) δ = 8.60 - 8.44 (m, 1H), 8.30 - 7.73 (m, 2H), 7.71 - 7.43 (m, 3H), 7.39 - 7.15 (m, 1H), 6.98 - 6.79 (m, 1H), 6.73 - 6.46 (m, 2H), 5.58 - 5.03 (m, 3H), 4.86 - 4.38 (m, 2H), 3.26 - 3.24 (m, 2H), 1.50 - 1.29 (m, 3H), 1.22 - 0.99 (m, 3H) LC-MS: Rt = 2.043 min, (ESI) *m/z.* $[M+H]^+$ 402.2; $C_{23}H_{23}N_5O_2$ | 8 | 1H NMR (400MHz, DMSO-d6) δ = 8.80 (d, J=4.9 Hz, 2H), 8.49 (d, J=6.8 Hz, 1H), 7.99 - 7.66 (m, 1H), 7.80 (br s, 2H), 7.59 (br d, J=8.3 Hz, 1H), 7.52 - 7.31 (m, 3H), 7.18 (br s, 1H), 6.85 (br t, J=6.6 Hz, 1H), 6.45 (br s, 2H), 5.46 - 5.27 (m, 1H), 5.04 - 4.06 (m, 2H), 2.20 (br s, 3H), 1.64 (br d, J=6.9 Hz, 3H).<br>LC-MS: Rt = 1.493 min, (ESI) *m/z.* $[M+H]^+$ 438.1; $C_{25}H_{23}N_7O$ |

(continued)

| | HNMR/LCMS | | HNMR/LCMS |
|---|---|---|---|
| 9 | 1H NMR (400 MHz, DMSO-d6) δ 8.10-8.59 (m, 3H), 7.68-7.97 (m, 1H), 7.52 (br d, J=8.78 Hz, 1H), 7.19-7.38 (m, 4H), 6.88 (t, J=6.65 Hz, 1H), 4.94-5.15 (m, 2H), 4.49-4.93 (m, 5H), 4.04-4.41 (m, 3H). LC-MS: Rt =1.188 min, (ESI) m/z. [M+H] + 446.2;C23H20FN7O2 | 10 | 1H NMR (400 MHz, DMSO-d6) δ 8.70 (br s, 1H), 8.50 (br d, J=6.00 Hz, 1H), 8.14-8.32 (m, 1H), 7.92 (br s, 1H), 7.56 (br d, J=8.76 Hz, 1H), 7.35 (br d, J=11.51 Hz, 1H), 7.24 (br s, 3H), 6.90 (br t, J=6.25 Hz, 1H), 5.92 (br s, 1H), 5.08 (br d, J=6.13 Hz, 1H), 4.36 (br s, 3H), 3.47 (br d, J=7.00 Hz, 1H), 3.13 (br s, 1H), 1.70 (br s, 1H), 1.52-1.64 (m, 2H), 1.09 (br s, 3H), 0.79 (br s, 1H).<br>LC-MS: Rt =1.155 min, (ESI) m/z. [M+H]+ 432.2 C23H22FN7O |
| 11 | 1H NMR (400MHz, DMSO-d6) δ 8.52 - 8.40 (m, 1H), 8.29 - 8.18 (m, 2H), 7.92 - 7.73 (m, 1H), 7.55 - 7.40 (m, 1H), 7.36 - 7.25 (m, 3H), 7.25 - 7.13 (m, 1H), 6.90 - 6.79 (m, 1H), 4.37 (d, J=10.8 Hz, 3H), 4.05 - 3.47 (m, 5H), 2.43 - 2.07 (m, 2H). LC-MS: Rt =2.206 min, (ESI) m/z. [M+H]+ 430.2;C23H20FN7O | 12 | 1H NMR (400 MHz, DMSO-d6) δ 8.45-8.61 (m, 2H), 8.17-8.29 (m, 1H), 7.90 (br s, 1H), 7.53 (d, J=9.03 Hz, 1H), 7.33 (d, J=11.80 Hz, 1H), 7.21-7.30 (m, 3H), 6.90 (t, J=6.65 Hz, 1H), 4.51-5.00 (m, 2H), 4.09-4.44 (m, 3H), 3.47-3.73 (m, 4H), 3.09-3.32 (m, 3H). LC-MS: Rt =1.305 min, (ESI) m/z. [M+H]+ 448.2;C23H22FN7O2 |
| 13 | 1H NMR (400 MHz, DMSO-d6) δ 8.54 (br s, 1H), 8.25 (br d, J=5.27 Hz, 2H), 7.86 (br s, 1H), 7.51 (br d, J=9.03 Hz, 1H), 7.14-7.41 (m, 4H), 6.89 (m, 1H), 3.90-5.15 (m, 5H), 1.50-2.28 (m, 7H). LC-MS: Rt =1.521 min, (ESI) m/z. [M+H]+ 456.2;C25H22FN7O | 14 | 1H NMR (400 MHz, DMSO-d6) δ 8.42-8.74 (m, 1H), 8.04-8.32 (m, 2H), 7.88 (s, 1H), 7.45-7.73 (m, 2H), 7.02-7.42 (m, 4H), 6.88 (t, J=6.65 Hz, 1H), 4.86-5.17 (m, 2H), 4.07-4.46 (m, 3H), 3.50-3.89 (m, 3H). LC-MS: Rt =1.228 min, (ESI) m/z. [M+H]+ 470.2; C24H20FN9O |
| 15 | 1H NMR(400 MHz, DMSO-d6) δ 8.41-8.58 (m, 2H), 8.35 (br s, 1H), 7.90 (br s, 1H), 7.44-7.68 (m, 3H), 7.16-7.28 (m, 1H), 6.74-6.93 (m, 3H), 5.19 (br s, 1H), 4.35 (s, 3H), 3.42 (br d, J=5.06 Hz, 1H), 3.16 (br dd, J=7.26, 13.64 Hz, 1H), 1.61 (br s, 3H), 1.07 (br s, 3H). LC-MS: Rt =1.395 min, (ESI) m/z. [M+H]+ 414.2;C23H23N7O | 16 | 1H NMR (400 MHz, DMSO-d6) δ 9.19 (s, 1H), 8.23 (s, 1H), 8.25-8.19 (m, 1H), 8.53-8.15 (m, 1H), 8.08 (s, 1H), 7.83-7.66 (m, 2H), 7.62 (br d, J = 7.6 Hz, 1H), 7.55-7.44 (m, 2H), 7.40-7.21 (m, 1H), 7.15 (s, 2H), 5.10 (s, 2H), 4.19 (s, 3H), 3.67 (br s, 3H). LC-MS: Rt = 2.362 min, (ESI) m/z. [M+H]+ 520.2; C25H20F3N9O |
| 17 | 1H NMR (400 MHz, DMSO-d6) δ 9.19 (s, 1H), 8.36 (br d, J = 2.0 Hz, 1H), 8.24 (s, 1H), 8.00 (br s, 1H), 7.76 (d, J = 9.4 Hz, 1H), 7.60 (br s, 2H), 7.49 (dd, J = 1.8, 9.5 Hz, 1H), 7.16 (s, 2H), 5.12-4.99 (m, 1H), 4.93 (br s, 2H), 4.78 (br t, J = 6.8 Hz, 2H), 4.64-4.58 (m, 2H), 4.25 (br s, 3H). LC-MS: Rt = 2.329 min, (ESI) *m/z.* $[M+H]^+$ 496.2; $C_{24}H_{20}F_3N_7O_2$ | 18 | 1H NMR (400 MHz, DMSO-d6) δ = 8.77 - 8.56 (m, 1H), 8.53 (d, J = 6.8 Hz, 1H), 8.23 (s, 1H), 7.97 (s, 1H), 7.66 (br s, 1H), 7.62 - 7.53 (m, 2H), 7.27 (br t, J = 7.1 Hz, 1H), 7.14 (br s, 2H), 6.91 (t, J = 6.7 Hz, 1H), 4.82 - 4.56 (m, 2H), 4.25 - 4.08 (m, 2H), 3.45 (q, J = 6.8 Hz, 3H), 1.15 (br t, J = 7.0 Hz, 3H) LC-MS: Rt = 1.964 min, (ESI) *m/z.* $[M+H]^+$ 400.2; $C_{22}H_{21}N_7O$ |
| 19 | 1H NMR (400 MHz, DMSO-d6) δ 8.43-9.02 (m, 2H), 8.22 (br s, 1H), 7.99 (br s, 1H), 7.54 (br d, J=9.03 Hz, 1H), 7.11-7.38 (m, 4H), 6.89 (br t, J=6.53 Hz, 1H), 4.07-5.87 (m, 5H), 3.45-3.94 (m, 4H), 0.93 (br d, J=6.53 Hz, 3H). LC-MS: Rt =1.340 min, (ESI) *m/z.* $[M+H]^+$ 460.2;<br><br>$C_{24}H_{22}FN_7O_2$ | 20 | 1H NMR (400 MHz, DMSO-d6) δ 9.22-9.44 (m, 1H), 8.43 (br d, J=7.26 Hz, 1H), 8.18-8.30 (m, 1H), 8.13 (br d, J=6.60 Hz, 1H), 7.92-8.05 (m, 1H), 7.69 (br d, J=8.80 Hz, 1H), 7.50 (br d, J=9.46 Hz, 1H), 7.17-7.38 (m, 3H), 4.49-4.94 (m, 2H), 4.17-4.40 (m, 3H), 3.42 (br s, 1H), 1.88-2.23 (m, 1H), 0.57-1.02 (m, 6H). LC-MS: Rt =1.546 min, (ESI) *m/z.* $[M+H]^+$ 471.2; $C_{25}H_{23}FN_8O$ |

(continued)

| | HNMR/LCMS | | HNMR/LCMS |
|---|---|---|---|
| 21 | 1H NMR (400 MHz, DMSO-d6) δ 8.42 (d, J=7.28 Hz, 2H), 8.20 (s, 1H), 7.80 (s, 1H), 7.51 (d, J=9.03 Hz, 1H), 7.29 (d, J=11.80 Hz, 1H), 7.19-7.26 (m, 3H), 6.85 (t, J=6.53 Hz, 1H), 4.90 (br d, J=5.77 Hz, 2H), 4.51 (s, 2H), 4.26 (br d, J=6.02 Hz, 2H), 4.21 (s, 3H), 4.16-4.23 (m, 1H), 1.54 (s, 3H). LC-MS: Rt =1.221 min, (ESI) m/z. [M+H]+ 460.2; C24H22FN7O2 | 22 | 1H NMR (400 MHz, DMSO-d6) δ 8.34-8.67 (m, 1H), 8.08-8.32 (m, 2H), 7.62-7.90 (m, 1H), 7.51 (br s, 1H), 7.10-7.40 (m, 4H), 6.86 (br s, 1H), 4.29-5.10 (m, 5H), 3.61-4.11 (m, 5H), 2.14 (br d, J=6.02 Hz, 2H). LC-MS: Rt =1.242 min, (ESI) m/z. [M+H]+ 460.2; C24H22FN7O2 |
| 23 | 1H NMR (400 MHz, DMSO-d6) δ 8.35-8.65 (m, 1H), 8.00-8.33 (m, 2H), 7.87 (br s, 1H), 7.52 (br d, J=9.03 Hz, 1H), 7.14-7.39 (m, 4H), 6.76-6.98 (m, 1H), 5.35-5.86 (m, 1H), 4.45-4.92 (m, 2H), 4.37 (s, 3H), 3.73-4.23 (m, 2H), 1.13-2.01 (m, 7H). LC-MS: Rt =1.366 min, (ESI) *m/z.* $[M+H]^+$ 474.2; $C_{25}H_{24}FN_7O_2$ | 24 | 1H NMR (400 MHz, DMSO-d6) δ 8.30-8.62 (m, 1H), 7.87-8.28 (m, 2H), 7.54 (br d, J=9.03 Hz, 1H), 7.06-7.39 (m, 5H), 6.66-6.98 (m, 1H), 4.21-4.91 (m, 5H), 3.71 (br s, 1H), 3.41-3.61 (m, 2H), 1.82-2.07 (m, 1H), 1.43-1.79 (m, 4H), 0.79-1.40 (m, 3H). LC-MS: Rt =1.518 min, (ESI) *m/z.* $[M+H]^+$ 488.3; $C_{26}H_{26}FN_7O_2$ |
| 25 | 1H NMR (400 MHz, DMSO-d6) δ 8.52 (d, J=6.82 Hz, 1H), 8.45 (s, 1H), 8.40 (br s, 1H), 7.97 (s, 1H), 7.59-7.65 (m, 1H), 7.57 (d, J=9.02 Hz, 2H), 7.17-7.25 (m, 1H), 6.81-6.90 (m, 2H), 5.24 (s, 1H), 4.66 (br s, 1H), 4.34 (s, 4H), 3.61-3.89 (m, 3H), 0.97 (d, J=6.82 Hz, 3H). LC-MS: Rt =1.305 min, (ESI) *m/z.* $[M+H]^+$ 442.2; $C_{24}H_{23}N_7O_2$ | 26 | 1H NMR (400 MHz, DMSO-d6) δ 8.52 (br dd, J=6.90, 15.94 Hz, 2H), 8.23 (s, 1H), 7.89 (s, 1H), 7.55 (d, J=9.03 Hz, 1H), 7.18-7.42 (m, 4H), 6.79-6.98 (m, 1H), 6.11-6.54 (m, 1H), 4.56-5.05 (m, 2H), 4.13-4.44 (m, 3H), 3.90 (br s, 2H). LC-MS: Rt =1.409 min, (ESI) m/z. [M+H]+ 454.2; C22H18F3N7O |
| 27 | 1H NMR (400 MHz, DMSO-d6) δ 8.50 (br dd, J=7.15, 14.68 Hz, 2H), 8.22 (s, 1H), 7.87 (s, 1H), 7.55 (d, J=9.03 Hz, 1H), 7.17-7.40 (m, 4H), 6.81-6.95 (m, 1H), 4.64 (br s, 2H), 4.23 (s, 3H), 3.57-4.12 (m, 2H), 1.74 (br t, J=19.20 Hz, 3H). LC-MS: Rt =1.468 min, (ESI) m/z. [M+H]+ 468.2; C23H20F3N7O | 28 | 1H NMR (400 MHz, DMSO-d6) δ 8.01-8.73 (m, 3H), 7.65 (br s, 1H), 7.48 (br d, J=8.03 Hz, 1H), 7.13-7.37 (m, 4H), 6.78 (br s, 1H), 4.97-5.69 (m, 1H), 3.79-4.88 (m, 4H), 1.46 (br s, 3H). LC-MS: Rt =1.535 min, (ESI) *m/z.* $[M+H]^+$ 486.2; $C_{23}H_{19}F_4N_7O$ |
| 29 | 1H NMR (400 MHz, DMSO-d6) δ 8.52 (br d, J=6.82 Hz, 1H), 8.03-8.50 (m, 2H), 7.93 (s, 1H), 7.39-7.71 (m, 3H), 7.24 (br t, J=7.26 Hz, 1H), 6.76-6.98 (m, 3H), 4.51-4.89 (m, 2H), 4.35 (s, 3H), 3.40-3.54 (m, 2H), 1.14 (br t, J=6.82 Hz, 3H). LC-MS: Rt =1.337 min, (ESI) m/z. [M+H]+ 400.2; C22H21N7O | 30 | 1H NMR (400 MHz, DMSO-d6) δ 8.63 (d, J=7.53 Hz, 1H), 8.10-8.33 (m, 2H), 7.49-7.64 (m, 1H), 7.15-7.42 (m, 4H), 6.82-7.02 (m, 1H), 4.88 (br s, 1H), 4.55 (s, 1H), 4.20-4.44 (m, 3H), 3.43-3.57 (m, 1H), 3.26 (br d, J=7.28 Hz, 1H), 2.55 (s, 1H), 2.26 (s, 2H), 0.98-1.18 (m, 3H). LC-MS: Rt =1.407 min, (ESI) m/z. [M+H]+ 432.2; C23H22FN7O |
| 31 | 1H NMR (400 MHz, DMSO-d6) δ 8.62-8.88 (m, 1H), 8.51 (d, J=6.60 Hz, 1H), 8.23 (br s, 1H), 8.13 (br s, 1H), 7.91 (br s, 1H), 7.56 (br s, 1H), 7.32 (br d, J=11.88 Hz, 1H), 7.25 (br s, 3H), 6.89 (br s, 1H), 5.29 (br s, 1H), 4.38 (br s, 3H), 1.78 (br d, J=1.54 Hz, 10H), 1.14-1.29 (m, 1H). LC-MS: Rt =1.259 min, (ESI) m/z. [M+H]+ 470.2; C26H24FN7O | 32 | 1H NMR (400MHz, DMSO-d6) δ = 8.47 (br d, J=6.5 Hz, 1H), 8.03 - 7.80 (m, 3H), 7.79 - 7.56 (m, 2H), 7.54 - 7.29 (m, 3H), 7.26 - 7.13 (m, 1H), 7.03 (br s, 1H), 6.85 (dt, J=1.0, 6.8 Hz, 1H), 6.55 - 6.39 (m, 2H), 5.24 (br s, 1H), 4.84 (br s, 1H), 4.34 - 4.04 (m, 1H), 2.22 - 2.17 (m, 3H), 1.60 (d, J=7.0 Hz, 3H). LC-MS: Rt = 1.656 min, (ESI) *m/z.* $[M+H]^+$ 455.2; $C_{26}H_{23}FN_6O$ |

(continued)

| | HNMR/LCMS | | HNMR/LCMS |
|---|---|---|---|
| 33 | 1H NMR (400 MHz, DMSO-d6) δ ppm 8.51 (d, J=6.75 Hz, 1 H) 7.82 - 7.98 (m, 2 H) 7.68 (br s, 1 H) 7.58 (d, J=8.63 Hz, 2 H) 7.21 - 7.28 (m, 1 H) 6.89 (t, J=6.75 Hz, 1 H) 6.57 (s, 2 H) 5.36 - 5.45 (m, 2 H) 5.29 (br s, 1 H) 5.22 (br d, J=13.76 Hz, 1 H) 3.10 - 3.24 (m, 2 H) 1.59 (br s, 3 H) 1.40 (t, J=6.00 Hz, 3 H) 1.05 (br s, 3 H<br>LC-MS: Rt = 2.295 min ,<br>(ESI) m/z = 416.2 [M+H]+;<br>C24H25N5O2 | 34 | 1H NMR (400 MHz, DMSO-d6) δ 9.12-9.33 (m, 1H), 8.40 (d, J=7.26 Hz, 1H), 8.19-8.28 (m, 1H), 7.97-8.17 (m, 1H), 7.75 (br d, J=9.24 Hz, 1H), 7.15-7.46 (m, 4H), 4.50-4.96 (m, 2H), 4.16-4.39 (m, 3H), 3.10-3.36 (m, 2H), 1.85-2.21 (m, 1H), 0.59-1.03 (m, 6H). LC-MS: Rt =1.937 min, (ESI) m/z. [M+H]+ 564.2;<br>C26H23F6N7O |
| 35 | 1H NMR (400 MHz, DMSO-d6) δ 8.49 (d, J=7.48 Hz, 1H), 8.09-8.33 (m, 2H), 7.69-7.84 (m, 1H), 7.45 (d, J=9.68 Hz, 1H), 7.18-7.36 (m, 3H), 7.07 (dd, J=2.20, 9.68 Hz, 1H), 4.43-4.86 (m, 2H), 4.22-4.40 (m, 3H), 4.00-4.12 (m, 2H), 3.63-3.73 (m, 2H), 3.32 (s, 5H), 1.90-2.24 (m, 1H), 0.60-1.00 (m, 6H). LC-MS: Rt =1.564 min, (ESI) m/z. [M+H] + 520.3;<br>C27H30FN7O3 | 36 | 1H NMR (400 MHz, DMSO-d6) δ 9.08-9.34 (m, 1H), 8.11-8.49 (m, 2H), 7.91-8.10 (m, 1H), 7.69-7.77 (m, 1H), 7.47 (d, J=9.46 Hz, 1H), 7.32 (d, J=11.66 Hz, 1H), 7.13-7.30 (m, 2H), 4.50-4.93 (m, 2H), 4.20-4.38 (m, 3H), 3.02-3.37 (m, 2H), 1.92-2.18 (m, 1H), 0.57-1.02 (m, 6H). LC-MS: Rt =1.777 min, (ESI) m/z. [M+H]+ 514.2;<br>C25H23F4N7O |
| 37 | 1H NMR (400 MHz, DMSO-d6) δ 8.53 (m, 1.7H), 8.14-8.29 (m, 1.3H), 7.90 (s, 1H), 7.53 (d, J=9.24 Hz, 1H), 7.13-7.40 (m, 4H), 6.90 (t, J=6.38 Hz, 1H), 4.47-4.88 (m, 2H), 4.19-4.44 (m, 3H). LC-MS: Rt =1.231 min, (ESI) m/z. [M+H]+ 407.2;<br>C21H15D3FN7O | 38 | 1H NMR (400 MHz, DMSO-d6) δ = 8.75 (d, J = 4.8 Hz, 2H), 8.44 (br s, 1H), 7.75 - 7.61 (m, 3H), 7.41 (br d, J = 9.2 Hz, 1H), 7.34 (t, J = 4.9 Hz, 1H), 7.21 - 7.09 (m, 2H), 6.81 (t, J = 6.5 Hz, 1H), 6.28 (br s, 2H), 5.76 - 4.73 (m, 2H), 4.69 - 4.35 (m, 1H), 2.20 (s, 3H), 1.66 (br d, J = 6.8 Hz, 3H) LC-MS: Rt = 2.219 min, (ESI) m/z. [M+H]+ 456.2;<br>C25H22FN7O |
| 39 | $^1$H NMR (400 MHz, DMSO-*d*$_6$) δ = 9.30 - 9.09 (m, 1H), 8.87 - 8.71 (m, 2H), 8.01 - 7.82 (m, 1H), 7.79 - 7.49 (m, 3H), 7.46 - 7.35 (m, 2H), 7.23 - 6.95 (m, 1H), 6.74 - 6.42 (m, 2H), 5.74 - 4.89 (m, 2H), 4.79 - 4.28 (m, 1H), 2.19 (s, 2H), 1.72 - 1.55 (m, 3H)<br>LC-MS: Rt = 2.585 min, (ESI) *m/z.*<br>$[M+H]^+$ 524.2; $C_{26}H_{21}F_4N_7O$ | 40 | 1H NMR (400 MHz, DMSO-d6) δ ppm 8.45 - 8.56 (m, 1 H) 7.60 - 7.98 (m, 3 H) 7.49 - 7.58 (m, 1 H) 7.14 - 7.27 (m, 2 H) 6.83 - 6.91 (m, 1 H) 6.58 (br s, 2 H) 4.77 - 5.97 (m, 1 H) 3.52 (dq, J=13.57, 6.82 Hz, 1 H) 3.08 - 3.18 (m, 1 H) 2.19 (s, 3 H) 1.53 - 1.70 (m, 3 H) 0.63 - 1.14 (m, 3 H)<br>LC-MS: Rt = 2.357 min ,<br>(ESI) m/z = 392.2 [M+H]+;<br>C25H24F3N7O |
| 41 | 1H NMR (400 MHz, DMSO-d6) δ = 9.23 (s, 1H), 8.15 - 7.59 (m, 4H), 7.47 - 7.47 (m, 1H), 7.58 - 7.43 (m, 1H), 6.59 (s, 2H), 5.49 - 5.12 (m, 3H), 4.92 - 4.62 (m, 2H), 3.56 (br s, 4H), 3.29 - 3.07 (m, 3H), 1.39 (br d, J = 5.7 Hz, 3H) LC-MS: Rt = 2.585 min ,<br>(ESI) m/z = 500.1 [M+H]+;<br>C25H24F3N5O3 | 42 | 1H NMR (400MHz, DMSO-d6) δ 9.19 (s, 1H), 8.04 (s, 1H), 7.74 (br d, J=9.4 Hz, 1H), 7.58 (br s, 2H), 7.47 (br dd, J=1.2, 9.4 Hz, 2H), 7.43 - 7.11 (m, 2H), 6.61 (s, 2H), 5.45 - 5.35 (m, 1H), 5.28 - 5.20 (m, 1H), 5.18 - 5.11 (m, 1H), 5.05 (s, 2H), 3.67 (br s, 2H), 1.39 (d, J=6.1 Hz, 3H) ; LC-MS: Rt = 1.714 min, (ESI) m/z. [M+H]+ 522.1;<br>C26H22F3N7O2 |

(continued)

| | HNMR/LCMS | | HNMR/LCMS |
|---|---|---|---|
| 43 | 1H NMR (400 MHz, DMSO-d6) δ = 9.21 (s, 1H), 8.03 (s, 1H), 7.74 (d, J = 9.4 Hz, 1H), 7.66 (br s, 1H), 7.54 (br s, 2H), 7.47 (br d, J = 9.4 Hz, 1H), 6.59 (s, 2H), 5.39 (br d, J = 4.3 Hz, 1H), 5.32 - 5.04 (m, 2H), 4.71 (br s, 2H), 2.29 (br s, 1H), 1.88 (br s, 6H), 1.39 (br d, J = 6.1 Hz, 3H) LC-MS: Rt = 2.758 min, (ESI) m/z. [M+H]+ 508.1; C27H24F3N5O2 | 44 | 1H NMR (400 MHz, DMSO-d6) δ = 9.21 (br s, 1H), 8.04 (br s, 1H), 7.91 - 7.38 (m, 5H), 6.58 (br s, 2H), 5.58 - 4.98 (m, 3H), 4.85 - 4.52 (m, 2H), 4.46 - 4.05 (m, 1H), 3.69 - 3.39 (m, 2H), 3.25 - 2.98 (m, 3H), 1.40 (br s, 3H), 1.13 (br s, 3H) LC-MS: Rt = 2.600 min, (ESI) m/z. [M+H]+ 514.3; C26H26F3N5O3 |
| 45 | 1H NMR (400 MHz, DMSO-d6) δ 8.09-8.63 (m, 3H), 7.75-7.87 (m, 1H), 7.45-7.57 (m, 1H), 7.09-7.40 (m, 4H), 6.74-6.97 (m, 1H), 4.49-5.01 (m, 2H), 3.83-4.47 (m, 4H), 3.55-3.80 (m, 1H), 2.93-3.16 (m, 3H), 2.540-2.62 (m, 1.5H), 1.92-2.43 (m, 2.5H). LC-MS: Rt =1.342 min, (ESI) m/z. [M+H]+ 474.2; C25H24FN7O2 | 46 | 1H NMR (400 MHz, DMSO-d6) δ = 8.53 (d, J = 6.8 Hz, 1H), 7.92 (s, 2H), 7.72 - 7.51 (m, 3H), 7.31 - 7.19 (m, 1H), 6.90 (t, J = 6.6 Hz, 1H), 6.65 - 6.52 (m, 2H), 5.41 (br s, 1H), 5.34 - 5.10 (m, 2H), 4.80 - 4.51 (m, 2H), 3.45 (br d, J = 4.6 Hz, 2H), 1.39 (br d, J = 5.9 Hz, 3H), 1.12 (t, J = 7.1 Hz, 3H) LC-MS: Rt = 1.405 min, (ESI) *m/z*. $[M+H]^+$ 402.2; $C_{23}H_{23}N_5O_2$<br>$C_{26}H_{22}F_3N_7O_2$ |
| 49 | 1H NMR (400MHz, DMSO-d6) δ 9.18 (s, 1H), 8.03 (s, 1H), 7.73 (d, J=9.4 Hz, 1H), 7.58 (br s, 2H), 7.46 (br dd, J=1.4, 9.5 Hz, 2H), 7.42 - 7.00 (m, 2H), 6.59 (s, 2H), 5.43 - 5.34 (m, 1H), 5.28 - 5.19 (m, 1H), 5.17 - 5.11 (m, 1H), 5.05 (s, 2H), 3.66 (br s, 3H), 1.39 (d, J=6.1 Hz, 3H) ; LC-MS: Rt = 1.738 min, (ESI) m/z. [M+H]+ 522.0; C26H22F3N7O2 | 50 | 1H NMR (400 MHz, DMSO-d6) δ = 9.22 (s, 1H), 8.10 - 7.92 (m, 2H), 7.77 (br d, J = 8.3 Hz, 1H), 7.67 (br s, 1H), 7.57 - 7.54 (m, 1H), 7.49 (br d, J = 8.0 Hz, 1H), 6.58 (s, 2H), 5.41 (br s, 1H), 5.23 (br dd, J = 1.8, 19.9 Hz, 2H), 4.81 - 4.61 (m, 2H), 1.39 (br d, J = 6.1 Hz, 3H) LC-MS: Rt = 2.457 min, (ESI) m/z. [M+H]+ 459.2; C23H17D3F3N5O2 |
| 51 | 1H NMR (400 MHz, DMSO-d6) δ ppm 9.23 (s, 1 H) 8.06 (s, 1 H) 7.27 - 7.96 (m, 5 H) 6.59 (s, 2 H) 5.42 (br s, 1 H) 5.26 (br s, 2 H) 4.57 - 4.85 (m, 2 H) 3.39 - 3.46 (m, 2 H) 1.40 (br d, J=6.13 Hz, 3 H) 1.13 (t, J=7.00 Hz, 3 H)<br>LC-MS: Rt = 2.562 min ,<br>(ESI) m/z = 470.2 [M+H]+;<br>C24H22F3N5O | 52 | 1H NMR (400 MHz, DMSO-d6) δ ppm 9.21 (br s, 1 H) 7.87 - 8.02 (m, 1 H) 7.77 (d, J=9.38 Hz, 1 H) 7.64 (br s, 1 H) 7.43 - 7.61 (m, 3 H) 6.58 (br s, 2 H) 5.41 (br s, 1 H) 5.24 (br d, J=17.76 Hz, 2 H) 4.55 - 4.87 (m, 2 H) 3.27 (br s, 2 H) 1.90 - 2.20 (m, 1 H) 1.40 (br d, J=5.88 Hz, 3 H) 0.65 - 0.94 (m, 6 H)<br>LC-MS: Rt = 2.769 min ,<br>(ESI) *m/z* = 498.2 [M+H]+;<br>$C_{26}H_{26}F_3N_5O_2$ |
| 53 | 1H NMR (400 MHz, DMSO-d6) δ ppm 8.52 (br d, J=6.38 Hz, 1 H) 7.77 - 8.01 (m, 2 H) 7.67 (br s, 1 H) 7.55 (br d, J=9.13 Hz, 2 H) 7.25 (br t, J=7.75 Hz, 1 H) 6.89 (td, J=6.75, 1.13 Hz, 1 H) 6.57 (br s, 2 H) 5.13 - 5.47 (m, 3 H) 4.52 - 4.81 (m, 2 H) 3.30 (br d, J=3.50 Hz, 2 H) 2.10 (br d, J=11.63 Hz, 1 H) 1.40 (br d, J=6.00 Hz, 3 H) 0.60 - 0.97 (m, 6 H)<br>LC-MS: Rt = 2.428 min ,<br>(ESI) *m/z* = 430.2 [M+H]+;<br>$C_{25}H_{27}N_5O_2$ | 54 | 1H NMR (400 MHz, DMSO-d6) δ ppm 8.52 (br d, J=6.38 Hz, 1 H) 7.72 - 8.09 (m, 2 H) 7.45 - 7.71 (m, 3 H) 7.25 (br t, J=7.75 Hz, 1 H) 6.84 - 6.93 (m, 1 H) 6.57 (br s, 2 H) 5.12 - 5.48 (m, 3 H) 4.51 - 4.81 (m, 2 H) 3.14 - 3.31 (m, 2 H) 2.07 (br s, 1 H) 1.40 (br d, J=5.88 Hz, 3 H) 0.58 - 0.97 (m, 6 H)<br>LC-MS: Rt = 2.429 min,<br>(ESI) m/z = 430.2 [M+H]+;<br>$C_{25}H_{27}N_5O_2$ |

(continued)

| | HNMR/LCMS | | HNMR/LCMS |
|---|---|---|---|
| 55 | 1H NMR (400 MHz, DMSO-d6) δ ppm 8.52 (br d, J=6.38 Hz, 1 H) 7.80 - 8.04 (m, 2 H) 7.42 - 7.73 (m, 3 H) 7.25 (br t, J=7.82 Hz, 1 H) 6.89 (td, J=6.75, 1.00 Hz, 1 H) 6.57 (br s, 2 H) 5.11 - 5.47 (m, 3 H) 4.50 - 4.84 (m, 2 H) 3.28 (br s, 2 H) 2.08 (br s, 1 H) 1.40 (br d, J=5.88 Hz, 3 H) 0.63 - 0.98 (m, 6 H) LC-MS: Rt = 2.430 min, (ESI) m/z = 430.2 [M+H]+; C25H27N5O2 | 56 | 1H NMR (400 MHz, DMSO-d6) δ = 8.53 (d, J = 6.8 Hz, 1H), 7.88 (s, 1H), 7.70 (br s, 1H), 7.60 - 7.51 (m, 3H), 7.26 - 7.20 (m, 1H), 6.89 (dt, J = 0.9, 6.8 Hz, 1H), 6.57 (s, 2H), 5.41 (br dd, J = 4.4, 8.6 Hz, 1H), 5.26 - 5.09 (m, 2H), 4.67 (br s, 2H), 2.30 (br s, 1H), 1.91 (br s, 6H), 1.39 (d, J = 6.1 Hz, 3H) LC-MS: Rt = 1.649 min, (ESI) m/z. [M+H]+ 440.2; C26H25N5O2 |
| 57 | 1H NMR (400 MHz, DMSO-d6) δ = 8.49 (d, J = 6.9 Hz, 1H), 8.14 (s, 1H), 7.88 (s, 1H), 7.73 - 7.36 (m, 4H), 7.35 - 6.99 (m, 2H), 6.87 (dt, J = 1.0, 6.8 Hz, 1H), 6.60 (s, 2H), 5.39 (ddd, J = 1.6, 4.0, 5.9 Hz, 1H), 5.27 - 5.18 (m, 1H), 5.17 - 5.08 (m, 1H), 5.00 (s, 2H), 3.66 (br s, 3H), 1.38 (d, J = 6.1 Hz, 3H) LC-MS: Rt = 2.044 min, (ESI) *m/z.* $[M+H]^+$ 454.2; $C_{25}H_{23}N_7O_2$ | 58 | 1H NMR (400MHz, DMSO-d6) δ 8.53 - 7.97 (m, 3H), 7.53 (br d, J=9.1 Hz, 1H), 7.38 - 7.17 (m, 4H), 7.10 - 6.87 (m, 1H), 4.94 - 4.47 (m, 2H), 4.39 - 4.20 (m, 3H), 3.35 - 3.31 (m, 2H), 2.26 - 1.94 (m, 1H), 1.06 - 0.62 (m, 6H). LC-MS: RT =2.439 min, (ESI) *m/z.* $[M+H]^+$ 464.2; $C_{24}H_{23}F_2N_7O$ |
| 59 | 1H NMR (400 MHz, DMSO-d6) δ 8.11-8.58 (m, 3H), 7.85 (s, 1H), 7.51 (d, J=9.02 Hz, 1H), 7.18-7.39 (m, 4H), 6.81-6.95 (m, 1H), 3.85-5.07 (m, 5H), 1.85-2.49 (m, 6H). 19F NMR (376.5 MHz, DMSO-d6) δ -116.31,-116.95,-166.45,-170.46. LC-MS: RT =2.690 min, (ESI) *m/z.* $[M+H]^+$ 474.2; $C_{25}H_{21}F_2N_7O$ | 60 | 1H NMR (400 MHz, DMSO-d6) δ 8.40-8.85 (m, 1.7 H), 8.19-8.29 (m, 1.3H), 7.79-8.05 (m, 1H), 7.54 (d, J=9.02 Hz, 1H), 7.08-7.46 (m, 4H), 6.89 (t, J=6.60 Hz, 1H), 5.75-5.86 (m, 0.15H), 5.01-5.12 (m, 0.85H), 4.27-4.47 (m, 3H), 2.77-3.46 (m, 7H), 1.43-1.76 (m, 3H). 19F NMR (376.5 MHz, DMSO-d6) δ -110.40, -112.23, -111.62. LC-MS: RT =1.372 min, (ESI) *m/z.* $[M+H]^+$ 462.2; $C_{24}H_{2470}FN_7O_2$ |
| 61 | 1H NMR (400 MHz, DMSO-d6) δ 8.13-8.58 (m, 3H), 7.84 (s, 1H), 7.51 (d, J=9.02 Hz, 1H), 7.17-7.41 (m, 4H), 6.89 (t, J=6.60 Hz, 1H), 3.95-5.01 (m, 5H), 2.52-2.71 (m, 3H), 1.97-2.46 (m, 3H). 19F NMR (376.5 MHz, DMSO-d6) δ -116.32, -117.13. LC-MS: RT =2.442 min, (ESI) *m/z.* $[M+H]^+$ 481.2; $C_{26}H_{21}FN_8O$ | 62 | 1H NMR (400 MHz, DMSO-d6) δ 8.04-8.64 (m, 3H), 7.87 (s, 1H), 7.52 (d, J=9.24 Hz, 1H), 7.08-7.45 (m, 4H), 6.81-6.96 (m, 1H), 3.79-5.12 (m, 5H), 1.76-2.47 (m, 6H). 19F NMR (376.5 MHz, DMSO-d6) δ -64.86, -111.54, -112.23. LC-MS: Rt =1.697 min, (ESI) *m/z.* $[M+H]^+$ 524.2; $C_{26}H_{21}F_4N_7O$ |
| 63 | 1H NMR (400 MHz, DMSO-d6) δ 8.59 (br s, 1H), 8.51 (d, J=6.82 Hz, 1H), 8.22 (s, 1H), 8.00 (s, 1H), 7.65-7.73 (m, 1H), 7.59 (d, J=8.36 Hz, 1H), 7.54 (d, J=9.02 Hz, 1H), 7.20-7.27 (m, 1H), 7.09 (br s, 2H), 6.88 (t, J=6.82 Hz, 1H), 5.01-5.36 (m, 1H), 4.51-4.68 (m, 1H), 4.35-4.52 (m, 1H), 4.26 (s, 3H), 3.80 (dd, J=3.74, 11.88 Hz, 1H), 3.58-3.74 (m, 2H), 0.95 (d, J=7.04 Hz, 3H). LC-MS: Rt =1.284 min, (ESI) *m/z.* $[M+H]^+$ 442.2; $C_{24}H_{23}N_7O_2$ | 64 | 1H NMR (400 MHz, DMSO-d6) δ 8.45-8.59 (m, 1H), 7.95 (s, 1H), 7.81-7.99 (m, 1H), 7.66-7.74(m, 1H), 7.49-7.59 (m, 2H), 7.19-7.25 (m, 1H), 6.84-6.90 (t, J=6.71 Hz, 1H), 6.55 (s, 2H), 4.82-5.58 (m, 4H), 4.51-4.72 (m, 1H), 4.10-4.46 (m, 1H), 3.76-3.83 (m, 1H), 3.62-3.71 (m, 2H), 1.21-1.40 (m, 3H), 0.87-0.99 (m, 3H). LC-MS: Rt =1.337 min, (ESI) *m/z.* $[M+H]^+$ 444.3; $C_{25}H_{25}N_5O_3$ |

(continued)

| | HNMR/LCMS | | HNMR/LCMS |
|---|---|---|---|
| 65 | 1H NMR (400 MHz, DMSO-d6) δ = 8.41 (t, J = 5.9 Hz, 1H), 7.73 - 7.63 (m, 2H), 7.55 (s, 2H), 7.49 (d, J = 9.2 Hz, 1H), 7.23 - 7.18 (m, 1H), 6.83 (t, J = 6.7 Hz, 1H), 6.31 (s, 2H), 5.46 - 5.37 (m, 1H), 5.28 - 5.02 (m, 3H), 4.91 - 4.82 (m, 1H), 4.59 (br d, J = 16.6 Hz, 1H), 1.49 (br d, J = 6.4 Hz, 3H), 1.41 (dd, J = 4.1, 6.1 Hz, 3H) LC-MS: Rt = 1.660 min, (ESI) *m/z.* $[M+H]^+$ 470.1; $C_{24}H_{22}F_3N_5O_2$ | 66 | 1H NMR (400 MHz, DMSO-d6) δ = 8.53 (d, J = 6.8 Hz, 1H), 8.07 (br s, 1H), 7.91 (s, 1H), 7.77 - 7.64 (m, 1H), 7.60 - 7.54 (m, 2H), 7.27 (br t, J = 7.8 Hz, 1H), 6.91 (t, J = 6.3 Hz, 1H), 6.61 (s, 2H), 6.49 - 6.13 (m, 1H), 5.45 - 5.37 (m, 1H), 5.32 - 5.13 (m, 2H), 4.70 (br s, 2H), 3.85 (br d, J = 3.8 Hz, 2H), 1.39 (d, J = 6.1 Hz, 3H) LC-MS: Rt = 2.340 min, (ESI) *m/z.* $[M+H]^+$ 438.2; $C_{23}H_{21}F_2N_5O_2$ |
| 67 | 1H NMR (400 MHz, DMSO-d6) δ 9.19 (s, 1H), 8.10 (s, 1H), 7.79-8.04 (m, 1H), 7.77 (dd, J=4.39, 9.41 Hz, 1H), 7.65-7.73 (m, 1H), 7.55 (d, J=8.28 Hz, 1H), 7.44-7.50 (m, 1H), 6.57 (s, 2H), 5.06-5.54 (m, 4H), 4.01-4.82 (m, 2H), 3.78-3.92 (m, 1H), 3.57-3.77 (m, 2H), 1.33-1.42 (m, 3H), 0.87-0.93 (m 3H). 19F NMR (376.5 MHz, DMSO-d6) δ -60.43. LC-MS: Rt =1.653 min, (ESI) *m/z.* $[M+H]^+$ 512.2;<br>$C_{26}H_{24}F_3N_5O_3$ | 68 | 1H NMR (400 MHz, DMSO-d6) δ = 9.12 (s, 1H), 8.01 (s, 1H), 7.70 (d, J = 9.4 Hz, 1H), 7.62 (s, 2H), 7.56 - 7.49 (m, 1H), 7.48 - 7.37 (m, 2H), 7.22 (s, 1H), 6.40 (s, 2H), 5.21 (t, J = 3.1 Hz, 2H), 5.06 (s, 2H), 5.01 (t, J = 3.0 Hz, 2H), 3.67 (s, 3H) LC-MS: Rt = 2.381 min, (ESI) *m/z.* $[M+H]^+$ 508.2; $C_{25}H_{20}F_3N_7O_2$ |
| 69 | 1H NMR (400 MHz, DMSO-d6) δ 8.65 (d, J=6.16 Hz, 1H), 8.49 (br d, J=6.60 Hz, 1H), 8.30 (s, 1H), 7.84-7.93 (m, 2H), 7.58 (br d, J=17.83 Hz, 2H), 7.47-7.53 (m, 1H), 7.20-7.26 (m, 1H), 6.88 (q, J=7.41 Hz, 1H), 4.78 (br d, J=6.16 Hz, 2H), 3.46-3.62 (m, 2H), 2.88-3.00 (m, 3H), 1.15-1.25 (m, 3H) LC-MS: Rt =1.640 min, (ESI) *m/z.* $[M+H]^+$ 401.2;<br>$C_{21}H_{20}N_8O$ | 70 | 1H NMR (400 MHz, DMSO-d6) δ 9.23 (br s, 1H), 8.66 (br s, 1H), 8.45 (br s, 1H), 7.97-8.15 (m, 2H), 7.84-7.88 (m, 1H), 7.70 (br d, J=14.30 Hz, 2H), 7.57 (br s, 2H), 7.45 (br s, 1H), 7.11 (br s, 1H), 5.05-5.30 (m, 2H), 3.56-3.88 (m, 3H), 2.91 (s, 3H). LC-MS: Rt =1.484 min, (ESI) *m/z.* $[M+H]^+$ 521.3;<br>$C_{24}H_{19}F_3N_{10}O$ |
| 71 | 1H NMR (400 MHz, DMSO-d6) δ = 8.78 (s, 1H), 7.70 (br d, J = 9.7 Hz, 1H), 7.60 (s, 2H), 7.52 - 7.47 (m, 1H), 7.45 - 7.39 (m, 2H), 7.20 (s, 1H), 6.31 (s, 2H), 5.42 (ddd, J = 1.7, 4.1, 6.0 Hz, 1H), 5.29 - 5.21 (m, 1H), 5.17 - 5.11 (m, 1H), 5.08 (s, 2H), 3.67 (s, 3H), 1.42 (d, J = 6.2 Hz, 3H) LC-MS: Rt = 2.486 min,<br>(ESI) *m/z* = 540.2 [M+H]+;<br>$C_{26}H_{21}F_4N_7O_2$ | 72 | 1H NMR (400MHz, DMSO-d6) δ 7.98 - 7.64 (m, 3H), 7.64 - 7.45 (m, 3H), 7.45 - 7.04 (m, 3H), 6.61 (s, 2H), 5.44 - 5.35 (m, 1H), 5.27 - 5.18 (m, 1H), 5.18 - 5.11 (m, 1H), 5.08 (s, 2H), 3.67 (br s, 3H), 1.41 - 1.36 (m, 3H) ; LC-MS: Rt = 1.826 min, (ESI) m/z. $[M+H]^+$ 522.2; $C_{26}H_{22}F_3N_7O_2$ |
| 73 | 1H NMR (400MHz, DMSO-d6) δ 7.97 - 7.66 (m, 3H), 7.65 - 7.45 (m, 3H), 7.44 - 6.99 (m, 3H), 6.60 (s, 2H), 5.43 - 5.34 (m, 1H), 5.27 - 5.17 (m, 1H), 5.17 - 5.09 (m, 1H), 5.07 (s, 2H), 3.73 - 3.54 (m, 3H), 1.38 (d, J=6.1 Hz, 3H) ; LC-MS: Rt = 1.828 min, (ESI) m/z. $[M+H]^+$ 522.2; $C_{26}H_{22}F_3N_7O$ | 74 | 1H NMR (400 MHz, DMSO-d6) δ = 8.04 - 7.81 (m, 3H), 7.69 - 7.31 (m, 6H), 6.62 (s, 2H), 5.43 - 5.35 (m, 1H), 5.26 - 5.11 (m, 2H), 5.08 (s, 2H), 3.66 (br s, 3H), 1.38 (d, J = 6.1 Hz, 3H) LC-MS: Rt = 2.352 min, (ESI) *m/z.* $[M+H]^+$ 522.2; $C_{26}H_{22}F_3N_7O_2$ |
| 75 | 1H NMR (400 MHz, DMSO-d6) δ = 8.60 (s, 1H), 8.36 - 7.99 (m, 4H), 7.95 (d, J = 9.1 Hz, 2H), 7.65 (br d, J = 9.3 Hz, 2H), 6.01 - 5.93 (m, 1H), 5.89 (br s, 2H), 5.83 (br d, J = 14.1 Hz, 1H), 5.74 - 5.67 (m, 1H), 5.58 (br s, 2H), 4.20 (br s, 3H), 2.84 (s, 3H), 2.00 (d, J = 6.3 Hz, 3H)LC-MS: Rt = 2.165 min, (ESI) *m/z.* $[M+H]^+$ 468.2; $C_{26}H_{25}N_7O_2$ | 76 | 1H NMR (400MHz, DMSO-d6) δ 9.18 (s, 1H), 8.47 (br s, 1H), 8.33 - 8.14 (m, 1H), 8.08 (s, 1H), 7.87 - 7.68 (m, 5H), 7.47 (br d, J=8.4 Hz, 1H), 5.09 (s, 2H), 3.68 (br s, 3H), 2.71 (s, 3H). 19F NMR (376MHz, DMSO-d6) δ -60.427. LC-MS: Rt =3.535 min, (ESI) *m/z.* $[M+H]^+$ 521.3; $C_{24}H_{19}F_3N_{10}O$ |

(continued)

| | HNMR/LCMS | | HNMR/LCMS |
|---|---|---|---|
| 77 | (400MHz, DMSO-d6) δ 8.77 - 8.46 (m, 3H), 7.98 (s, 1H), 7.88 - 7.65 (m, 3H), 7.55 (br d, J=9.0 Hz, 1H), 7.28 (br s, 1H), 6.91 (br t, J=6.5 Hz, 1H), 4.63 (br s, 2H), 3.73 - 3.51 (m, 2H), 3.03 - 2.62 (m, 3H), 1.25 - 1.06 (m, 3H). LC-MS: Rt =2.973 min, (ESI) *m/z.* $[M+H]^+$ 401.2; $C_{21}H_{20}N_8O$ | 78 | 1H NMR (400 MHz, DMSO-d6) δ = 8.86 (s, 1H), 8.01 (s, 1H), 7.73 - 7.31 (m, 6H), 7.28 - 6.93 (m, 2H), 6.60 (s, 2H), 5.46 - 5.35 (m, 1H), 5.27 - 5.19 (m, 1H), 5.18 - 5.10 (m, 1H), 5.03 (s, 2H), 3.66 (br s, 3H), 1.38 (d, J = 6.1 Hz, 3H) LC-MS: Rt = 2.254 min, (ESI) *m/z* = 504.0 [M+H]+; $C_{26}H_{23}F_2N_7O_2$ |
| 79 | 1H NMR (400MHz, DMSO-d6) δ = 8.36 (s, 1H), 7.78 (s, 1H), 7.55 (s, 1H), 7.29 - 6.91 (m, 1H), 6.69 - 6.51 (m, 1H), 5.75 (s, 1H), 5.40 (s, 1H), 4.45 (s, 1H), 4.15 (s, 1H), 3.86 (br s, 2H), 3.16 (br d, J=4.4 Hz, 2H), 2.03 (s, 2H), 1.62 (s, 2H), 1.39 (br s, 3H), 1.11 (s, 3H). LC-MS: Rt = 1.702 min, (ESI) *m/z.* $[M+H]^+$ 502.2; $C_{28}H_{31}N_5O_4$ | 80 | 1H NMR (400MHz, DMSO-d6) δ 9.28 - 9.12 (m, 1H), 8.16 - 7.86 (m, 1H), 7.78 - 7.50 (m, 3H), 7.46 (br d, J=9.7 Hz, 1H), 7.38 - 7.02 (m, 2H), 6.81 (br s, 2H), 5.16 (br d, J=2.2 Hz, 2H), 5.10 - 4.88 (m, 4H), 3.79 (s, 3H). 19F NMR (376MHz, DMSO-d6) δ -60.408, -60.457; -113.851, -115.468. LC-MS: Rt =4.112 (ESI) *m/z.* $[M+H]^+$ 526.3 |
| 81 | $^1$H NMR (400 MHz, DMSO-d6) δ 8.51 (br s, 1H), 7.82-8.05 (m, 1H), 7.44-7.75 (m, 1H), 7.15-7.39 (m, 2H), 6.68-6.94 (m, 3H), 5.24-5.73 (m, 2H), 4.97 (br s, 3H), 4.37-4.80 (m, 2H), 3.49-3.91 (m, 4H), 0.93 (br s, 3H). 19F NMR (376 MHz, DMSO-d6) δ -116.33 (br s, 1F). LC-MS: Rt =1.395 min, (ESI) *m/z.* $[M+H]^+$ 448.2; $C_{24}H_{22}FN_5O_3$ | 82 | 1H NMR (400MHz, DMSO-d6) δ 8.59 - 8.41 (m, 1H), 8.15 - 7.81 (m, 1H), 7.81 - 7.44 (m, 3H), 7.36 - 7.01 (m, 3H), 6.93 - 6.63 (m, 3H), 5.13 (td, J=1.5, 3.0 Hz, 2H), 5.06 - 4.85 (m, 4H), 3.85 - 3.50 (m, 3H).19F NMR (376MHz, DMSO-d6) δ -113.938, -115.488. LC-MS: Rt =3.269 min, (ESI) *m/z.* $[M+H]^+$ 458.2; $C_{24}H_{20}FN_7O_2$ |
| 83 | 1H NMR (400MHz, DMSO-d6) δ 8.32 - 7.93 (m, 1H), 7.68 - 7.43 (m, 3H), 7.39 - 7.17 (m, 1H), 7.16 - 6.93 (m, 3H), 6.82 (br s, 2H), 5.28 (br s, 2H), 5.09 - 4.93 (m, 4H), 3.86 - 3.52 (m, 3H). 19F NMR (376MHz, DMSO-d6) δ -113.863,-114.252, -115.681, -115.827; -158.023. LC-MS: Rt =0.809 min, (ESI) *m/z.* $[M+H]^+$ 476.3; $C_{24}H_{19}F_2N_7O_2$ | 84 | 1H NMR (400MHz, DMSO-d6) δ 9.00 - 8.77 (m, 1H), 7.72 (br d, J=9.5 Hz, 1H), 7.65 - 7.53 (m, 1H), 7.47 (br d, J=9.9 Hz, 2H), 7.38 - 7.01 (m, 2H), 6.79 (br s, 2H), 5.33 - 5.20 (m, 2H), 5.14 - 4.86 (m, 4H), 3.82 - 3.53 (m, 3H). 19F NMR (376MHz, DMSO-d6) δ -60.560, -61.047, -61.162; -113.827, -115.669; -154.764. LC-MS: Rt =4.270 min, (ESI) *m/z.* $[M+H]^+$ 544.2; $C_{25}H_{15}F_5N_7O_2$ |
| 85 | 1H NMR (400 MHz, DMSO-d6) δ 9.13-9.35 (m, 1H), 7.87-8.20 (m, 1H), 7.68-7.84 (m, 1H), 7.43-7.58 (m, 1H), 7.30 (dd, J=11.88, 18.51 Hz, 1H), 6.81 (br d, J=11.63 Hz, 1H), 5.04-5.42 (m, 2H), 4.44-5.04 (m, 4H), 3.74 (s, 1H), 3.51 (br s, 2H), 3.03 (s, 1H), 1.00-1.19 (m, 3H). 19F NMR (376 MHz, DMSO-d6) δ -60.54--60.31 (m, 3F), -115.78 (br d, J=15.71 Hz, 1F). LC-MS: Rt =1.659 min, (ESI) *m/z.* $[M+H]^+$ 474.2; $C_{23}H_{19}F_4N_5O_2$ | 86 | 1H NMR (400 MHz, DMSO-d6) δ 8.46 - 8.57 (m, 1 H) 7.84 - 7.91 (m, 1 H) 7.47 - 7.81 (m, 2 H) 7.19 - 7.35 (m, 2 H) 6.78 (br s, 3 H) 5.14 - 5.37 (m, 2 H) 4.74 - 5.03 (m, 3 H) 4.47 (s, 1 H) 3.51 (br d, J=6.38 Hz, 2 H) 1.01 - 1.16 (m, 3 H). 19F NMR (376 MHz, DMSO-d6) δ -115.78 (br d, J=11.45 Hz, 1 F). LC-MS: Rt =0.721 min, (ESI) *m/z.* $[M+H]^+$ 406.2; $C_{22}H_{20}FN_5O_2$ |
| 87 | 1H NMR (400 MHz, DMSO-d6) δ 7.96 - 8.37 (m, 2 H) 7.70 - 7.81 (m, 1 H) 7.48 - 7.69 (m, 2 H) 7.27 - 7.46 (m, 1 H) 6.99 - 7.13 (m, 2 H) 6.70 - 6.89 (m, 2 H) 5.06 - 5.37 (m, 2 H) 4.81 - 5.04 (m, 4 H) 3.51 - 3.82 (m, 3 H) 2.17 - 2.29 (m, 3 H). 19F NMR (376 MHz, DMSO-d6) δ -116.26 - -113.01 (m, 1 F). LC-MS: Rt =0.754 min, (ESI) *m/z.* $[M+H]^+$ 472.4; $C_{25}H_{22}FN_7O_2$ | 88 | 1H NMR (400 MHz, DMSO-d6) δ 9.20 (br s, 1H), 7.09-8.20 (m, 6H), 6.89 (br s, 2H), 5.19 (br s, 4H), 4.97 (br s, 2H), 3.98 (br s, 3H). 19F NMR (376 MHz, DMSO-d6) δ -60.46 (br s, 1F), -114.10 (br s, 1F). LC-MS: Rt =1.655 min, (ESI) *m/z.* $[M+H]^+$ 527.2; $C_{24}H_{15}F_4N_8O_2$ |

(continued)

| | HNMR/LCMS | | HNMR/LCMS |
|---|---|---|---|
| 89 | 1H NMR (400 MHz, DMSO-d6) δ 9.22 (s, 1H), 8.06 (br s, 1H), 7.72 (br d, J=9.46 Hz, 1H), 7.60 (s, 1H), 7.23-7.57 (m, 4H), 6.72 (s, 2H), 5.20 (br s, 2H), 4.99 (br d, J=2.86 Hz, 4H), 3.60 (s, 3H), 1.65 (br s, 3H). 19F NMR (376 MHz, DMSO-d6) δ -60.41 (br s, 1F). LC-MS: Rt =0.781 min, (ESI) *m/z.* $[M+H]^+$ 522.3; $C_{26}H_{22}F_3N_7O_2$ | 90 | 1H NMR (400 MHz, DMSO-d6) δ9.22 (s, 1H), 8.06 (br s, 1H), 7.72 (br d, J=9.24 Hz, 1H), 7.60 (s, 1H), 7.13-7.55 (m, 4H), 6.63 (s, 2H), 5.39 (br d, J=4.84 Hz, 1H), 5.07-5.31 (m, 2H), 5.00 (br s, 2H), 3.60 (s, 3H), 1.67 (br s, 3H), 1.39 (d, J=6.16 Hz, 3H). 19F NMR (376 MHz, DMSO-d6) δ -60.41 (br s, 1F). LC-MS: Rt =0.802 min, (ESI) *m/z.* $[M+H]^+$ 536.3; $C_{27}H_{24}F_3N_7O_2$ |
| 91 | 1H NMR (400 MHz, DMSO-d6) δ 8.88 (s, 1H), 8.17 (s, 1H), 6.96-7.83 (m, 7H), 6.70 (s, 2H), 5.22 (s, 2H), 5.06 (s, 2H), 4.98 (s, 2H), 3.65 (s, 3H). 19F NMR (376.5 MHz, DMSO-d6) δ -60.57, -155.01. LC-MS: Rt =1.545 min, (ESI) *m/z.* $[M+H]^+$ 526.3; $C_{25}H_{19}F_4N_7O_2$ | 92 | 1H NMR (400 MHz, DMSO-d6) δ 8.25 (d, J=6.82 Hz, 1H), 7.31-7.89 (m, 5H), 7.02-7.30 (m, 2H), 6.98 (t, J=6.71 Hz, 1H), 6.69 (s, 2H), 5.18-5.27 (m, 2H), 5.03 (s, 2H), 4.98 (m, 2H), 3.66 (s, 3H). 19F NMR (376.5 MHz, DMSO-d6) δ -158.28. LC-MS: Rt =1.300 min, (ESI) *m/z.* $[M+H]^+$ 458.3; $C_{24}H_{20}FN_7O_2$ |
| 93 | 1H NMR (400 MHz, DMSO-d6) δ 8.90 (s, 1H), 7.71 (br d, J=9.38 Hz, 1H), 7.61 (s, 1H), 7.30-7.49 (m, 4H), 6.71 (s, 2H), 5.19 (br s, 2H), 4.93-5.06 (m, 4H), 3.61 (s, 3H), 1.66 (br s, 3H). 19F NMR (376 MHz, DMSO-d6) δ -60.91--60.37 (m, 3F), -154.84 (br s, 1F). LC-MS: Rt =1.570 min, (ESI) *m/z.* $[M+H]^+$ 540.4; $C_{26}H_{21}F_4N_7O_2$ | 94 | 1H NMR (400 MHz, DMSO-d6) δ 8.92-9.41 (m, 1H), 7.11-8.15 (m, 5H), 6.68-6.99 (m, 2H), 4.27-5.42 (m, 7H), 3.42-4.06 (m, 4H), 2.08 (br d, J=7.26 Hz, 2H). 19F NMR (376 MHz, DMSO-d6) δ -64.04--58.45, -116.36. LC-MS: Rt =0.928 MIN, 1.033 min, (ESI) *m/z.* $[M+H]^+$ 516.2; $C_{25}H_{21}F_4N_5O_3$ |
| 95 | 1H NMR (400 MHz, DMSO-d6) δ 9.21 (br s, 1H), 8.05 (br s, 1H), 7.71 (br d, J=9.24 Hz, 1H), 7.63 (s, 1H), 7.30-7.54 (m, 4H), 6.71 (br s, 2H), 5.18 (br s, 2H), 4.90-5.08 (m, 4H), 3.87 (q, J=6.68 Hz, 2H), 1.64 (br s, 3H), 1.16 (br t, J=6.49 Hz, 3H). 19F NMR (376 MHz, DMSO-d6) δ -60.42. LC-MS: Rt =0.942 min, (ESI) *m/z.* $[M+H]^+$ 536.2; $C_{27}H_{24}F_3N_7O_2$ | 96 | 1H NMR (400 MHz, DMSO-d6) 8.25 (d, J=6.88 Hz, 1H), 7.62 (s, 1H), 7.32-7.55 (m, 4H), 7.19-7.27 (m, 1H), 6.98 (t, J=6.73 Hz, 1H), 6.70 (s, 2H), 5.20 (br s, 2H), 4.98 (br s, 4H), 3.61 (s, 3H), 1.64 (br s, 3H). 19F NMR (376 MHz, DMSO-d6) -158.13 (br s, 1F). LC-MS: Rt =0.801 min, (ESI) m/z. $[M+H]^+$ 472.1; $C_{25}H_{22}FN_7O_2$ |
| 97 | 1H NMR (400 MHz, DMSO-d6) δ 9.22 (s, 1H), 8.06 (br s, 1H), 7.72 (br d, J=9.46 Hz, 1H), 7.62 (s, 1H), 7.30-7.57 (m, 4H), 6.73 (s, 2H), 4.68-5.36 (m, 6H), 3.63 (s, 3H), 2.00 (br s, 2H), 0.73 (br s, 3H). 19F NMR (376 MHz, DMSO-d6) δ -60.41 (br s, 1F). LC-MS: Rt =1.003 min, (ESI) *m/z.* $[M+H]^+$ 536.3; $C_{27}H_{24}F_3N_7O$ | 98 | 1H NMR (400 MHz, DMSO-d6) δ 9.21 (s, 1H), 8.17 (s, 1H), 8.06 (br s, 1H), 7.74 (br d, J=9.46 Hz, 1H), 7.21-7.67 (m, 4H), 6.80 (br s, 2H), 6.06 (s, 1H), 4.70-5.40 (m, 6H), 3.20 (br s, 3H), 2.03 (s, 3H). 19F NMR (376 MHz, DMSO-d6) δ -60.46 (br s, 1F). LC-MS: Rt =0.949 min, (ESI) *m/z.* $[M+H]^+$ 522.3; $C_{26}H_{22}F_3N_7O_2$ |
| 99 | 1H NMR (400 MHz, DMSO-d6) δ 9.21 (br s, 1H), 8.21-8.59 (m, 1H), 8.10 (s, 1H), 7.69-7.88 (m, 2H), 6.99-7.66 (m, 5H), 6.73 (br s, 2H), 4.77-5.50 (m, 6H), 1.97-2.42 (m, 3H). 19F NMR (376 MHz, DMSO-d6) δ -60.45 (br s, 1F). LC-MS: Rt =0.786 min, (ESI) *m/z.* $[M+H]^+$ 519.3; $C_{27}H_{21}F_3N_6O_2$ | 100 | 1H NMR (400 MHz, DMSO-d6) δ 9.22 (br s, 1H), 8.25 (br s, 1H), 7.94-8.18 (m, 1H), 7.69-7.91 (m, 2H), 7.13-7.66 (m, 5H), 6.65 (br s, 2H), 4.72-5.49 (m, 5H), 1.73-2.40 (m, 4H), 1.36 (br s, 3H). 19F NMR (376 MHz, DMSO-d6) δ -60.44 (br s, 1F). LC-MS: Rt =0.801 min, (ESI) *m/z.* $[M+H]^+$ 533.3; $C_{28}H_{23}F_3N_6O_2$ |

(continued)

| | HNMR/LCMS | | HNMR/LCMS |
|---|---|---|---|
| 101 | 1H NMR (400 MHz, DMSO-d6) δ 8.90 (s, 1H), 7.71 (br d, J=9.38 Hz, 1H), 7.63 (s, 1H), 7.43-7.52 (m, 2H), 7.29-7.43 (m, 2H), 6.71 (s, 2H), 5.18 (br s, 2H), 4.90-5.08 (m, 4H), 3.88 (q, J=7.13 Hz, 2H), 1.65 (br s, 3H), 1.16 (br t, J=7.07 Hz, 3H). 19F NMR (376 MHz, DMSO-d6) δ -61.06--60.17 (m, 3F), -154.91 (br s, 1F). LC-MS: Rt =1.62 min, (ESI) *m/z.* $[M+H]^+$ 554.3; $C_{27}H_{23}F_4N_7O_2$ | 102 | 1H NMR (400 MHz, DMSO-d6) δ 9.20 (br s, 1H), 8.05 (br s, 1H), 7.38-7.75 (m, 4H), 6.61 (br s, 2H), 5.13-5.49 (m, 2H), 4.92-5.13 (m, 2H), 3.41-3.97 (m, 7H), 1.07-1.74 (m, 7H). 19F NMR (376 MHz, DMSO-d6) δ -60.42. LC-MS: Rt =2.666 min, (ESI) *m/z.* $[M+H]^+$ 550.2; $C_{28}H_{26}F_3N_7O_2$ |
| 103 | 1H NMR (400 MHz, DMSO-d6) δ 9.22 (s, 1H), 8.06 (br s, 1H), 7.59-7.78 (m, 3H), 7.58 (s, 1H), 7.18-7.50 (m, 3H), 6.49 (s, 2H), 4.99 (br s, 2H), 3.58 (s, 3H), 2.18 (s, 3H), 1.68 (br s, 3H). LC-MS: Rt =0.802 min, (ESI) *m/z.* $[M+H]^+$ 494.4; $C_{25}H_{22}F_3N_7O$ | 104 | 1H NMR (400 MHz, DMSO-d6) δ 9.25 (s, 1H), 8.22 (s, 1H), 8.01-8.13 (m, 2H), 7.72 (d, J=9.68 Hz, 1H), 7.62 (s, 1H), 7.46 (br d, J=9.46 Hz, 1H), 7.28 (br s, 2H), 7.14 (d, J=12.10 Hz, 1H), 5.04 (br s, 2H), 4.28 (s, 3H), 3.52 (s, 3H), 1.77 (s, 3H) LC-MS: (ESI)m/z=552.3 [M+1]+, RT= 0.764 min C26H21F4N9O |
| 105 | 1H NMR (400 MHz, DMSO-d6) δ 9.22 (s, 1H), 8.39 (s, 1H), 8.07 (br s, 2H), 7.72 (br d, J=9.46 Hz, 1H), 7.63 (s, 1H), 7.45 (dd, J=1.43, 9.57 Hz, 1H), 7.36 (br d, J=6.60 Hz, 2H), 6.88 (s, 2H), 5.01 (br s, 2H), 4.33 (s, 3H), 3.56 (s, 3H), 1.67 (br s, 3H).LC-MS: (ESI) m/z=534.3 [M+1]+, RT= 0.762 min.C26H22F3N9O | 106 | 1H NMR (400 MHz, DMSO-d6) δ 9.20 (s, 1H), 8.05 (br s, 1H), 7.73 (br d, J=9.24 Hz, 1H), 7.34-7.65 (m, 4H), 7.30 (d, J=1.76 Hz, 1H), 6.70 (s, 2H), 6.20 (d, J=1.76 Hz, 1H), 5.32-5.46 (m, 1H), 4.97-5.31 (m, 4H), 3.33 (s, 3H), 1.37 (d, J=6.16 Hz, 3H). LC-MS: Rt =0.836 min, (ESI) *m/z.* $[M+H]^+$ 522.3; $C_{26}H_{22}F_3N_7O_2$ |
| 107 | 1H NMR (400 MHz, DMSO-d6) δ 9.20 (s, 1H), 8.05 (br s, 1H), 7.73 (br d, J=9.24 Hz, 1H), 7.46 (br d, J=9.46 Hz, 4H), 7.30 (d, J=1.32 Hz, 1H), 6.79 (s, 2H), 6.20 (d, J=1.76 Hz, 1H), 5.05-5.22 (m, 4H), 4.97 (br s, 2H), 3.34 (s, 3H). LC-MS: Rt =0.799 min, (ESI) *m/z.* $[M+H]^+$ 508.4; $C_{25}H_{20}F_3N_7O_2$ | 108 | 1H NMR (400 MHz, DMSO-d6) δ 10.17-10.63 (m, 1H), 9.21 (s, 1H), 7.93-8.12 (m, 1H), 7.60-7.82 (m, 2H), 7.51-7.59 (m, 1H), 7.42-7.50 (m, 1H), 7.22 (d, J=1.88 Hz, 1H), 5.41-5.67 (m, 2H), 4.81-5.04 (m, 2H), 3.55-3.71 (m, 3H), 1.96-2.07 (m, 3H), 1.82-1.95 (m, 3H). 19F NMR (376 MHz, DMSO-d6) δ 60.416. LC-MS: Rt =2.320 min, (ESI) *m/z.* $[M+H]^+$ 487.2; $C_{22}H_{21}F_3N_8O_2$ |
| 109 | 1H NMR (400 MHz, DMSO-d6) δ 9.21 (s, 1H), 8.06 (br s, 1H), 7.71 (d, J=9.46 Hz, 1H), 7.67 (br s, 1H), 7.58 (s, 1H), 7.44 (dd, J=1.43, 9.57 Hz, 1H), 7.36 (br s, 1H), 7.25 (br d, J=8.80 Hz, 1H), 6.48 (s, 2H), 4.97 (br s, 2H), 4.78 (br t, J=9.02 Hz, 2H), 3.58 (s, 3H), 3.09 (br t, J=9.13 Hz, 2H), 1.67 (br s, 3H) 19F NMR (376 MHz, DMSO-d6) δ -60.99- -60.11 (m, 3F). LC-MS: Rt =2.54 min, (ESI) *m/z.* $[M+H]^+$ 522.2; $C_{26}H_{22}F_3N_7O_2$ | 110 | 1H NMR (400 MHz, DMSO-d6) δ 9.18 (s, 1H), 7.88 (s, 1H), 7.67 (d, J=9.54 Hz, 1H), 7.45-7.56 (m, 2H), 7.36-7.43 (m, 2H), 7.32 (s, 1H), 6.32 (s, 2H), 5.35-5.47 (m, 1H), 5.26-5.35 (m, 1H), 5.15-5.26 (m, 1H), 4.12 (d, J=6.53 Hz, 4H), 3.56 (s, 3H), 2.11 (s, 3H), 1.39 (d, J=6.02 Hz, 3H) . LC-MS: Rt =0.840 min, (ESI) *m/z.* $[M+H]^+$ 522.3; $C_{27}H_{26}F_3N_7O$ |
| 111 | 1H NMR (400 MHz, DMSO-d6) δ 9.24 (br s, 1H), 8.22 (s, 1H), 7.95-8.16 (m, 2H), 7.74 (br d, J=9.02 Hz, 1H), 7.47 (br d, J=9.90 Hz, 1H), 6.92-7.38 (m, 4H), 6.19 (br s, 1H), 5.14 (br s, 2H), 4.29 (br s, 3H), 3.48 (br s, 3H).. LC-MS: Rt =0.786 min, (ESI) *m/z.* $[M+H]^+$ 538.3; $C_{25}H_{19}F_4N_9O$ | 112 | 1H NMR (400 MHz, DMSO-d6) δ 9.21 (s, 1H), 7.97-8.28 (m, 1H), 7.54-7.84 (m, 2H), 7.15-7.48 (m, 4H), 6.57 (s, 2H), 6.17 (d, J=1.88 Hz, 1H), 5.08 (br s, 2H), 4.78 (t, J=9.07 Hz, 2H), 3.09 (br t, J=9.07 Hz, 2H). 19F NMR (376 MHz, DMSO-d6) δ -60.46 (s, 3F). LC-MS: Rt =0.755 min, (ESI) *m/z.* $[M+H]^+$ 508.4; $C_{25}H_{20}F_3N_7O_2$ |

(continued)

| | HNMR/LCMS | | HNMR/LCMS |
|---|---|---|---|
| 113 | 1H NMR (400 MHz, DMSO-d6) δ 9.18 (s, 1H), 8.05 (s, 1H), 7.72 (br d, J=9.26 Hz, 3H), 7.45 (dd, J=1.75, 9.51 Hz, 2H), 7.03-7.36 (m, 2H), 6.50 (s, 2H), 5.05 (s, 2H), 4.78 (br t, J=9.07 Hz, 2H), 3.54-3.82 (m, 3H), 3.10 (t, J=9.07 Hz, 2H).19F NMR (376 MHz, DMSO-d6) δ -60.44 (s, 1F). LC-MS: Rt =0.807 min, (ESI) *m/z.* $[M+H]^+$ 508.2; $C_{25}H_{20}F_3N_7O_2$ | 114 | 1H NMR (400 MHz, DMSO-d6) δ 10.49-11.14 (m, 1H), 8.52 (br d, J=5.94 Hz, 1H), 7.79-8.13 (m, 2H), 7.45-7.65 (m, 2H), 7.15-7.33 (m, 1H), 6.83-6.97 (m, 1H), 5.55-5.71 (m, 2H), 5.26-5.50 (m, 1H), 4.53-4.88 (m, 1H), 3.92-4.36 (m, 1H), 3.71-3.87 (m, 1H), 3.67 (br d, J=17.39 Hz, 2H), 1.97-2.07 (m, 3H), 0.78-1.11 (m, 3H). LC-MS: Rt =1.628 min, (ESI) *m/z.* $[M+H]^+$ 395.3; $C_{20}H_{22}N_6O_3$ |
| 115 | 1H NMR (400 MHz, DMSO-d6) δ 9.25 (s, 1H), 8.24 (s, 1H), 8.11 (s, 2H), 7.73 (d, J=9.38 Hz, 1H), 7.42-7.65 (m, 3H), 7.36 (d, J=4.25 Hz, 1H), 7.26 (s, 2H), 4.99-5.24 (m, 2H), 4.27 (s, 3H), 3.53-3.62 (m, 3H). 19F NMR (376 MHz, DMSO-d6) δ -60.47 (s, 1F), -174.46 (br s, 1F). LC-MS: RT =0.786 min, (ESI) *m/z.* $[M+H]^+$ 538.3; $C_{25}H_{19}N_9OF_4$ | 116 | $^1$H NMR (400 MHz, MeOD) δ 8.75 (s, 2H), 8.40 (s, 2H), 7.87 - 7.61 (m, 2H), 7.55 (d, *J* = 8.8 Hz, 1H), 7.32 (s, 1H), 7.18 (s, 1H), 6.83 (s, 1H), 6.51 (s, 1H), 5.68 (s, 1H), 5.40 (s, 1H), 4.73 - 4.53 (m, 2H), 3.24 (s, 2H), 2.94 (s, 2H), 2.41 - 2.13 (m, 2H), 1.84 - 1.65 (m, 3H). LC-MS: Rt =0.981 min, (ESI) m/z. 464.2 $[M+H]^+$. $C_{27}H_{25}N_7O$ |
| 117 | 1H NMR (400MHz, DMSO-d6) δ 8.68 (d, J=6.8 Hz, 1H), 8.54 (s, 1H), 8.26 (br s, 1H), 7.65 (d, J=9.0 Hz, 1H), 7.63 - 7.60 (m, 1H), 7.63 - 7.60 (m, 1H), 7.57 - 7.55 (m, 1H), 7.54 (br s, 1H), 7.28 - 7.15 (m, 1H), 6.93 - 6.82 (m, 3H), 6.58 (br s, 1H), 5.26 (br s, 1H), 4.36 (s, 3H), 3.55 - 3.43 (m, 1H), 3.21 - 3.09 (m, 1H), 1.67 (br s, 3H), 1.08 (br s, 2H). LC-MS: Rt =2.296 min, (ESI) *m/z.* $[M+H]^+$ 413.9; $C_{23}H_{23}N_7O$ | 118 | 1H NMR (400 MHz, DMSO-d6) δ 8.67 (br d, J=6.50 Hz, 1H), 8.02-8.62 (m, 2H), 7.66 (d, J=8.88 Hz, 1H), 7.58 (br s, 2H), 7.15-7.28 (m, 1H), 6.76-6.94 (m, 3H), 6.60 (br s, 1H), 4.57-4.98 (m, 2H), 4.36 (s, 3H), 3.37-3.61 (m, 2H), 1.15 (br s, 3H) . LC-MS: Rt =1.324 min, (ESI) *m/z.* $[M+H]^+$ 400.1; $C_{22}H_{21}N_7O$ |
| 119 | 1H NMR (400 MHz, DMSO-d6) δ = 8.67 (d, J = 7.0 Hz, 1H), 7.74 - 7.53 (m, 4H), 7.26 - 7.15 (m, 1H), 6.92 - 6.83 (m, 1H), 6.67 (s, 2H), 6.57 (br s, 1H), 5.46 - 5.19 (m, 3H), 5.00 (br s, 2H), 3.31 - 3.25 (m, 1H), 3.14 (dd, J = 7.0, 14.1 Hz, 1H), 1.63 (br d, J = 3.2 Hz, 3H), 1.20 - 0.90 (m, 3H) LC-MS: Rt = 2.426 min, (ESI) *m/z.* $[M+H]^+$ 402.1; $C_{23}H_{23}N_5O_2$ | 120 | 1H NMR (400 MHz, DMSO-d6) δ = 8.80 (d, J = 4.9 Hz, 2H), 8.62 - 8.50 (m, 1H), 7.79 (s, 2H), 7.66 - 7.54 (m, 2H), 7.52 - 7.42 (m, 1H), 7.39 (br t, J = 4.8 Hz, 1H), 7.17 (br s, 1H), 6.81 (br s, 1H), 6.61 - 6.41 (m, 3H), 5.38 (br d, J = 6.8 Hz, 1H), 5.02 - 4.88 (m, 1H), 4.54 - 4.21 (m, 1H), 2.21 (s, 3H), 1.63 (d, J = 7.1 Hz, 3H) LC-MS: Rt = 2.341 min, (ESI) *m/z.* $[M+H]^+$ 438.2; $C_{25}H_{23}N_7O$ |
| 121 | 1H NMR (400 MHz, DMSO-d6) δ = 8.66 (br d, J = 6.3 Hz, 1H), 7.84 - 7.44 (m, 4H), 7.22 (br t, J = 7.6 Hz, 1H), 6.93 - 6.82 (m, 1H), 6.58 (br s, 3H), 5.51 - 5.02 (m, 3H), 4.93 - 4.57 (m, 2H), 3.60 - 3.34 (m, 2H), 1.39 (br d, J = 5.8 Hz, 3H), 1.12 (br s, 3H) LC-MS: Rt = 2.385 min, (ESI) *m/z.* $[M+H]^+$ 402.1; $C_{23}H_{23}N_5O_2$ | 122 | 1H NMR (400 MHz, DMSO-d6) δ = 8.56 (br d, J = 7.2 Hz, 1H), 7.93 (q, J = 8.1 Hz, 1H), 7.85 - 7.70 (m, 2H), 7.62 - 7.54 (m, 2H), 7.51 - 7.24 (m, 2H), 7.20 - 7.13 (m, 1H), 7.09 - 6.96 (m, 1H), 6.81 (dt, J = 1.3, 6.8 Hz, 1H), 6.46 (br s, 3H), 5.24 (br d, J = 3.8 Hz, 1H), 4.98 - 4.14 (m, 2H), 2.20 (s, 3H), 1.58 (d, J = 7.0 Hz, 3H) LC-MS: Rt = 2.623 min, (ESI) *m/z.* $[M+H]^+$ 455.1; $C_{26}H_{23}FN_6O$ |
| 123 | 1H NMR (500 MHz, DMSO-d6) δ 8.65 (br s, 1H), 8.27-8.61 (m, 1H), 8.23 (s, 1H), 7.65 (d, J=8.54 Hz, 1H), 7.27-7.49 (m, 1H), 7.27 (br s, 2H), 7.14-7.22 (m, 1H), 6.83-6.90 (m, 1H), 6.66 (br s, 1H), 4.07-5.97 (m, 6H), 3.51-4.00 (m, 3H), 0.75-1.05 (m, 3H). 19F NMR (376.5 MHz, DMSO-d6) δ -114.28, -116.70. LC-MS: Rt =1.553 min, (ESI) *m/z.* $[M+H]^+$ 460.2; $C_{24}H_{22}FN_7O_2$ | 124 | 1H NMR (400 MHz, DMSO-d6) δ 8.25-8.80 (m, 1H), 7.64 (br d, J=8.53 Hz, 2H), 7.06-7.47 (m, 2H), 6.68-6.92 (m, 3H), 6.51-6.67 (m, 1H), 5.35 (br s, 1H), 4.35-5.13 (m, 3H), 3.43-3.98 (m, 6H), 0.88 (br d, J=7.03 Hz, 3H). 19F NMR (376 MHz, DMSO-d6) δ -116.47 (br s, 1F). LC-MS: Rt =1.539 min, (ESI) *m/z.* $[M+H]^+$ 448.3; $C_{24}H_{22}FN_5O_3$ |

(continued)

| | HNMR/LCMS | | HNMR/LCMS |
|---|---|---|---|
| 125 | 1H NMR (400 MHz, DMSO) δ 9.27 (s, 1H), 7.86 (d, J=9.24 Hz, 1H), 7.62 (s, 1H), 7.29-7.56 (m, 4H), 6.61-6.88 (m, 3H), 5.20 (br s, 2H), 5.06 (br s, 2H), 4.97 (br s, 2H), 3.59 (s, 3H), 1.64 (br s, 3H). LC-MS: Rt =1.109 min, (ESI) *m/z.* $[M+H]^+$ 522.1; $C_{26}H_{22}F_3N_7O_2$ | 126 | |
| 127 | $^1$HNMR (400 MHz, DMSO-$d_6$) δ 11.84 (s, 1H), 7.80-7.68 (m, 2H), 7.59 - 7.51 (m, 2H), 7.46 (s, 1H), 7.18 - 7.13 (m, 2H), 6.45 (s, 2H), 4.98 (s, 2H), 4.46 - 4.33 (m, 2H), 3.70 - 3.62 (m, 2H), 3.48-3.38 (m, 2H), 3.09 (s, 3H), 2.21 (s, 3H), 1.05-0.99 (m, 1H), 0.57 - 0.33 (m, 2H), 0.31 - 0.11 (m, 2H). LC-MS: Rt = 0.90 min, (ESI) *m/z.* $[M+H]^+$ 430.2 ; C25H27N5O2 | 128 | $^1$H NMR (400 MHz, dmso-$d_6$) δ 8.90 - 8.71 (m, 1H), 8.53 (s, 3H), 8.00 - 7.76 (m, 1H), 7.59 - 7.43 (m, 3H), 7.23 (s, 2H), 5.64 - 5.45 (m, 1H), 5.30 - 5.09 (m, 1H), 5.06 - 4.99 (m, 1H), 4.95 - 4.89 (m, 1H), 3.29 - 3.18 (m, 2H), 3.00 - 2.77 (m, 2H), 2.31 (s, 1H), 2.03 (s, 1H), 1.79 - 1.20 (m, 3H). LC-MS: Rt = 0.918 min, (ESI) m/z. $[M+H]^+$, 464.2. $C_{27}H_{25}N_7O$ |
| 129 | $^1$H NMR (400 MHz, MeOD) δ 8.49 (s, 3H), 8.01 (s, 1H), 7.75 (d, *J* = 7.2 Hz, 1H), 7.68 - 7.57 (m, 3H), 7.24 (dd, *J* = 5.2, 2.8 Hz, 2H), 5.49 (s, 1H), 5.21 - 5.08 (m, 4H), 3.66 (d, *J* = 8.0 Hz, 2H), 3.49 (d, *J* = 8.4 Hz, 2H), 3.18 (s, 3H), 2.97 (s, 2H), 2.34 (s, 2H), 1.37 (d, *J* = 6.4 Hz, 1H), 1.00 (s, 1H), 0.53 (s, 2H), 0.29 (s, 1H). LC-MS: Rt =1.000 min, (ESI) *m/z.* $[M+H]^+$ 456.3; $C_{27}H_{29}N_5O_2$ | 130 | $^1$H NMR (400 MHz, DMSO-$d_6$) δ 12.10 - 12.08 (m, 1H), 7.76 - 7.72 (m, 1H), 7.56 - 7.54 (m, 4H), 7.16 - 7.14 (m, 2H), 6.44 - 6.43 (m, 2H), 4.74 - 4.69 (m, 2H), 3.15 - 3.10 (m, 1H), 2.85 - 2.80 (m, 2H), 2.13 - 2.05 (m, 2H), 1.26 - 1.20 (m, 2H), 1.20 - 1.16 (m, 6H). LC-MS: Rt =0.905 min, (ESI) m/z. $[M+H]^+$ 401.2. $C_{24}H_{25}N_5O$ |
| 131 | 1H NMR (400 MHz, DMSO-d6) δ = 12.77 (br s, 1H), 8.13 (s, 1H), 8.05 - 7.83 (m, 1H), 7.82 - 7.66 (m, 1H), 7.82 - 7.45 (m, 3H), 6.70 (br s, 2H), 5.51 - 5.22 (m, 1H), 5.19 - 5.04 (m, 1H), 5.03 - 4.84 (m, 3H), 4.78 (br d, J = 7.1 Hz, 1H), 3.42 (br s, 2H), 2.17 - 1.85 (m, 1H), 1.08 - 0.57 (m, 6H) LC-MS: Rt = 2.023 min, (ESI) *m/z.* $[M+H]^+$ 484.2; $C_{25}H_{24}F_3N_5O_2$ | 132 | 1H NMR (400 MHz, DMSO-d6) δ = 13.16 - 12.43 (m, 1H), 8.52 - 8.29 (m, 1H), 8.28 - 8.18 (m, 1H), 8.17 - 8.11 (m, 1H), 8.07 - 7.84 (m, 1H), 7.83 - 7.65 (m, 1H), 7.65 - 7.55 (m, 2H), 7.55 - 7.42 (m, 1H), 7.26 - 7.01 (m, 2H), 5.09 - 4.69 (m, 2H), 4.42 - 4.02 (m, 3H), 3.33 - 3.32 (m, 2H), 2.17 - 1.91 (m, 1H), 1.05 - 0.47 (m, 6H) LC-MS: Rt = 2.130 min, (ESI) *m/z.* $[M+H]^+$496.1; $C_{25}H_{24}F_3N_7O$ |
| 133 | 1H NMR (400 MHz, DMSO-d6) δ 12.85 (br s, 1H), 8.20-8.29 (m, 1H), 8.17 (s, 2H), 7.63-7.82 (m, 2H), 7.60 (br s, 1H), 7.26-7.37 (m, 2H), 7.22 (br s, 1H), 5.00 (br s, 1H), 4.73 (br s, 1H), 4.38 (s, 1H), 3.91-4.11 (m, 2H), 3.33 (s, 3H), 3.17-3.26 (m, 1H), 2.05-2.20 (m, 1H), 1.90-2.05 (m, 1H), 0.96 (br d, J=6.50 Hz, 4H), 0.88 (br d, J=6.63 Hz, 1H), 0.69 (br d, J=6.25 Hz, 2H). LC-MS: Rt =1.761 min, (ESI) *m/z.* $[M+H]^+$ 572.1; $C_{24}H_{23}F_6N_7OS$ | 134 | 1H NMR (400 MHz, DMSO-d6) δ = 12.77 (br dd, J = 2.4, 5.9 Hz, 1H), 7.93 (br s, 1H), 7.82 - 7.42 (m, 5H), 6.60 (br s, 2H), 5.40 (br s, 3H), 4.95 - 4.63 (m, 2H), 3.48 (br s, 2H), 1.38 (br d, J = 3.9 Hz, 3H), 1.14 (br t, J = 6.8 Hz, 3H) LC-MS: Rt = 2.601 min , (ESI) *m/z* = 470.2 [M+H]+; $C_{24}H_{22}F_3N_5O_2$ |
| 135 | 1H NMR (400 MHz, DMSO-d6) δ ppm 12.35 (br s, 1H) 7.41 - 7.90 (m, 5 H) 7.19 (br s, 2 H) 6.60 (br s, 2 H) 4.67 - 5.48 (m, 5 H) 3.48 (br s, 2 H) 1.39 (br d, J=4.50 Hz, 3 H) 1.14 (br t, J=6.63 Hz, 3 H) LC-MS: Rt = 2.254 min , (ESI) *m/z* = 402.2 [M+H]+; $C_{23}H_{23}N_5O_2$ | 136 | 1H NMR (400 MHz, DMSO-d6) δ = 12.44 - 11.68 (m, 1H), 7.67 (s, 1H), 7.62 - 7.47 (m, 4H), 7.17 (dd, J = 3.2, 6.0 Hz, 2H), 6.28 (s, 2H), 5.48 - 5.36 (m, 1H), 5.29 - 5.06 (m, 2H), 4.78 (s, 2H), 3.34 (br d, J = 7.4 Hz, 2H), 2.05 (td, J = 6.8, 13.8 Hz, 1H), 1.42 (d, J = 6.1 Hz, 3H), 0.83 (br d, J = 6.3 Hz, 6H) LC-MS: Rt = 2.453 min, (ESI) *m/z* = 430.2 [M+H]+; $C_{25}H_{27}N_5O_2$ |

(continued)

| | HNMR/LCMS | | HNMR/LCMS |
|---|---|---|---|
| 137 | 1H NMR (400 MHz, DMSO-d6) δ = 8.99 - 8.68 (m, 1H), 8.27 - 8.08 (m, 1H), 8.00 - 7.40 (m, 4H), 7.18 - 7.09 (m, 1H), 7.07 - 6.94 (m, 2H), 4.99 - 4.71 (m, 2H), 2.62 (s, 1H), 2.58 (s, 1H), 2.50 (br s, 3H), 0.95 (d, J = 6.5 Hz, 3H), 0.88 - 0.80 (m, 1H), 0.69 (d, J = 6.5 Hz, 3H) LC-MS: Rt = 1.617 min, (ESI) *m/z.* $[M+H]^+$ 514.3; $C_{25}H_{23}F_4N_7O$ | 138 | 1H NMR (400 MHz, DMSO-d6) δ = 12.73 - 12.43 (m, 1H), 8.02 (br d, J = 6.4 Hz, 1H), 7.90 - 7.82 (m, 1H), 7.78 (br s, 1H), 7.73 - 7.64 (m, 1H), 7.47 (br d, J = 8.5 Hz, 1H), 7.20 (d, J = 10.9 Hz, 1H), 7.17 (br s, 2H), 4.95 - 4.69 (m, 2H), 3.46 - 3.22 (m, 2H), 2.91 - 2.57 (m, 3H), 2.16 - 2.03 (m, 1H), 0.99 - 0.78 (m, 6H) LC-MS: Rt = 2.498 min, (ESI) *m/z.* $[M+H]^+$ 514.2; $C_{25}H_{23}F_4N_7O$ |
| 139 | 1H NMR (400 MHz, DMSO-d6) δ 9.01 (s, 0.7H), 8.66 (s, 0.3H), 8.53 (d, J=6.60 Hz, 0.7H), 8.31 (d, J=6.60 Hz, 0.3H), 8.04-8.20 (m, 1H), 7.35-7.86 (m, 2H), 7.08-7.26 (m, 2H), 6.91-7.08 (m, 2H), 6.71-6.91 (m, 1H), 4.19-5.11 (m, 3H), 2.75-2.86 (m, 1H), 2.63-2.70 (m, 1H), 2.56-2.63 (m, 3H), 2.34-2.46 (m, 1H), 2.15-2.27 (m, 3H), 1.92-2.15 (m, 2H). 19F NMR (376.5 MHz, DMSO-d6) δ -118.93, -119.11, -119.89. LC-MS: Rt =1.824 min, (ESI) *m/z.* $[M+H]^+$ 572.3;<br>$C_{24}H_{23}F_6N_7OS$ | 140 | 1H NMR (400 MHz, DMSO-d6) δ 7.35 - 8.50 (m, 7 H) 6.96 - 7.35 (m, 1 H) 4.53 - 5.22 (m, 2 H) 2.60 - 2.98 (m, 3 H) 1.05 - 1.27 (m, 3 H). 19F NMR (376 MHz, DMSO-d6) δ -58.95 - -58.53 (m, 1 F) -120.17 - -118.91 (m, 1 F). LC-MS: Rt =1.613 min, (ESI) *m/z.* $[M+H]^+$ 486.2;<br><br>$C_{23}H_{19}F_4N_7O$ |
| 141 | 1H NMR (400 MHz, DMSO-d6) δ 12.20-12.38 (m, 1H), 8.75-8.97 (m, 1H), 8.14-8.65 (m, 1H), 7.56-7.67 (m, 2H), 7.35-7.54 (m, 1H), 7.12-7.22 (m, 2H), 7.03 (br s, 2H), 6.95 (d, J=11.04 Hz, 1H), 4.82-5.13 (m, 2H), 3.51-3.70 (m, 3H), 2.59-2.70 (m, 3H), 1.75-2.06 (m, 3H). 19F NMR (376.5 MHz, DMSO-d6) δ -116.39, -118.62 LC-MS: Rt =0.790 min, (ESI) *m/z.* $[M+H]^+$ 484.1;<br>$C_{25}H_{22}FN_9O$ | 142 | 1H NMR (400 MHz, DMSO-d6) δ 12.99 (br s, 1H), 7.87-8.05 (m, 2H), 7.69-7.84 (m, 2H), 7.48-7.60 (m, 3H), 7.22 (br s, 1H), 6.97-7.13 (m, 1H), 6.22 (br s, 1H), 5.25 (br s, 2H), 3.54-3.66 (m, 3H), 2.87 (br s, 3H). LC-MS: Rt =0.774 min, (ESI) *m/z.* $[M+H]^+$ 538.2;<br><br>$C_{25}H_{19}F_4N_9O$ |
| 143 | 1H NMR (400 MHz, DMSO-d6) δ 9.25 - 9.27 (m, 1 H) 9.26 (s, 1 H) 9.14 - 9.19 (m, 1 H) 8.87 - 9.01 (m, 1 H) 8.06 (s, 1 H) 6.96 - 7.21 (m, 3 H) 4.62 - 4.95 (m, 2 H) 3.47 - 3.60 (m, 2 H) 2.58 - 2.63 (m, 3 H) 1.05 - 1.20 (m, 3 H). 19F NMR (376 MHz, DMSO-d6) δ -65.75 - -65.59 (m, 1 F) -119.33 - -119.19 (m, 1 F). LC-MS: Rt =1.520 min, (ESI) *m/z.* $[M+H]^+$ 487.3;<br>$C_{22}H_{18}F_4N_8O$ | 144 | 1H NMR (400 MHz, DMSO-d6) δ = 9.05 - 8.85 (m, 1H), 8.44 - 8.25 (m, 1H), 8.21 - 8.13 (m, 1H), 7.82 - 7.67 (m, 1H), 7.44 (d, J = 9.8 Hz, 1H), 7.16 (t, J = 11.3 Hz, 1H), 7.01 (br dd, J = 2.3, 9.8 Hz, 3H), 4.76 (s, 1H), 4.49 (s, 1H), 3.76 (d, J = 14.4 Hz, 3H), 3.51 (br d, J = 6.6 Hz, 1H), 3.30 (br s, 1H), 2.61 (d, J = 3.9 Hz, 3H), 1.19 - 1.03 (m, 3H) LC-MS: Rt = 2.228 min, (ESI) m/z. [M+H]+ 448.2; $C_{23}H_{22}FN_7O_2$ |
| 145 | 1H NMR (400 MHz, DMSO-d6) δ 8.02-8.43 (m, 2H), 7.62-8.02 (m, 3H), 7.19-7.62 (m, 4H), 6.83-7.19 (m, 2H), 4.75-5.19 (m, 1H), 4.75-5.19 (m, 1H), 3.51-3.83 (m, 6H), 2.68-2.97 (m, 3H) LC-MS: Rt =1.319 min, (ESI) *m/z.* $[M+H]^+$ 500.3<br>$C_{25}H_{22}FN_9O_2$ | 146 | LC-MS: Rt =1.056 min, (ESI) m/z. 447.2 $[M+H]^+$. $C_{25}H_{26}N_4O_2S$ require 446.57. HPLC Purity: 99.72% (214 nm), 99.84% (254 nm). |
| 147 | $^1H$ NMR (400 MHz, DMSO-$d_6$) δ 8.09 - 8.07 (m, 2H), 7.97 - 7.95 (m, 1H), 7.70 (s, 1H), 7.52 - 7.48 (m, 3H), 7.43 - 7.40 (m, 1H), 6.47 (s, 2H), 5.11 (s, 2H), 3.75 - 3.70 (m, 1H), 3.18 (s, 3H), 3.12 (s, 2H), 2.84 - 2.81 (m, 2H), 2.19 - 2.17 (m, 2H), 2.03 - 1.98 (m, 1H), 1.23 (s, 2H), 0.50 - 0.47 (m, 1H), 0.36 - 0.34 (m, 1H), 0.12 - 0.07 (m, 2H). LC-MS: Rt =1.156 min, (ESI) m/z. 473.2 $[M+H]^+$.<br>$C_{27}H_{28}N_4O_2S$ | 148 | $^1H$ NMR (400 MHz, DMSO-$d_6$) δ 8.12 (d, *J* = 4.0 Hz, 1H), 7.99 (d, *J* = 8.0 Hz, 1H), 7.82 (s, 1H), 7.69 (m, 1H), 7.55 - 7.43 (m, 4H), 6.50 (s, 2H), 5.07 (s, 2H), 2.25 (s, 1H), 2.22 (s, 3H), 1.78 (s, 6H). LC-MS: Rt = 1.079 min, (ESI) m/z. [M+H]+, 415.2. $C_{24}H_{22}N_4OS$ |

(continued)

| | HNMR/LCMS | | HNMR/LCMS |
|---|---|---|---|
| 149 | $^1$H NMR (400 MHz, DMSO-$d_6$) δ 7.98 - 7.96 (m, 2H), 7.79 (m, 1H), 7.67 (s, 1H), 7.49 - 7.46 (m, 3H), 6.45 (s, 2H), 4.92 - 4.80 (m, 2H), 3.62 - 3.56 (m, 1H), 3.40 (s, 2H), 3.12 (s, 3H), 2.79 (s, 3H), 2.21 (s, 2H), 1.02 - 0.96 (m, 1H), 0.45 (s, 1H), 0.32 - 0.27 (m, 1H), 0.04 (s, 2H). LC-MS: Rt =1.156 min, (ESI) m/z. 473.2 $[M+H]^+$. $C_{26}H_{28}N_4O_2S$ | 150 | $^1$H NMR (400 MHz, DMSO-$d_6$) δ 8.37 - 8.31 (m, 2H), 7.79 - 7.67 (m, 3H), 7.41 (s, 2H), 6.49 (s, 2H), 5.16 (s, 2H), 3.70 - 3.46 (m, 3H), 3.17 (s, 3H), 2.22 (s, 3H), 1.15 - 1.09 (m, 1H), 0.45 - 0.34 (m, 2H), 0.32 - 0.14 (m, 2H). LC-MS: Rt = 1.133 min, (ESI) m/z. $[M+H]^+$ 515.1. $C_{26}H_{25}F_3N_4O_2S$ |
| 151 | $^1$H NMR (400 MHz, DMSO-$d_6$) 1H NMR (400 MHz, DMSO) δ 8.19 (s, 1H), 8.07 (d, *J* = 8.4 Hz, 1H), 7.78 (s, 1H), 7.68 (s, 1H), 7.59 (dd, *J* = 8.4, 2.0 Hz, 1H), 7.46 (s, 2H), 6.52 (s, 2H), 5.12 (s, 2H), 3.69 (t, *J* = 9.6 Hz, 1H), 3.46 (d, *J* = 7.6 Hz, 1H), 3.26 (s, 1H), 3.16 (s, 3H), 2.21 (s, 3H), 1.15 - 1.03 (m, 1H), 0.48 (s, 1H), 0.37 - 0.29 (m, 1H), 0.07 (s, 2H). LC-MS: Rt =1.156 min, (ESI) m/z. $[M+H]^+$ 526.1; $[M+2+H]^+$ 528.1. $C_{25}H_{25}BrN_4O_2S$ | 152 | $^1$H NMR (400 MHz, DMSO-d6) δ 9.66 (s, 1H), 8.94 - 8.61 (m, 3H), 8.07 - 7.89 (m, 2H), 7.87 - 7.54 (m, 2H), 7.47 - 7.22 (m, 2H), 7.21 - 6.96 (m, 1H), 5.28 (s, 1H), 5.04 - 4.86 (m, 1H), 4.76 (m, 1H), 4.48 (d, *J* = 16.0 Hz, 1H), 3.57 (d, *J* = 8.0 Hz, 2H), 3.45 - 3.37 (m, 1H), 3.29 (s, 1H), 3.20 - 3.07 (m, 2H), 3.02 - 2.03 (m, 5H), 2.40 - 2.13 (m, 4H), 1.99 (d, *J* = 16.0 Hz, 2H), 1.59 (s, 3H). LC-MS: Rt =0.926 min, (ESI) m/z. 578.4 $[M+H]^+$. $C_{33}H_{35}N_7OS$ |
| 153 | $^1$H NMR (400 MHz, MeOD) δ 8.77 (s, 2H), 8.39 (s, 1H), 8.24 - 8.12 (m, 2H), 8.10 - 7.94 (m, 1H), 7.92 - 7.78 (m, 1H), 7.70 - 7.66 (m, 1H), 7.42 - 7.28 (m, 1H), 5.55 - 5.40 (m, 0.5H), 5.35 - 5.20 (m, 0.5H), 5.17 - 5.02 (m, 1H), 4.67 - 4.47 (m, 1H), 3.06 - 2.88 (m, 2H), 2.42 - 2.22 (m, 2H), 1.77 (s, 3H), 1.41 - 1.24 (m, 2H). $^{19}$F NMR (376 MHz, MeOD) δ -63.20, 74.02, 75.91. LC-MS: Rt =1.277 min, (ESI) m/z. $[M+H]^+$ 549.2. $C_{28}H_{23}F_3N_6OS$ | 154 | $^1$H NMR (400 MHz, $CD_3OD$) δ 8.32 - 8.11 (m, 2H), 7.92 (s, 1H), 7.75 - 7.55 (m, 3H), 5.36 - 5.18 (m, 2H), 3.85-3.73 (m, 1H), 3.58 (d, *J* = 8.0 Hz, 1H), 3.44 - 3.35 (s, 1H), 3.26 (s, 3H), 3.00 - 2.90 (m, 2H), 2.40 - 2.20 (m, 2H), 1.40 - 1.25 (m, 2H), 1.11 (s, 1H), 0.95 - 0.85 (m, 1H), 0.63 - 0.53 (m, 1H), 0.45 - 0.33 (m, 1H), 0.13 - 0.08 (m, 1H). LC-MS: Rt = 0.859 min, (ESI) m/z. $[M+H]^+$ 541.2. $C_{28}H_{27}F_3N_4O_2S$ |
| 155 | $^1$H NMR (400 MHz, DMSO-$d_6$) δ 8.90 (s, 1H), 8.80 (d, *J* = 4.0 Hz, 2H), 7.95 - 7.60 (m, 3H), 7.55 - 7.30 (m, 3H), 7.18 (s, 1H), 6.45 (s, 2H), 5.70 - 5.30 (m, 1H), 5.20 - 4.50 (m, 2H), 3.05 - 2.60 (m, 5H), 2.25 - 1.95 (m, 2H), 1.66 (s, 3H). LC-MS: Rt = 0.942 min, (ESI) m/z. $[M+H]^+$ 465.2, $C_{26}H_{24}N_8O$ | 156 | $^1$H NMR (400 MHz, $CD_3OD$) δ 8.76 (s, 1H), 8.75 (s, 1H), 7.99 (s, 1H), 7.40 - 7.35 (m, 2H), 7.34 (s, 1H), 6.01 (s, 1H), 5.53 - 5.40 (m, 2H), 4.49 (s, 2H), 4.58 - 4.50 (m, 2H), 4.06 - 4.01 (m, 3H), 3.31 - 3.18 (m, 2H), 3.15 - 2.93 (m, 2H),2.36 - 2.29 (m, 2H), 1.76 - 1.65(m, 3H).LC-MS: Rt = 0.911 min, (ESI) m/z. $[M+H]^+$ 470.2; $C_{26}H_{27}N_7O_2$ |
| 157 | $^1$H NMR (400 MHz, DMSO-d6) δ 8.79 (d, *J* = 4.0 Hz, 2H), 8.06 - 7.69 (m, 1H), 7.59 - 7.53 (m, 1H), 7.49 (s, 1H), 7.39 (s, 1H), 7.12 - 6.85 (m, 1H), 6.47 (s, 2H), 5.34 - 5.16 (m, 1H), 4.79 - 4.40 (m, 4H), 3.96 (s, 5H), 3.02 (d, *J* = 8.0 Hz, 2H), 2.81 (t, *J* = 8.0 Hz, 2H), 2.25 - 2.08 (m, 2H), 1.63 (d, *J* = 8.0 Hz, 3H). LC-MS: Rt = 0.671 min, (ESI) m/z. $[M+H]^+$ 470.2, | 158 | NT |
| 159 | 1H NMR (400 MHz, DMSO-d6) δ 8.66 (d, J=6.88 Hz, 1H), 8.12 (s, 1H), 7.78 (s, 1H), 7.66 (d, J=8.88 Hz, 1H), 7.47 (s, 2H), 7.30 (br s, 2H), 7.17-7.25 (m, 1H), 6.89 (br t, J=6.69 Hz, 1H), 6.59 (s, 1H), 4.77 (br s, 2H), 3.41-3.57 (m, 2H), 2.54-3.11 (m, 3H), 1.17 (t, J=7.00 Hz, 3H). LC-MS: Rt =1.262 min, (ESI) *m/z.* $[M+H]^+$ 400.1; $C_{22}H_{21}N_7O$ | 162 | 1H NMR (400MHz, DMSO-d6) δ 9.26 (s, 1H), 8.53 (d, J=5.3 Hz, 1H), 8.20 (d, J=5.3 Hz, 1H), 8.07 (br s, 1H), 7.81 (br s, 1H), 7.47 (br s, 1H), 7.44 - 7.39 (m, 1H), 7.34 (br s, 2H), 5.13 (s, 2H), 3.41 (br d, J=7.3 Hz, 2H), 3.33 (br s, 1H), 2.92 (br s, 2H), 2.13 - 1.91 (m, 1H), 0.76 (br s, 6H). LC-MS: Rt =0.723 min, (ESI) *m/z.* $[M+H]^+$ 446.3; $C_{23}H_{23}N_7OS$ |

(continued)

| | HNMR/LCMS | | HNMR/LCMS |
|---|---|---|---|
| 163 | 1H NMR (400 MHz, DMSO-d6) δ 8.50 (br d, J=6.60 Hz, 2H), 7.91 (br s, 1H), 7.79 (s, 1H), 7.53 (d, J=8.80 Hz, 1H), 7.41 (s, 2H), 7.23-7.30 (m, 2H), 6.89 (t, J=6.71 Hz, 1H), 5.03 (br d, J=6.82 Hz, 1H), 3.45-3.53 (m, 2H), 2.93 (s, 3H), 1.58 (br d, J=6.82 Hz, 3H), 0.72-1.15 (m, 3H). LC-MS: Rt =3.743 min, (ESI) *m/z.* $[M+H]^+$ 432.3; | 164 | 1H NMR (400MHz, DMSO-d6) δ 9.10 - 8.60 (m, 3H), 8.52 - 8.27 (m, 1H), 8.13 (s, 1H), 8.05 - 7.88 (m, 2H), 7.65 (br s, 2H), 7.52 (br s, 1H), 5.10 - 4.72 (m, 2H), 3.45 - 3.18 (m, 2H), 3.14 - 2.84 (m, 3H), 2.68 (s, 3H), 2.16 - 1.88 (m, 1H), 1.08 - 0.62 (m, 6H). LC-MS: Rt =1.623 min, (ESI) *m/z.* $[M+H]^+$ 442.2;<br>$C_{25}H_{27}N_7O$ |
| 165 | 1H NMR (400 MHz, DMSO-d6) δ 8.45-8.60 (m, 1H), 7.82-8.31 (m, 2H), 7.42-7.82 (m, 5H), 7.13-7.31 (m, 1H), 6.83-6.92 (m, 1H), 4.30-5.09 (m, 2H), 3.11-3.33 (m, 2H), 2.55 (br s, 3H), 1.87-2.23 (m, 1H), 0.59-1.04 (m, 6H). LC-MS: Rt =1.568 min, (ESI) *m/z.* $[M+H]^+$ 429.2;<br>$C_{23}H_{24}N_8O$ | 166 | 1H NMR (400MHz, DMSO-d6) δ 9.10 - 8.60 (m, 3H), 8.52 - 8.27 (m, 1H), 8.13 (s, 1H), 8.05 - 7.88 (m, 2H), 7.65 (br s, 2H), 7.52 (br s, 1H), 5.10 - 4.72 (m, 2H), 3.45 - 3.18 (m, 2H), 3.14 - 2.84 (m, 3H), 2.68 (s, 3H), 2.16 - 1.88 (m, 1H), 1.08 - 0.62 (m, 6H). LC-MS: Rt =1.623 min, (ESI) *m/z.* $[M+H]^+$ 442.2;<br>$C_{25}H_{27}N_7O$ |
| 167 | 1H NMR (400 MHz, DMSO-d6) δ 9.00 (s, 1H), 8.49 (s, 1H), 8.01-8.11 (m, 1H), 7.39-7.52 (m, 3H), 7.28-7.39 (m, 2H), 6.76 (br s, 2H), 5.49-6.49 (m, 1H), 4.61-4.80 (m, 2H), 2.87-2.95 (m, 3H), 2.64-2.70 (m, 3H), 2.58 (s, 3H). 19F NMR (376 MHz, DMSO-d6) δ -59.29 (s, 3F). LC-MS: Rt =1.840 min, (ESI) *m/z.* $[M+H]^+$ 522.2;<br>$C_{26}H_{22}F_3N_7O_2$ | 168 | 1H NMR (400 MHz, DMSO-d6) δ 8.42-8.65 (m, 1H), 7.93-8.31 (m, 1H), 7.85-7.93 (m, 1H), 7.69-7.85 (m, 1H), 7.35-7.62 (m, 3H), 7.11-7.34 (m, 2H), 6.88 (t, J=6.71 Hz, 1H), 4.50-4.93 (m, 2H), 3.44-3.68 (m, 4H), 3.10-3.32 (m, 3H), 2.73-2.97 (m, 3H). 19F NMR (376.5 MHz, DMSO-d6) δ -119.21, -119.78. LC-MS: Rt =1.289 min, (ESI) *m/z.* $[M+H]^+$ 448.3;<br>$C_{23}H_{22}FN_7O_2$ |
| 169 | 1H NMR (400 MHz, DMSO-d6) δ 8.41 - 8.63 (m, 1 H),7.92 - 8.15 (m, 1 H), 7.71 - 7.91 (m, 2 H),7.33 - 7.56 (m, 3 H), 7.14 - 7.27 (m, 2 H), 6.88 (t, J=6.75 Hz, 1 H), 4.34 - 5.02 (m, 2 H), 2.82 - 3.18 (m, 2 H), 2.52 - 2.81 (m, 2 H), 1.93 - 2.26 (m, 3 H), 1.56 - 1.87 (m, 3 H). LC-MS: Rt =1.462 min, (ESI) *m/z.* $[M+H]^+$ 456.2;<br>$C_{25}H_{22}FN_7O$ | 170 | 1H NMR (400 MHz, DMSO-d6) δ 8.97 (s, 1H), 8.44-8.55 (m, 2H), 7.96 (s, 1H), 7.51-7.60 (m, 2H), 7.38 (d, J=8.14 Hz, 1H), 7.16-7.26 (m, 1H), 6.88 (t, J=6.27 Hz, 1H), 6.82 (br s, 2H), 5.01-5.39 (m, 1H), 4.53-4.71 (m, 1H), 4.11-4.48 (m, 1H), 3.84 (dd, J=3.85, 11.77 Hz, 1H), 3.62-3.77 (m, 2H), 2.61 (s, 3H), 0.94 (d, J=7.04 Hz, 3H). LC-MS: Rt =2.003 min, (ESI) *m/z.* $[M+H]^+$ 442.2;<br>$C_{24}H_{23}N_7O_2$ |
| 171 | 1H NMR (400 MHz, DMSO-d6) δ 8.47-8.58 (m, 1H), 7.85-8.46 (m, 2H), 7.75-7.85 (s, 1H), 7.50-7.58 (m, 1H), 7.43 (br s, 2H), 7.20-7.32 (m, 2H), 6.89 (t, J=6.71 Hz, 1H), 4.85-5.92 (m, 1H), 3.39-3.62 (m, 3H), 3.20 (s, 3H), 3.05-3.28 (m, 1H) 2.92 (s, 3H), 1.50-1.75 (m, 3H). 19F NMR (376.5 MHz, DMSO-d6) δ -118.53, -119.65, -120.69. LC-MS: Rt =1.383 min, (ESI) *m/z.* $[M+H]^+$ 462.3;<br>$C_{24}H_{24}FN_7O_2$ | 172 | 1H NMR (400 MHz, DMSO-d6) δ 9.10-9.39 (m, 1H), 9.00 (s, 1H), 8.16-8.74 (m, 1H), 8.06 (s, 1H), 7.80 (d, J=7.53 Hz, 1H), 7.48 (d, J=9.54 Hz, 1H), 6.74-7.36 (m, 3H), 4.16-5.89 (m, 2H), 3.49-3.96 (m, 4H), 2.55-2.68 (m, 3H), 0.92 (d, J=6.53 Hz, 3H). 19F NMR (376.5 MHz, DMSO-d6) δ -60.43, -118.60, -119.72. LC-MS: Rt =1.583 min, (ESI) *m/z.* $[M+H]^+$ 528.2;<br>$C_{25}H_{21}F_4N_7O_2$ |

(continued)

| | HNMR/LCMS | | HNMR/LCMS |
|---|---|---|---|
| 173 | 1H NMR (400 MHz, DMSO-d6) δ 9.17-9.31 (m, 1H), 7.94-8.30 (m, 2H), 7.77-7.87 (m, 1H), 7.69-7.76 (m, 1H), 7.38-7.56 (m, 3H), 7.23 (d, J=11.00 Hz, 1H), 4.54-4.89 (m, 2H), 2.78-2.98 (m, 3H). 19F NMR (376.5 MHz, DMSO-d6) δ -60.38, -60.44, -118.63, -119.29. LC-MS: Rt =1.503 min, (ESI) *m/z.* $[M+H]^+$ 475.2; $C_{22}H_{14}D_3F_4N_7O$ | 174 | 1H NMR (400 MHz, DMSO-d6) δ 8.22-8.61 (m, 2H), 7.87-8.07 (m, 1H), 7.78 (s, 1H), 7.47-7.55 (m, 1H), 7.41 (br s, 2H), 7.11-7.30 (m, 2H), 6.88 (t, J=6.65 Hz, 1H), 4.12-5.23 (m, 2H), 3.51-3.92 (m, 3H), 2.92 (s, 3H), 0.88 (d, J=6.78 Hz, 3H). 19F NMR (376.5 MHz, DMSO-d6) δ -119.26, -120.20. LC-MS: Rt =2.304 min, (ESI) *m/z.* $[M+H]^+$ 460.2; $C_{24}H_{22}FN_7O_2$ |
| 175 | 1H NMR (400 MHz, DMSO-d6) δ 8.40-8.64 (m, 1H), 7.95-8.32 (m, 1H), 7.69-7.94 (m, 2H), 7.40-7.61 (m, 3H), 7.15-7.36 (m, 2H), 6.89 (t, J=6.60 Hz, 1H), 6.11-6.50 (m, 1H), 4.53-5.04 (m, 2H), 3.63-4.10 (m, 2H), 2.72-2.98 (m, 3H). 19F NMR (376.5 MHz, DMSO-d6) δ -119.26, -119.63, -120.56, -121.90. LC-MS: Rt =1.400 min, (ESI) *m/z.* $[M+H]^+$ 452.2; $C_{22}H_{18}F_3N_7O$ | 176 | 1H NMR (400 MHz, DMSO-d6) δ 8.86-9.15 (m, 1H), 8.17-8.80 (m, 2H), 7.92 (br s, 1H), 7.57 (d, J=7.78 Hz, 1H), 6.72-7.34 (m, 5H), 5.41-5.85 (m, 0.2H), 4.28-5.16 (m, 1.8H), 3.53-3.92 (m, 4H), 2.60 (s, 3H), 0.94 (d, J=6.53 Hz, 3H). 19F NMR (376.5 MHz, DMSO-d6) δ -119.70, -120.05. LC-MS: Rt =2.166 min, (ESI) *m/z.* $[M+H]^+$ 460.2; $C_{24}H_{22}FN_7O_2$ |
| 177 | 1H NMR (400 MHz, DMSO-d6) δ 8.41-8.58 (m, 1H), 8.21 (s, 0.21H, HCOOH), 7.91-8.15 (m, 1H), 7.65-7.90 (m, 2H), 7.33-7.59 (m, 3H), 7.13-7.32 (m, 2H), 6.87 (t, J=6.53 Hz, 1H), 4.57-4.98 (m, 2H), 4.05-4.57 (m, 1H), 3.71-4.01 (m, 1H), 3.44-3.52 (m, 3H), 2.91-3.16 (m, 3.9H), 2.58-2.65 (m, 0.6H), 1.91-2.03 (m, 2.5H). 19F NMR (376.5 MHz, DMSO-d6) δ -119.18, -119.36, -119.98. LC-MS: Rt =1.349 min, (ESI) *m/z.* $[M+H]^+$ 474.3; $C_{24}H_{23}F_6N_7OS$ | 178 | 1H NMR (400 MHz, DMSO-d6) δ 8.22-8.61 (m, 2H), 7.87-8.07 (m, 1H), 7.78 (s, 1H), 7.47-7.55 (m, 1H), 7.41 (br s, 2H), 7.11-7.30 (m, 2H), 6.88 (t, J=6.65 Hz, 1H), 4.12-5.23 (m, 2H), 3.51-3.92 (m, 3H), 2.92 (s, 3H), 0.88 (d, J=6.78 Hz, 3H). 19F NMR (376.5 MHz, DMSO-d6) δ -119.26, -120.20. LC-MS: Rt =2.304 min, (ESI) *m/z.* $[M+H]^+$ 460.2; $C_{24}H_{22}FN_7O_2$ |
| 179 | 1H NMR (400 MHz, DMSO-d6) δ 8.41-8.61 (m, 1H), 7.93-8.13 (m, 1H), 7.69-7.89 (m, 2H), 7.34-7.58 (m, 3H), 7.12-7.31 (m, 2H), 6.87 (t, J=6.71 Hz, 1H), 4.11-5.02 (m, 3H), 2.57-3.02 (m, 3H), 1.78-2.31 (m, 4H), 1.30-1.70 (m, 2H). 19F NMR (376.5 MHz, DMSO-d6) δ -119.35, -119.94. LC-MS: Rt =1.452 min, (ESI) *m/z.* $[M+H]^+$ 444.3; $C_{24}H_{22}FN_7O$ | 180 | 1H NMR (400 MHz, DMSO-d6) δ 9.15-9.31 (m, 1H), 7.94-8.28 (m, 2H), 7.66-7.88 (m, 2H), 7.34-7.55 (m, 3H), 7.16-7.29 (m, 1H), 4.52-4.91 (m, 2H), 3.43-3.52 (m, 2H), 2.78-3.00 (m, 3H), 1.03-1.19 (m, 3H). 19F NMR (376.5 MHz, DMSO-d6) δ -60.37, -60.44, -119.21, -119.92. LC-MS: Rt =1.580 min, (ESI) *m/z.* $[M+H]^+$ 486.3; $C_{23}H_{19}F_4N_7O$ |
| 181 | 1H NMR (400 MHz, DMSO-d6) δ 9.13 - 9.28 (m, 1 H) 7.98 (br s, 2 H) 7.54 - 7.88 (m, 3 H) 7.36 - 7.53 (m, 3 H) 7.12 - 7.32 (m, 1 H) 7.02 (br d, J=10.88 Hz, 1 H) 4.90 - 5.20 (m, 2 H) 3.54 - 3.82 (m, 3 H) 2.73 - 2.94 (m, 3 H). 19F NMR (376 MHz, DMSO-d6) δ -60.66 (s, 1 F) -119.61 - -117.01 (m, 1 F). LC-MS: Rt =1.496 min, (ESI) *m/z.* $[M+H]^+$ 538.2; $C_{25}H_{19}F_4N_9O$ | 182 | 1H NMR (400 MHz, DMSO-d6) δ 8.40-8.61 (m, 1H), 7.97-8.28 (m, 1H), 7.69-7.92 (m, 2H), 7.40-7.62 (m, 3H), 7.19-7.34 (m, 2H), 6.88 (t, J=6.49 Hz, 1H), 4.59-5.05 (m, 2H), 4.10-4.59 (m, 2H), 2.71-2.96 (m, 3H). 19F NMR (376.5 MHz, DMSO-d6) δ -68.10, -68.99, -119.46, -120.09. LC-MS: Rt =1.337 min, (ESI) *m/z.* $[M+H]^+$ 444.3; $C_{22}H_{17}F_4N_7O$ |

(continued)

| | HNMR/LCMS | | HNMR/LCMS |
|---|---|---|---|
| 183 | 1H NMR (400 MHz, DMSO-d6) δ 9.13 - 9.28 (m, 1 H) 7.98 (br s, 2 H) 7.54 - 7.88 (m, 3 H) 7.36 - 7.53 (m, 3 H) 7.12 - 7.32 (m, 1 H) 7.02 (br d, J=10.88 Hz, 1 H) 4.90 - 5.20 (m, 2 H) 3.54 - 3.82 (m, 3 H) 2.73 - 2.94 (m, 3 H). 19F NMR (376 MHz, DMSO-d6) δ -60.66 (s, 1 F) -119.61 - -117.01 (m, 1 F). LC-MS: Rt =1.496 min, (ESI) *m/z.* $[M+H]^+$ 538.2; $C_{25}H_{19}F_4N_9O$ | 184 | 1H NMR (400 MHz, DMSO-d6) δ 9.12-9.33 (m, 1H), 8.05-8.17 (m, 1H), 7.90-8.02 (m, 1H), 7.67-7.88 (m, 2H), 7.36-7.49 (m, 3H), 7.23 (d, J=10.78 Hz, 1H), 4.47-4.96 (m, 2H), 3.40 (br s, 2H), 2.71-2.96 (m, 3H), 1.90-2.20 (m, 1H), 0.66-0.99 (m, 6H). 19F NMR (376 MHz, DMSO-d6) δ -84.67--83.60 (m, 3F), -113.70--112.30 (m, 2F), -120.55--118.62 (m, 1F). LC-MS: Rt =1.887 min, (ESI) *m/z.* $[M+H]^+$ 564.3; $C_{26}H_{23}F_6N_7O$ |
| 185 | 1H NMR (400 MHz, DMSO-d6) δ 8.37-8.66 (m, 1H), 7.93-8.18 (m, 1H), 7.67-7.89 (m, 2H), 7.33-7.59 (m, 3H), 7.06-7.31 (m, 2H), 6.77-6.95 (m, 1H), 3.77-4.89 (m, 4H), 2.99-3.23 (m, 3H), 2.68-2.99 (m, 3H), 1.18-2.05 (m, 6H). 19F NMR (376.5 MHz, DMSO-d6) δ -119.06, -119.59, -120.35. LC-MS: Rt =1.454 min, (ESI) *m/z.* $[M+H]^+$ 488.3; $C_{26}H_{26}FN_7O_2$ | 186 | 1H NMR (400 MHz, DMSO-d6) δ 8.41-8.61 (m, 1H), 7.93-8.34 (m, 1H), 7.85-7.93 (m, 1H), 7.71-7.85 (m, 1H), 7.32-7.71 (m, 3H), 7.13-7.32 (m, 2H), 6.88 (t, J=6.63 Hz, 1H), 4.39-4.89 (m, 2H), 3.38-3.58 (m, 2H), 2.75-3.04 (m, 3H), 1.01-1.21 (m, 3H). 19F NMR (376.5 MHz, DMSO-d6) δ -119.31, -119.99. LC-MS: Rt =1.329 min, (ESI) *m/z.* $[M+H]^+$ 418.2; $C_{22}H_{20}FN_7O$ |
| 187 | 1H NMR (400 MHz, DMSO-d6) δ 8.43-8.63 (m, 1H), 7.98-8.29 (m, 1H), 7.85-7.94 (m, 1H), 7.74-7.84 (m, 1H), 7.35-7.62 (m, 3H), 7.15-7.32 (m, 2H), 6.89 (t, J=6.65 Hz, 1H), 4.55-4.94 (m, 2H), 3.57-3.85 (m, 2H), 2.70-2.97 (m, 5H). 19F NMR (376.5 MHz, DMSO-d6) δ -119.41. LC-MS: Rt =1.241 min, (ESI) *m/z.* $[M+H]^+$ 443.2; $C_{23}H_{19}FN_8O$ | 188 | (400MHz, DMSO-d6) δ 8.77 - 8.46 (m, 3H), 7.98 (s, 1H), 7.88 - 7.65 (m, 3H), 7.55 (br d, J=9.0 Hz, 1H), 7.28 (br s, 1H), 6.91 (br t, J=6.5 Hz, 1H), 4.63 (br s, 2H), 3.73 - 3.51 (m, 2H), 3.03 - 2.62 (m, 3H), 1.25 - 1.06 (m, 3H). LC-MS: Rt =2.973 min, (ESI) *m/z.* $[M+H]^+$ 401.2; $C_{21}H_{20}N_8O$ |
| 189 | 1H NMR (400 MHz, DMSO-d6) δ 9.01 (s, 0.7H), 8.66 (s, 0.3H), 8.53 (d, J=6.60 Hz, 0.7H), 8.31 (d, J=6.60 Hz, 0.3H), 8.04-8.20 (m, 1H), 7.35-7.86 (m, 2H), 7.08-7.26 (m, 2H), 6.91-7.08 (m, 2H), 6.71-6.91 (m, 1H), 4.19-5.11 (m, 3H), 2.75-2.86 (m, 1H), 2.63-2.70 (m, 1H), 2.56-2.63 (m, 3H), 2.34-2.46 (m, 1H), 2.15-2.27 (m, 3H), 1.92-2.15 (m, 2H). 19F NMR (376.5 MHz, DMSO-d6) δ -118.93, -119.11, -119.89. LC-MS: Rt =1.824 min, (ESI) *m/z.* $[M+H]^+$ 572.3; $C_{24}H_{23}F_6N_7OS$ | 190 | 1H NMR (400MHz, DMSO-d6) δ 8.37 - 7.75 (m, 2H), 8.39 - 7.73 (m, 1H), 7.58 - 7.39 (m, 3H), 7.33 - 7.19 (m, 2H), 7.07 - 6.93 (m, 1H), 6.95 - 6.42 (m, 1H), 4.90 - 4.47 (m, 2H), 3.27 - 3.03 (m, 1H), 3.33 - 3.03 (m, 1H), 2.89 (s, 3H), 2.23 - 1.95 (m, 1H), 1.00 - 0.66 (m, 6H). 19F NMR (376MHz, DMSO-d6) δ -119.073, -120.192; 158.442, -159.220. LC-MS: Rt =1.97 min, (ESI) *m/z.* $[M+H]^+$ 464.1; $C_{24}H_{23}F_2N_7O$ |
| 191 | 1H NMR (400 MHz, DMSO-d6) δ 8.65 (d, J=6.16 Hz, 1H), 8.49 (br d, J=6.60 Hz, 1H), 8.30 (s, 1H), 7.84-7.93 (m, 2H), 7.58 (br d, J=17.83 Hz, 2H), 7.47-7.53 (m, 1H), 7.20-7.26 (m, 1H), 6.88 (q, J=7.41 Hz, 1H), 4.78 (br d, J=6.16 Hz, 2H), 3.46-3.62 (m, 2H), 2.88-3.00 (m, 3H), 1.15-1.25 (m, 3H) LC-MS: Rt =1.640 min, (ESI) *m/z.* $[M+H]^+$ 401.2; $C_{21}H_{20}N_8O$ | 192 | 1H NMR (400 MHz, CHLOROFORM-d) δ 8.28-8.68 (m, 1H), 7.57-8.19 (m, 3H), 7.07-7.57 (m, 5H), 6.75-6.98 (m, 1H), 5.49-5.86 (m, 1H), 4.44-5.01 (m, 2H), 3.74-4.41 (m, 2H), 2.93 (s, 3H), 1.18-1.96 (m, 6H). 19F NMR (376.5 MHz, DMSO-d6) δ -118.56, -120.12. LC-MS: Rt =1.365 min, (ESI) *m/z.* $[M+H]^+$ 474.3; $C_{25}H_{24}FN_7O_2$ |

(continued)

| | HNMR/LCMS | | HNMR/LCMS |
|---|---|---|---|
| 193 | 1H NMR (400MHz, DMSO-d6) δ 9.18 (s, 1H), 8.47 (br s, 1H), 8.33 - 8.14 (m, 1H), 8.08 (s, 1H), 7.87 - 7.68 (m, 5H), 7.47 (br d, J=8.4 Hz, 1H), 5.09 (s, 2H), 3.68 (br s, 3H), 2.71 (s, 3H). 19F NMR (376MHz, DMSO-d6) δ -60.427. LC-MS: Rt =3.535 min, (ESI) *m/z.* $[M+H]^+$ 521.3; $C_{24}H_{19}F_3N_{10}O$ | 194 | 1H NMR (400 MHz, DMSO-d6) δ 8.43-8.60 (m, 1H), 7.93-8.23 (m, 1H), 7.84-7.93 (m, 1H), 7.75-7.84 (m, 1H), 7.35-7.56 (m, 4H), 7.18-7.29 (m, 1H), 6.82-6.92 (m, 1H), 3.84-5.07 (m, 2H), 3.01-3.32 (m, 2H), 2.78-2.96 (m, 3H), 1.00-1.23 (m, 3H). LC-MS: Rt =1.390 min, (ESI) *m/z.* $[M+H]^+$ 434.2; $C_{22}H_{20}ClN_7O$ |
| 195 | 1H NMR (400 MHz, DMSO-d6) δ 8.82 (br d, J=5.72 Hz, 1H), 8.06 (br s, 1H), 7.97 (br d, J=5.72 Hz, 1H), 7.65-7.84 (m, 3H), 7.47 (br s, 2H), 7.25 (br s, 1H), 7.04 (br d, J=5.94 Hz, 2H), 5.10 (br s, 2H), 3.60 (br s, 3H), 2.78-3.06 (m, 3H). LC-MS: Rt =1.453 min, (ESI) *m/z.* $[M+H]^+$ 538.2; $C_{25}H_{19}F_4N_9O$ | 196 | 1H NMR (400MHz, DMSO-d6) δ 8.37 - 7.75 (m, 2H), 8.39 - 7.73 (m, 1H), 7.58 - 7.39 (m, 3H), 7.33 - 7.19 (m, 2H), 7.07 - 6.93 (m, 1H), 6.95 - 6.42 (m, 1H), 4.90 - 4.47 (m, 2H), 3.27 - 3.03 (m, 1H), 3.33 - 3.03 (m, 1H), 2.89 (s, 3H), 2.23 - 1.95 (m, 1H), 1.00 - 0.66 (m, 6H). 19F NMR (376MHz, DMSO-d6) δ -119.073, -120.192; 158.442, -159.220. LC-MS: Rt =1.97 min, (ESI) *m/z.* $[M+H]^+$ 464.1; $C_{24}H_{23}F_2N_7O$ |
| 197 | 1H NMR (400 MHz, DMSO-d6) δ 8.33-8.61 (m, 1H), 8.20 (s, 0.6H, HCOOH), 7.63-8.05 (m, 3H), 7.33-7.61 (m, 3H), 7.09-7.30 (m, 2H), 6.72-6.96 (m, 1H), 4.34-5.03 (m, 3H), 2.63-3.02 (m, 6H), 1.92-2.35 (m, 6H). 19F NMR (376.5 MHz, DMSO-d6) δ -119.22, -122.68. LC-MS: Rt =1.344 min, (ESI) *m/z.* $[M+H]^+$ 473.3; $C_{25}H_{25}FN_8O$ | 198 | 1H NMR (400 MHz, DMSO-d6) δ 9.33 (br s, 1H), 7.88-8.42 (m, 2H), 7.55-7.85 (m, 3H), 7.37-7.55 (m, 3H), 6.91-7.36 (m, 2H), 4.92-5.14 (m, 2H), 3.48-3.85 (m, 3H), 2.74-2.93 (m, 3H). 19F NMR (376 MHz, DMSO-d6) δ -120.51--115.64 (m, 1F). LC-MS: Rt =1.204 min, (ESI) *m/z.* $[M+H]^+$ 495.3; $C_{25}H_{19}FN_{10}O$ |
| 199 | 1H NMR (400 MHz, DMSO-d6) δ 8.41 - 8.56 (m, 2 H) 8.21 - 8.32 (m, 1 H) 7.99 - 8.14 (m, 1 H) 7.87 - 7.95 (m, 1 H) 7.76 - 7.86 (m, 2 H) 7.44 - 7.56 (m, 3 H) 7.16 (s, 2 H) 6.81 - 7.03 (m, 2 H) 5.15 - 5.26 (m, 2 H) 2.88 (s, 3 H). 19F NMR (376 MHz, DMSO-d6) δ -117.24 - -115.16 (m, 1 F). LC-MS: Rt =1.271 min, (ESI) *m/z.* $[M+H]^+$ 467.2; $C_{25}H_{19}FN_8O$ | 200 | 1H NMR (400 MHz, DMSO-d6) δ 9.23 (br s, 1H), 8.66 (br s, 1H), 8.45 (br s, 1H), 7.97-8.15 (m, 2H), 7.84-7.88 (m, 1H), 7.70 (br d, J=14.30 Hz, 2H), 7.57 (br s, 2H), 7.45 (br s, 1H), 7.11 (br s, 1H), 5.05-5.30 (m, 2H), 3.56-3.88 (m, 3H), 2.91 (s, 3H). LC-MS: Rt =1.484 min, (ESI) *m/z.* $[M+H]^+$ 521.3; $C_{24}H_{19}F_3N_{10}O$ |
| 201 | 1H NMR (400MHz, DMSO-d6) δ 8.53 - 8.36 (m, 1H), 8.28 - 7.92 (m, 1H), 7.88 - 7.75 (m, 2H), 7.55 - 7.37 (m, 3H), 7.29 - 7.16 (m, 2H), 4.87 - 4.43 (m, 2H), 3.58 (br t, J=4.2 Hz, 4H), 3.52 - 3.36 (m, 4H), 3.00 - 2.76 (m, 3H), 2.45 - 2.29 (m, 4H), 1.19 - 1.05 (m, 3H). 19F NMR (376MHz, DMSO-d6) δ -119.304, -120.046. LC-MS: Rt =3.633 min, (ESI) *m/z.* $[M+H]^+$ 517.3 $C_{27}H_{20}FN_3O_2$ | 202 | 1H NMR (400 MHz, DMSO-d6) δ 8.02-8.43 (m, 2H), 7.62-8.02 (m, 3H), 7.19-7.62 (m, 4H), 6.83-7.19 (m, 2H), 4.75-5.19 (m, 1H), 4.75-5.19 (m, 1H), 3.51-3.83 (m, 6H), 2.68-2.97 (m, 3H) LC-MS: Rt =1.319 min, (ESI) *m/z.* $[M+H]^+$ 500.3 $C_{25}H_{22}FN_9O_2$ |
| 203 | 1H NMR (400 MHz, DMSO-d6) δ 8.55-8.69 (m, 1H), 8.17 (br s, 1H), 7.79 (s, 1H), 7.64 (d, J=8.80 Hz, 1H), 7.44 (br s, 2H), 7.10-7.31 (m, 2H), 6.86 (t, J=6.60 Hz, 1H)124, 6.61 (br s, 1H), 4.61-5.85 (m, 2H), 3.50-4.04 (m, 3H), 3.28-3.36 (m, 1H), 2.68-3.06 (m, 3H), 0.80-1.01 (m, 3H). 19F NMR (376.5 MHz, DMSO-d6) δ -119.63 LC-MS: Rt =1.479 min, (ESI) *m/z.* $[M+H]^+$ 460.3; $C_{24}H_{22}FN_7O_2$ | 204 | 1H NMR (400 MHz, DMSO-d6) δ 8.31-8.45 (m, 1H), 7.92-8.17 (m, 1H), 7.72-7.85 (m, 2H), 7.58-7.69 (m, 1H), 7.44 (br s, 3H), 7.14-7.32 (m, 2H), 7.02 (br d, J=11.13 Hz, 1H), 4.83-5.08 (m, 2H), 3.53-3.86 (m, 3H), 3.39-3.48 (m, 2H), 2.64-2.95 (m, 3H), 2.16 (br s, 6H). 19F NMR (376 MHz, DMSO-d6) δ -120.02--116.80 (m, 1F).LC-MS: Rt =1.342 (ESI) *m/z.* $[M+H]^+$ 527.4; $C_{27}H_{27}FN_{10}O$ |

(continued)

| | HNMR/LCMS | | HNMR/LCMS |
|---|---|---|---|
| 205 | 1H NMR (400MHz, DMSO-d6) δ 9.06 - 8.84 (m, 1H), 8.31 (d, J=6.6 Hz, 1H), 8.24 - 8.11 (m, 1H), 7.52 (br t, J=7.5 Hz, 1H), 7.33 - 6.89 (m, 5H), 4.94 - 4.45 (m, 2H), 3.60 (br s, 2H), 2.61 (d, J=2.4 Hz, 3H), 1.22 - 1.07 (m, 3H).19F NMR (376MHz, DMSO-d6) δ -119.164, -119.681; -157.688, -158.649. C-MS: Rt =3.824 (ESI) *m/z.* $[M+H]^+$ 436.3<br>$C_{22}H_{19}F_2N_7O$ | 206 | 1H NMR (400 MHz, DMSO-d6) δ 8.14-8.38 (m, 2H), 7.75-8.01 (m, 1H), 7.35-7.55 (m, 3H), 7.18-7.31 (m, 2H), 6.85 (br d, J=7.04 Hz, 1H), 5.53-5.67 (m, 1H), 4.45-4.94 (m, 2H), 3.44 (br s, 2H), 2.77-2.97 (m, 3H), 1.48-1.70 (m, 6H), 0.98-1.26 (m, 3H). 19F NMR (376 MHz, DMSO-d6) δ -121.18--118.30 (m, 1F). LC-MS: Rt =1.370 min, (ESI) *m/z.* $[M+H]^+$ 476.3;<br>$C_{25}H_{26}FN_7O_2$ |
| 207 | 1H NMR (400 MHz, DMSO-d6) δ 9.25 - 9.27 (m, 1 H) 9.26 (s, 1 H) 9.14 - 9.19 (m, 1 H) 8.87 - 9.01 (m, 1 H) 8.06 (s, 1 H) 6.96 - 7.21 (m, 3 H) 4.62 - 4.95 (m, 2 H) 3.47 - 3.60 (m, 2 H) 2.58 - 2.63 (m, 3 H) 1.05 - 1.20 (m, 3 H). 19F NMR (376 MHz, DMSO-d6) δ -65.75 - -65.59 (m, 1 F) -119.33 - -119.19 (m, 1 F). LC-MS: Rt =1.520 min, (ESI) *m/z.* $[M+H]^+$ 487.3;<br>$C_{22}H_{18}F_4N_8O$ | 208 | 1H NMR (400 MHz, DMSO-d6) δ 8.14-8.38 (m, 2H), 7.75-8.01 (m, 1H), 7.35-7.55 (m, 3H), 7.18-7.31 (m, 2H), 6.85 (br d, J=7.04 Hz, 1H), 5.53-5.67 (m, 1H), 4.45-4.94 (m, 2H), 3.44 (br s, 2H), 2.77-2.97 (m, 3H), 1.48-1.70 (m, 6H), 0.98-1.26 (m, 3H). 19F NMR (376 MHz, DMSO-d6) δ -121.18--118.30 (m, 1F). LC-MS: Rt =1.370 min, (ESI) *m/z.* $[M+H]^+$ 476.3;<br>$C_{25}H_{26}FN_7O_2$ |
| 209 | 1H NMR (400 MHz, DMSO-d6) δ 12.55-12.90 (m, 1H), 8.89-9.00 (m, 1H), 8.12-8.30 (m, 1H), 7.95-8.10 (m, 1H), 7.76-7.90 (m, 1H), 7.52-7.73 (m, 2H), 7.46 (s, 2H), 7.16-7.37 (m, 1H), 6.97-7.10 (m, 1H), 5.10-5.25 (s, 2H), 2.60-2.98 (m, 3H). 19F NMR (376.5 MHz, DMSO-d6) δ -60.70, -60.82, -117.24, -119.11. LC-MS: Rt =1.490 min, (ESI) *m/z.* $[M+H]^+$ 485.3;<br>$C_{22}H_{16}F_4N_8O$ | 210 | 1H NMR (400MHz, DMSO-d6) δ 8.50 - 8.26 (m, 1H), 8.21 - 7.93 (m, 1H), 7.81 (br d, J=5.9 Hz, 2H), 7.63 (br s, 1H), 7.44 (s, 3H), 7.35 - 7.13 (m, 2H), 7.02 (br d, J=10.3 Hz, 1H), 5.12 - 4.86 (m, 2H), 3.86 - 3.46 (m, 9H), 2.96 - 2.69 (m, 3H), 2.44 - 2.30 (m, 4H). 19F NMR (376MHz, DMSO-d6) δ -117.195, -119.505. LC-MS: Rt =1.673 min, (ESI) *m/z.* $[M+H]^+$ 569.2;<br>$C_{29}H_{29}FN_{10}O_2$ |
| 211 | **1H** NMR (400 MHz, DMSO-d6) δ 8.50 (br s, 1H), 7.74-8.27 (m, 2H), 7.62 (br s, 1H), 7.29-7.52 (m, 3H), 6.91-7.29 (m, 2H), 4.74-5.27 (m, 6H), 3.59 (br s, 3H), 2.62-2.98 (m, 3H). LC-MS: Rt =1.982 min, (ESI) *m/z.* $[M+H]^+$ 473.3;<br>$C_{24}H_{21}FN_8O_2$ | 212 | 1H NMR (400 MHz, DMSO-d6) δ 9.15-9.31 (m, 1H), 8.33 (s, 1H), 8.07-8.16 (m, 1H), 7.92-8.03 (m, 1H), 7.76-7.86 (m, 1H), 7.58-7.75 (m, 2H), 7.41-7.55 (m, 3H), 7.18-7.33 (m, 1H), 4.71-5.17 (m, 2H), 3.53-3.83 (m, 3H), 2.80-2.97 (m, 3H). LC-MS: Rt =1.544 min, (ESI) *m/z.* $[M+H]^+$ 554.2;<br>$C_{25}H_{19}ClF_3N_9O$ |
| 213 | 1H NMR (400 MHz, DMSO-d6) δ 8.83 - 9.07 (m, 1 H) 8.34 - 8.50 (m, 2 H) 8.15 - 8.29 (m, 1 H) 7.62 - 7.72 (m, 1 H) 7.09 - 7.24 (m, 1 H) 6.96 - 7.07 (m, 2 H) 4.61 - 4.95 (m, 2 H) 3.51 - 3.61 (m, 2 H) 2.58 - 2.64 (m, 3 H) 1.05 - 1.19 (m, 3 H). 19F NMR (376 MHz, DMSO-d6) δ -65.19 - -64.80 (m, 1 F) -119.50 (br d, J=20.60 Hz, 1 F). LC-MS: Rt =1.633 min, (ESI) *m/z.* $[M+H]^+$ 487.3;<br>$C_{22}H_{18}F_4N_8O$ | 214 | 1H NMR (400 MHz, DMSO-d6) δ 7.35 - 8.50 (m, 7 H) 6.96 - 7.35 (m, 1 H) 4.53 - 5.22 (m, 2 H) 2.60 - 2.98 (m, 3 H) 1.05 - 1.27 (m, 3 H). 19F NMR (376 MHz, DMSO-d6) δ -58.95 - -58.53 (m, 1 F) -120.17 - -118.91 (m, 1 F). LC-MS: Rt =1.613 min, (ESI) *m/z.* $[M+H]^+$ 486.2;<br>$C_{23}H_{19}F_4N_7O$ |
| 215 | 1H NMR (400 MHz, DMSO-d6) δ 8.99 (s, 1H), 8.06-8.47 (m, 1H), 7.57 (br d, J=7.75 Hz, 1H), 7.33 (br d, J=7.63 Hz, 1H), 7.24 (s, 1H), 7.18 (br d, J=10.76 Hz, 1H), 7.05 (br s, 2H), 5.49-6.15 (m, 1H), 4.50-5.06 (m, 2H), 3.17 (br dd, J=7.75, 14.88 Hz, 2H), 2.63 (s, 3H), 0.83-1.04 (m, 3H) LC-MS: Rt =0.78 min, (ESI) *m/z.* $[M+H]^+$ 474.3;<br>$C_{23}H_{19}F_4N_5O_2$ | 216 | 1H NMR (400 MHz, DMSO-d6) δ 8.15 (s, 0.16H, HCOOH), 7.76-7.99 (m, 2H), 7.35-7.52 (m, 2H), 6.85-7.27 (m, 3H), 6.62-6.78 (m, 1H), 4.30-4.74 (m, 4H), 3.01-3.21 (m, 4H), 2.71-2.96 (m, 3H), 0.93-1.21 (m, 3H). 19F NMR (376.5 MHz, DMSO-d6) δ -119.74, -119.89. LC-MS: Rt =1.623 min, (ESI) *m/z.* $[M+H]^+$ 420.3;<br>$C_{23}H_{22}FN_5O_2$ |

(continued)

| | HNMR/LCMS | | HNMR/LCMS |
|---|---|---|---|
| 217 | 1H NMR (400 MHz, DMSO-d6) δ 8.75-9.25 (m, 1H), 8.14-8.49 (m, 2H), 7.64-7.86 (m, 1H), 7.46 (br d, J=7.82 Hz, 1H), 7.12-7.24 (m, 2H), 7.02 (br s, 2H), 4.31-4.93 (m, 2H), 3.49-3.65 (m, 4H), 3.00-3.32 (m, 3H), 2.54-2.77 (m, 6H), 2.43 (br s, 4H), 1.04-1.18 (m, 3H), ~90% of purity. LC-MS: Rt =0.774 min, (ESI) *m/z.* $[M+H]^+$ 531.3; $C_{23}H_{31}FN_3O$; | 218 | 1H NMR (400 MHz, DMSO-d6) δ 8.15 (s, 0.16H, HCOOH), 7.76-7.99 (m, 2H), 7.35-7.52 (m, 2H), 6.85-7.27 (m, 3H), 6.62-6.78 (m, 1H), 4.30-4.74 (m, 4H), 3.01-3.21 (m, 4H), 2.71-2.96 (m, 3H), 0.93-1.21 (m, 3H). 19F NMR (376.5 MHz, DMSO-d6) δ -119.74, -119.89. LC-MS: Rt =1.623 min, (ESI) *m/z.* $[M+H]^+$ 420.3; $C_{23}H_{22}FN_5O_2$ |
| 219 | 1H NMR (400 MHz, DMSO-d6) δ 7.88-8.15 (m, 1H), 7.81 (s, 1H), 7.41-7.60 (m, 3H), 7.14-7.28 (m, 1H), 6.84-7.13 (m, 3H), 6.62-6.77 (m, 1H), 4.87 (s, 2H), 4.38-4.61 (m, 2H), 3.51-3.91 (m, 3H), 3.00-3.22 (m, 2H), 2.64-2.97 (m, 3H). 19F NMR (376.5 MHz, DMSO-d6) δ -117.53, -119.22. LC-MS: Rt =1.720 min, (ESI) *m/z.* $[M+H]^+$ 472.1; $C_{25}H_{22}FN_7O_2$ | 220 | 1H NMR (400 MHz, DMSO-d6) δ 9.13-9.40 (m, 1H), 8.86-9.09 (m, 1H), 8.11-8.31 (m, 1H), 7.71-7.98 (m, 1H), 7.42 (br dd, J=9.35, 19.50 Hz, 1H), 7.16 (br dd, J=10.94, 19.62 Hz, 1H), 7.01 (br d, J=8.93 Hz, 2H), 6.56-6.83 (m, 1H), 4.54-5.07 (m, 2H), 3.53 (br d, J=5.38 Hz, 1H), 3.29 (br s, 1H), 2.61 (br d, J=9.54 Hz, 3H), 1.02-1.19 (m, 3H). LC-MS: Rt =0.899 min, (ESI) *m/z.* $[M+H]^+$ 486.3; $C_{23}H_{19}F_4N_7O$ |
| 221 | 1H NMR (400 MHz, DMSO-d6) δ 8.93 (s, 1H), 8.13 (br d, J=6.16 Hz, 1H), 7.47 (br d, J=7.26 Hz, 1H), 7.29 (s, 1H), 7.22 (t, J=7.70 Hz, 1H), 6.86-7.13 (m, 4H), 6.81 (s, 1H), 6.72 (d, J=7.92 Hz, 1H), 6.56 (br d, J=6.16 Hz, 1H), 4.77 (br t, J=9.35 Hz, 1H), 4.49 (br dd, J=3.30, 10.34 Hz, 1H), 2.60 (s, 3H). 19F NMR (376 MHz, DMSO-d6) δ -117.76 (br s, 1F). LC-MS: Rt =0.802 min, (ESI) *m/z.* $[M+H]^+$458.3; $C_{24}H_{20}FN_7O_2$ | 222 | 1H NMR (400 MHz, DMSO-d6) δ 8.81-9.21 (m, 1H), 8.33-8.63 (m, 1H), 7.74-8.31 (m, 3H), 7.40-7.68 (m, 1H), 6.91-7.28 (m, 3H), 5.37-5.90 (m, 1H), 4.71-5.05 (m, 1H), 4.38-4.70 (m, 1H), 3.80-3.93 (m, 3H), 3.22 (br d, J=6.75 Hz, 2H), 2.61 (s, 3H), 0.69-1.12 (m, 3H). LC-MS: Rt =2.220 min, (ESI) *m/z.* $[M+H]^+$ 487.3; $C_{25}H_{23}FN_8O_2$ |
| 223 | 1H NMR (400 MHz, DMSO-d6) δ 9.00 (s, 1H), 8.89 (br d, J=16.51 Hz, 1H), 8.57 (br d, J=3.67 Hz, 1H), 8.15-8.48 (m, 1H), 7.95-8.15 (m, 1H), 7.41-7.66 (m, 2H), 6.83-7.41 (m, 5H), 5.45-6.29 (m, 1H), 4.61 (br d, J=10.15 Hz, 2H), 3.15-3.29 (m, 2H), 2.63 (s, 3H), 0.78-1.12 (m, 3H). LC-MS: Rt =0.807 min, (ESI) *m/z.* $[M+H]^+$ 483.3; $C_{27}H_{23}FN_6O_2$ | 224 | 1H NMR (400 MHz, DMSO-d6) δ 7.92-8.19 (m, 1H), 7.83 (s, 1H), 7.57 (br s, 1H), 7.47 (s, 2H), 7.31 (br d, J=7.83 Hz, 1H), 7.13-7.28 (m, 2H), 5.48-6.23 (m, 1H), 4.49-5.02 (m, 2H), 3.18 (br d, J=8.44 Hz, 2H), 2.96 (br s, 3H), 0.81-1.11 (m, 3H). LC-MS: Rt =0.78 min, (ESI) *m/z.* $[M+H]^+$ 474.3; $C_{23}H_{19}F_4N_5O_2$ |
| 225 | 1H NMR (400 MHz, DMSO-d6) δ 8.93 (s, 1H), 8.13 (br d, J=6.16 Hz, 1H), 7.47 (br d, J=7.26 Hz, 1H), 7.29 (s, 1H), 7.22 (t, J=7.70 Hz, 1H), 6.86-7.13 (m, 4H), 6.81 (s, 1H), 6.72 (d, J=7.92 Hz, 1H), 6.56 (br d, J=6.16 Hz, 1H), 4.77 (br t, J=9.35 Hz, 1H), 4.49 (br dd, J=3.30, 10.34 Hz, 1H), 2.60 (s, 3H). 19F NMR (376 MHz, DMSO-d6) δ -117.76 (br s, 1F). LC-MS: Rt =0.802 min, (ESI) *m/z.* $[M+H]^+$458.3; $C_{24}H_{20}FN_7O_2$ | 226 | 1H NMR (400 MHz, DMSO-d6) δ 9.00 (s, 1H), 8.25 (br d, J=6.38 Hz, 1H), 7.14-7.51 (m, 3H), 6.85-7.13 (m, 4H), 5.40-6.21 (m, 1H), 4.36-4.88 (m, 2H), 2.99-3.20 (m, 1H), 3.07 (br d, J=6.82 Hz, 1H), 2.63 (s, 3H), 0.55-1.09 (m, 3H). 19F NMR (376 MHz, DMSO-d6) δ -119.88 (br d, J=16.02 Hz, 1F). LC-MS: Rt =0.842 min, (ESI) *m/z.* $[M+H]^+$ 406.2; $C_{22}H_{20}FN_5O_2$ |

(continued)

| | HNMR/LCMS | | HNMR/LCMS |
|---|---|---|---|
| 227 | 1H NMR (400 MHz, DMSO-d6) δ 8.60 (d, J=7.04 Hz, 1H), 7.89 (d, J=6.38 Hz, 1H), 7.81 (s, 1H), 7.57-7.72 (m, 2H), 7.48 (s, 2H), 7.15-7.34 (m, 1H), 7.07 (d, J=11.22 Hz, 1H), 6.85 (t, J=6.71 Hz, 1H), 6.58 (s, 1H), 5.07 (s, 2H), 3.54 (s, 3H), 2.85 (s, 3H), 1.77 (s, 3H). 19F NMR (376 MHz, DMSO-d6) δ -117.27 (br s, 1F). LC-MS: Rt =0.719 min, (ESI) *m/z.* $[M+H]^+$ 484.4; $C_{25}H_{22}FN_9O$ | 228 | 1H NMR (400 MHz, DMSO-d6) δ 9.00 (s, 1H), 8.06-8.52 (m, 2H), 7.86 (d, J=7.95 Hz, 1H), 6.83-7.48 (m, 6H), 5.14-6.64 (m, 1H), 4.39-4.93 (m, 2H), 3.85 (d, J=5.01 Hz, 3H), 2.56-2.70 (m, 6H). LC-MS: Rt =0.761 min, (ESI) *m/z.* $[M+H]^+$ 472.2; $C_{25}H_{22}FN_7O_2$ |
| 229 | 1H NMR (400 MHz, DMSO-d6) 8.52 (br s, 1H), 8.03 (br s, 2H), 7.91 (br s, 1H), 7.82 (br s, 1H), 7.75 (br s, 1H), 7.49 (br s, 3H), 7.22 (br s, 1H), 6.97 (br s, 1H), 6.87 (br s, 1H), 6.68 (br s, 1H), 5.17 (br s, 2H), 3.63-3.82 (m, 3H), 2.92 (br s, 3H). 19F NMR (376 MHz, DMSO-d6) -116.39 (s, 1F) LC-MS: Rt =0.800 min, (ESI) *m/z.* $[M+H]^+$ 497.1; $C_{26}H_{21}FN_3O_2$ | 230 | H NMR (400 MHz, DMSO-d6) δ 8.99 (s, 1H), 8.08-8.54 (m, 2H), 7.62 (d, J=1.63 Hz, 1H), 7.16 (d, J=10.88 Hz, 1H), 7.04 (br s, 2H), 5.42-5.78 (m, 1H), 4.75-5.07 (m, 1H), 4.54-4.75 (m, 1H), 3.28 (br d, J=7.38 Hz, 1H), 3.18 (br d, J=7.50 Hz, 1H), 2.61 (d, J=3.13 Hz, 3H), 0.87-1.04 (m, 3H). 19F NMR (376 MHz, DMSO-d6) δ -120.15-118.26 (m, 1F). LC-MS: Rt =0.847 min, (ESI) *m/z.* $[M+H]^+$ 485.4; $C_{21}H_{18}BrFN_6O_2$ |
| 231 | 1H NMR (400 MHz, DMSO-d6) δ 8.50 (br d, J=3.38 Hz, 1H), 8.18 (s, 1H), 8.08 (d, J=8.63 Hz, 1H), 7.90 (d, J=6.38 Hz, 1H), 7.79 (s, 1H), 7.65 (s, 1H), 7.46 (s, 2H), 7.22 (dd, J=4.44, 9.19 Hz, 1H), 7.05 (d, J=11.13 Hz, 1H), 5.04 (br s, 2H), 3.47-3.71 (m, 3H), 2.85 (s, 3H), 1.79 (s, 3H). 19F NMR (376 MHz, DMSO-d6) δ -120.22--116.68 (m, 1F). LC-MS: Rt =1.24 min, (ESI) *m/z.* $[M+H]^+$ 485.3; $C_{24}H_{21}FN_{10}O$ | 232 | 1H NMR (400 MHz, DMSO-d6) δ8.87-9.41 (m, 1H), 7.64-8.23 (m, 4H), 7.35-7.59 (m, 3H), 7.07-7.32 (m, 1H), 4.36-5.16 (m, 3H), 3.47-4.08 (m, 4H), 2.60-3.08 (m, 3H), 1.97-2.30 (m, 2H). 19F NMR (376 MHz, DMSO-d6) δ -60.42 (br d, J=43.49 Hz, 3F), -122.45--117.86 (m, 1F). LC-MS: Rt =1.009 min, (ESI) *m/z.* $[M+H]^+$ 528.3; $C_{25}H_{21}F_4N_7O_2$ |
| 233 | 1H NMR (400 MHz, DMSO-d6) δ 9.25 (s, 1H), 8.97 (s, 1H), 8.15 (d, J=6.38 Hz, 1H), 8.04 (s, 1H), 7.73 (d, J=9.46 Hz, 1H), 7.57 (s, 1H), 7.47 (dd, J=1.54, 9.46 Hz, 1H), 6.63-7.24 (m, 3H), 5.01 (s, 2H), 3.52 (s, 3H), 2.60 (s, 3H), 1.79 (s, 3H). 19F NMR (376 MHz, DMSO-d6) δ -60.40 (br s, 3F), -116.73 (br s, 1F). LC-MS: Rt =0.994 min, (ESI) *m/z.* $[M+H]^+$ 522.3; $C_{26}H_{21}F_4N_9O$ | 234 | 1H NMR (400 MHz, DMSO-d6) δ 8.60 (br s, 1H), 8.23 (s, 1H), 7.70-8.10 (m, 2H), 7.38-7.70 (m, 3H), 7.04-7.38 (m, 2H), 6.84 (br s, 1H), 6.27-6.60 (m, 1H), 4.36-5.18 (m, 3H), 3.79-4.04 (m, 2H), 3.45 (br s, 2H), 2.97 (br s, 2H), 2.38-2.50 (m, 1H), 2.11 (br s, 2H). LC-MS: Rt =1.081 min, (ESI) *m/z.* $[M+H]^+$ 460.4; $C_{24}H_{22}FN_7O_2$ |
| 235 | 1H NMR (400 MHz, DMSO) δ 9.10 (s, 1H), 8.88 (s, 1H), 8.09 (d, J=6.38 Hz, 1H), 7.82 (d, J=9.24 Hz, 1H), 7.57 (s, 1H), 7.38 (d, J=9.24 Hz, 1H), 6.94 (d, J=10.78 Hz, 1H), 6.65-6.88 (m, 3H), 5.03 (br s, 2H), 3.46 (s, 3H), 2.56 (s, 3H), 1.73 (s, 3H). LC-MS: Rt =1.113 min, (ESI) *m/z.* $[M+H]^+$ 552.1; $C_{26}H_{21}F_4N_9O$ | 236 | 1H NMR (400 MHz, DMSO-d6) δ 9.25 (s, 1H), 8.04 (s, 1H), 7.88 (br d, J=6.38 Hz, 1H), 7.81 (s, 1H), 7.73 (d, J=9.46 Hz, 1H), 7.62 (s, 1H), 7.37-7.56 (m, 3H), 7.07 (d, J=11.22 Hz, 1H), 5.04 (br s, 2H), 3.55 (s, 3H), 2.84 (s, 3H), 1.79 (s, 3H). 19F NMR (376 MHz, DMSO-d6) δ -60.40, -117.07. LC-MS: Rt =1.017 min, (ESI) *m/z.* $[M+H]^+$ 522.3; $C_{26}H_{21}F_4N_9O$ |

(continued)

| | HNMR/LCMS | | HNMR/LCMS |
|---|---|---|---|
| 237 | 1H NMR (400 MHz, DMSO-d6) δ 12.20-12.38 (m, 1H), 8.75-8.97 (m, 1H), 8.14-8.65 (m, 1H), 7.56-7.67 (m, 2H), 7.35-7.54 (m, 1H), 7.12-7.22 (m, 2H), 7.03 (br s, 2H), 6.95 (d, J=11.04 Hz, 1H), 4.82-5.13 (m, 2H), 3.51-3.70 (m, 3H), 2.59-2.70 (m, 3H), 1.75-2.06 (m, 3H). 19F NMR (376.5 MHz, DMSO-d6) δ -116.39, -118.62 LC-MS: Rt =0.790 min, (ESI) *m/z.* $[M+H]^+$ 484.1; $C_{25}H_{22}FN_9O$ | 238 | 1H NMR (400 MHz, DMSO) δ 9.26 (s, 1H), 7.84-7.96 (m, 2H), 7.80 (s, 1H), 7.64 (s, 1H), 7.48 (s, 2H), 7.42 (dd, J=1.21, 9.35 Hz, 1H), 7.06 (d, J=11.22 Hz, 1H), 6.78 (s, 1H), 5.10 (s, 2H), 3.53 (s, 3H), 2.84 (s, 3H), 1.77 (s, 3H). LC-MS: Rt =1.113 min, (ESI) *m/z.* $[M+H]^+$ 552.1; $C_{26}H_{21}F_4N_9O$ |
| 239 | 1H NMR (400 MHz, DMSO-d6) δ 9.23 (br s, 1H), 7.87-8.56 (m, 2H), 7.68-7.84 (m, 2H), 7.43-7.55 (m, 3H), 7.01-7.37 (m, 2H), 6.17 (br s, 1H), 4.84-5.28 (m, 2H), 3.45-3.83 (m, 3H), 2.86 (br s, 3H). LC-MS: Rt =0.811 min, (ESI) *m/z.* $[M+H]^+$ 538.3; $C_{25}H_{19}F_4N_9O$ | 240 | 1H NMR (400 MHz, DMSO-d6) 8.91 (br s, 1H), 8.23 (br d, J=8.13 Hz, 1H), 7.97 (br d, J=6.75 Hz, 1H), 7.89 (br s, 1H), 7.81 (s, 1H), 7.34 (br s, 2H), 7.13 (br d, J=11.26 Hz, 1H), 6.50 (s, 1H), 5.17 (br s, 2H), 3.23-3.28 (m, 3H), 2.87 (s, 3H), 1.96 (br s, 3H) 19F NMR (376 MHz, DMSO-d6) -60.85 (s, 3F), -118.24 (br s, 1F). LC-MS: Rt =0.759 min, (ESI) *m/z.* $[M+H]^+$ 513.3; $C_{24}H_{20}F_4N_8O$ |
| 241 | 1H NMR (400 MHz, DMSO-d6) 9.28 (br s, 1H), 8.10 (br s, 1H), 7.92 (br s, 1H), 7.82 (s, 1H), 7.73 (d, J=9.76 Hz, 1H), 7.55 (s, 2H), 7.47 (br d, J=8.63 Hz, 1H), 7.29 (br s, 1H), 7.11 (br d, J=10.38 Hz, 1H), 5.19 (br s, 1H), 5.04-5.14 (m, 1H), 3.59 (s, 3H), 2.86 (br s, 3H). 19F NMR (376 MHz, DMSO-d6) -60.47 (s, 3F), -118.15 (s, 1F), -173.54 (s, 1F). LC-MS: Rt =1.961 min, (ESI) *m/z.* $[M+H]^+$ *556.1;* $C_{25}H_{18}F_5N_9O$ | 242 | 1H NMR (400 MHz, DMSO-d6) δ 9.24 (br s, 1H), 8.22 (s, 1H), 7.95-8.16 (m, 2H), 7.74 (br d, J=9.02 Hz, 1H), 7.47 (br d, J=9.90 Hz, 1H), 6.92-7.38 (m, 4H), 6.19 (br s, 1H), 5.14 (br s, 2H), 4.29 (br s, 3H), 3.48 (br s, 3H).. LC-MS: Rt =0.786 min, (ESI) *m/z.* $[M+H]^+$ 538.3; $C_{25}H_{19}F_4N_9O$ |
| 243 | 1H NMR (400 MHz, DMSO-d6) δ 8.43 (br s, 1H), 7.76-7.97 (m, 1H), 7.72-8.01 (m, 2H), 7.39-7.66 (m, 3H), 7.03-7.36 (m, 3H), 6.17 (br s, 1H), 4.77-5.16 (m, 2H), 3.57 (br t, J=4.29 Hz, 4H), 3.37-3.50 (m, 6H), 2.86 (br s, 2H), 2.31-2.39 (m, 4H). LC-MS: Rt =0.614 min, (ESI) *m/z.* $[M+H]^+$ 569.4; $C_{29}H_{29}FN_{10}O_2$ | 244 | 1H NMR (400 MHz, DMSO-d6) δ 9.23 (br s, 1H), 7.86-8.62 (m, 2H), 7.71-7.84 (m, 2H), 7.40-7.64 (m, 3H), 7.28 (br s, 1H), 7.09 (br d, J=10.38 Hz, 1H), 6.27 (br s, 1H), 4.76-5.34 (m, 2H), 3.47-4.17 (m, 2H), 2.85 (br s, 3H), 0.90-1.26 (m, 3H). LC-MS: Rt =0.801 min, (ESI) *m/z.* $[M+H]^+$ 552.3; $C_{26}H_{21}F_4N_9O$ |
| 245 | 1H NMR (400 MHz, DMSO-d6) δ 12.99 (br s, 1H), 7.87-8.05 (m, 2H), 7.69-7.84 (m, 2H), 7.48-7.60 (m, 3H), 7.22 (br s, 1H), 6.97-7.13 (m, 1H), 6.22 (br s, 1H), 5.25 (br s, 2H), 3.54-3.66 (m, 3H), 2.87 (br s, 3H). LC-MS: Rt =0.774 min, (ESI) *m/z.* $[M+H]^+$ 538.2; $C_{25}H_{19}F_4N_9O$ | 246 | 1H NMR (400 MHz, DMSO-d6) δ8.52 (br d, J=4.63 Hz, 1H), 7.62-8.03 (m, 3H), 7.44-7.59 (m, 3H), 7.02-7.39 (m, 3H), 6.72-6.99 (m, 1H), 6.18 (br s, 1H), 4.67-5.25 (m, 2H), 3.44-3.79 (m, 3H), 2.87 (br s, 3H). 19F NMR (376 MHz, DMSO-d6) δ -120.00--116.92 (m, 1F). LC-MS: Rt =0.645 min, (ESI) *m/z.* $[M+H]^+$ 470.3; $C_{24}H_{20}FN_9O$ |
| 247 | $^1H$ NMR (400 MHz, DMSO-$d_6$) δ 8.84-8.73 (m, 1H), 8.33-8.10 (m, 2H), 8.00-7.86 (m, 1H), 7.84-7.74 (m, 2H), 7.58-7.48 (m, 2H), 7.48-7.38 (m, 2H), 7.29-7.17 (m, 1H), 4.87-4.49 (m, 2H), 3.89 (s, 3H), 3.58-3.35 (m, 2H), 2.99-2.85 (m, 3H), 1.18-1.07 (m, 3H). LC-MS: Rt = 2.109 min, (ESI) *m/z.* $[M+H]^+$ 498.1; $C_{26}H_{24}FN_9O$ | 248 | 1H NMR (400 MHz, DMSO-d6) δ 9.15-9.28 (m, 1H), 8.05 (br s, 2H), 7.68-7.89 (m, 3H), 7.37-7.62 (m, 4H), 7.05 (br s, 1H), 6.42 (br s, 1H), 4.92-5.33 (m, 2H), 2.90 (br s, 3H). LC-MS: Rt =0.814 min, (ESI) *m/z.* $[M+H]^+$ 574.3; $C_{25}H_{17}F_6N_9O$ |

(continued)

| | HNMR/LCMS | | HNMR/LCMS |
|---|---|---|---|
| 249 | 1H NMR (400 MHz, DMSO-d6) δ = 9.04 - 8.82 (m, 1H), 8.55 - 8.15 (m, 2H), 7.94 - 7.74 (m, 1H), 7.53 (br t, J = 9.5 Hz, 1H), 7.32 - 7.11 (m, 2H), 7.02 (br s, 2H), 4.80 (s, 1H), 4.52 (s, 1H), 3.75 - 3.38 (m, 8H), 2.70 - 2.51 (m, 7H), 1.20 - 1.03 (m, 3H) LC-MS: Rt = 2.142 min, (ESI) *m/z.* $[M+H]^+$ 517.3; $C_{27}H_{20}FN_8O_2$ | 250 | 1H NMR (400 MHz, DMSO-d6) δ9.58-9.37 (m, 1H), 9.35-9.17 (m, 2H), 9.00-8.73 (m, 2H), 8.73-8.53 (m, 1H), 8.52-8.33 (m, 1H), 8.12-7.73 (m, 2H), 7.48 (br d, J = 9.7 Hz, 1H), 5.13-4.75 (m, 2H), 3.43 (br d, J = 2.4 Hz, 2H), 3.40 (br s, 1H), 3.13-2.98 (m, 4H), 2.73 (s, 3H), 2.12-1.95 (m, 4H), 1.16 (br s, 3H). LC-MS: Rt = 1.343 min, (ESI) *m/z.* $[M+H]^+$ 501.3; $C_{27}H_{30}ClFN_8O$ |
| 251 | 1H NMR (400 MHz, DMSO-d6) δ = 9.07 - 8.88 (m, 1H), 8.55 - 8.40 (m, 2H), 7.90 - 7.77 (m, 1H), 7.48 - 7.39 (m, 1H), 7.35 - 7.26 (m, 1H), 7.16 (t, J = 10.7 Hz, 1H), 7.00 (br s, 2H), 5.22 - 5.18 (m, 1H), 4.78 (s, 1H), 4.50 (s, 1H), 3.74 (s, 1H), 3.51 (br d, J = 5.8 Hz, 1H), 2.61 (d, J = 4.9 Hz, 3H), 1.46 (t, J = 5.7 Hz, 6H), 1.16 - 1.04 (m, 3H) LC-MS: Rt = 2.138 min, (ESI) *m/z.* $[M+H]^+$ 476.2; $C_{25}H_{26}FN_7O_2$ | 252 | 1H NMR (400 MHz, DMSO-d6) δ ppm 8.56 (d, J=7.00 Hz, 2 H) 7.98 (d, J=6.38 Hz, 1 H) 7.81 (s, 1 H) 7.62 (br d, J=8.88 Hz, 1 H) 7.13 - 7.26 (m, 4 H) 6.77 - 6.90 (m, 1 H) 6.51 (br s, 1 H) 4.76 (br s, 2 H) 3.48 (br s, 2 H) 2.67 - 2.93 (m, 3 H) 1.15 (br s, 3 H) LC-MS: Rt = 2.357 min, (ESI) *m/z* = 418.2 [M+H]+ ; $C_{22}H_{20}FN_7O$ |
| 253 | 1H NMR (400 MHz, DMSO-d6) δ = 9.35 - 9.22 (m, 1H), 8.99 - 8.81 (m, 1H), 8.22 - 8.10 (m, 1H), 8.01 - 7.84 (m, 1H), 7.73 - 7.66 (m, 1H), 7.58 (s, 1H), 7.50 (br dd, J = 1.2, 9.3 Hz, 1H), 7.17 (s, 1H), 7.01 (br s, 2H), 6.94 (br d, J = 10.9 Hz, 1H), 5.08 (s, 2H), 3.58 (s, 3H), 2.60 (s, 3H) LC-MS: Rt = 2.092 min, (ESI) *m/z.* $[M+H]^+$ 495.2; $C_{25}H_{19}FN_{10}O$ | 254 | 1H NMR (400 MHz, DMSO-d6) δ = 8.63 - 8.43 (m, 1H), 8.39 - 8.07 (m, 1H), 7.95 - 7.68 (m, 2H), 7.59 - 7.36 (m, 3H), 7.33 - 7.18 (m, 2H), 6.90 (t, J = 6.6 Hz, 1H), 6.49 - 6.15 (m, 1H), 4.64 (br s, 2H), 3.89 (br s, 2H), 2.95 - 2.73 (m, 3H) LC-MS: Rt = 2.178 min, (ESI) *m/z.* $[M+H]^+$ 454.1; $C_{22}H_{18}F_3N_7O$ |
| 255 | 1H NMR (400 MHz, DMSO-d6) δ = 8.96 (s, 1H), 8.53 (d, J = 6.8 Hz, 1H), 8.15 (d, J = 6.5 Hz, 1H), 7.87 (s, 1H), 7.50 (d, J = 9.0 Hz, 1H), 7.26 - 7.17 (m, 2H), 7.03 - 6.84 (m, 4H), 4.97 (s, 2H), 3.44 (s, 3H), 2.59 (s, 3H), 1.85 (s, 3H) LC-MS: Rt = 2.088 min, (ESI) *m/z.* $[M+H]^+$ 484.2; $C_{25}H_{22}FN_9O$ | 256 | 1H NMR (400 MHz, DMSO-d6) δ = 9.04 - 8.80 (m, 1H), 8.46 - 8.31 (m, 1H), 8.23 - 8.09 (m, 1H), 7.83 (s, 1H), 7.70 - 7.55 (m, 1H), 7.47 (br d, J = 9.1 Hz, 1H), 7.25 - 7.12 (m, 2H), 7.08 - 6.89 (m, 3H), 5.06 - 4.93 (m, 2H), 3.58 (br s, 9H), 2.60 (s, 3H), 2.39 (br s, 4H) LC-MS: Rt = 2.046 min, (ESI) *m/z.* $[M+H]^+$ 569.3; $C_{29}H_{29}FN_{10}O_2$ |
| 257 | 1H NMR (400 MHz, DMSO-d6) δ = 9.04 - 8.78 (m, 1H), 8.62 - 8.40 (m, 1H), 8.26 - 8.07 (m, 1H), 7.91 - 7.74 (m, 1H), 7.71 - 7.56 (m, 1H), 7.51 (br d, J = 8.5 Hz, 1H), 7.29 - 7.12 (m, 2H), 7.11 - 6.78 (m, 4H), 5.14 - 4.93 (m, 2H), 3.85 - 3.53 (m, 3H), 2.60 (br s, 3H) LC-MS: Rt = 2.060 min, (ESI) *m/z.* $[M+H]^+$ 470.2; $C_{24}H_{20}FN_9O$ | 258 | 1H NMR (400 MHz, DMSO-d6) δ = 9.03 - 8.87 (m, 1H), 8.52 - 8.14 (m, 1H), 7.70 (br d, J = 5.5 Hz, 1H), 7.56 (s, 1H), 7.39 - 7.26 (m, 1H), 7.20 - 7.05 (m, 2H), 7.05 - 6.94 (m, 2H), 5.46 - 5.07 (m, 1H), 4.74 (s, 1H), 4.61 - 4.37 (m, 2H), 3.57 - 3.39 (m, 2H), 2.85 (br dd, J = 4.8, 19.3 Hz, 2H), 2.61 (br d, J = 2.3 Hz, 3H), 1.23 - 1.15 (m, 6H), 1.14 - 1.04 (m, 3H) LC-MS: Rt = 2.119 min, (ESI) *m/z.* $[M+H]^+$ 505.2; $C_{26}H_{29}FN_8O_2$ |
| 259 | 1H NMR (400 MHz, DMSO-d6) δ = 9.29 (br s, 2H), 8.81 - 8.32 (m, 3H), 8.30 - 8.08 (m, 1H), 7.84 (br d, J = 4.5 Hz, 1H), 7.73 - 7.52 (m, 2H), 7.43 - 7.12 (m, 2H), 5.13 (br s, 2H), 3.64 (br s, 3H), 2.70 (s, 3H) LC-MS: Rt = 1.695 min, (ESI) *m/z.* $[M+H]^+$ 538.2; $C_{25}H_{19}F_4N_9O$ | 260 | H NMR (400 MHz, DMSO-$d_6$) δ 9.15-8.91 (m, 1H), 8.90-8.72 (m, 1H), 8.55-8.23 (m, 1H), 8.20 (d, *J* = 11.0 Hz, 1H), 7.97-7.74 (m, 2H), 7.66-7.58 (m, 1H), 7.56-7.50 (m, 1H), 7.23 (dd, *J* = 10.9, 13.2 Hz, 1H), 7.06 (br s, 2H), 4.93-4.51 (m, 2H), 3.94 (d, *J* = 3.9 Hz, 3H), 3.41 (br s, 2H), 2.67 (d, *J* = 8.4 Hz, 3H), 1.30-1.03 (m, 3H). LC-MS: Rt = 2.166 min, (ESI) *m/z.* $[M+H]^+$ 498.2; $C_{26}H_{24}FN_9O$ |

(continued)

| | HNMR/LCMS | | HNMR/LCMS |
|---|---|---|---|
| 261 | 1H NMR (400 MHz, DMSO-d6) δ = 9.08 - 8.71 (m, 1H), 8.59 - 8.39 (m, 1H), 8.29 - 8.09 (m, 1H), 7.85 - 7.66 (m, 1H), 7.58 - 7.39 (m, 1H), 7.30 - 7.14 (m, 2H), 7.12 - 6.94 (m, 2H), 6.91 - 6.78 (m, 1H), 5.53 - 5.30 (m, 1H), 4.90 - 4.79 (m, 1H), 4.67 - 4.38 (m, 1H), 4.30 - 4.15 (m, 1H), 3.89 (br dd, J = 1.9, 3.7 Hz, 1H), 2.64 - 2.57 (m, 3H), 1.90 - 1.78 (m, 1H), 1.70 - 1.40 (m, 2H), 1.28 - 1.12 (m, 1H) LC-MS: Rt = 1.996 min, (ESI) *m/z.* $[M+H]^+$ 460.1; $C_{24}H_{22}FN_7O_2$ | 262 | 1H NMR (400 MHz, DMSO-d6) δ = 9.07 - 8.85 (m, 1H), 8.48 - 8.14 (m, 1H), 8.12 - 7.95 (m, 1H), 7.80 - 7.59 (m, 1H), 7.44 (br d, J = 9.6 Hz, 1H), 7.24 (br d, J = 9.1 Hz, 1H), 7.16 (dt, J = 3.1, 10.7 Hz, 1H), 7.00 (br s, 2H), 4.80 - 4.44 (m, 2H), 3.57 - 3.41 (m, 2H), 3.18 (br d, J = 3.3 Hz, 4H), 3.09 - 2.90 (m, 4H), 2.61 (br s, 6H), 1.18 - 1.03 (m, 3H) LC-MS: Rt = 2.158 min, (ESI) *m/z.* $[M+H]^+$ 516.3; $C_{27}H_{30}FN_9O$ |
| 263 | 1H NMR (400 MHz, DMSO-d6) δ = 9.05 - 8.85 (m, 1H), 8.44 - 8.25 (m, 1H), 8.21 - 8.13 (m, 1H), 7.82 - 7.67 (m, 1H), 7.44 (d, J = 9.8 Hz, 1H), 7.16 (t, J = 11.3 Hz, 1H), 7.01 (br dd, J = 2.3, 9.8 Hz, 3H), 4.76 (s, 1H), 4.49 (s, 1H), 3.76 (d, J = 14.4 Hz, 3H), 3.51 (br d, J = 6.6 Hz, 1H), 3.30 (br s, 1H), 2.61 (d, J = 3.9 Hz, 3H), 1.19 - 1.03 (m, 3H) LC-MS: Rt = 2.228 min, (ESI) m/z. $[M+H]^+$ 448.2; $C_{23}H_{22}FN_7O_2$ | 264 | 1H NMR (400 MHz, DMSO-d6) δ = 9.13 - 8.79 (m, 1H), 8.60 - 8.21 (m, 2H), 7.79 (br s, 1H), 7.59 - 7.16 (m, 2H), 7.15 - 6.92 (m, 3H), 6.85 (br t, J = 6.7 Hz, 1H), 5.17 (s, 2H), 4.11 - 3.84 (m, 3H), 2.60 (s, 3H) LC-MS: Rt = 2.170 min, (ESI) *m/z.* $[M+H]^+$ 471.2; $C_{23}H_{19}FN_{10}O$ |
| 265 | 1H NMR (400 MHz, DMSO-d6) δ = 9.07 - 8.82 (m, 1H), 8.52 - 8.46 (m, 1H), 8.36 (br d, J = 6.6 Hz, 1H), 8.26 - 8.17 (m, 1H), 8.14 - 8.05 (m, 2H), 7.26 - 7.15 (m, 2H), 7.00 (br d, J = 6.4 Hz, 1H), 4.91 - 4.50 (m, 2H), 3.54 (br d, J = 5.9 Hz, 2H), 2.61 (d, J = 6.3 Hz, 3H), 1.17 - 1.06 (m, 3H) LC-MS: Rt = 2.131 min, (ESI) *m/z.* $[M+H]^+$ 419.2; $C_{21}H_{19}FN_8O$ | 266 | 1H NMR (400 MHz, DMSO-d6) δ = 9.00 - 8.80 (m, 1H), 8.62 - 8.12 (m, 2H), 7.97 - 7.73 (m, 1H), 7.54 (br d, J = 9.0 Hz, 1H), 7.28 - 7.14 (m, 2H), 7.03 (br s, 2H), 6.93 - 6.82 (m, 1H), 4.92 - 4.57 (m, 2H), 3.81 - 3.62 (m, 2H), 2.93 - 2.84 (m, 2H), 2.61 (s, 3H) LC-MS: Rt = 1.308 min, (ESI) *m/z.* $[M+H]^+$ 443.2; $C_{23}H_{19}FN_8O$ |
| 267 | 1H NMR (400 MHz, DMSO-d6) δ = 9.06 - 8.85 (m, 1H), 8.81 - 8.66 (m, 1H), 8.41 - 8.16 (m, 1H), 7.93 - 7.75 (m, 1H), 7.67 - 7.55 (m, 1H), 7.37 - 7.28 (m, 1H), 7.16 (dd, J = 10.9, 14.9 Hz, 1H), 7.01 (br d, J = 1.1 Hz, 2H), 4.79 (s, 1H), 4.52 (s, 1H), 3.52 (br d, J = 6.1 Hz, 2H), 2.61 (d, J = 4.6 Hz, 3H), 1.20 - 1.03 (m, 3H) LC-MS: Rt = 2.244 min, (ESI) m/z. [M+H]+ 436.2; $C_{22}H_{19}F_2N_7O$ | 268 | 1H NMR (400MHz, DMSO-d6) δ = 9.19 (s, 1H), 8.16 (br s, 1H), 8.05 (s, 1H), 7.73 (d, J=9.5 Hz, 2H), 7.57 - 7.40 (m, 3H), 7.35 (s, 1H), 7.29 (br s, 2H), 7.31 - 7.09 (m, 1H), 5.09 (s, 2H), 3.66 (br s, 3H), 2.72 - 2.58 (m, 3H). LC-MS: Rt = 1.711 min, (ESI) *m/z.* $[M+H]^+$ 520.1; $C_{25}H_{20}F_3N_9O$ |
| 269 | 1H NMR (400 MHz, DMSO-d6) δ = 8.99 - 8.68 (m, 1H), 8.27 - 8.08 (m, 1H), 8.00 - 7.40 (m, 4H), 7.18 - 7.09 (m, 1H), 7.07 - 6.94 (m, 2H), 4.99 - 4.71 (m, 2H), 2.62 (s, 1H), 2.58 (s, 1H), 2.50 (br s, 3H), 0.95 (d, J = 6.5 Hz, 3H), 0.88 - 0.80 (m, 1H), 0.69 (d, J = 6.5 Hz, 3H) LC-MS: Rt = 1.617 min, (ESI) *m/z.* $[M+H]^+$ 514.3; $C_{25}H_{23}F_4N_7O$ | 270 | 1H NMR (400 MHz, DMSO-d6) δ = 8.47 (br d, J = 5.0 Hz, 1H), 8.03 - 7.94 (m, 1H), 7.84 (br s, 1H), 7.77 (s, 1H), 7.48 (br d, J = 8.9 Hz, 1H), 7.33 (s, 1H), 7.28 - 7.19 (m, 1H), 6.98 (br s, 2H), 6.87 (t, J = 7.1 Hz, 1H), 4.77 - 4.28 (m, 2H), 3.63 - 3.34 (m, 2H), 2.83 (br s, 3H), 2.32 (s, 3H), 1.22 - 1.11 (m, 3H) LC-MS: Rt = 2.057 min, (ESI) *m/z.* $[M+H]^+$ 414.2; C23H23N7O |
| 271 | 1H NMR (400 MHz, DMSO-d6) δ 9.04 (s, 1H), 9.10-8.76 (m, 1H), 8.61-8.39 (m, 1H), 8.31-8.14 (m, 1H), 8.34-8.11 (m, 1H), 7.91-7.72 (m, 1H), 7.53-7.45 (m, 1H), 7.56-7.42 (m, 1H), 7.24-7.06 (m, 2H), 7.05-6.90 (m, 2H), 6.89-6.76 (m, 1H), 4.83-4.52 (m, 2H), 4.00-3.58 (m, 1H), 3.52-3.42 (m, 1H), 3.27-3.05 (m, 4H), 2.64-2.57 (m, 3H), 1.23-1.13 (m, 3H). LC-MS: Rt = 1.104 min, (ESI) *m/z.* $[M+H]^+$ 462.3; $C_{24}H_{24}FN_7O_2$ | 272 | 1H NMR (400MHz, DMSO-d6) δ 9.06 - 8.86 (m, 2H), 8.56 - 8.50 (m, 1H), 8.48 - 8.18 (m, 1H), 7.89 - 7.73 (m, 1H), 7.26 - 7.11 (m, 1H), 7.10 - 6.95 (m, 3H), 4.87 - 4.54 (m, 2H), 3.62 - 3.34 (m, 2H), 2.62 (d, J=6.5 Hz, 3H), 1.21 - 1.06 (m, 3H) ; LC-MS: Rt = 1.889 min, (ESI) m/z. [M+H]+ 419.1; $C_{21}H_{19}FN_8O$ |

(continued)

| | HNMR/LCMS | | HNMR/LCMS |
|---|---|---|---|
| 273 | 1H NMR (400 MHz, DMSO-d6) δ = 9.05 - 8.75 (m, 1H), 8.53 (br d, J = 7.4 Hz, 1H), 8.16 (br d, J = 6.9 Hz, 1H), 7.82 - 7.69 (m, 1H), 7.53 (br d, J = 9.4 Hz, 1H), 7.26 - 7.14 (m, 2H), 7.04 (br d, J = 1.9 Hz, 2H), 6.88 (s, 1H), 4.83 (br s, 2H), 3.89 (br dd, J = 3.8, 8.3 Hz, 1H), 3.41 - 3.35 (m, 1H), 3.21 - 2.87 (m, 4H), 2.65 - 2.56 (m, 4H), 2.18 - 1.97 (m, 2H) LC-MS: Rt = 2.127 min, (ESI) *m/z.* $[M+H]^+$ 474.2; $C_{25}H_{24}FN_7O_2$ | 274 | 1H NMR (400 MHz, DMSO-d6) δ = 9.04 - 8.85 (m, 1H), 8.52 - 8.18 (m, 1H), 7.81 - 7.59 (m, 1H), 7.54 - 7.41 (m, 1H), 7.34 - 7.14 (m, 2H), 7.04 (br s, 2H), 6.89 - 6.73 (m, 1H), 4.85 (s, 1H), 4.54 (s, 1H), 3.53 (br dd, J = 1.9, 14.5 Hz, 2H), 2.64 - 2.53 (m, 6H), 1.24 - 1.05 (m, 3H) LC-MS: Rt = 2.265 min, (ESI) m/z. [M+H]+ 432.2; $C_{23}H_{22}FN_7O$ |
| 275 | $^1$H NMR (400 MHz, DMSO-*d*$_6$) δ = 9.10 (s, 1H), 8.84 (s, 1H), 8.23 (s, 1H), 7.90 (br s, 1H), 7.71 (d, *J* = 9.5 Hz, 1H), 7.44 - 7.41 (m, 1H), 7.40 - 7.36 (m, 2H), 6.55 (br s, 2H), 4.93 (br s, 2H), 4.62 (br s, 1H), 3.54 (s, 2H), 2.64 (s, 3H), 1.19 (br s, 6H)<br>LC-MS: Rt = 2.316 min, (ESI) *m/z.* $[M+H]^+$ 512.2; $C_{25}H_{24}F_3N_7O_2$ | 276 | 1H NMR (400 MHz, DMSO-d6) δ = 9.04 - 8.87 (m, 1H), 8.60 - 8.46 (m, 1H), 8.45 - 8.17 (m, 1H), 7.94 - 7.79 (m, 1H), 7.57 - 7.49 (m, 1H), 7.30 - 7.11 (m, 2H), 7.02 (br s, 2H), 6.95 - 6.84 (m, 1H), 4.82 - 4.51 (m, 2H), 3.57 - 3.35 (m, 2H), 2.64 - 2.59 (m, 3H), 1.17 - 1.05 (m, 3H) LC-MS: Rt = 1.051 min, (ESI) *m/z.* $[M+H]^+$ 518.2; $C_{22}H_{20}FN_7O$ |
| 277 | 1H NMR (400 MHz, DMSO-d6) δ ppm 8.75 - 8.99 (m, 1 H) 8.15 - 8.56 (m, 2 H) 7.74 (br s, 1 H) 7.50 (br d, J=9.06 Hz, 1 H) 7.09 - 7.26 (m, 2 H) 6.84 (br s, 1 H) 6.73 (br s, 2 H) 4.31 - 4.94 (m, 2 H) 3.37 (br s, 2 H) 2.63 (s, 3 H) 2.15 (brs, 1 H) 0.84 - 0.85 (m, 1 H) 0.61 - 1.06 (m, 5 H)<br>LC-MS: Rt = 2.387 min,<br>(ESI) *m/z* = 446.2 [M+H]+;<br>$C_{24}H_{24}FN_7O$ | 278 | 1H NMR (400 MHz, DMSO-d6) δ = 12.73 - 12.43 (m, 1H), 8.02 (br d, J = 6.4 Hz, 1H), 7.90 - 7.82 (m, 1H), 7.78 (br s, 1H), 7.73 - 7.64 (m, 1H), 7.47 (br d, J = 8.5 Hz, 1H), 7.20 (d, J = 10.9 Hz, 1H), 7.17 (br s, 2H), 4.95 - 4.69 (m, 2H), 3.46 - 3.22 (m, 2H), 2.91 - 2.57 (m, 3H), 2.16 - 2.03 (m, 1H), 0.99 - 0.78 (m, 6H) LC-MS: Rt = 2.498 min, (ESI) *m/z.* $[M+H]^+$ 514.2;<br>$C_{25}H_{23}F_4N_7O$ |
| 279 | 1H NMR (400MHz, DMSO-d6) δ= 8.52 (br d, J=6.6 Hz, 1H), 8.46 - 8.11 (m, 1H), 7.95 (s, 1H), 7.66 - 7.46 (m, 3H), 7.40 - 7.12 (m, 4H), 6.90 (br t, J=6.6 Hz, 1H), 4.62 (br s, 2H), 3.45 (br d, J=6.4 Hz, 2H), 2.98 - 2.55 (m, 3H), 1.15 (br t, J=6.9 Hz, 3H).<br>LC-MS: Rt = 1.454 min, (ESI) *m/z.* $[M+H]^+$ 400.2; $C_{22}H_{21}N_7O$ | 280 | 1H NMR (400 MHz, DMSO-d6) δ = 8.97 - 8.71 (m, 1H), 8.54 - 8.39 (m, 1H), 8.15 (br s, 1H), 7.79 (br s, 1H), 7.47 (br d, J = 9.1 Hz, 1H), 7.25 - 7.06 (m, 2H), 6.83 (br t, J = 6.4 Hz, 1H), 6.72 (br s, 2H), 4.80 - 4.52 (m, 2H), 4.26 - 3.82 (m, 2H), 3.13 (br s, 3H), 2.63 (s, 3H), 1.87 - 1.81 (m, 2H), 1.73 - 1.33 (m, 4H) LC-MS: Rt = 2.317 min, (ESI) *m/z.* $[M+H]^+$ 488.2; $C_{26}H_{26}FN_7O_2$ |
| 281 | $^1$H NMR (400 MHz, DMSO-*d*$_6$) δ = 9.09 - 8.94 (m, 1H), 8.72 (br dd, *J* = 2.3, 3.5 Hz, 1H), 8.09 (d, *J* = 6.4 Hz, 1H), 7.83 - 7.60 (m, 2H), 7.45 - 7.31 (m, 1H), 7.11 (d, *J*= 11.1 Hz, 1H), 6.71 (br s, 2H), 4.80 (br s, 2H), 4.49 (d, *J* = 2.1 Hz, 1H), 3.60 (br s, 2H), 2.62 (s, 3H), 1.40 - 1.13 (m, 6H)<br>LC-MS: Rt = 2.356 min, (ESI) *m/z.* $[M+H]^+$ 530.2; $C_{25}H_{23}F_4N_7O_2$ | 282 | 1H NMR (400 MHz, DMSO-d6) δ = 9.32 - 9.07 (m, 1H), 9.00 - 8.73 (m, 1H), 8.15 (br s, 1H), 8.02 - 7.82 (m, 1H), 7.78 - 7.63 (m, 1H), 7.50 - 7.39 (m, 1H), 7.19 (d, J = 10.8 Hz, 1H), 7.09 - 6.93 (m, 2H), 5.40 - 5.26 (m, 1H), 4.98 - 4.55 (m, 2H), 4.29 - 4.12 (m, 1H), 4.00 - 3.82 (m, 1H), 2.63 (s, 3H), 1.90 - 1.79 (m, 1H), 1.69 - 1.52 (m, 2H), 1.28 - 1.11 (m, 1H) LC-MS: Rt = 2.344 min, (ESI) *m/z.* $[M+H]^+$ 527.1; $C_{25}H_{21}F_4N_7O_2$ |
| 283 | 1H NMR (400 MHz, DMSO-d6) δ = 9.30 - 9.13 (m, 1H), 9.04 - 8.86 (m, 1H), 8.36 - 8.16 (m, 1H), 8.07 - 7.89 (m, 1H), 7.74 (t, J = 8.8 Hz, 1H), 7.51 - 7.43 (m, 1H), 7.17 (dd, J = 10.9, 17.5 Hz, 1H), 7.05 - 6.96 (m, 2H), 4.87 - 4.56 (m, 2H), 3.40 - 3.33 (m, 2H), 2.64 - 2.60 (m, 3H), 1.20 - 1.06 (m, 3H) LC-MS: Rt = 2.497 min, (ESI) *m/z.* $[M+H]^+$ 486.2; $C_{23}H_{19}F_4N_7O$ | 284 | 1H NMR (400 MHz, DMSO-d6) δ = 9.32 - 9.09 (m, 1H), 9.05 - 8.85 (m, 1H), 8.31 - 8.13 (m, 1H), 8.11 - 7.83 (m, 1H), 7.81 - 7.67 (m, 1H), 7.55 - 7.40 (m, 1H), 7.21 - 7.11 (m, 1H), 7.09 - 6.87 (m, 2H), 4.57 (s, 2H), 3.29 - 3.11 (m, 2H), 2.64 - 2.60 (m, 3H), 2.18 - 1.97 (m, 1H), 0.97 - 0.69 (m, 6H) LC-MS: Rt = 1.965 min, (ESI) *m/z.* $[M+H]^+$ 514.2; $C_{25}H_{23}F_4N_7O$ |

(continued)

| | HNMR/LCMS | | HNMR/LCMS |
|---|---|---|---|
| 285 | 1H NMR (400MHz, DMSO-d6) δ= 9.29 - 8.54 (m, 2H), 8.18 - 7.78 (m, 2H), 7.66 (br s, 1H), 7.38 (br d, J=7.9 Hz, 1H), 7.13 (br s, 1H), 6.72 (br s, 2H), 4.95 - 4.39 (m, 2H), 4.22 - 3.82 (m, 1H), 3.52 (br s, 2H), 3.31 - 3.10 (m, 3H), 2.63 (s, 3H), 1.21 (br s, 3H).<br><br>LC-MS: Rt = 2.517 min, (ESI) *m*/*z*. $[M+H]^+$ 530.2; $C_{25}H_{23}F_4N_7O_2$ | 286 | $^1$H NMR (400 MHz, DMSO-d6) δ = 9.33 - 9.07 (m, 1H), 9.05 - 8.75 (m, 1H), 8.22 - 8.09 (m, 1H), 8.05 - 7.92 (m, 1H), 7.88 - 7.70 (m, 1H), 7.69 - 7.27 (m, 2H), 7.25 - 7.08 (m, 2H), 4.92 - 4.60 (m, 2H), 4.32 - 3.91 (m, 2H), 3.18 - 2.96 (m, 3H), 2.64 - 2.58 (m, 3H), 2.14 - 1.69 (m, 3H), 1.51 - 1.14 (m, 1H)<br>LC-MS: Rt = 1.843 min, (ESI) *m*/*z*. $[M+H]^+$ 542.2; $C_{26}H_{23}F_4N_7O_2$ |
| 287 | 1H NMR (400 MHz, DMSO-d6) δ = 9.15 (s, 1H), 8.82 (s, 1H), 8.19 (d, J = 1.5 Hz, 1H), 8.01 (s, 1H), 7.73 (d, J = 9.3 Hz, 1H), 7.45 (dd, J = 1.8, 9.5 Hz, 1H), 7.37 - 7.24 (m, 2H), 6.79 (br s, 2H), 4.70 (s, 2H), 3.71 (s, 2H), 3.32 (s, 3H), 2.58 (s, 3H), 1.45 (s, 6H) LC-MS: Rt = 2.611 min, (ESI) *m*/*z*. $[M+H]^+$ 526.2; $C_{26}H_{26}F_3N_7O_2$ | 288 | 1H NMR (400 MHz, DMSO-d6) δ = 9.22 (br s, 1H), 9.01 - 8.75 (m, 1H), 8.22 (br d, J = 2.0 Hz, 1H), 8.00 (br s, 1H), 7.73 (d, J = 9.4 Hz, 1H), 7.46 (dd, J = 1.6, 9.5 Hz, 1H), 7.42 - 7.27 (m, 2H), 6.89 (br s, 2H), 4.85 (br d, J = 3.9 Hz, 2H), 4.12 (br dd, J = 1.4, 7.7 Hz, 2H), 3.06 (s, 3H), 2.62 (s, 3H), 1.98 - 1.66 (m, 3H), 1.38 - 1.08 (m, 1H) LC-MS: Rt = 2.518 min, (ESI) *m*/*z*. $[M+H]^+$ 524.2; $C_{26}H_{24}F_3N_7O_2$ |
| 289 | 1H NMR (400 MHz, DMSO-d6) δ9.23-9.10 (m, 1H), 8.97 (s, 1H), 8.46-8.14 (m, 1H), 8.05-7.84 (m, 1H), 7.75 (d, J = 9.5 Hz, 1H), 7.48 (dd, J = 1.8, 9.5 Hz, 1H), 7.44 (br s, 2H), 6.90 (br s, 2H), 5.15 - 4.81 (m, 3H), 4.77 (br s, 2H), 4.60 (br t, J = 6.8 Hz, 2H), 2.63 (s, 3H). LC-MS: Rt = 2.285 min, (ESI) *m*/*z*. $[M+H]^+$ 496.2; $C_{24}H_{20}F_3N_7O_2$ | 290 | $^1$H NMR (400 MHz, DMSO-d6) δ = 9.28 - 8.80 (m, 2H), 8.39 - 7.94 (m, 2H), 7.73 (br d, J = 9.3 Hz, 1H), 7.54 - 7.26 (m, 3H), 6.85 (br s, 2H), 4.87 - 4.57 (m, 2H), 4.16 (br d, J = 1.4 Hz, 1H), 3.50 (br s, 1H), 3.31 - 3.02 (m, 4H), 2.67 - 2.56 (m, 3H), 1.16 (br s, 3H)<br>LC-MS: Rt = 2.461 min, (ESI) *m*/*z*. $[M+H]^+$ 512.2; $C_{25}H_{24}F_3N_7O_2$ |
| 291 | 1H NMR (400 MHz, DMSO-d6) δ = 9.13 - 8.80 (m, 1H), 8.65 (br d, J = 6.6 Hz, 1H), 8.41 - 8.12 (m, 1H), 7.65 (d, J = 8.9 Hz, 1H), 7.39 (br s, 2H), 7.24 - 7.17 (m, 1H), 6.92 - 6.78 (m, 3H), 6.59 (br s, 1H), 4.98 - 4.52 (m, 2H), 3.56 - 3.40 (m, 2H), 2.62 (s, 3H), 1.13 (br s, 3H) LC-MS: Rt = 2.283 min ,<br>(ESI) *m*/*z* = 400.2 [M+H]+;<br>$C_{22}H_{21}N_7O$ | 292 | 1H NMR (400 MHz, DMSO-d6) δ ppm 9.18 (br s, 1 H) 8.07 - 8.25 (m, 1 H) 7.81 - 8.06 (m, 2 H) 7.74 (br d, J=8.13 Hz, 1 H) 7.28 - 7.65 (m, 3 H) 6.97 (br s, 2 H) 4.50 - 4.99 (m, 2 H) 3.30 (br s, 2 H) 2.50 (br s, 3 H) 1.91 - 2.19 (m, 1 H) 0.64 - 1.00 (m, 1 H) 0.64 - 1.00 (m, 5 H)<br>LC-MS: Rt = 2.838 min ,<br>(ESI) *m*/*z* = 496.2 [M+H]+;<br>$C_{25}H_{24}F_3N_7O$ |
| 293 | 1H NMR (400 MHz, DMSO-d6) δ9.24 (br d, *J*= 17.9 Hz, 2H), 9.09-8.57 (m, 2H), 8.35 (br s, 1H), 8.08 (br d, *J* = 10.5 Hz, 1H), 7.79 (br d, *J* = 9.4 Hz, 1H), 7.62 (br s, 1H), 7.55 (br d, *J* = 7.4 Hz, 1H), 7.51-7.30 (m, 2H), 7.18 (br s, 1H), 5.09 (br s, 2H), 3.62 (br s, 3H), 2.71-2.68 (m, 3H). LC-MS: Rt = 1.707 min, (ESI) *m*/*z*. $[M+H]^+$ 520.1; $C_{25}H_{20}F_3N_9O$ | 294 | 1H NMR (400 MHz, DMSO-d6) δ = 9.15 (s, 1H), 8.93 (s, 1H), 8.10 (br d, J = 1.6 Hz, 1H), 7.99 (s, 1H), 7.78 (d, J = 9.5 Hz, 1H), 7.47 (dd, J = 1.6, 9.5 Hz, 1H), 7.40 (s, 1H), 7.29 - 7.14 (m, 2H), 6.98 (s, 1H), 6.84 (br s, 2H), 6.27 - 6.05 (m, 1H), 3.56 (s, 3H), 2.61 (s, 3H), 1.51 (d, J = 7.0 Hz, 3H) LC-MS: Rt = 2.371 min, (ESI) *m*/*z*. $[M+H]^+$ 534.2; $C_{26}H_{22}F_3N_9O$ |
| 295 | 1H NMR (400 MHz, DMSO-d6) δ = 8.65 (d, J = 6.9 Hz, 1H), 8.33 - 8.04 (m, 1H), 7.93 (br s, 1H), 7.66 (d, J = 8.8 Hz, 1H), 7.57 - 7.40 (m, 2H), 7.29 - 7.16 (m, 1H), 7.09 - 6.78 (m, 3H), 6.57 (s, 1H), 4.91 - 4.60 (m, 2H), 3.61 - 3.33 (m, 2H), 2.53 - 2.51 (m, 3H), 1.29 (d, J = 12.2 Hz, 1H), 1.14 (br t, J = 6.3 Hz, 3H) LC-MS: Rt = 2.468 min ,<br>(ESI) *m*/*z* = 400.1 [M+H]+;<br>$C_{22}H_{21}N_7O$ | 296 | 1H NMR (400 MHz, DMSO-d6) δ = 9.22 (br s, 1H), 8.95 (br s, 1H), 8.21 (br d, J = 5.0 Hz, 1H), 8.05 (s, 1H), 7.73 (d, J = 9.4 Hz, 1H), 7.53 - 7.31 (m, 3H), 6.87 (br s, 2H), 4.71 (br s, 2H), 4.19 - 4.06 (m, 1H), 4.04 - 3.84 (m, 1H), 3.11 (s, 3H), 2.62 (s, 3H), 2.00 - 1.72 (m, 3H), 1.68 - 1.22 (m, 3H) LC-MS: Rt = 2.581 min, (ESI) *m*/*z*. $[M+H]^+$ 538.2; $C_{27}H_{26}F_3N_7O_2$ |

(continued)

| | HNMR/LCMS | | HNMR/LCMS |
|---|---|---|---|
| 297 | 1H NMR (400 MHz, DMSO-d6) δ ppm:8.99 (br s, 1H), 8.61-8.42 (m, 2H), 7.88 (br s, 1H), 8.00-7.79 (m, 1H), 7.61-7.48 (m, 2H), 7.39 (br d, J = 7.9 Hz, 1H), 7.25 (br t, J = 7.7 Hz, 1H), 6.89 (dt, J = 0.9, 6.8 Hz, 1H), 6.85 (br s, 2H), 4.62 (br s, 2H), 3.30-3.18 (m, 2H), 2.63 (s, 3H), 2.14-1.93 (m, 1H), 0.99-0.61 (m, 6H). LC-MS: Rt = 2.316 min, (ESI) *m/z.* $[M+H]^+$ 428.3; $C_{24}H_{25}N_7O$ | 298 | 1H NMR (400 MHz, DMSO-d6) δ ppm: 9.16 (br s, 1H), 9.07 (s, 1H), 8.36 (br s, 1H), 7.97 (br s, 1H), 7.77 (d, J = 9.5 Hz, 1H), 7.59 - 7.39 (m, 3H), 6.85 (br s, 2H), 5.24 (br d, J = 4.4 Hz, 1H), 3.26 (dd, J = 7.2, 13.6 Hz, 1H), 2.91 - 2.75 (m, 1H), 2.63 (s, 3H), 2.07 - 1.87 (m, 1H), 1.64 (br s, 3H), 1.01 - 0.55 (m, 6H) LC-MS: Rt = 2.726 min , (ESI) *m/z* = 510.2 [M+H]+; $C_{26}H_{26}F_3N_7O$ |
| 299 | 1H NMR (400 MHz, DMSO-d6) δ ppm: 9.18 (br s, 1H), 9.13-9.03 (m, 1H), 8.47 (br s, 1H), 8.21-8.11 (m, 1H), 7.98 (brs, 1H), 7.77 (br d, J = 9.4 Hz, 1H), 7.56-7.42 (m, 4H), 4.85-4.64 (m, 3H), 3.27-3.15 (m, 2H), 2.65 (s, 3H), 2.15-2.02 (m, 1H), 0.93 (br s, 4H), 0.70 (br s, 2H). LC-MS: Rt = 2.642 min, (ESI) *m/z.* $[M+H]^+$ 496.3; $C_{25}H_{24}F_3N_7O$ | 300 | 1H NMR (400 MHz, DMSO-d6) δ ppm 9.02 (br s, 1 H) 8.39 - 8.53 (m, 2 H) 7.91 (br s, 1 H) 7.49 - 7.62 (m, 2H) 7.41 (d, J=8.25 Hz, 1 H) 7.21 - 7.28 (m, 1 H) 6.74 - 7.01 (m, 3 H) 5.18 (br s, 1 H) 3.02 - 3.28 (m, 2 H) 2.62 (s, 3 H) 1.61 (br s, 3 H) 0.84 - 1.20 (m, 3 H) LC-MS: Rt = 2.178 min , (ESI) *m/z* = 414.2 [M+H]+; $C_{23}H_{23}N_7O$ |
| 301 | 1H NMR (400 MHz, DMSO-$d_6$) δ 9.39 (br s, 1H), 9.16 (s, 1H), 8.24-8.43 (m, 1H), 7.78-7.99 (m, 2H), 7.55 (s, 2H), 7.31 (br s, 1H), 7.10 (br d, J=11.29 Hz, 1H), 5.08-5.39 (m, 2H), 3.61 (s, 3H), 2.85 (br s, 3H). LC-MS: (ESI) m/z=557.3 [M+1]+, RT= 0.749 min | 302 | 1H NMR (400 MHz, DMSO-$d_6$) δppm: 9.24 (s, 1H), 8.09 (s, 1H), 7.99 (br s, 1H), 7.80 (s, 1H), 7.72 (br d, J=9.38 Hz, 1H), 7.33-7.50 (m, 6H), 5.03-5.19 (m, 2H), 3.55 (s, 3H), 2.84 (s, 3H) 19F NMR (376 MHz, DMSO-$d_6$) δ ppm: -60.48 (s, 1F), -174.29 (br s, 1F) LC-MS: (ESI) m/z=538.1 $[M+1]^+$; RT=2.897 min |
| 303 | 1H NMR (400 MHz, DMSO-d6) δ ppm : 8.93 (br s, 1H), 8.00-8.15 (m, 1H), 7.92 (br d, J=4.88 Hz, 1H), 7.81 (s, 1H), 7.65 (d, J=9.38 Hz, 1H), 7.54 (s, 2H), 7.37 (br d, J=9.51 Hz, 1H), 6.90-7.32 (m, 3H), 4.94-5.30 (m, 2H), 3.57 (s, 3H), 2.86 (br s, 3H), 2.08 (s, 1H) 19F NMR (376 MHz, DMSO-d6) δ-109.29 (br s, 1F), -118.19 (br s, 1F), -173.53 (br s, 1F) LC-MS: (ESI) m/z= 538.3 [M+1]+, RT=0.723 min | 304 | $^1$H NMR (400 MHz, DMSO-$d_6$) δ ppm: 9.26 - 9.11 (m, 1H), 8.81 (d, *J* = 4.9 Hz, 2H), 8.49 (br d, *J* = 1.1 Hz, 1H), 8.13 (br d, *J*= 5.1 Hz, 1H), 7.74 - 7.57 (m, 3H), 7.42 (t, *J* = 4.9 Hz, 1H), 6.73 (br s, 2H), 5.50 - 5.09 (m, 4H), 5.00 (br s, 2H), 4.96 - 4.87 (m, 1H), 1.68 (br d, *J* = 7.0 Hz, 3H); LC-MS, $[MH]^+$ 484.2 |
| 305 | $^1$H NMR (400 MHz, DMSO-$d_6$) δ ppm: 9.21 (s, 1H), 8.05 (br s, 1H), 7.72 (br d, J=9.38 Hz, 1H), 7.59 (s, 1H), 7.28-7.53 (m, 4H), 6.61 (s, 2H), 5.33-5.50 (m, 1H), 5.09-5.32 (m, 2H), 4.99 (br s, 2H), 3.60 (s, 3H), 1.68 (br s, 3H), 1.39 (d, J=6.13 Hz, 3H). $^{19}$F NMR (376 MHz, DMSO-$d_6$) δ -60.40 (s, 3F). LC-MS: (ESI) m/z=536.3 $[M+H]^+$, RT =0.71 min; SFC: RT=2.052 min | 306 | $^1$H NMR (400 MHz, DMSO-$d_6$) δ ppm: 9.21 (s, 1H), 8.06 (br s, 1H), 7.72 (br d, J=9.38 Hz, 1H), 7.59 (s, 1H), 7.25-7.55 (m, 4H), 6.62 (s, 2H), 5.39 (br d, J=4.25 Hz, 1H), 5.03-5.28 (m, 2H), 3.60 (s, 3H), 1.66 (br s, 3H), 1.39 (d, J=6.25 Hz, 3H) $^{19}$F NMR (376 MHz, DMSO-$d_6$) δ ppm: -60.40 (s, 3F). LCMS: (ESI) m/z=538.1 [M+1]+, RT=0.672 min. SFC: RT=3.599 min |
| 313 | $^1$H NMR (400 MHz, DMSO-$d_6$) δ ppm: 9.21 (s, 1H), 8.06 (br s, 1H), 7.72 (br d, J=9.38 Hz, 1H), 7.59 (s, 1H), 7.27-7.55 (m, 4H), 6.61 (s, 2H), 5.33-5.50 (m, 1H), 5.08-5.28 (m, 2H), 4.99 (br s, 2H), 3.60 (s, 3H), 1.68 (br s, 3H), 1.39 (d, J=6.13 Hz, 3H).$^{19}$F NMR (376 MHz, DMSO-$d_6$) δ -60.40 (s, 3F). LC-MS:(ESI)m/z=536.3 $[M+1]^+$, RT=0.71 min; | | |

**Biological Test Example 1 PRMTS inhibitory activity assay in vitro**

**Experimental Materials:**

PRMT5 (Active Motiv, catalog number 31921), [$^3$H] - SAM (Perkin Elmer, catalog number NET155V001MC), SAM (Sigma, catalog number A7007), MTA (Sigma, catalog number D5011), SAH (Sigma, catalog number A9384), 384-well plate (Perkin Elmer, catalog number 6007299), Echo 550 (manufacturer: Labcyte, model: Echo 550), 384-well Flashplate (manufacturer: Perkin Elmer, model: SMP410A001PK)

**Experimental method:**

1. Enzyme reaction process

(1) 1x Assay buffer (modified Tris buffer) was configured.
(2) Dilute compound: the compound was dissolved in 100% DMSO and the compound solution was added to a 384-well plate using Echo 550.
(3) Configure enzyme solution: PRMT5 was added to 1x assay buffer to prepare enzyme solution 1; PRMT5 and MTA was added to 1x assay buffer to prepare enzyme solution 2.
(4) Configure substrate solution: peptide segments and [3H] - SAM was added to 1x assay buffer.
(5) 15 μL of enzyme solution was added to the 384-well plate, and 15 μL of 1x assay buffer was added to the negative control well, and incubated at room temperature for 30 minutes.
(6) 15 μL of substrate solution was added to each well, and incubated at room temperature for 90 minutes.
(7) Configure termination reaction solution: pre-cooled SAM was added to 1x assay buffer.
(8) 10 μL of termination reaction solution was added to each well to terminate the reaction.
(9) 25 μL/well of the mixed solution was transferred to Flashplate and incubated for 1 hour at room temperature.
(10) the Flashplate three was washed times with dH2O + 0.1% Tween-20 solution.
(11) the radiometric values was readed with Microbeta.

2. Data analysis

(1) The raw data were converted to % inhibition according to Equation 1.

$$\text{Equation 1: \% inhibition}=(\text{Max-Signal})/(\text{Max-Min}) * 100$$

(2) the % inhibition data were entered into XL-Fit Equation 2 to obtain $IC_{50}$ value:

$$\text{Equation 2: } Y=\text{Bottom}+(\text{Top-Bottom})/(1+(IC_{50}/X) * \text{HillSlope})$$

wherein, Y is the % inhibition, and X is the concentration of the compound.

The biological activities of some of the compounds were measured by experimental methods and were shown in Table 3

Table 3

| Example No. | Enzyme activity inhibition rate PRMT5 MTA (2.0 um) $IC_{50}$ (nm) |
|---|---|
| 1 | 5.1 |
| 2 | 3.0 |
| 4 | 1.7 |
| 127 | 3.7 |

(continued)

| Example No. | Enzyme activity inhibition rate PRMT5 MTA (2.0 um) $IC_{50}$ (nm) |
|---|---|
| 132 | 4.4 |
| 146 | 2.5 |
| 147 | 2.9 |
| 148 | 2.6 |

**Biological Test Example 2 Proliferation Inhibition of HCT116 and HCT116 MTAP-KO cells in vitro**

**Experimental Materials:**

HCT116 cell line was purchased from the Chinese Academy of Sciences Cell Bank, and the MTAP gene was knocked out by CRISPR/Cas9 technology to obtain HCT116-MTAP-KO cell line.

McCoy's 5A medium (Gibco, catalog No.16600082), fetal bovine serum (Gibco, catalog No.10099141C), penicillin-streptomycin double antibody (Gibco, catalog No.15140122), pancreatic enzyme (Gibco, catalog No.25200056), CellTiter Glo assay kit (Promega, catalog No.G7572), 384-well transparent flat-bottomed black walled cell culture plate (Corning, catalog No.3764), ultra micro sampler (Tecan, catalog No.D300e), Multimode reader (Biotek, catalog No. SynergyHTX)

**Experimental method:**

1. Cell culture: HCT116 and HCT116-MTAP-KO cells were cultured in McCoy'5A medium + 10% fetal bovine serum + 1% penicillin-streptomycin double antibody; to ensure that they were always in the logarithmic growth phase and the cell viability greater than 95%.
2. Compound concentration gradient preparation: The compound to be tested was added to a 384-well plate using an ultra micro sampler, starting from 30 μM (HCT116 cells) or 3 μM (HCT116-MTAP-KO cells), and diluted with DMSO at 3 times for a total of 9 concentrations and set up three duplicate wells.
3. Treatment of cells with compounds: Trypsin-digested HCT116 or HCT116-MTAP-KO cell suspension was added to the 384-well plates spotted with the compounds to be tested at 40 μL per well, i.e., 100 cells per well, and the final concentration of DMSO was 0.4%. The cell culture plate was incubated at 37°C in a 5% $CO_2$ incubator for 6 days.
4. Detection: 20 μL of CellTiter Glo reagent was added to each well of the cell culture plate and incubated at room temperature for 30 minutes. A multimode reader was used to detect the luminescence signal at 578 nm.
5. Data analysis:
The data was fitted with GraphPad Prism 8.0 software using a four parameter inhibitor-reaction model to obtain the $IC_{50}$ value (half inhibitory concentration) of the test compounds.

The biological activities of some of the compounds were measured by experimental methods. "A" represents $IC_{50}$ (nm)<100, "B" represents 100<$IC_{50}$ (nm)<1000, "C" represents 1000<$IC_{50}$ (nm)<10000, as shown in Table 4. The first column represents cell proliferation inhibition rate HCT116 MTAP WT $IC_{50}$ (nm), and the second column represents cell proliferation inhibition rate HCT116-MTAP null IC50 (nm):

Table 4

| | Cell proliferation inhibition rate HCT116 MTAP WT IC50 (nm) | Cell proliferation inhibition rate HCT116-MT AP null IC50 (nm) | | Cell proliferation inhibition rate HCT116 MTAP WT IC50 (nm) | Cell proliferation inhibition rate HCT116-MT AP null IC50 (nm) |
|---|---|---|---|---|---|
| 1 | C | A | 2 | C | A |
| 3 | C | A | 4 | C | A |
| 5 | C | A | 6 | C | A |
| 7 | C | A | 8 | C | A |
| 9 | C | B | 10 | C | A |

(continued)

| | Cell proliferation inhibition rate HCT116 MTAP WT IC50 (nm) | Cell proliferation inhibition rate HCT116-MT AP null IC50 (nm) | | Cell proliferation inhibition rate HCT116 MTAP WT IC50 (nm) | Cell proliferation inhibition rate HCT116-MT AP null IC50 (nm) |
|---|---|---|---|---|---|
| 11 | C | A | 12 | B | A |
| 13 | B | A | 14 | C | A |
| 15 | C | B | 16 | C | B |
| 19 | B | A | 18 | C | A |
| 23 | C | A | 20 | B | A |
| 25 | C | B | 22 | C | B |
| 27 | C | A | 24 | C | A |
| 29 | C | B | 26 | C | A |
| 31 | C | A | 28 | C | A |
| 33 | C | B | 30 | C | A |
| 35 | C | A | 32 | C | A |
| 37 | C | A | 34 | A | A |
| 39 | C | A | 36 | B | A |
| 41 | C | A | 38 | C | A |
| 43 | C | A | 40 | C | A |
| 45 | C | A | 42 | C | A |
| 47 | C | A | 44 | C | A |
| 49 | C | A | 50 | C | B |
| 51 | C | B | 52 | C | A |
| 53 | C | A | 54 | C | B |
| 55 | C | A | 56 | C | B |
| 57 | C | B | 58 | B | A |
| 59 | C | A | 60 | C | A |
| 61 | C | A | 62 | C | A |
| 63 | C | A | 64 | C | B |
| 65 | C | B | 66 | C | B |
| 67 | C | B | 68 | C | A |
| 69 | C | B | 74 | C | B |
| 71 | C | A | 78 | C | A |
| 73 | C | A | 80 | C | A |
| 75 | C | A | 82 | C | A |
| 81 | C | A | 84 | C | A |
| 83 | B | A | 86 | C | A |
| 85 | C | A | 88 | C | A |
| 87 | C | A | 90 | B | A |
| 89 | C | A | 92 | C | A |
| 91 | C | A | 94 | C | B |

(continued)

| | Cell proliferation inhibition rate HCT116 MTAP WT IC50 (nm) | Cell proliferation inhibition rate HCT116-MT AP null IC50 (nm) | | Cell proliferation inhibition rate HCT116 MTAP WT IC50 (nm) | Cell proliferation inhibition rate HCT116-MT AP null IC50 (nm) |
|---|---|---|---|---|---|
| 93 | C | A | 96 | C | A |
| 95 | C | A | 98 | C | A |
| 97 | C | A | 100 | C | A |
| 99 | C | A | 102 | C | A |
| 101 | C | A | 104 | B | A |
| 103 | C | B | 106 | C | A |
| 105 | C | A | 108 | C | A |
| 107 | C | A | 110 | C | B |
| 109 | C | A | 112 | B | A |
| 111 | A | A | 116 | C | A |
| 113 | C | B | 118 | C | B |
| 115 | B | A | 120 | C | A |
| 117 | C | B | 122 | C | A |
| 119 | C | A | 124 | C | A |
| 121 | C | B | 126 | C | A |
| 123 | B | A | 128 | B | B |
| 125 | C | A | 130 | C | A |
| 127 | C | A | 132 | C | A |
| 129 | C | A | 134 | C | A |
| 131 | C | B | 136 | C | B |
| 133 | B | A | 138 | C | B |
| 135 | C | B | 140 | C | B |
| 137 | C | B | 142 | C | B |
| 139 | C | A | 144 | C | B |
| 141 | C | B | 146 | C | B |
| 143 | C | A | 150 | C | B |
| 147 | C | B | 154 | C | B |
| 151 | C | B | 156 | C | B |
| 153 | C | B | 158 | C | B |
| 155 | C | B | 160 | C | B |
| 157 | C | B | 162 | C | B |
| 159 | C | B | 166 | B | A |
| 163 | C | B | 168 | C | B |
| 167 | C | B | 170 | B | A |
| 169 | B | A | 172 | B | A |
| 171 | C | B | 174 | A | B |
| 173 | C | B | 176 | C | B |

(continued)

| | Cell proliferation inhibition rate HCT116 MTAP WT IC50 (nm) | Cell proliferation inhibition rate HCT116-MT AP null IC50 (nm) | | Cell proliferation inhibition rate HCT116 MTAP WT IC50 (nm) | Cell proliferation inhibition rate HCT116-MT AP null IC50 (nm) |
|---|---|---|---|---|---|
| 175 | C | B | 178 | C | B |
| 177 | C | B | 180 | C | B |
| 179 | C | A | 182 | B | A |
| 181 | C | B | 184 | C | B |
| 183 | C | A | 188 | C | A |
| 185 | C | A | 190 | C | B |
| 189 | C | A | 192 | C | B |
| 195 | C | B | 194 | C | A |
| 199 | C | B | 210 | C | B |
| 203 | C | A | 212 | C | B |
| 205 | C | A | 214 | C | B |
| 207 | C | A | 216 | C | B |
| 209 | C | A | 218 | C | A |
| 211 | C | B | 222 | C | A |
| 213 | C | B | 224 | C | B |
| 215 | C | A | 226 | C | B |
| 217 | C | B | 228 | C | B |
| 219 | C | B | 230 | C | B |
| 221 | C | A | 232 | C | B |
| 223 | C | B | 234 | C | A |
| 225 | C | A | 236 | C | A |
| 227 | C | A | 238 | C | B |
| 229 | C | A | 240 | C | A |
| 231 | C | B | 242 | B | A |
| 233 | C | A | 244 | C | A |
| 235 | A | A | 246 | C | A |
| 237 | C | B | 250 | C | A |
| 239 | C | A | 252 | C | B |
| 241 | B | A | 254 | C | B |
| 243 | C | B | 258 | C | B |
| 245 | C | A | 262 | C | A |
| 247 | C | B | 264 | C | B |
| 249 | C | B | 268 | C | B |
| 253 | C | B | 272 | C | B |
| 255 | C | B | 274 | C | B |
| 257 | C | B | 276 | C | B |
| 259 | C | A | 278 | C | A |

(continued)

| | Cell proliferation inhibition rate HCT116 MTAP WT IC50 (nm) | Cell proliferation inhibition rate HCT116-MT AP null IC50 (nm) | | Cell proliferation inhibition rate HCT116 MTAP WT IC50 (nm) | Cell proliferation inhibition rate HCT116-MT AP null IC50 (nm) |
|---|---|---|---|---|---|
| 261 | C | B | 280 | C | B |
| 263 | C | B | 282 | C | A |
| 267 | C | A | 284 | B | A |
| 269 | C | B | 286 | C | A |
| 271 | C | A | 288 | C | A |
| 273 | C | A | 290 | C | A |
| 277 | C | A | 292 | C | B |
| 279 | C | B | 294 | C | A |
| 283 | C | A | 296 | C | B |
| 285 | B | A | 298 | C | B |
| 293 | C | B | 300 | C | A |
| 297 | C | A | 302 | B | A |
| 299 | C | A | 304 | B | A |
| 303 | B | A | | | |

All documents mentioned in the present invention are cited as references in this application, just as each document is individually cited as a reference. In addition, it should be understood that, after reading the above teaching content of the present invention, those skilled in the art can make various changes or modifications to the present invention, and these equivalent forms also fall within the scope defined by the appended claims of the present application.

## Claims

**1.** A compound of formula I, or a pharmaceutically acceptable salt thereof or a deuterated product thereof:

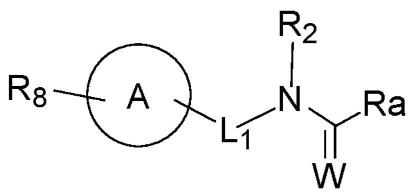

I

wherein,

Ra is selected from the group consisting of

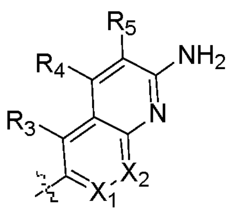 and 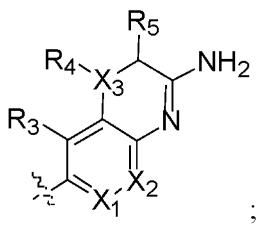 ;

W is O or S;

$X_1$, $X_2$ are each independently selected from the group consisting of CR and N; $X_3$ is N;
$L_1$ is selected from the group consisting of: chemical bonds, -O-, -CHR-, and -C(R)R-;
Ring A is selected from the group consisting of: substituted or unsubstituted 8-12 membered fused bicyclic heterocyclyl (including carbocycle or heterocycle, preferably five-membered fused six-membered ring), and substituted or unsubstituted 7-10 membered fused bicyclic heteroaryl (preferably five-membered fused six-membered ring);
$R_8$ is selected from the group consisting of: H, deuterium, halogen, cyano, amino, nitro, hydroxyl, thiol, aldehyde, carboxyl, $C_2$-$C_6$ alkynyl, $SF_5$, substituted or unsubstituted or halogenated $C_1$-$C_6$ alkyl, and unsubstituted or halogenated $C_1$-$C_6$ alkoxyl, or $R_8$ is

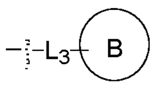
;

$L_3$ is selected from the group consisting of: chemical bonds, -O-, -CHR-, -C(R)R-, carbonyl, S, and -NH-;
Ring B is selected from the group consisting of: substituted or unsubstituted benzene ring, substituted or unsubstituted 5-6-membered heteroaromatic ring, substituted or unsubstituted $C_3$-$C_6$ carbocycle (including saturated and partially unsaturated situations), substituted or unsubstituted 3-7-membered heterocycle (including saturated and partially unsaturated situations);
$R_2$ is selected from the group consisting of: $R_7$, and $-L_2R_7$; wherein, $L_2$ is selected from the group consisting of: -O-, -CHR-, -C(R)R-, and carbonyl; wherein, $R_7$ is selected from the group consisting of: hydrogen, none, substituted or unsubstituted $C_1$-$C_6$ alkyl, substituted or unsubstituted $C_{6-10}$ aromatic ring, and substituted or unsubstituted 5-12 membered (preferably 5-6 membered or 8-10 membered) heteroaromatic ring, substituted or unsubstituted $C_3$-$C_{10}$ carbocycle (including saturated and partially unsaturated situations, including single ring, fused ring, spiro ring and bridged ring), and substituted or unsubstituted 3-10 membered heterocycle (including saturated and partially unsaturated situations, including single ring, fused ring, spiro ring and bridged ring);
$R_3$ is selected from the group consisting of H, deuterium, halogen, cyano, and substituted or unsubstituted $C_1$-$C_6$ alkyl;
$R_4$ and $R_5$ are each independently selected from the group consisting of: H, halogen, cyano, substituted or unsubstituted $C_1$-$C_6$ alkyl, substituted or unsubstituted $C_1$-$C_6$ alkoxyl, substituted or unsubstituted $C_3$-$C_6$ carbocycle (including saturated and partially unsaturated situations), and substituted or unsubstituted 3-6 membered heterocycle; or $R_4$ and $R_5$ together with the connected ring atom form a 5-12 membered saturated or unsaturated ring, and the ring can be substituted or unsubstituted;
R is H, deuterium, halogen, substituted or unsubstituted $C_1$-$C_4$ alkyl, substituted or unsubstituted $C_1$-$C_4$ alkoxyl, and substituted or unsubstituted $C_3$-$C_6$ cycloalkyl;
unless otherwise specified, in the above formulas, the substituted refers to hydrogen atoms on the corresponding group are substituted by one or more substituents selected from the group consisting of: deuterium, tritium, halogen, hydroxyl, carboxyl, thiol, benzyl, $C_1$-$C_{12}$ alkoxycarbonyl, $C_1$-$C_6$ aldehyde, amino, $C_1$-$C_6$ amide, nitro, cyano, unsubstituted or halogenated $C_1$-$C_6$ alkyl, unsubstituted or halogenated $C_3$-$C_8$ cycloalkyl, $C_2$-$C_{10}$ alkenyl, $C_1$-$C_6$ alkoxyl, $C_1$-$C_6$ alkyl-amino, $C_6$-$C_{10}$ aryl, five-membered or six-membered heteroaryl, five-membered or six-membered non-aromatic heterocyclyl, -O-($C_6$-$C_{10}$ aryl), -O- (five-membered or six-membered heteroaryl), $C_1$-$C_{12}$ alkylamino carbonyl, unsubstituted or halogenated $C_2$-$C_{10}$ acyl, sulfonyl ($-SO_2$-OH), phosphoryl-($-PO_3$-OH), unsubstituted or halogenated $C_1$-$C_4$ alkyl-$S(O)_2$-, unsubstituted or halogenated $C_1$-$C_4$ alkyl-SO-, and $-SF_5$.

**2.** The compound according to claim 1, or a pharmaceutically acceptable stereoisomer, a salt or a deuterated product thereof, wherein, Ring A is selected from the group consisting of:

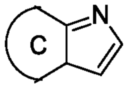
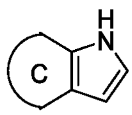
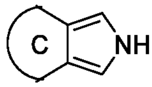
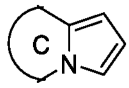
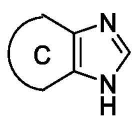

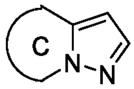
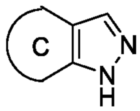
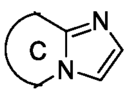
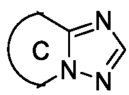
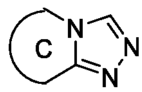

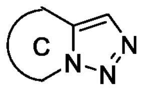
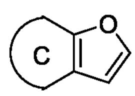
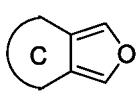
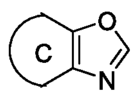
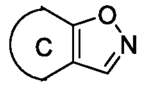

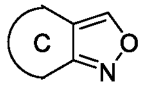
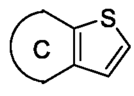
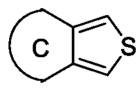
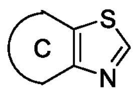
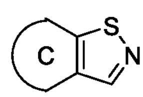

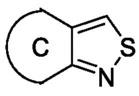
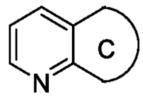
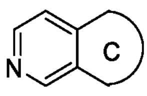
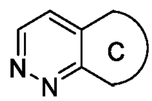
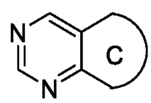

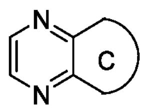
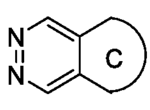
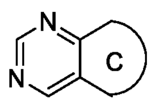
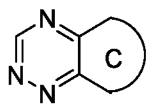
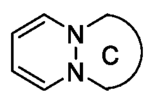

**3.** The compound according to claim 1, or a pharmaceutically acceptable salt or a deuterated product thereof, wherein, Ra is selected from the group consisting of:

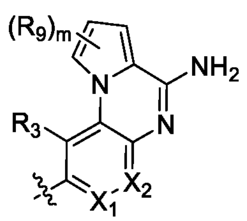
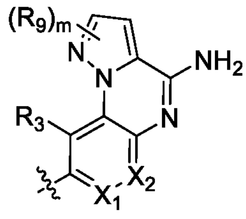
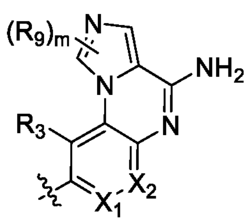
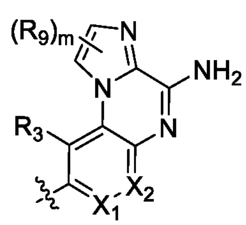

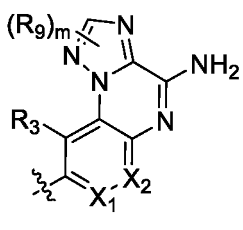
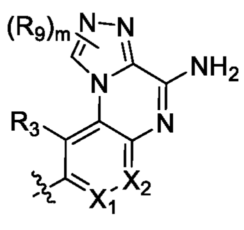
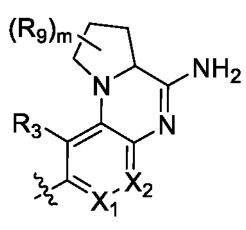
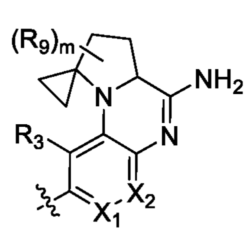

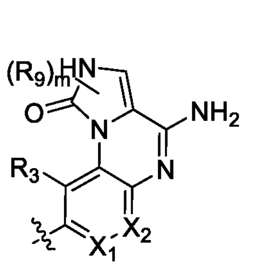
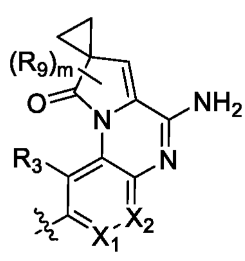
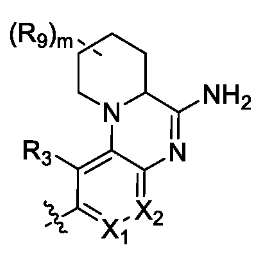
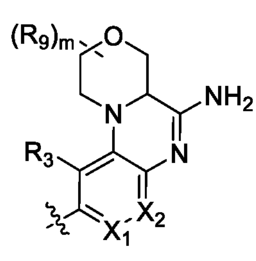

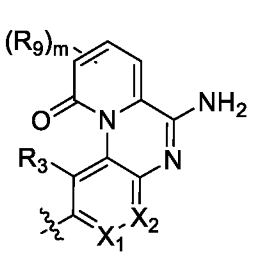
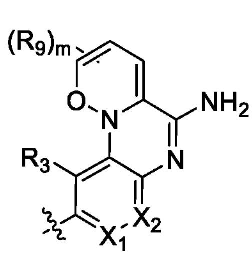
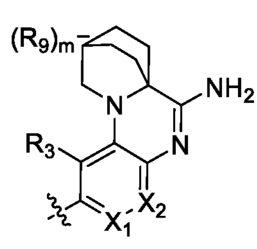
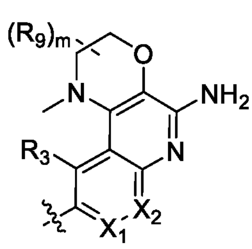

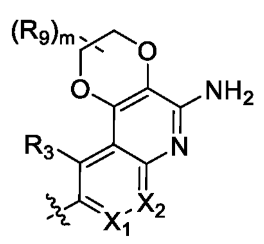
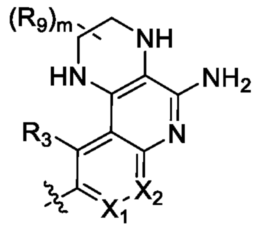
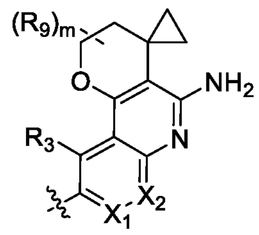
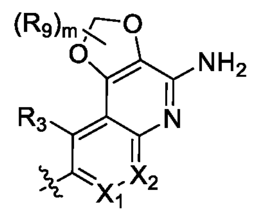
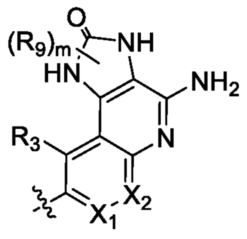
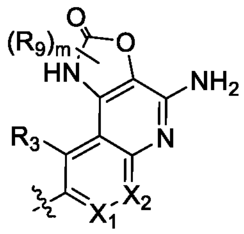
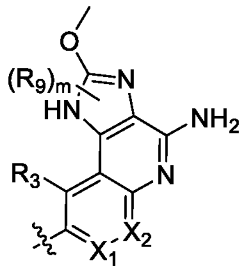
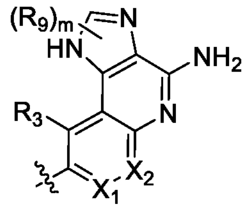
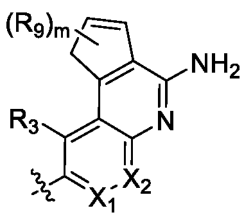
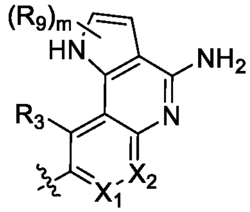
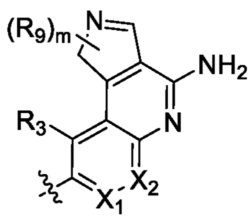
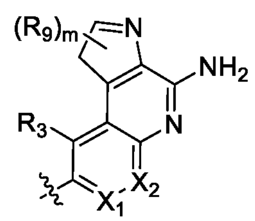
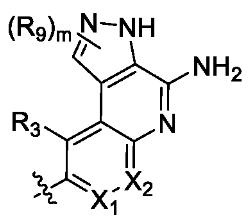
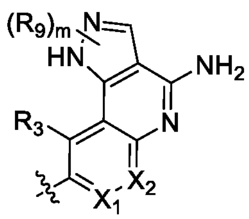
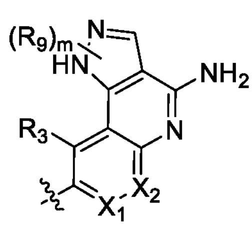
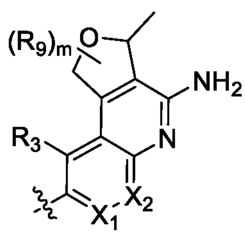
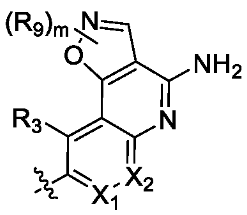
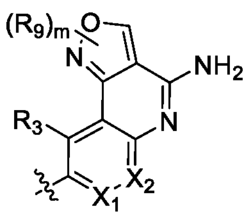
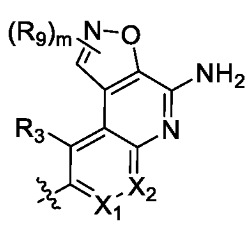
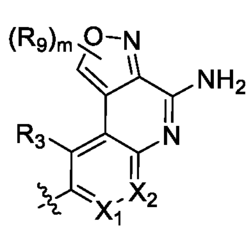
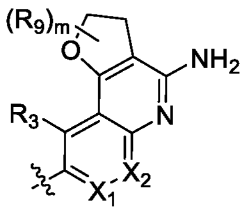
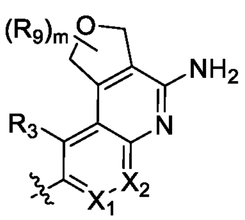
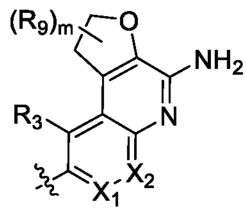
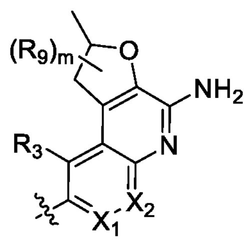

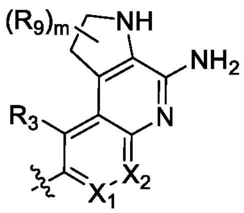 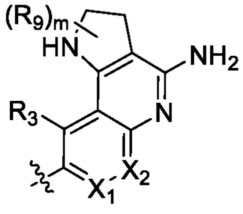 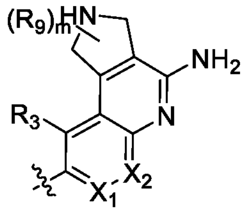 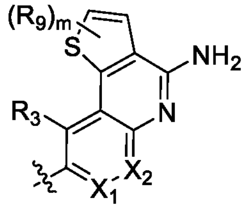

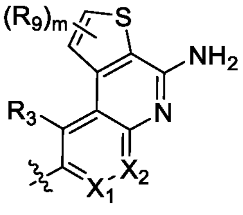 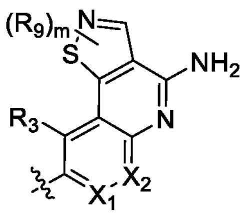 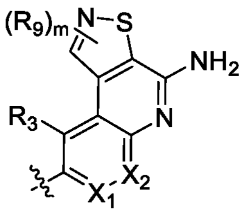 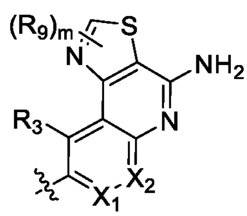

wherein, $R_9$ is selected from the group consisting of: deuterium, tritium, halogen, hydroxyl, carboxyl, unsubstituted or halogenated $C_1$-$C_6$ alkyl, unsubstituted or halogenated $C_1$-$C_6$ alkoxyl, unsubstituted or halogenated $C_1$-$C_6$ alkyl-OH, -NH (unsubstituted or halogenated $C_1$-$C_6$ alkyl), and- N (unsubstituted or halogenated $C_1$-$C_6$ alkyl)$_2$; m is selected from 0, 1, 2, and 3.

4. The compound according to claim 1, or a pharmaceutically acceptable salt or a deuterated product thereof, wherein, $L_1$ is -$CH_2$-, or -$CH(CH_3)$-; ring A is selected from the group consisting of:

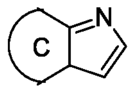 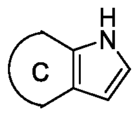 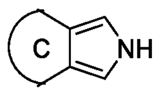 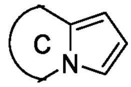 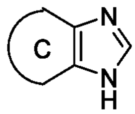

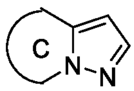 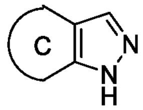 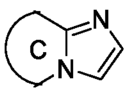 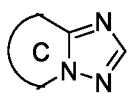 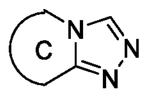

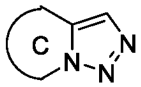 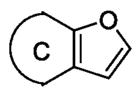 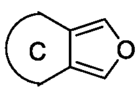 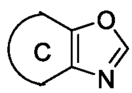 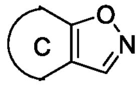

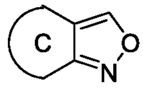 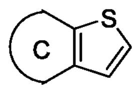 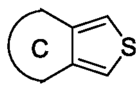 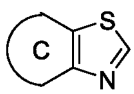 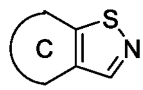

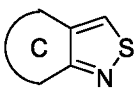 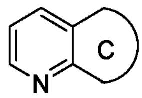 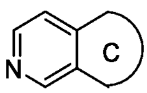 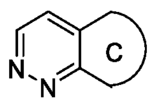 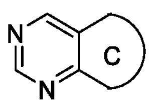

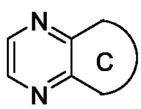 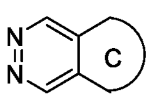 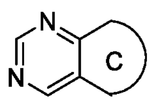 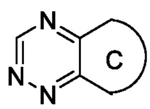 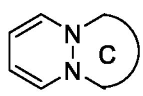

wherein ring C is selected from the group consisting of: substituted or unsubstituted benzene ring, substituted or unsubstituted 5-6-membered heteroaromatic ring, substituted or unsubstituted $C_3$-$C_6$ carbocycle (including saturated and partially unsaturated situations), and substituted or unsubstituted 3-6-membered heterocycle (including saturated and partially unsaturated situations).

**5.** The compound according to claim 1, or a pharmaceutically acceptable stereoisomer, a salt or a deuterated product thereof, wherein, $R_2$ is ortho-substituted 5-membered or 6-membered heteroaromatic ring, as shown below:

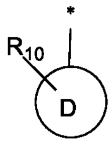

wherein, $R_{10}$ is a substituent located adjacent to the connecting site, and selected from the group consisting of: hydrogen, deuterium, halogen, unsubstituted or halogenated $C_1$-$C_3$ alkyl, and unsubstituted or halogenated $C_1$-$C_3$ alkoxyl;

preferably, ring D is selected from the group consisting of: substituted or unsubstituted benzene ring, substituted or unsubstituted 5-6 membered heteroaromatic ring, more preferably, ring D is selected from the group consisting of:

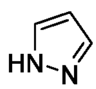 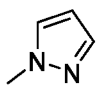 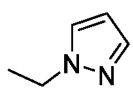 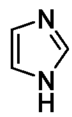 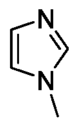

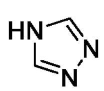 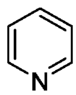 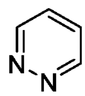 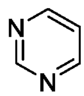

and ring A is selected from the group consisting of: substituted or unsubstituted 8-12 membered fused bicyclic heterocyclyl (including carbocycle or heterocycle, preferably five-membered fused six-membered ring), and substituted or unsubstituted 7-10 membered fused bicyclic heteroaryl (preferably five-membered fused six-membered ring); preferably, ring A is selected from the group consisting of:

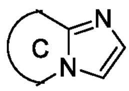 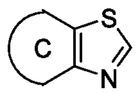 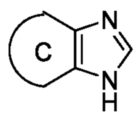

wherein ring C is selected from the group consisting of substituted or unsubstituted benzene ring, and substituted or unsubstituted 5-6 membered heteroaryl ring; and $R_8$ is $CF_3$.

**6.** The compound according to claim 1, or a pharmaceutically acceptable stereoisomer, a salt or a deuterated product thereof, wherein, $R_2$ is selected from the group consisting of: $R_7$, and $-L_2R_7$; wherein, $L_2$ is selected from the group consisting of: -O-, -CHR-, carbonyl, S, and -NH-; wherein, $R_7$ is selected from the group consisting of: substituted or unsubstituted $C_1$-$C_6$ alkyl, substituted or unsubstituted $C_{6-10}$ aromatic ring, and substituted or unsubstituted 5-12 membered heteroaromatic ring.

**7.** The compound according to claim 1, or a pharmaceutically acceptable stereoisomer, a salt or a deuterated product thereof, wherein, $R_7$ is selected from the group consisting of: substituted or unsubstituted $C_1$-$C_6$ alkyl, substituted or unsubstituted phenyl, and substituted or unsubstituted 5-7 membered heteroaromatic ring.

**8.** The compound according to claim 1, or a pharmaceutically acceptable stereoisomer, a salt or a deuterated product thereof, wherein, $R_2$ is selected from the group consisting of $R_7$, and $-(CHR)R_7$; wherein, $R_7$ is selected from the group consisting of: substituted or unsubstituted $C_{6-10}$ aromatic ring, and substituted or unsubstituted 5-12 membered heteroaromatic ring.

**9.** The compound according to claim 1, or a pharmaceutically acceptable stereoisomer, a salt or a deuterated product thereof, wherein, $R_2$ is substituted or unsubstituted 5-7 membered heteroaromatic ring; ring A is selected from the group consisting of: substituted or unsubstituted 7-10 membered fused bicyclic heteroaryl; and $R_8$ is $CF_3$.

**10.** The compound according to claim 1, or a pharmaceutically acceptable stereoisomer, a salt or a deuterated product thereof, wherein, Ra has a structure as shown in the following formula:

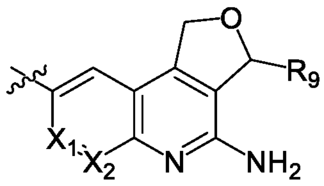 or 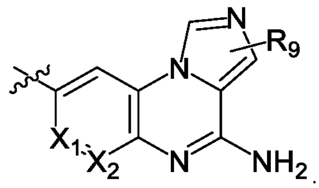.

**11.** The compound according to any one of claims 1-10, or a pharmaceutically acceptable stereoisomer, a salt or a deuterated product thereof, wherein, the compound has a structure selected from the following table:

Table 1

| NO. | Structure | NO. | Structure |
|---|---|---|---|
| 1 | 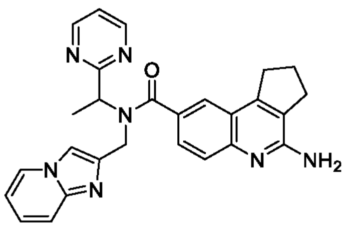 | 2 | 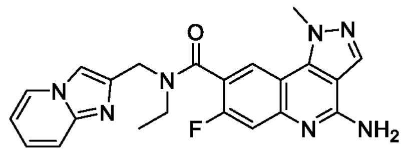 |
| 3 | 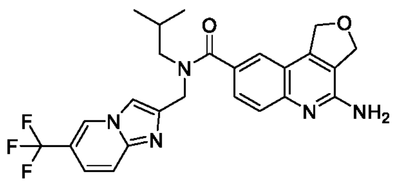 | 4 | 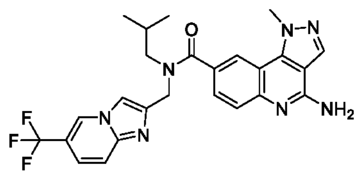 |
| 5 | 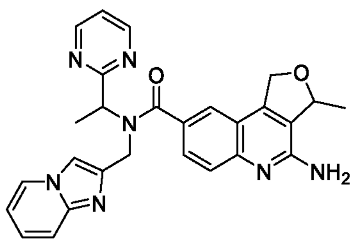 | 6 | 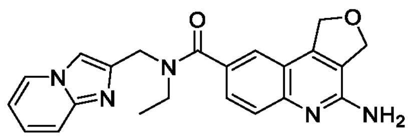 |
| 7 | 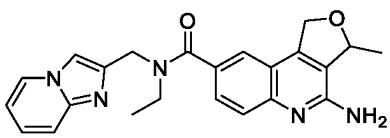 | 8 | 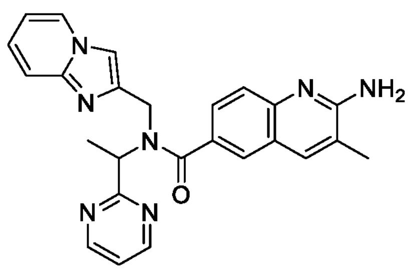 |
| 9 | 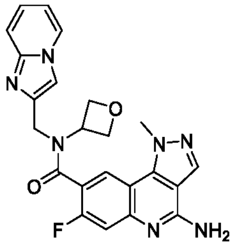 | 10 | 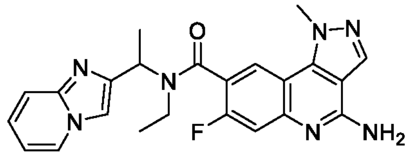 |

(continued)

| NO. | Structure | NO. | Structure |
|---|---|---|---|
| 11 | 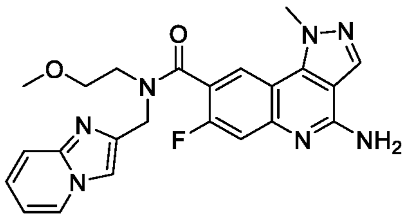 | 12 | 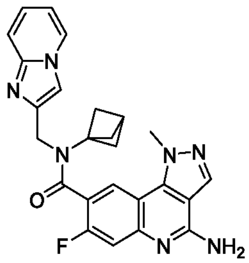 |
| 13 | 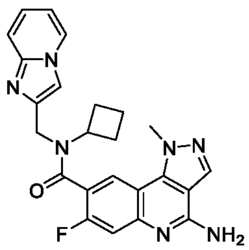 | 14 | 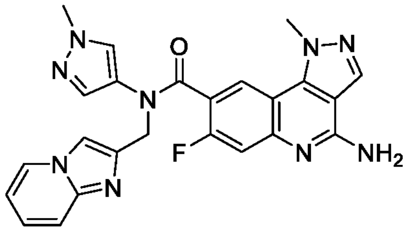 |
| 15 | 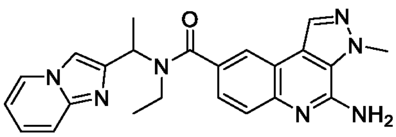 | 16 | 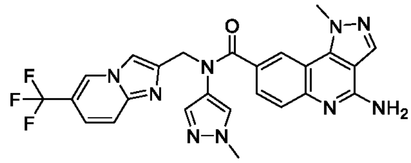 |
| 17 | 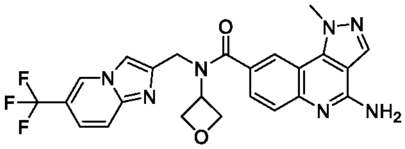 | 18 | 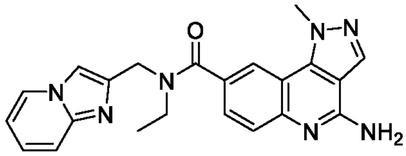 |
| 19 | 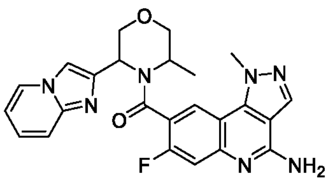 | 20 | 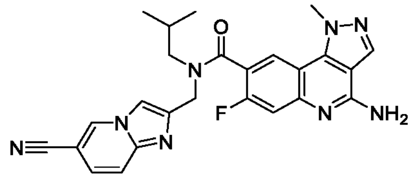 |
| 21 | 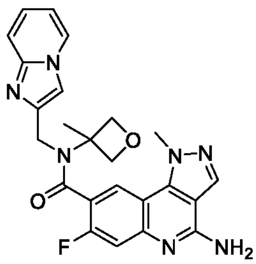 | 22 | 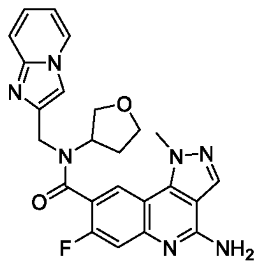 |
| 23 | 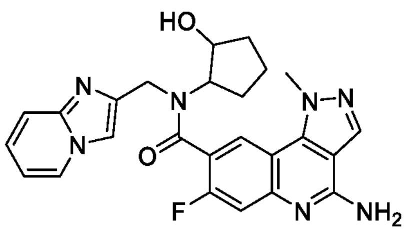 | 24 | 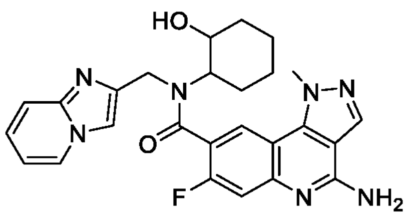 |

(continued)

| NO. | Structure | NO. | Structure |
| --- | --- | --- | --- |
| 25 | 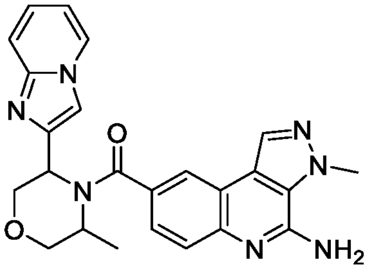 | 26 | 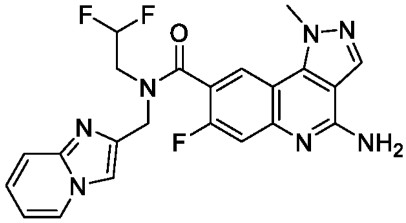 |
| 27 | 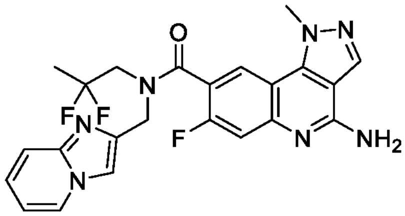 | 28 | 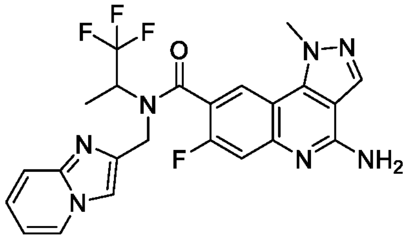 |
| 29 | 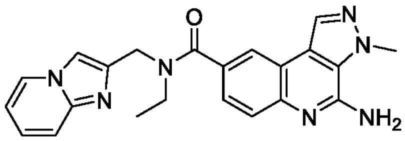 | 30 | 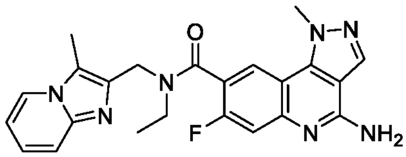 |
| 31 | 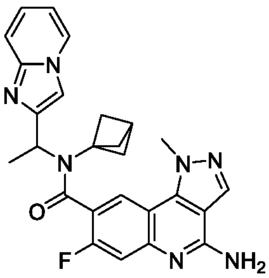 | 32 | 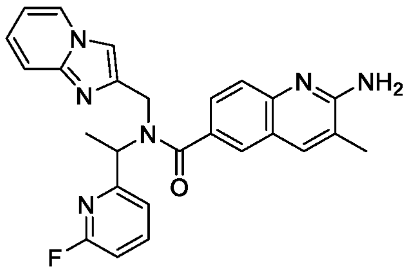 |
| 33 | 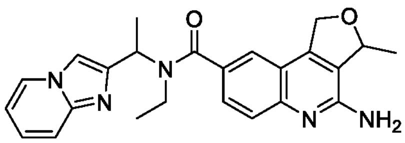 | 34 | 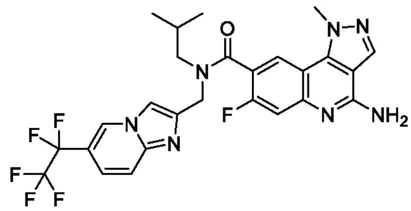 |
| 35 | 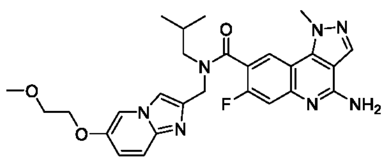 | 36 | 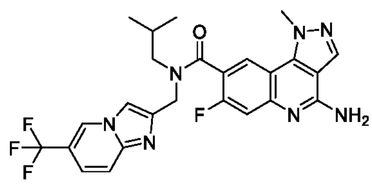 |
| 37 | 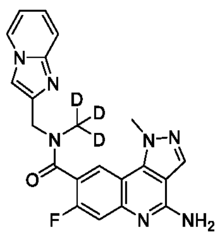 | 38 | 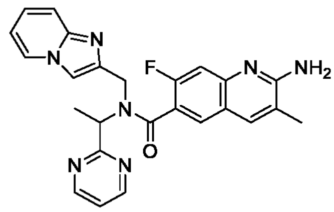 |

(continued)

| NO. | Structure | NO. | Structure |
|---|---|---|---|
| 39 | 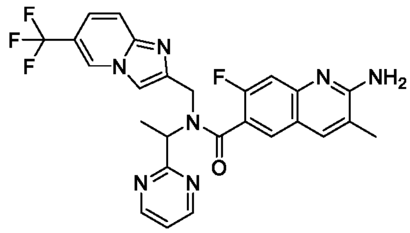 | 40 | 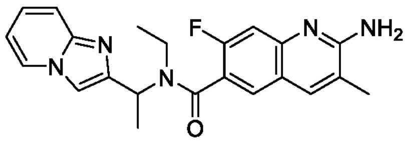 |
| 41 | 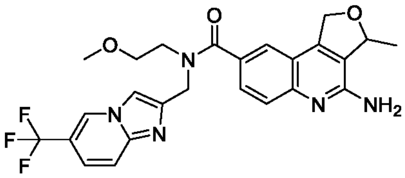 | 42 | 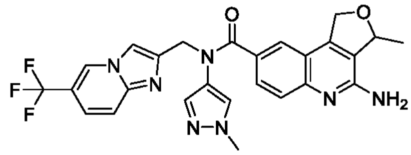 |
| 43 | 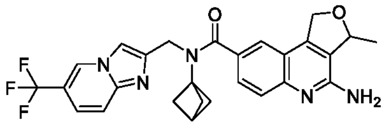 | 44 | 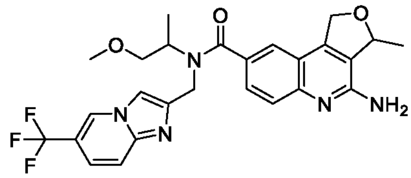 |
| 45 | 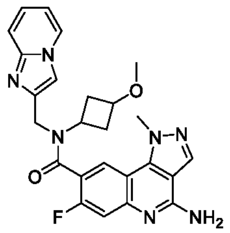 | 46 | 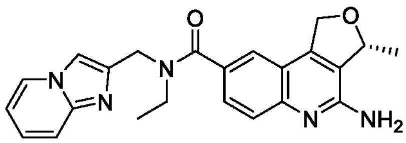 |
| 47 | 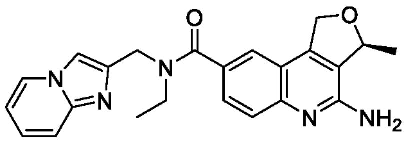 | 48 | 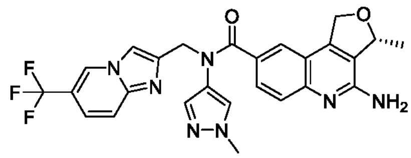 |
| 49 | 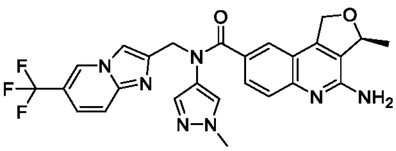 | 50 | 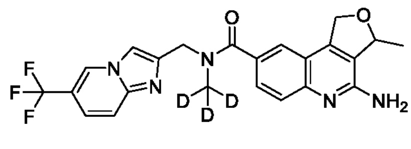 |
| 51 | 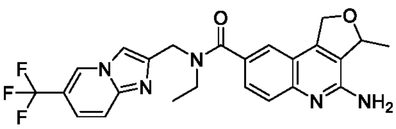 | 52 | 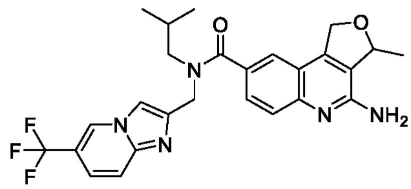 |
| 53 | 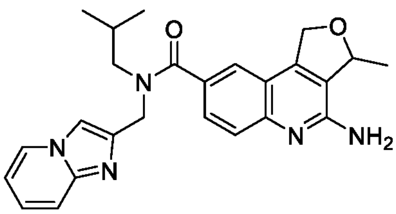 | 54 | 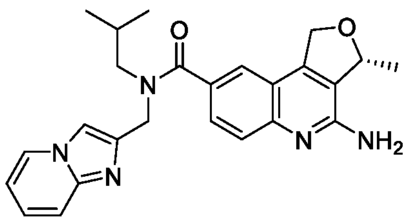 |

(continued)

| NO. | Structure | NO. | Structure |
|---|---|---|---|
| 55 | 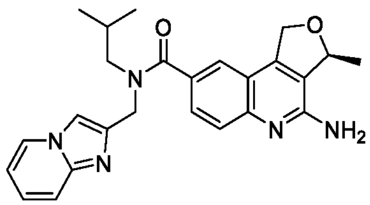 | 56 | 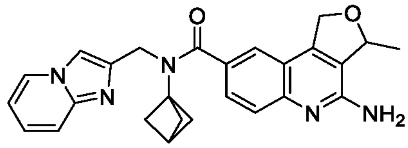 |
| 57 | 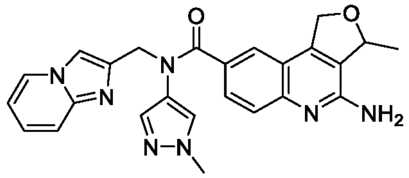 | 58 | 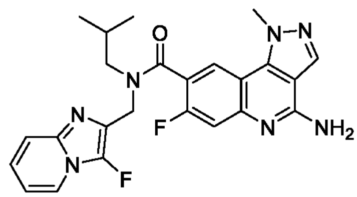 |
| 59 | 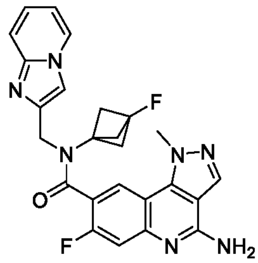 | 60 | 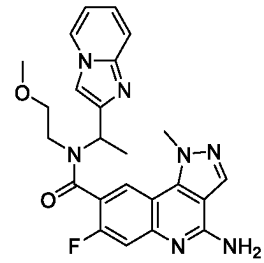 |
| 61 | 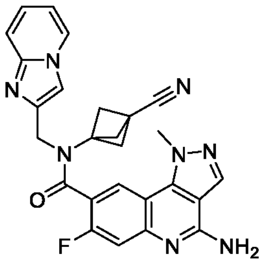 | 62 | 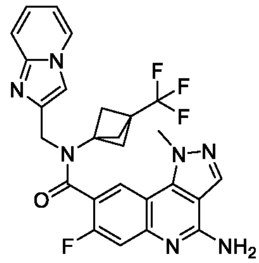 |
| 63 | 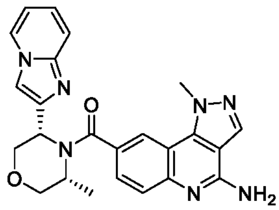 | 64 | 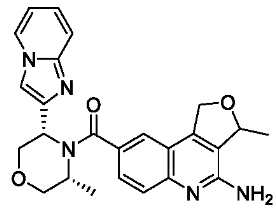 |
| 65 | 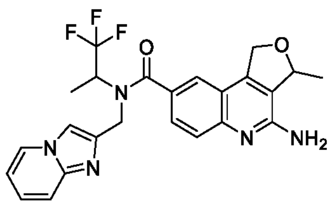 | 66 | 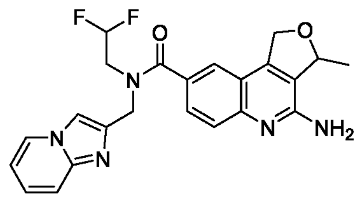 |
| 67 | 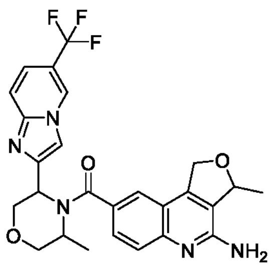 | 68 | 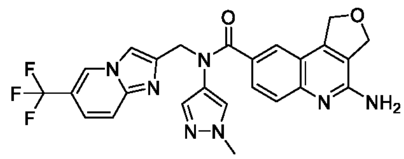 |

(continued)

| NO. | Structure | NO. | Structure |
|---|---|---|---|
| 69 | 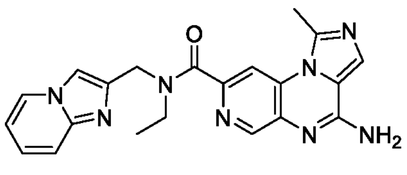 | 70 | 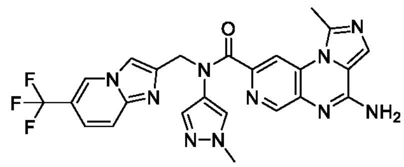 |
| 71 | 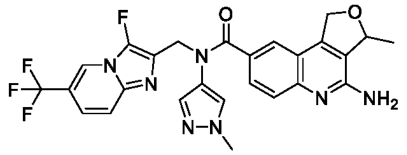 | 72 | 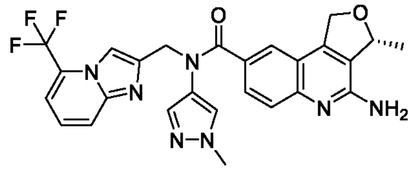 |
| 73 | 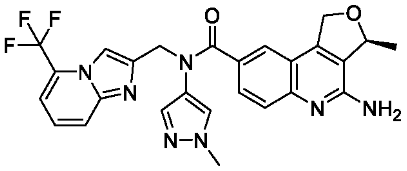 | 74 | 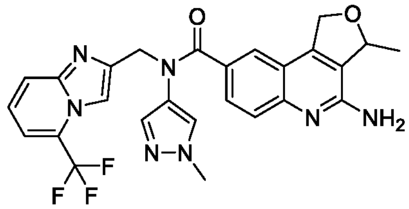 |
| 75 | 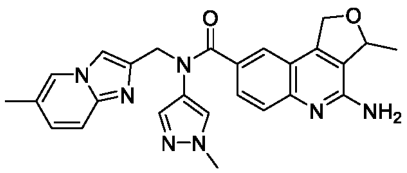 | 76 | 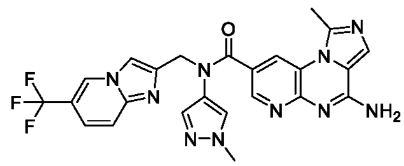 |
| 77 | 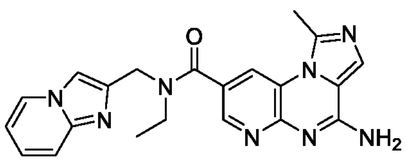 | 78 | 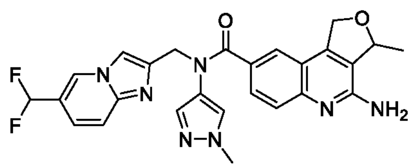 |
| 79 | 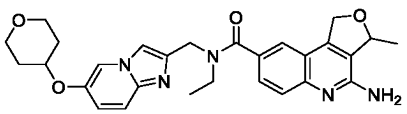 | 80 | 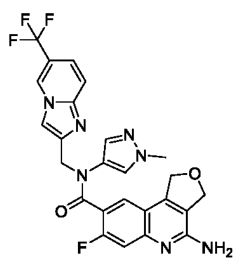 |
| 81 | 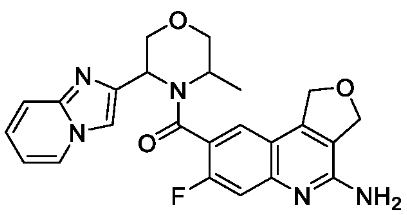 | 82 | 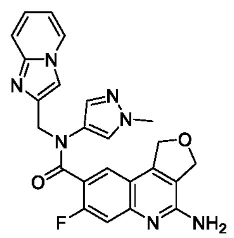 |

(continued)

| NO. | Structure | NO. | Structure |
|---|---|---|---|
| 83 | 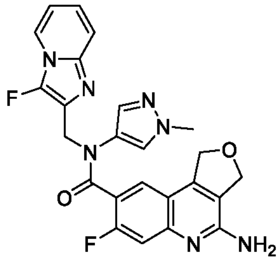 | 84 | 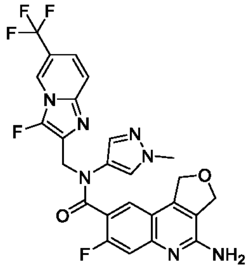 |
| 85 | 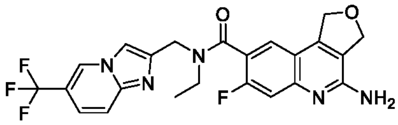 | 86 | 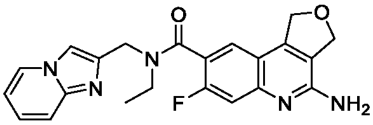 |
| 87 | 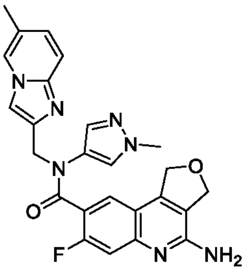 | 88 | 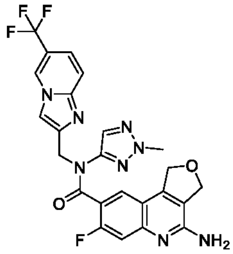 |
| 89 | 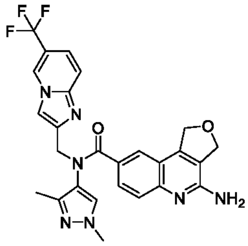 | 90 | 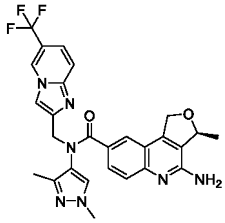 |
| 91 | 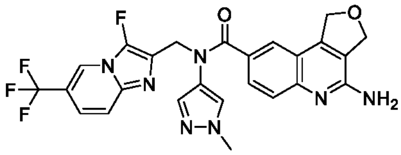 | 92 | 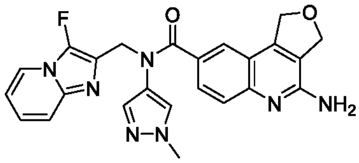 |
| 93 | 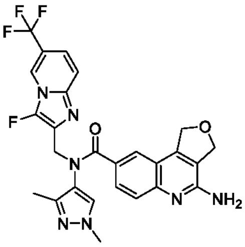 | 94 | 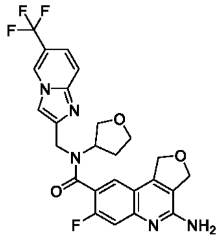 |
| 95 | 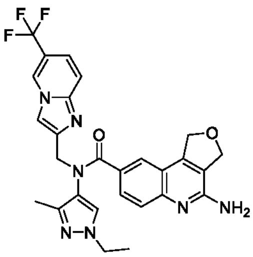 | 96 | 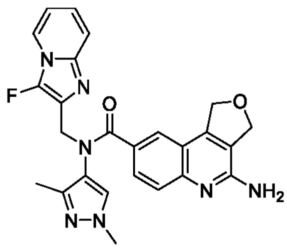 |

(continued)

| NO. | Structure | NO. | Structure |
|---|---|---|---|
| 97 | 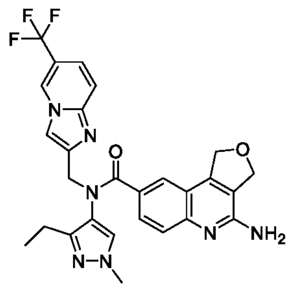 | 98 | 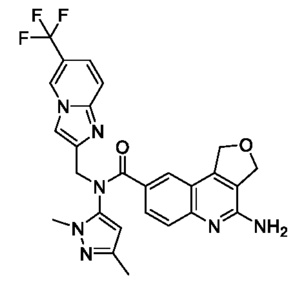 |
| 99 | 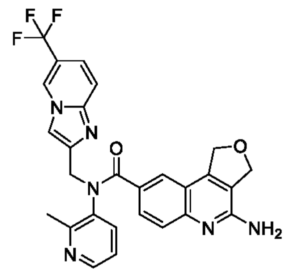 | 100 | 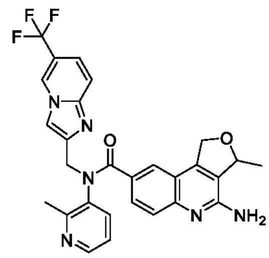 |
| 101 | 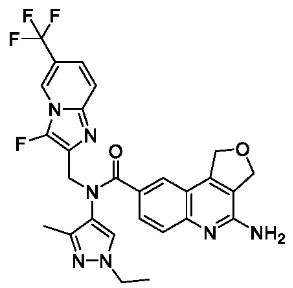 | 102 | 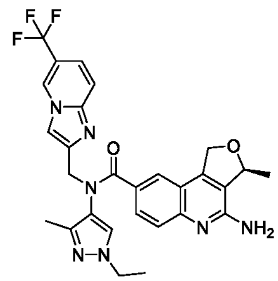 |
| 103 | 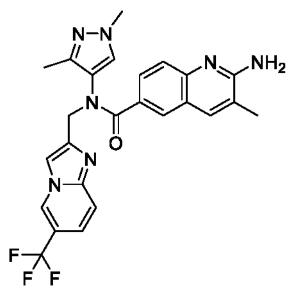 | 104 | 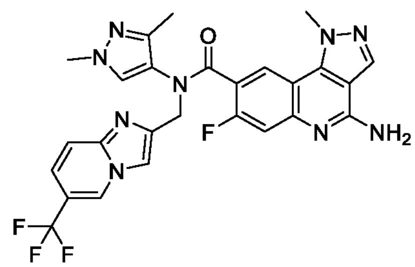 |
| 105 | 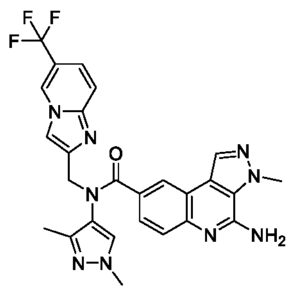 | 106 | 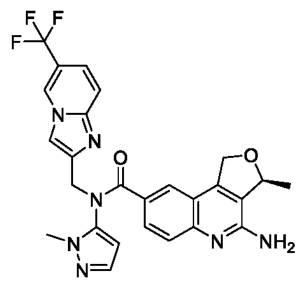 |

(continued)

| NO. | Structure | NO. | Structure |
|---|---|---|---|
| 107 | 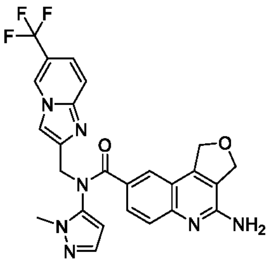 | 108 | 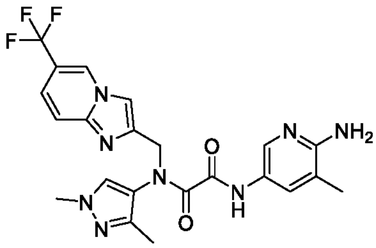 |
| 109 | 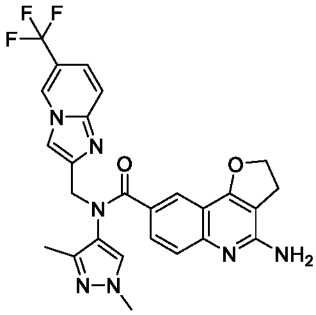 | 110 | 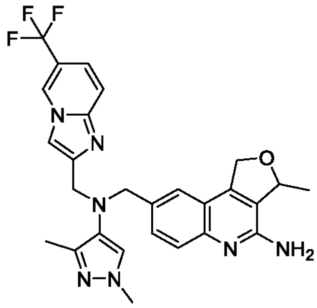 |
| 111 | 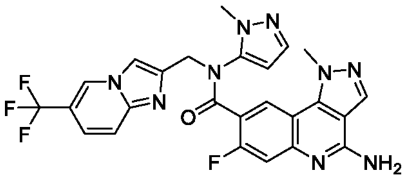 | 112 | 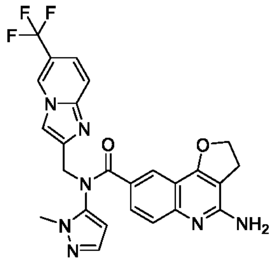 |
| 113 | 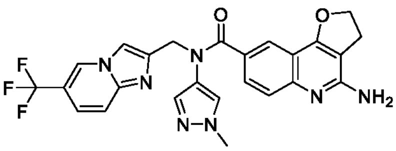 | 114 | 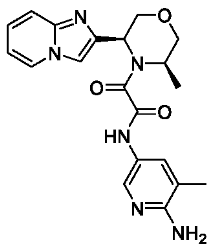 |
| 115 | 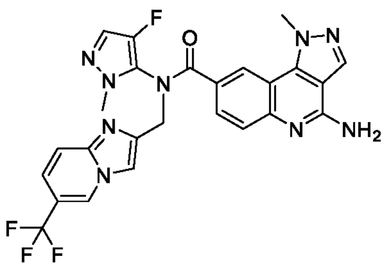 | 116 | 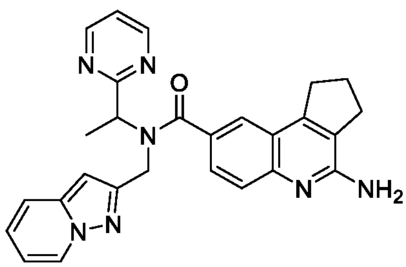 |
| 117 | 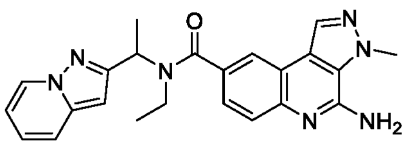 | 118 | 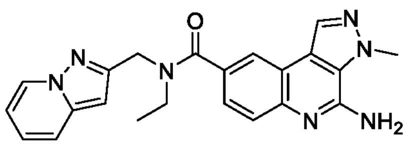 |

(continued)

| NO. | Structure | NO. | Structure |
|---|---|---|---|
| 119 | 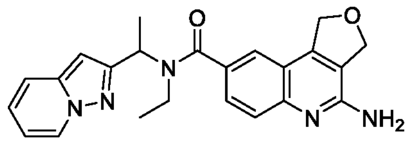 | 120 | 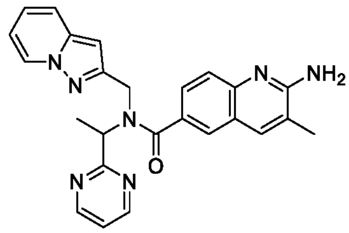 |
| 121 | 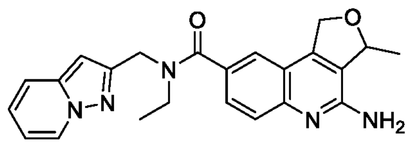 | 122 | 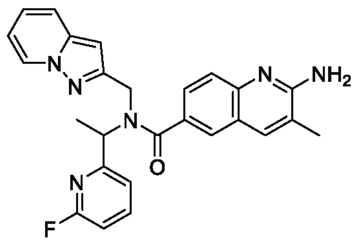 |
| 123 | 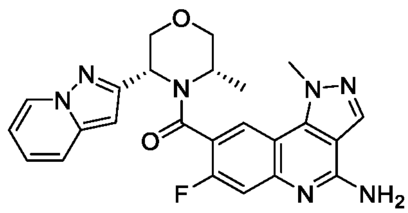 | 124 | 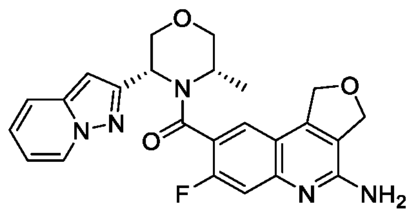 |
| 125 | 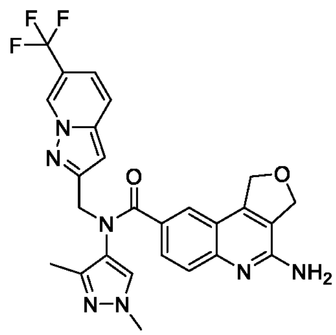 | 126 | 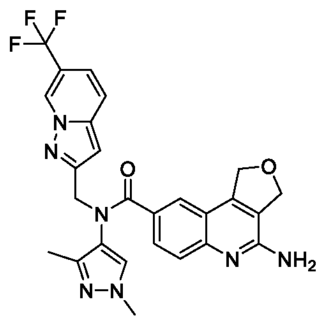 |
| 127 | 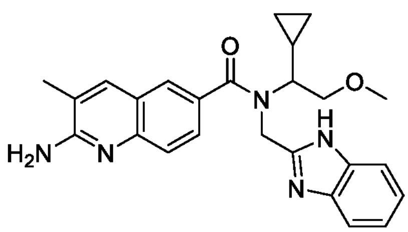 | 128 | 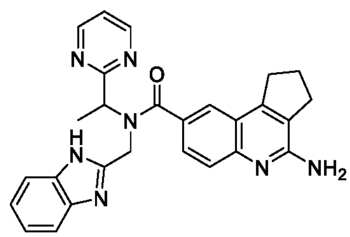 |
| 129 | 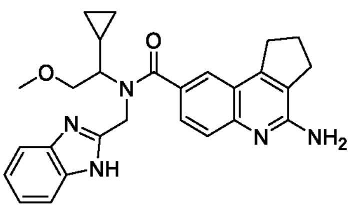 | 130 | 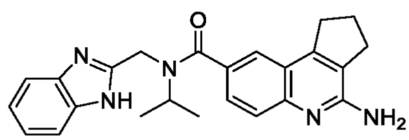 |

(continued)

| NO. | Structure | NO. | Structure |
|---|---|---|---|
| 131 | 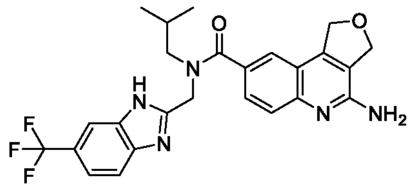 | 132 | 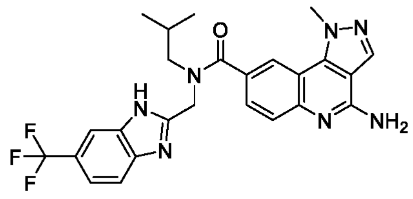 |
| 133 | 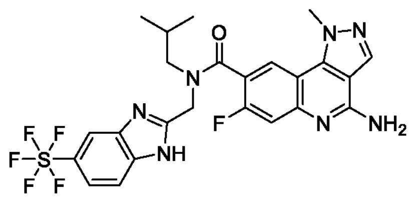 | 134 | 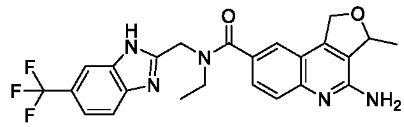 |
| 135 | 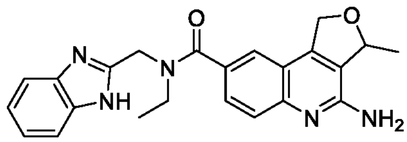 | 136 | 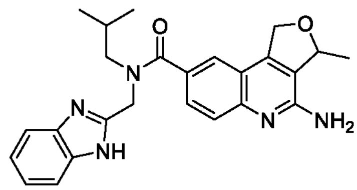 |
| 137 | 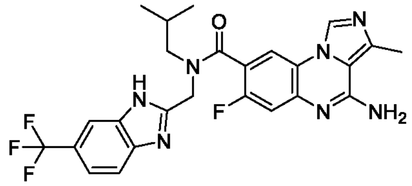 | 138 | 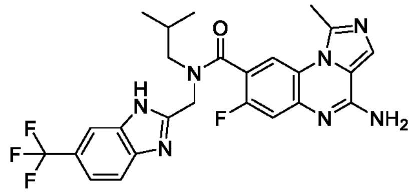 |
| 139 | 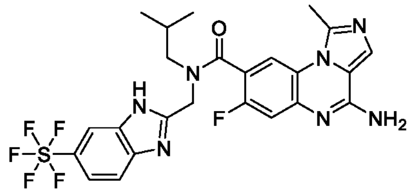 | 140 | 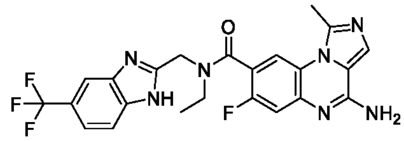 |
| 141 | 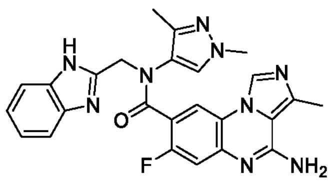 | 142 | 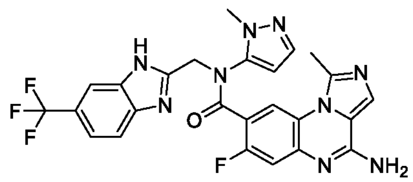 |
| 143 | 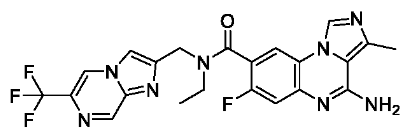 | 144 | 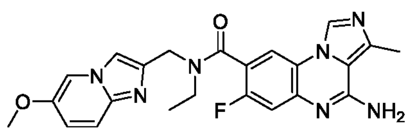 |
| 145 | 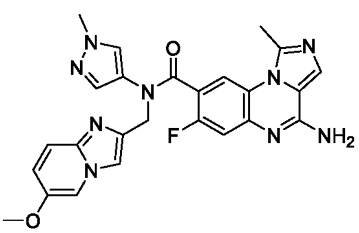 | 146 | 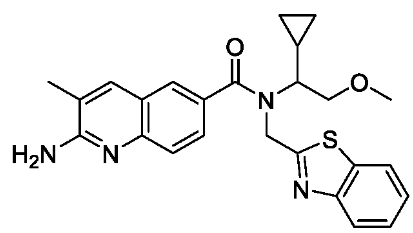 |

(continued)

| NO. | Structure | NO. | Structure |
|---|---|---|---|
| 147 | 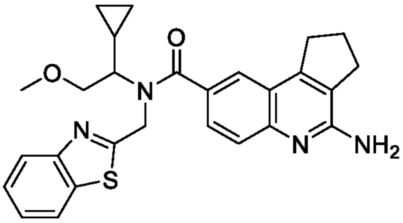 | 148 | 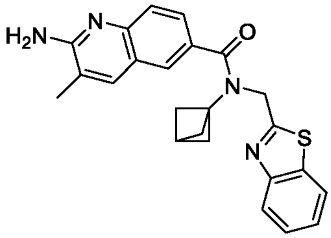 |
| 149 | 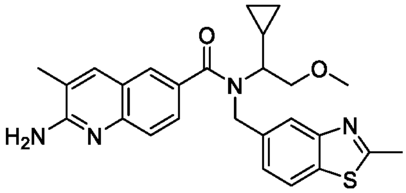 | 150 | 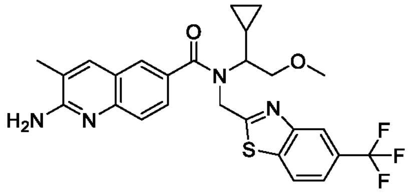 |
| 151 | 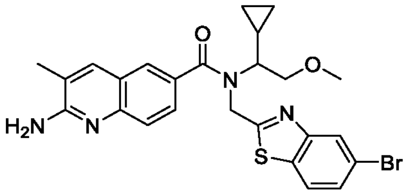 | 152 | 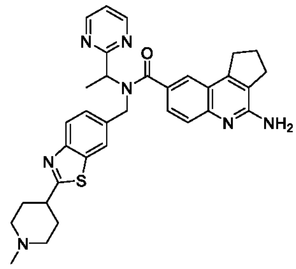 |
| 153 | 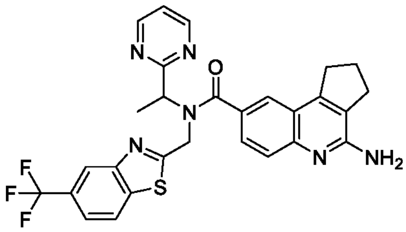 | 154 | 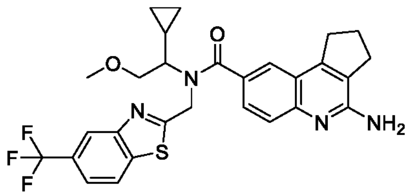 |
| 155 | 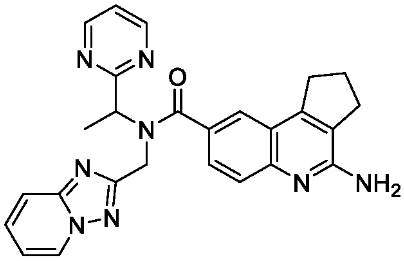 | 156 | 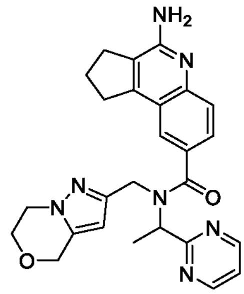 |
| 157 | 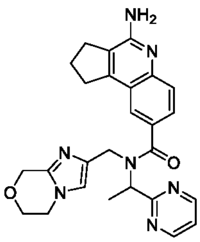 | 158 | 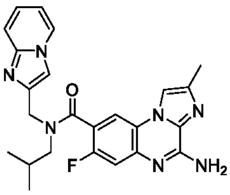 |

(continued)

| NO. | Structure | NO. | Structure |
|---|---|---|---|
| 159 | 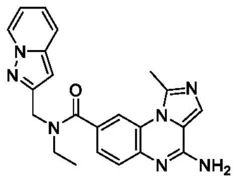 | 160 | 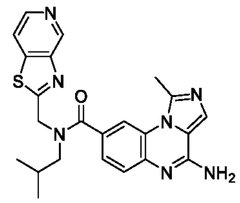 |
| 161 | 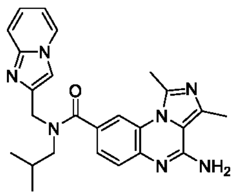 | 162 | 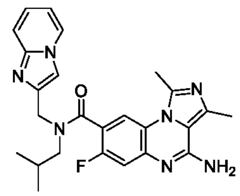 |
| 163 | 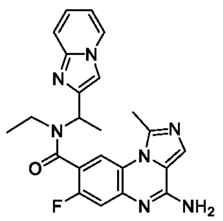 | 164 | 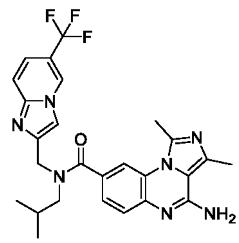 |
| 165 | 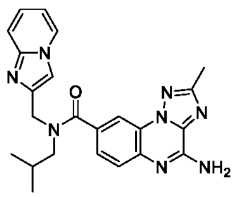 | 166 | 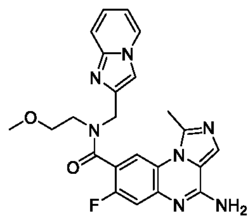 |
| 167 | 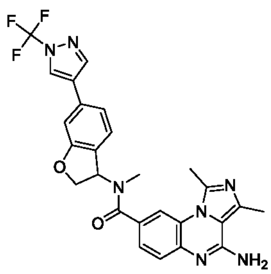 | 168 | 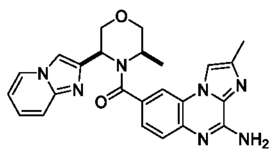 |
| 169 | 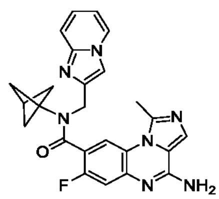 | 170 | 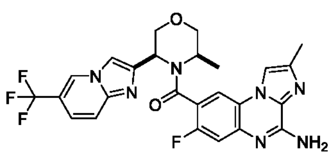 |
| 171 | 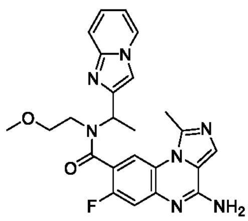 | 172 | 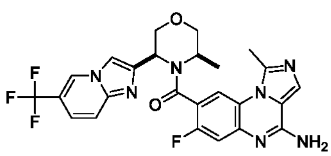 |

(continued)

| NO. | Structure | NO. | Structure |
|---|---|---|---|
| 173 | 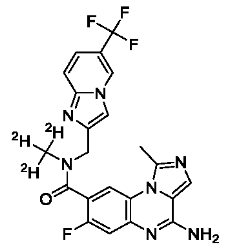 | 174 | 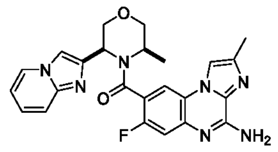 |
| 175 | 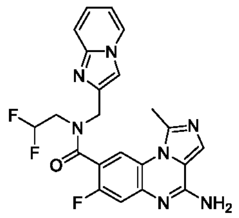 | 176 | 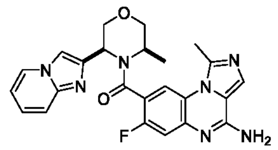 |
| 177 | 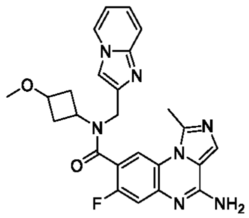 | 178 | 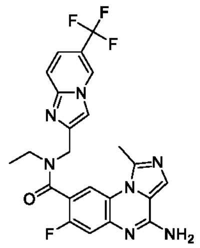 |
| 179 | 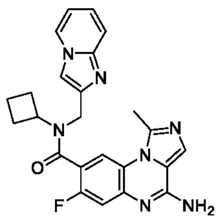 | 180 | 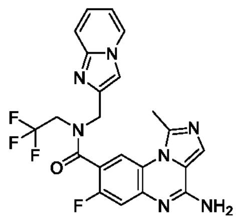 |
| 181 | 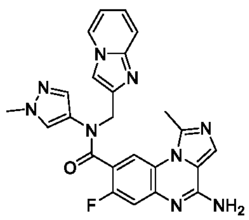 | 182 | 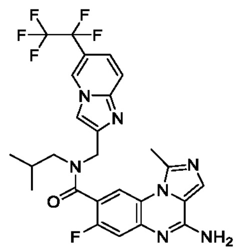 |
| 183 | 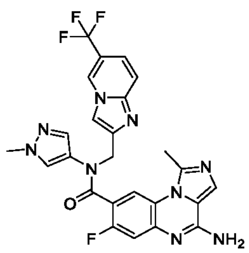 | 184 | 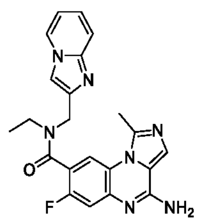 |

(continued)

| NO. | Structure | NO. | Structure |
|---|---|---|---|
| 185 | 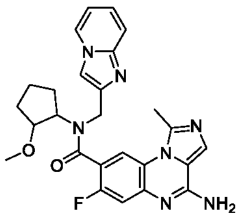 | 186 | 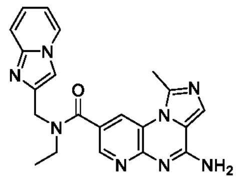 |
| 187 | 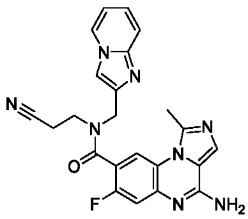 | 188 | 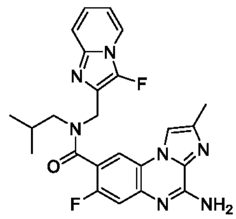 |
| 189 | 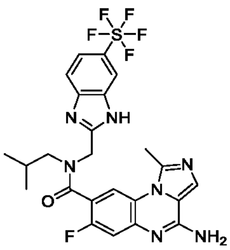 | 190 | 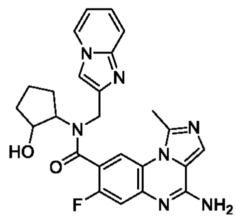 |
| 191 | 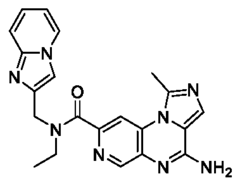 | 192 | 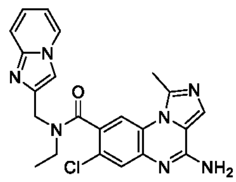 |
| 193 | 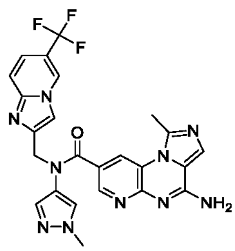 | 194 | 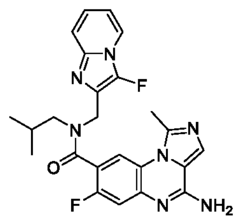 |
| 195 | 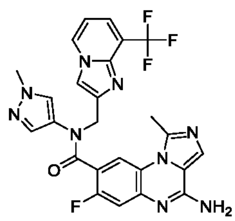 | 196 | 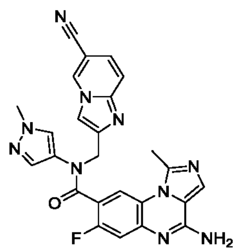 |

(continued)

| NO. | Structure | NO. | Structure |
|---|---|---|---|
| 197 | 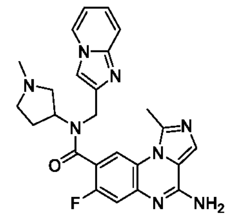 | 198 | 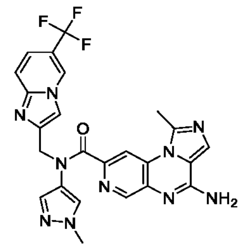 |
| 199 | 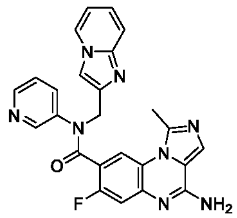 | 200 | 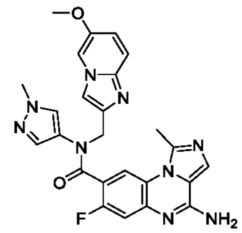 |
| 201 | 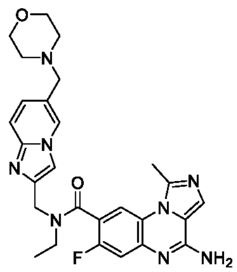 | 202 | 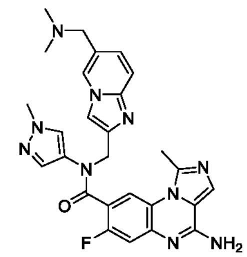 |
| 203 | 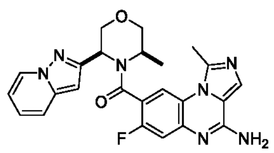 | 204 | 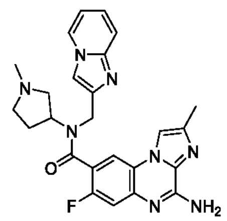 |
| 205 | 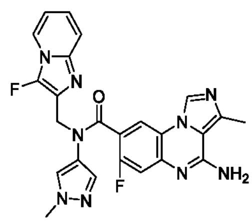 | 206 | 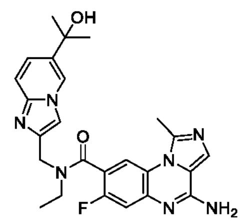 |
| 207 | 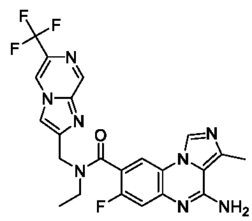 | 208 | 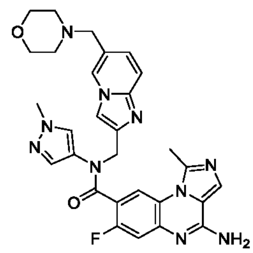 |

(continued)

| NO. | Structure | NO. | Structure |
|---|---|---|---|
| 209 | 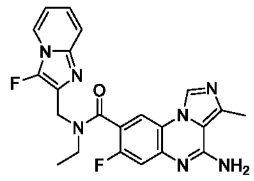 | 210 | 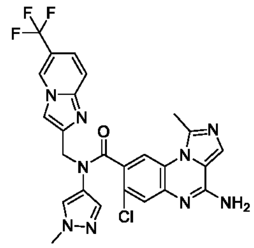 |
| 211 | 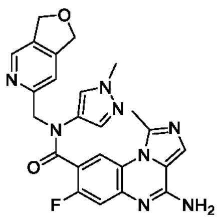 | 212 | 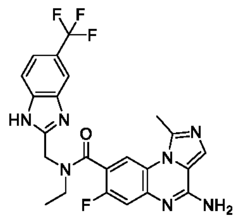 |
| 213 | 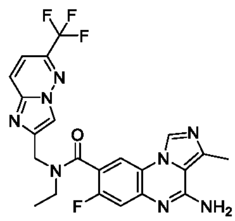 | 214 | 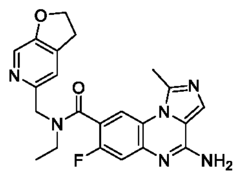 |
| 215 | 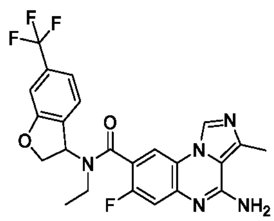 | 216 | 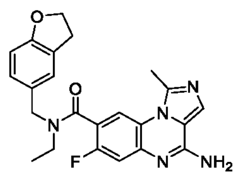 |
| 217 | 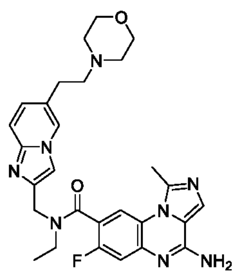 | 218 | 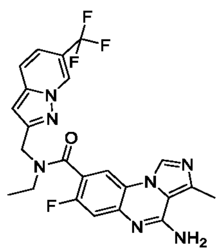 |
| 219 | 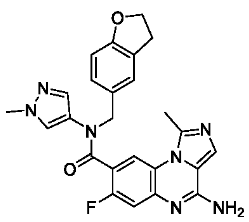 | 220 | 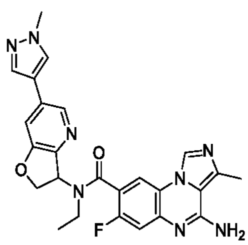 |

(continued)

| NO. | Structure | NO. | Structure |
|---|---|---|---|
| 221 | 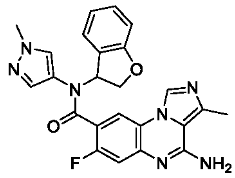 | 222 | 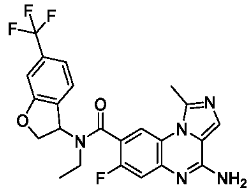 |
| 223 | 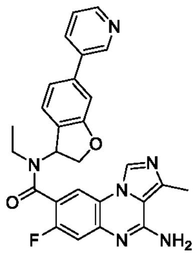 | 224 | 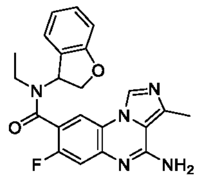 |
| 225 | 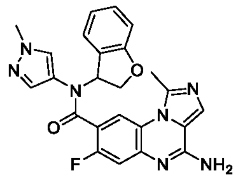 | 226 | 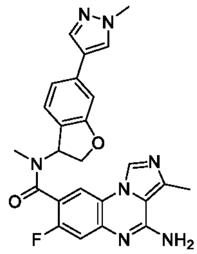 |
| 227 | 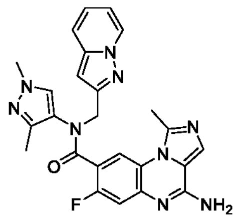 | 228 | 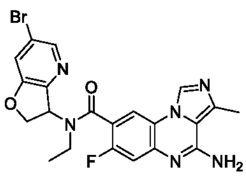 |
| 229 | 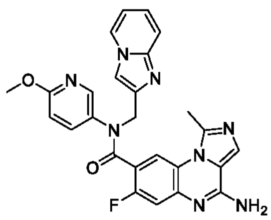 | 230 | 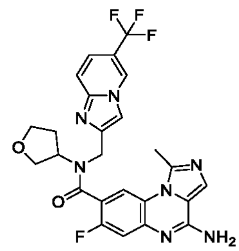 |
| 231 | 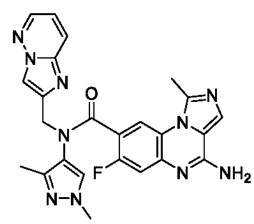 | 232 | 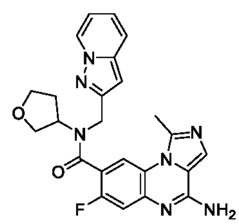 |

(continued)

| NO. | Structure | NO. | Structure |
|---|---|---|---|
| 233 | 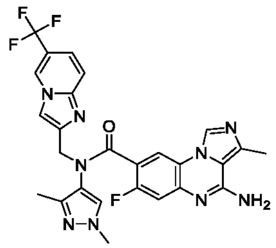 | 234 | 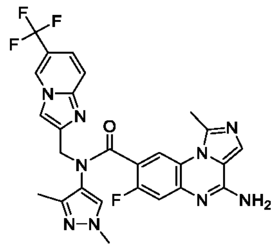 |
| 235 | 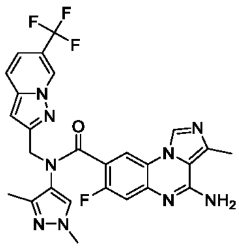 | 236 | 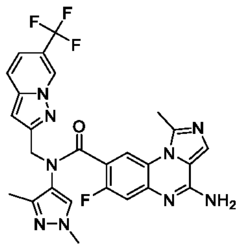 |
| 237 | 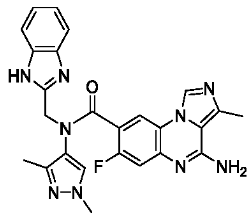 | 238 | 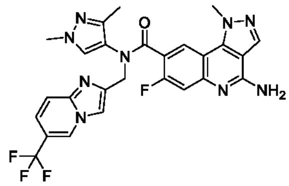 |
| 239 | 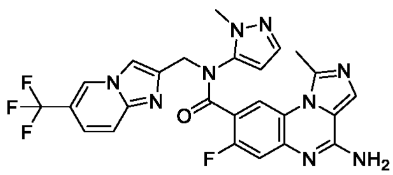 | 240 | 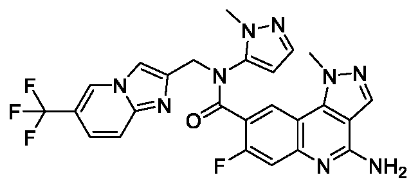 |
| 241 | 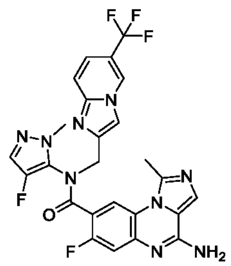 | 242 | 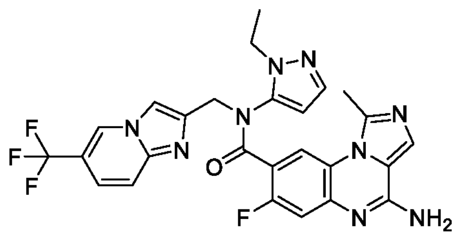 |
| 243 | 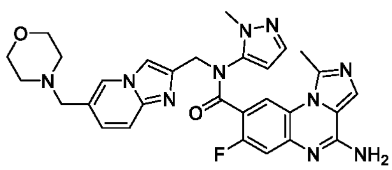 | 244 | 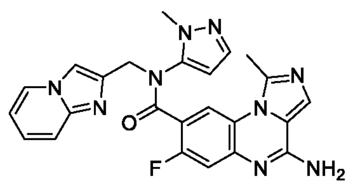 |
| 245 | 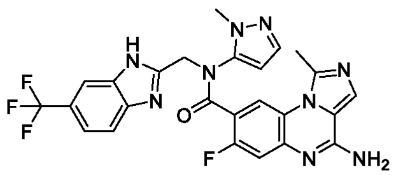 | 246 | 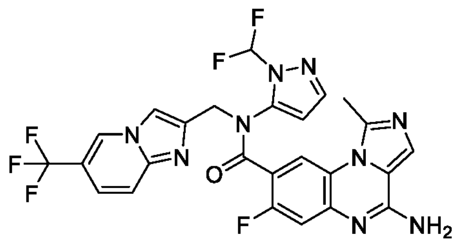 |

(continued)

| NO. | Structure | NO. | Structure |
|---|---|---|---|
| 247 | 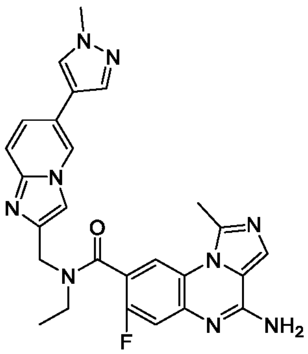 | 248 | 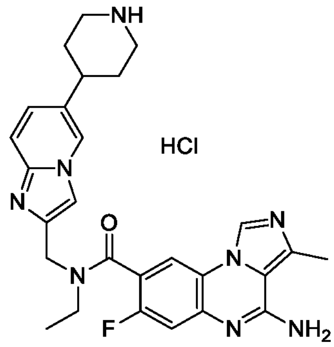 HCl |
| 249 | 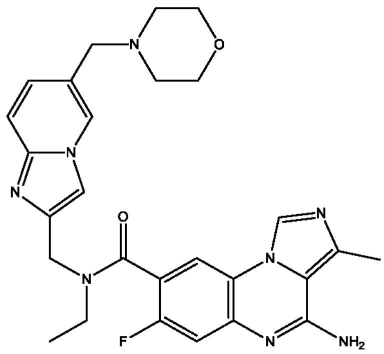 | 250 | 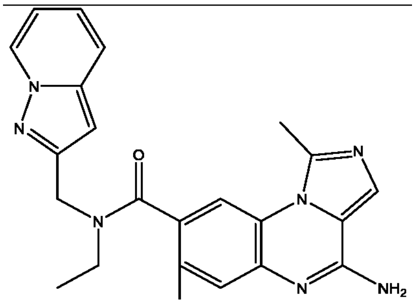 |
| 251 | 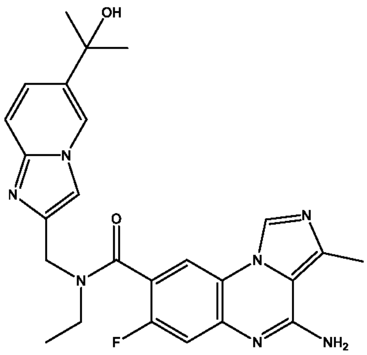 | 252 | 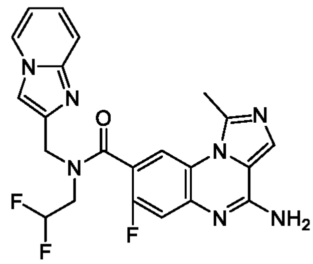 |
| 253 | 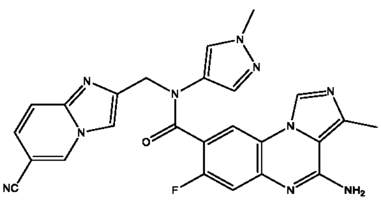 | 254 | 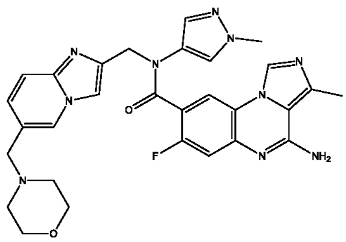 |
| 255 | 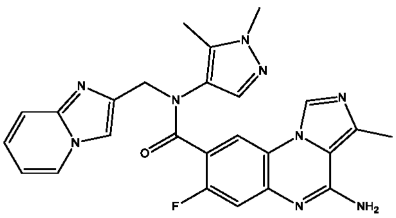 | 256 | 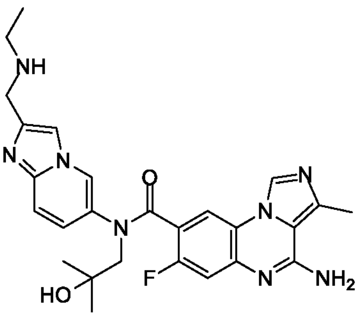 |

(continued)

| NO. | Structure | NO. | Structure |
|---|---|---|---|
| 257 | 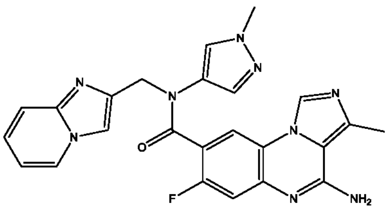 | 258 | 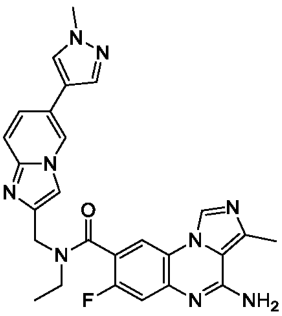 |
| 259 | 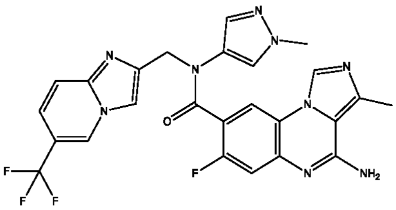 | 260 | 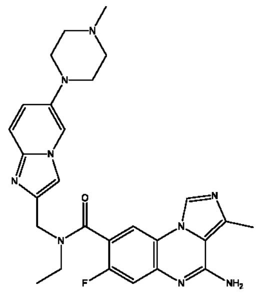 |
| 261 | 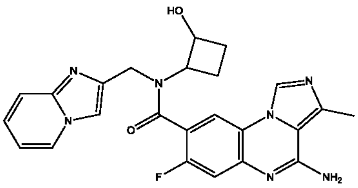 | 262 | 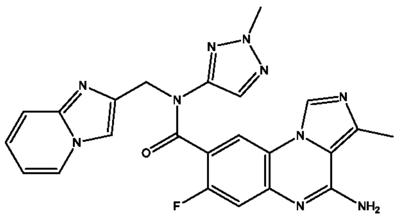 |
| 263 | 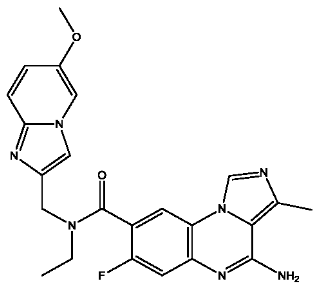 | 264 | 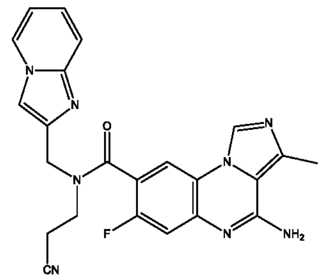 |
| 265 | 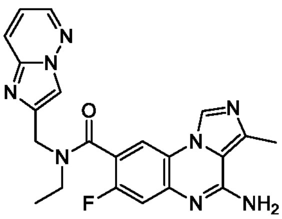 | 266 | 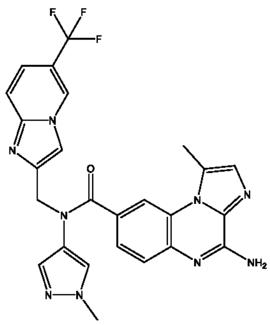 |

(continued)

| NO. | Structure | NO. | Structure |
|---|---|---|---|
| 267 | 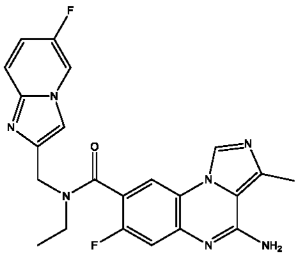 | 268 | 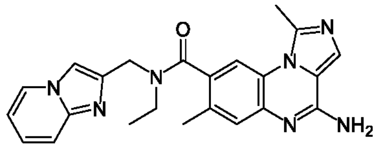 |
| 269 | 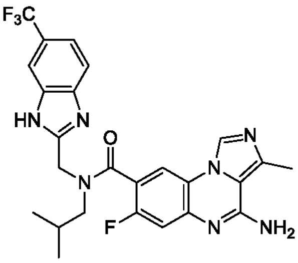 | 270 | 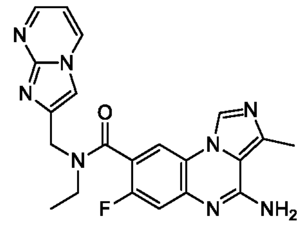 |
| 271 | 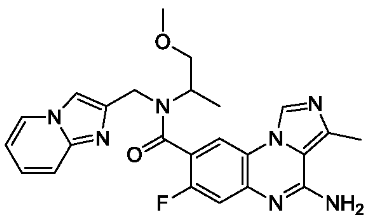 | 272 | 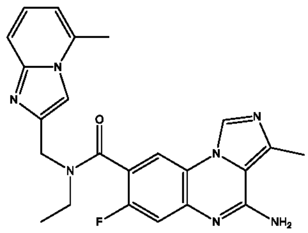 |
| 273 | 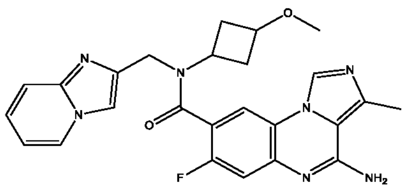 | 274 | 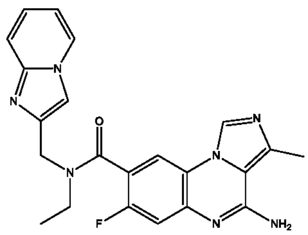 |
| 275 | 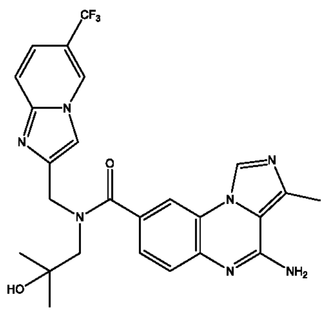 | 276 | 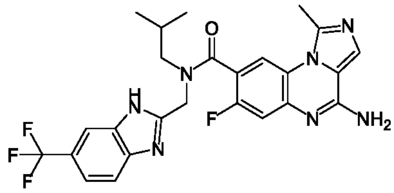 |
| 277 | 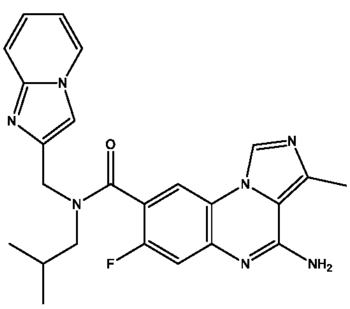 | 278 | 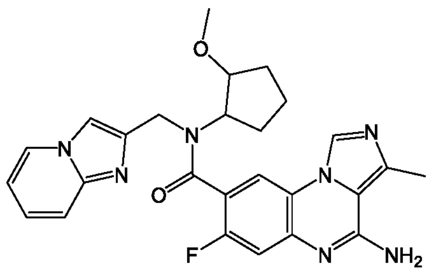 |

(continued)

| NO. | Structure | NO. | Structure |
|---|---|---|---|
| 279 | 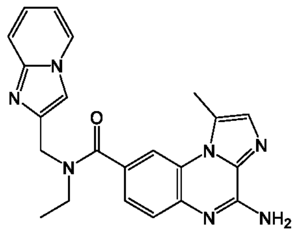 | 280 | 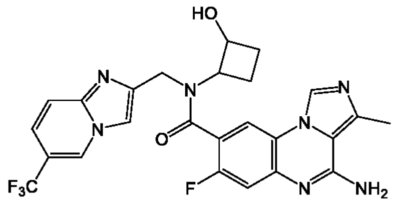 |
| 281 | 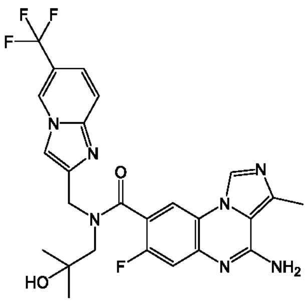 | 282 | 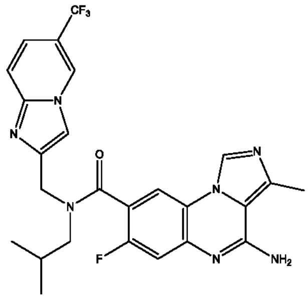 |
| 283 | 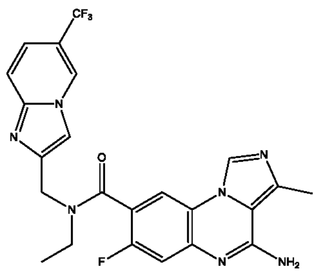 | 284 | 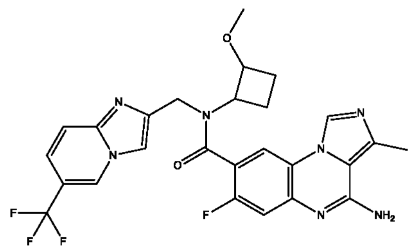 |
| 285 | 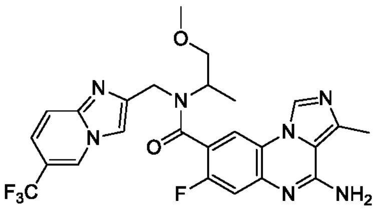 | 286 | 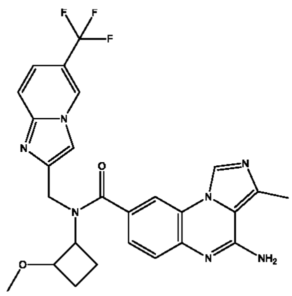 |
| 287 | 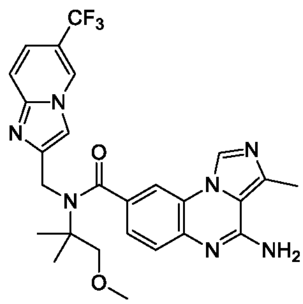 | 288 | 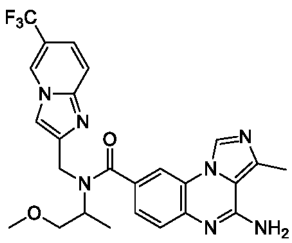 |

(continued)

| NO. | Structure | NO. | Structure |
|---|---|---|---|
| 289 | 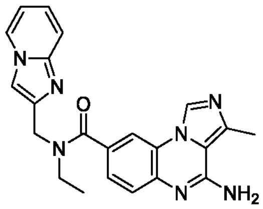 | 290 | 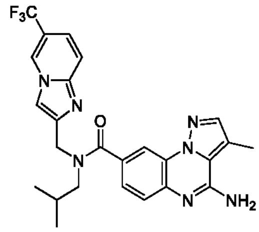 |
| 291 | 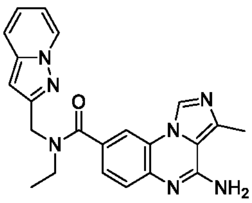 | 292 | 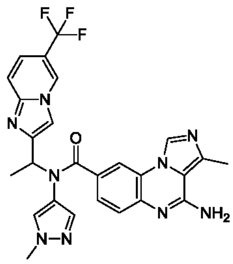 |
| 293 | 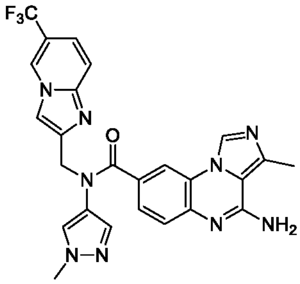 | 294 | 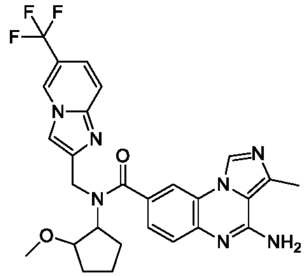 |
| 295 | 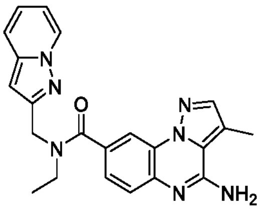 | 296 | 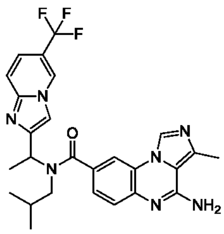 |
| 297 | 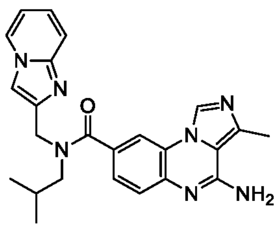 | 298 | 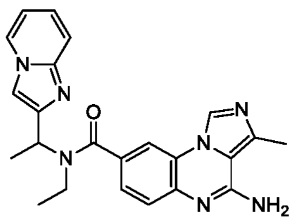 |
| 299 | 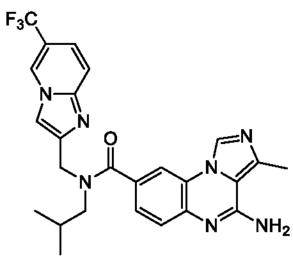 | 300 | 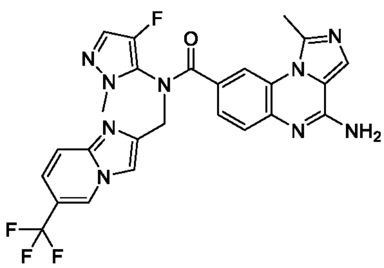 |

(continued)

| NO. | Structure | NO. | Structure |
|---|---|---|---|
| 301 | 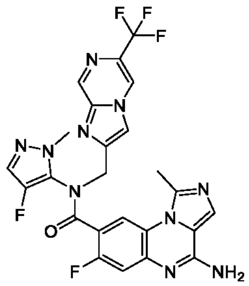 | 302 | 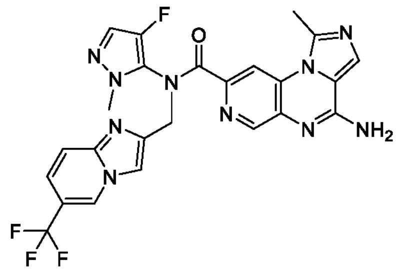 |
| 303 | 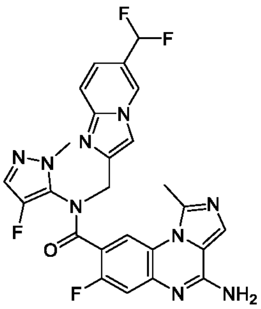 | 304 | 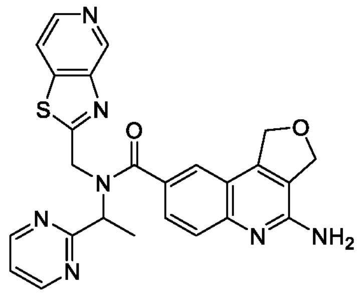 |
| 305 | 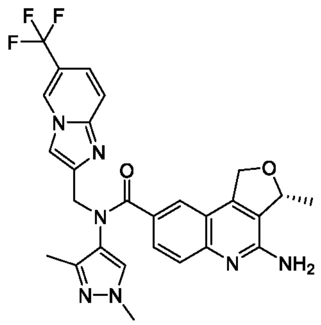 | 306 | 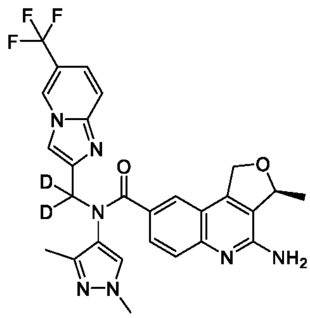 |
| 307 | 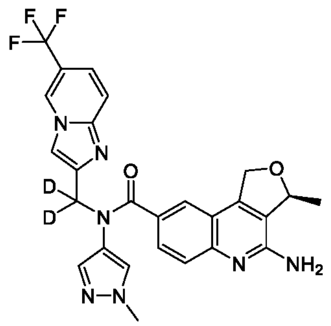 | 308 | 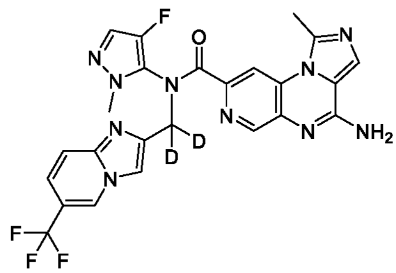 |
| 309 | 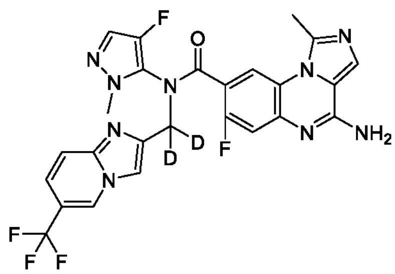 | 310 | 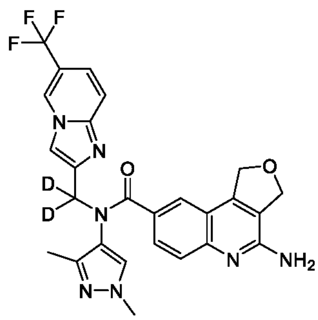 |

(continued)

| NO. | Structure | NO. | Structure |
|---|---|---|---|
| 311 | 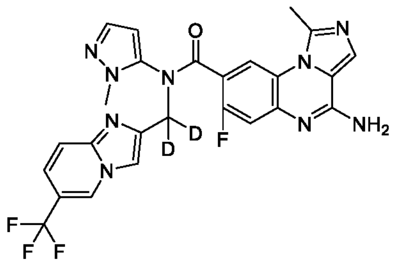 | 312 | 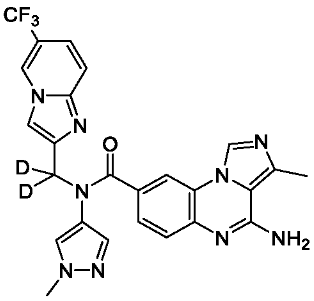 |

**12.** A pharmaceutical composition comprising a therapeutically effective amount of one or more of the compound according to any one of claims 1-11, a pharmaceutically acceptable salt, a racemate, an optical isomer, a stereoisomer, or a tautomer thereof, and one or more pharmaceutically acceptable carriers, excipients, adjuvants, accessories, and/or diluents.

**13.** A use of the compound according to any one of claims 1-11, a racemate, a stereoisomer, or a pharmaceutically acceptable salt thereof in the preparation of drugs for the treatment or prevention of diseases associated with abnormal gene levels or abnormal expression of PRMT5 (such as corresponding nucleic acid mutations, deletions, or abnormal MTAP gene level, or the methyltransferase is ectopic or fused or overexpressed).

**14.** The use according to claim 13, wherein, the disease is selected from the group consising of: the disease or disorder ovarian cancer, esophageal cancer, lung cancer, lymphatic cancer, glioblastoma, colon cancer, melanoma, gastric cancer, pancreatic cancer or bladder cancer.

**INTERNATIONAL SEARCH REPORT**

International application No.
**PCT/CN2023/105594**

**A. CLASSIFICATION OF SUBJECT MATTER**

C07D471/04(2006.01)i; C07D519/00(2006.01)i; C07D401/12(2006.01)i; C07D401/14(2006.01)i; C07D491/04(2006.01)i; C07D487/04(2006.01)i; C07D417/12(2006.01)i; A61K31/506(2006.01)i; A61K31/437(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXTC, DWPI, CNKI, STN: 上海赛岚生物科技有限公司, PRMT5, 抑制剂, 癌症, protein arginine methyltransferase, inhibit+, cancer, 结构检索, structure search

**C. DOCUMENTS CONSIDERED TO BE RELEVANT**

| Category* | Citation of document, with indication, where appropriate, of the relevant passages | Relevant to claim No. |
|---|---|---|
| X | WO 2022115377 A1 (AMGEN INC.) 02 June 2022 (2022-06-02)<br>see abstract, claims 1 and 22-23, and descxription, embodiments | 1-14 |
| X | WO 2021163344 A1 (AMGEN INC.) 19 August 2021 (2021-08-19)<br>see abstract, claims 1 and 20-25, and description, embodiments | 1-14 |
| X | WO 2021127166 A1 (THE ROCKEFELLER UNIVERSITY et al.) 24 June 2021 (2021-06-24)<br>see abstract, claim 1, and description, paragraph [0006], and embodiments | 1-2, 12 |
| X | KR 20090063869 A (KOREA INSTITUTE OF SCIENCE AND TECHNOLOGY et al.) 18 June 2009 (2009-06-18)<br>see abstract, claims 1 and 13-14, and description, embodiments | 1-2, 4, 6-7, 12 |
| PX | ACS. "RN 2814523-89-0"<br>*STN Registry,* 29 August 2022 (2022-08-29),<br>see pages 1-3 | 1, 3, 6-7 |
| PX | WO 2022169948 A1 (AMGEN INC.) 11 August 2022 (2022-08-11)<br>see abstract, claims 1 and 24-27, and description, embodiments | 1-14 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **05 September 2023** | **21 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
**PCT/CN2023/105594**

**C. DOCUMENTS CONSIDERED TO BE RELEVANT**

| Category* | Citation of document, with indication, where appropriate, of the relevant passages | Relevant to claim No. |
|---|---|---|
| PX | WO 2022256806 A1 (IDEAYA BIOSCIENCES, INC.) 08 December 2022 (2022-12-08) see description, pages 174-212 | 1-14 |

Form PCT/ISA/210 (second sheet) (July 2022)

**INTERNATIONAL SEARCH REPORT**
**Information on patent family members**

International application No.
**PCT/CN2023/105594**

| Patent document cited in search report | Publication date (day/month/year) | Patent family member(s) | Publication date (day/month/year) |
|---|---|---|---|
| WO 2022115377 A1 | 02 June 2022 | KR 20230113339 A | 28 July 2023 |
| | | AU 2021386149 A1 | 22 June 2023 |
| | | IL 302820 A | 01 July 2023 |
| | | CA 3202141 A1 | 02 June 2022 |
| WO 2021163344 A1 | 19 August 2021 | AU 2021219730 A1 | 25 August 2022 |
| | | JP 2023513580 A | 31 March 2023 |
| | | EP 4103558 A1 | 21 December 2022 |
| | | US 2023159510 A1 | 25 May 2023 |
| | | CA 3170321 A1 | 19 August 2021 |
| WO 2021127166 A1 | 24 June 2021 | CR 20220326 A | 10 February 2023 |
| | | EP 4076440 A1 | 26 October 2022 |
| | | BR 112022012273 A2 | 30 August 2022 |
| | | KR 20220129554 A | 23 September 2022 |
| | | US 2023027198 A1 | 26 January 2023 |
| | | JP 2023527487 A | 29 June 2023 |
| | | IL 293839 A | 01 August 2022 |
| | | CA 3162253 A1 | 24 June 2021 |
| | | AU 2020407589 A1 | 21 July 2022 |
| | | CO 2022009852 A2 | 11 October 2022 |
| KR 20090063869 A | 18 June 2009 | KR 100937738 B1 | 21 January 2010 |
| WO 2022169948 A1 | 11 August 2022 | AU 2022217791 A1 | 17 August 2023 |
| WO 2022256806 A1 | 08 December 2022 | None | |

Form PCT/ISA/210 (patent family annex) (July 2022)